(19) Europäisches Patentamt
European Patent Office
Office européen des brevets

(11) EP 4 482 280 A1

(12) EUROPEAN PATENT APPLICATION

(43) Date of publication:
**25.12.2024 Bulletin 2024/52**

(21) Application number: **23180120.0**

(22) Date of filing: **19.06.2023**

(51) International Patent Classification (IPC):
***H10K 85/60*** *(2023.01)* ***C09K 11/06*** *(2006.01)*

(52) Cooperative Patent Classification (CPC):
**H10K 85/60; C07C 255/51; C07D 213/57; C07D 213/85; C07D 239/26; C07D 241/12; C09K 11/06;** C07C 2601/02; H10K 50/17; H10K 50/19

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Novaled GmbH**
**01099 Dresden (DE)**

(72) Inventors:
- **Luschtinetz, Regina**
**01099 Dresden (DE)**
- **Nüllen, Max Peter**
**01099 Dresden (DE)**
- **Schulze, Benjamin**
**01099 Dresden (DE)**
- **Wudarczyk, Jakob Jacek**
**01099 Dresden (DE)**

(74) Representative: **Michalski Hüttermann & Partner Patentanwälte mbB**
**Kaistraße 16A**
**40221 Düsseldorf (DE)**

# (54) ORGANIC COMPOUND OF FORMULA (I) FOR USE IN ORGANIC ELECTRONIC DEVICES, A COMPOSITION COMPRISING A COMPOUND OF FORMULA (IV) AND AT LEAST ONE COMPOUND OF FORMULA (IVA) TO (IVD), AN ORGANIC SEMICONDUCTOR LAYER COMPRISING THE COMPOUND OR COMPOSITION, AN ORGANIC ELECTRONIC DEVICE COMPRISING THE ORGANIC SEMICONDUCTOR LAYER, AND A DISPLAY DEVICE COMPRISING THE ORGANIC ELECTRONIC DEVICE

(57) The present invention relates to a compound of formula (I) and an organic electroluminescent device a compound of formula (I).

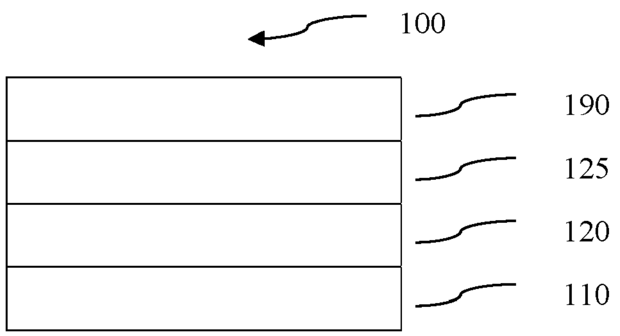


Fig. 1



Processed by Luminess, 75001 PARIS (FR)

## Description

## Technical Field

The present invention relates to an organic compound of formula (I) for use in organic electronic devices, a composition comprising a compound of formula (IV) and at least one compound of formula (IVa) to (IVd), an organic semiconductor layer comprising the compound or composition, an organic electronic device comprising the organic semiconductor layer, and a display device comprising the organic electronic device

## Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of metal complexes which are also contained in the semiconductor layer.

There remains a need to improve performance and stability of organic semiconductor materials, semiconductor layers, as well as organic electronic devices thereof, in particular to achieve improved brightness, higher lifetime, improved efficiency such as current efficiency and external quantum efficiency, improved operating voltage and/or voltage stability over time through improving the characteristics of the compounds comprised therein. The organic compounds of the present invention exhibit a remarkable energy gap (Egap), which is thedifference between the HOMO and LUMO energy level, and thus the compounds exhibits a very low absorption within the wavelength range of the visible light.

## DISCLOSURE

An aspect of the present invention provides an organic compound for use in organic electronic devices of formula (I):

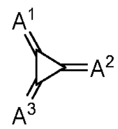

(I)

wherein

$A^1$ is selected from formula (II)

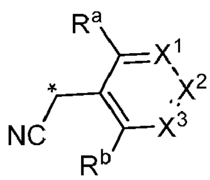

(II)

wherein

$X^1$ is selected from $CR^1$ or N,

$X^2$ is selected from $CR^2$ or N,

$X^3$ is selected from $CR^3$ or N,

$R^a$, and $R^b$ are independently selected from CN, $CF_3$, partially fluorinated $C_1$ to $C_8$ alkyl or perfluorinated $C_1$ to $C_8$ alkyl,

$R^1$, $R^2$, and $R^3$, if present, are independently selected from H, D, CN, $CF_3$, partially fluorinated $C_1$ to $C_8$ alkyl or

perfluorinated $C_1$ to $C_8$ alkyl,
whereby if in formula (II) any of $X^1$ to $X^3$ is N then there is no CN moiety in $\alpha$-position,
$A^2$ is different from $A^1$ and selected from formula (III)

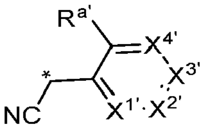 (III)

wherein
$X^{1'}$ is selected from $CR^{1'}$ or N,
$X^{2'}$ is selected from $CR^{2'}$ or N,
$X^{3'}$ is selected from $CR^{3'}$ or N,
$X^{4'}$ is selected from $CR^{4'}$ or N,
$R^{a'}$ is selected from CN, $CF_3$, partially fluorinated $C_1$ to $C_8$ alkyl or perfluorinated $C_1$ to $C_8$ alkyl,
$R^{1'}$, $R^{2'}$, $R^{3'}$ and $R^{4'}$, if present, are independently selected from H, D, CN, $CF_3$, partially fluorinated $C_1$ to $C_8$ alkyl or perfluorinated $C_1$ to $C_8$ alkyl,
whereby if in formula (III) any $X^{1'}$ to $X^{4'}$ is N then there is no CN moiety in $\alpha$-position,
$A^3$ is independently selected from formula (II), or formula (III),
wherein the compounds with the following combination of $A^1$ to $A^3$ are excluded:

| $A^1$ | $A^2$ | $A^3$ |
|---|---|---|
| 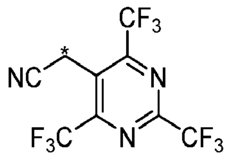 | 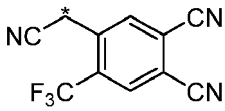 | 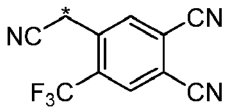 |
| 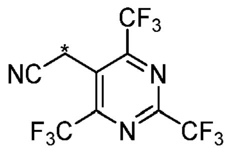 | 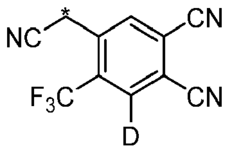 | 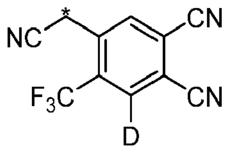 |
| 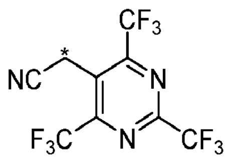 | 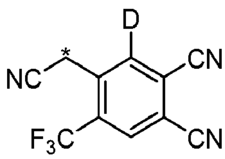 | 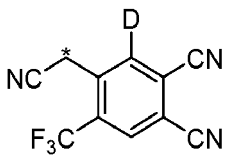 |
| 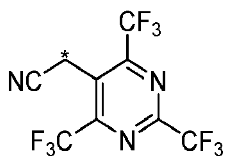 | 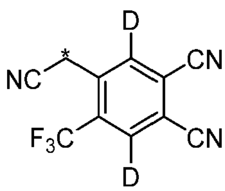 | 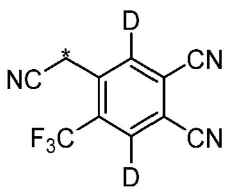 |
| 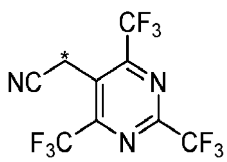 | 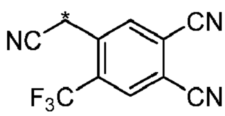 | 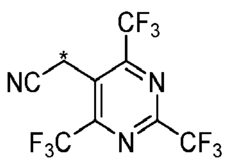 |
| 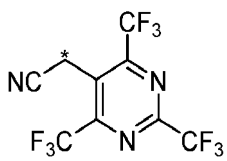 | 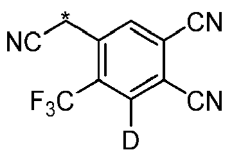 | 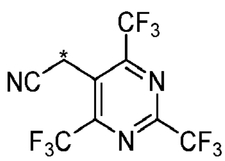 |

(continued)

| $A^1$ | $A^2$ | $A^3$ |
|---|---|---|
| 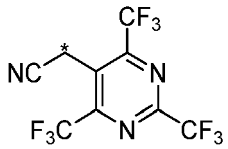 | 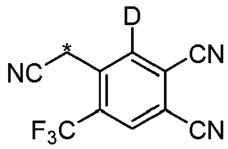 | 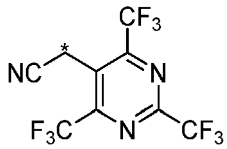 |
| 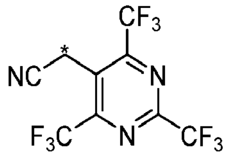 | 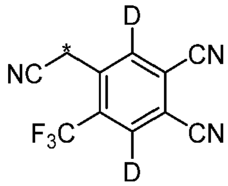 | 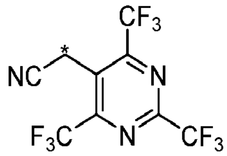 |
| 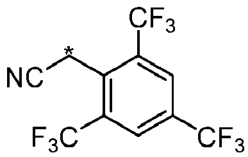 | 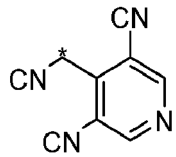 | 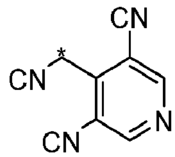 |
| 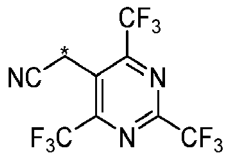 | 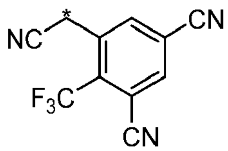 | 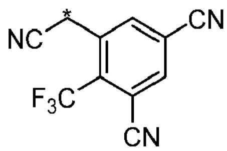 |

It should be noted that throughout the application and the claims any $A^n$, $B^n$, $R^n$, $X^n$ etc. always refer to the same moieties, unless otherwise noted.

In the present specification, when a definition is not otherwise provided, "partially fluorinated" refers to a $C_1$ to $C_8$ alkyl group in which only part of the hydrogen atoms are replaced by fluorine atoms.

In the present specification, when a definition is not otherwise provided, "perfluorinated" refers to a $C_1$ to $C_8$ alkyl group in which all hydrogen atoms are replaced by fluorine atoms.

In the present specification, when a definition is not otherwise provided, "substituted" refers to one substituted with a deuterium, $C_1$ to $C_{12}$ alkyl and $C_1$ to $C_{12}$ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like

phenyl or tolyl, polycyclic groups which comprise more aromatic rings linked by single bonds, like biphenyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common $sp^2$-hybridized carbon atoms

In the present specification, the single bond refers to a direct bond.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

## Advantageous Effects

Surprisingly, it was found that the organic compound of the present invention solves the problem underlying the present invention by enabling devices in various aspects superior over the organic electroluminescent devices known in the art, in particular with respect to improved brightness, higher lifetime, improved efficiency such as current efficiency and external quantum efficiency, operating voltage and voltage stability over time. The organic compounds of the present invention exhibit a remarkable energy gap (Egap), which is the difference between the HOMO and LUMO energy level, and thus the compounds exhibit a very low absorption within the wavelengthrange of the visible light. A low absorption results in a reduction of the external quantum efficiency, the current density, and/or the luminous flux.

Moreover, the compounds exhibit a suitable volatility as well as thermal stability for a good manufacturing of an OLED device.

Electroluminescent devices like OLEDs must not only match the color purity and long-term stability of competing technologies, but they must also provide a sigificant advantage in efficiency, especially in low-power, portable applications.

One main component to the power efficiency of an OLED is the external quantum efficiency (EQE) which is defined as EQE = extracted photons / injected electrons. The power efficiency is the ratio of the luminous power out, as detected by the human ecye, to the electrical power in.

The EQE is proportional connected to the luminous flux.

The luminous flux (given in lumen, lm) is defined as amount of light capable of sensitizing the human eye per unit of time, or the total photometric power emitted in all directions from a light source. It is standardized with the (ideal) eye's maximum sensitivity, at 555 nm (or 1/680 W).

The luminous intensity (measured in candela, cd) takes into account the colour of the light and its direction. It is the luminous flux emitted in to a specific solid angle.

The Luminance, L, is the luminous intensity, both per unit of area, respectively. In application, typical brightness levels of mobile displays are between 100-400 cd/m 2 while for general illuminations, higher values of around 5000 cd/m2 are required.

The current efficiency can be calculated as the amount of current flowing through the device with an emissive areas necessary to produce a certain luminance, L is expressed in cd/A.

A major limitation to the quantum efficiency, and current efficiency of OLEDs is the light output coupling fraction. The output coupling fraction is limited by absorption losses and guiding of electroluminescence within the device and its substrates, i.e. the use of less absorbing materials and transparent contacts increases the output coupling fraction and hence improve the external quantum efficiency, luminous flux, and current efficiency of the OLED.

It could be shown that the inventive compounds exhibiting a remarkable high energy gap, also exhibits a low

absorption within the wavelength range of the visible light.

According to one embodiment of the present invention, the compound is selected of the formula (IV)

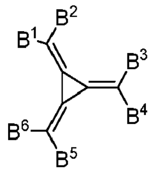

(IV)

whereby $B^1$ is selected from formula (II), $B^3$ is selected from formula (III), $B^5$ is selected from formula (II) or (III) and $B^2$, $B^4$ and $B^6$ are CN

According to one embodiment of the present invention, $A^3$ is the same as $A^1$ or $A^2$, subsequently according to one embodiment $B^5$ is the same as $B^1$ or $B^3$.

According to one embodiment of the present invention, formula (II) and formula (III) are not identical.

According to an embodiment, the compound of formula (I) has a LUMO energy level and a HOMO energy level, wherein the difference between the LUMO energy level and the HOMO energy level is ≥ 2.89 eV, preferably ≥ 2.92 eV, more preferably ≥ 2.95 eV, and most preferably ≥ 2.97 eV.

The HOMO and LUMO are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The dipole moment, the HOMO and LUMO energy levels of the molecular structures are determined by applying the hybrid functional B3LYP with a 6-31G* basis set from the optimized geometries obtained by applying the hybrid functional B3LYP with a 6-31G* basis set in the gas phase.

Compounds having a difference between the LUMO energy level and the HOMO energy level (Egap or energy gap) in the given range have a low absorption in range of visible light. The higher the difference between the LUMO energy level and the HOMO energy level, and thus a shift of the absorption in the direction of the blue area of electromagnetic spectrum, the lower the absorption in the range of visible light in an organic electroluminescent device. The luminous flux, the external quantum efficiency or the current efficiency of an organic electroluminescent device may be increased as a result of the lower absorption in the range of the visible light.

According to an embodiment the compound of formula (I) has a LUMO energy level of ≤ -4.90 eV, preferably ≤ -5.00 eV, more preferably ≤ -5.02 eV, and most preferably ≤ -5.04 eV.

The HOMO and LUMO are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The dipole moment, the HOMO and LUMO energy levels of the molecular structures are determined by applying the hybrid functional B3LYP with a 6-31G* basis set from the optimized geometries obtained by applying the hybrid functional B3LYP with a 6-31G* basis set in the gas phase.

A low or deep LUMO energy level further allows to reduce the operational voltage of an organic electronic device. The lower the LUMO energy level, the lower the operational voltage of an organic electronic device.

According to an embodiment the compound of formula (I) has a LUMO energy level and a HOMO energy level wherein the difference between the LUMO energy level and the HOMO energy level is ≥ 2.89 eV and the LUMO energy level is ≤ -4.90 eV.

According to a preferred embodiment, wherein the compound of formula (I) has a LUMO energy level and a HOMO energy level wherein the difference between the LUMO energy level and the HOMO energy level is ≥ 2.92 eV and the LUMO energy level is ≤ -5.00 eV.

According to a more embodiment, wherein the compound of formula (I) has a LUMO energy level and a HOMO energy level wherein the difference between the LUMO energy level and the HOMO energy level is ≥ 2.95 eV and the LUMO energy level is ≤ -5.02 eV.

According to a most preferred embodiment, wherein the compound of formula (I) has a LUMO energy level and a HOMO energy level wherein the difference between the LUMO energy level and the HOMO energy level is ≥ 2.97 eV and the LUMO energy level is ≤ -5.04 eV.

According to an embodiment the compound of formula (I) has a molecular weight of ≥500 g/mol to ≤1075 g/mol; preferably ≥550 g/mol to ≤1025 g/mol; more preferably ≥600 g/mol to ≤1000 g/mol; and most preferably ≥650 g/mol to ≤985 g/mol.

A molecular in the given range of the compound of formula (I) allows the evaporation of the compounds during the manufacturing of e.g. an organic electronic device. Moreover, a lower molecular weight corresponds to higher molar-% doping at same weight-% doping. Thus, less material (i.e. lower mass of material) needs to be evaporated to reach same

doping effect vs. a material of same doping strength but higher molecular weight. As a result, a lower evaporation rate (i.e. lower thermal stress) is required for a material with lower molecular weight.

According to an embodiment the compound of formula (I), the compound of formula (I) comprises at least 6 CN groups. A certain amount of CN groups ascertains a volatility of the compound of formula (I) which is high enough for the manufacturing conditions of an OLED. According to an embodiment, wherein the compound of formula (I) comprises 6 to 9 CN groups, preferably 6 to 8, and more preferably 7 to 8.

A certain amount of CN groups ascertains a volatility of the compound of formula (I) which is high enough for the manufacturing conditions of an OLED. If the amount of CN groups are limited to a certain extent, the compound of formula (I) would contain an evaporation temperature for which suitability for the manufacturing of an OLED device can be further improved. Moreover, the thermal stability can be increased when using at least 6, 6 to 8, 7 to 8 or 8 CN groups, i.e. the decomposition temperature can be further increased.

According to an embodiment the compound of formula (I) has a molecular weight of ≥500 g/mol to ≤1075 g/mol; preferably ≥550 g/mol to ≤1025 g/mol; more preferably ≥600 g/mol to ≤1000 g/mol; and most preferably ≥650 g/mol to ≤985 g/mol, and wherein the compound of formula (I) comprises 6 to 9 CN groups, preferably 6 to 8, and more preferably 7 to 8.

According to an embodiment the compound of formula (I) has a molecular weight of ≥500 g/mol to ≤1075 g/mol; preferably ≥550 g/mol to ≤1025 g/mol; more preferably ≥600 g/mol to ≤1000 g/mol; and most preferably ≥650 g/mol to ≤985 g/mol, and wherein the compound of formula (I) comprises 6 to 8 CN groups.

According to an embodiment the compound of formula (I) has a molecular weight of ≥500 g/mol to ≤1075 g/mol; preferably ≥550 g/mol to ≤1025 g/mol; more preferably ≥600 g/mol to ≤1000 g/mol; and most preferably ≥650 g/mol to ≤985 g/mol, and wherein the compound of formula (I) comprises 7 to 8 CN groups.

A molecular in the given range of the compound of formula (I) and the amount of CN groupds further allows the improvement of the evaporation of the compounds during the manufacturing of e.g. an organic electronic device.

According to an embodiment, the compound of formula (I) comprises 3 to 9 $CF_3$ groups or partially fluorinated $C_1$ to $C_8$ alkyl groups or perfluorinated $C_1$ to $C_8$ alkyl groups, preferably 4 to 8, more preferably 5 to 7, and most preferably 6 to 7.

According to an embodiment the compound of formula (I) has a molecular weight of ≥650 g/mol to ≤950 g/mol, the compound of formula (I) comprises 7 to 8 CN groups, and the compound of formula comprises 6 to 7 $CF_3$ groups or partially fluorinated $C_1$ to $C_8$ alkyl groups or perfluorinated $C_1$ to $C_8$ alkyl groups.

According to an embodiment the compound of formula (I) has a molecular weight of ≥650 g/mol to ≤950 g/mol, the compound of formula (I) comprises 8 CN groups, and the compound of formula comprises 6 to 7 $CF_3$ groups or partially fluorinated $C_1$ to $C_8$ alkyl groups or perfluorinated $C_1$ to $C_8$ alkyl groups.

According to an embodiment in formula (II) 1 to 3 of $R^a$, $R^b$, $R^1$, $R^2$, and $R^3$ are independently selected from $CF_3$ groups or partially fluorinated $C_1$ to $C_8$ alkyl groups or perfluorinated $C_1$ to $C_8$ alkyl groups.

According to an embodiment, whereby when in formula (II) one or two of X is N then formula (III) is selected from formula (IIIb)

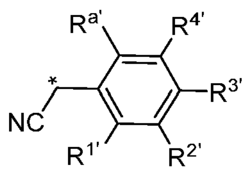

(IIIb)

$R^{a'}$ are independently selected from CN, $CF_3$, partially fluorinated $C_1$ to $C_8$ alkyl or perfluorinated $C_1$ to $C_8$ alkyl, $R^{1'}$, $R^{2'}$, $R^{3'}$ and $R^{4'}$ are independently selected from H, D, CN, $CF_3$, partially or perfluorinated $C_1$ to $C_8$ alkyl.

According to an embodiment, formula (III) is selected from formula (IIIb)

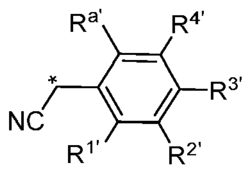

(IIIb)

$R^{a'}$ are independently selected from CN, $CF_3$, partially fluorinated $C_1$ to $C_8$ alkyl or perfluorinated $C_1$ to $C_8$ alkyl, $R^{1'}$, $R^{2'}$, $R^{3'}$ and $R^{4'}$ are independently selected from H, D, CN, $CF_3$, partially fluorinated $C_1$ to $C_8$ alkyl or perfluorinated $C_1$ to $C_8$ alkyl.

According to an embodiment, if $A^3$ is selected from formula (III) then $A^2$ and $A^3$ are independently selected from formula (IIIb)

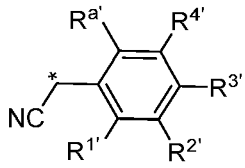

(IIIb)

$R^{a'}$ are independently selected from CN, $CF_3$, partially fluorinated $C_1$ to $C_8$ alkyl or perfluorinated $C_1$ to $C_8$ alkyl, $R^{1'}$, $R^{2'}$, $R^{3'}$ and $R^{4'}$ are independently selected from H, D, CN, $CF_3$, partially fluorinated $C_1$ to $C_8$ alkyl or perfluorinated $C_1$ to $C_8$ alkyl.

According to an embodiment in formula (II) at least one of $X^1$, $X^2$ or $X^3$ is selected from N, C-H, or C-D.
According to an embodiment in formula (II) one or two of $X^1$, $X^2$ or $X^3$ is selected from N, C-H, or C-D.
According to an embodiment, in formula (III) at least one of $X^{1'}$, $X^{2'}$, $X^{3'}$ and $X^{4'}$ is selected from N, C-H, or C-D.
According to an embodiment, in formula (III) one or two of $X^{1'}$, $X^{2'}$, $X^{3'}$ and $X^{4'}$ is selected from N, C-H, or C-D.
According to an embodiment, in formula (IIIb) at least one of $R^{1'}$, $R^{2'}$, $R^{3'}$ and $R^{4'}$ is selected from H or D.
According to an embodiment, in formula (IIIb) one or two of $R^{1'}$, $R^{2'}$, $R^{3'}$ and $R^{4'}$ is selected from H or D.
According to an embodiment, in formula (IIIb) at least one $R^{1'}$, $R^{2'}$, and $R^{3'}$ is selected from HorD.
According to an embodiment, in formula (IIIb) one or two $R^{1'}$, $R^{2'}$, and $R^{3'}$ is selected from HorD.
According to an embodiment, in formula (III) only one of $X^1$, $X^{2'}$, $X^{3'}$ and $X^{4'}$ is selected from C-H, or C-D.
According to an embodiment, in formula (IIIb) only one of $X^{1'}$, $X^{2'}$, $X^{3'}$ and $X^{4'}$ is selected from C-H, or C-D.
According to an embodiment, if $A^3$ is equal to $A^2$, then $A^2$ and $A^3$ are selected from formula (IIIc)

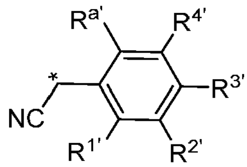

(IIIc)

whereby

$R^{a'}$ are independently selected from CN, $CF_3$, partially fluorinated $C_1$ to $C_8$ alkyl or perfluorinated $C_1$ to $C_8$ alkyl, $R^{1'}$, $R^{2'}$, $R^{3'}$ and $R^{4'}$ are independently selected from H, D, CN, $CF_3$, partially fluorinated $C_1$ to $C_8$ alkyl or perfluorinated $C_1$ to $C_8$ alkyl.

According to an embodiment, whereby when in formula (II) one or two of X is N then formula (III) is selected from formula (IIId)

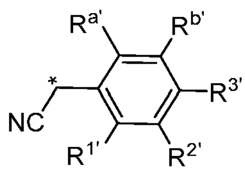

(IIId)

whereby $R^{a'}$, and $R^{b'}$ are independently selected from CN, $CF_3$, partially fluorinated $C_1$ to $C_8$ alkyl or perfluorinated $C_1$ to $C_8$ alkyl,
$R^{1'}$, $R^{2'}$, and $R^{3'}$ are independently selected from H, D, CN, $CF_3$, partially fluorinated $C_1$ to $C_8$ alkyl or perfluorinated $C_1$ to $C_8$ alkyl.

According to an embodiment, formula (III) is selected from formula (IIId).
According to an embodiment, if $A^3$ is selected from formula (III) then $A^2$ and $A^3$ are independently selected from formula (IIId).
According to an embodiment, if $A^3$ is equal to $A^2$, then $A^2$ and $A^3$ are selected from formula (IIId).

According to an embodiment, in formulae (III), (IIIb), (IIIc) and/or (IIId) 1 to 3 of $R^{1'}$, $R^{2'}$, $R^{3'}$ and - if present - $R^{4'}$ are independently selected form $CF_3$ or partially fluorinated $C_1$ to $C_8$ alkyl groups or perfluorinated $C_1$ to $C_8$ alkyl groups, preferably one to two.

According to an embodiment, the compound of formula (I) comprises 3 to 9 $CF_3$ groups; preferably 4 to 8; more preferably 5 to 7, and most preferably 6 to 7.

According to an embodiment in formula (II) 1 to 3 of $R^a$, $R^b$, $R^1$, $R^2$, and $R^3$ is independently selected from $CF_3$ groups.

According to an embodiment, in formulae (III), (IIIb), (IIIc) and/or (IIId) 1 to 3 of $R^{1'}$, $R^{2'}$, $R^{3'}$ and - if present - $R^4$ are independently selected form $CF_3$, preferably 1 to 2.

According to an embodiment, in compound of formula (I) the sum of $CF_3$ groups or partially fluorinated $C_1$ to $C_8$ alkyl groups or perfluorinated $C_1$ to $C_8$ alkyl groups of present $R^a$, $R^b$, $R^{a'}$ is from 1 to 5, preferably 2 to 5,

According to an embodiment, in compound of formula (I) the sum of $CF_3$ groups of present $R^a$, $R^b$, $R^{a'}$ is from 1 to 5, preferably 2 to 5.

According to an embodiment in formula (II) $R^1$, $R^2$, and $R^3$, if present, are independently selected from H, D, CN, or $CF_3$, and $R^a$ and $R^b$ are independently selected from $CF_3$ or CN.

According to an embodiment in formula (III), $R^{1'}$, $R^{2'}$, $R^{3'}$ and $R^{4'}$, if present, are independently selected from H, D, CN, or $CF_3$, and $R^{a'}$ is selected from CN or $CF_3$,

According to an embodiment in formula (IIIb), (IIIc) and/or (IIId) $R^{1'}$, $R^{2'}$, $R^{3'}$ and - if present - $R^{4'}$, if present, are independently selected from H, D, CN, or $CF_3$, and $R^{a'}$ is selected from CN or $CF_3$

According to an embodiment in formula (IIIc) $R^{1'}$, $R^{2'}$, and $R^{3'}$ are independently selected from H, D, CN, or $CF_3$, and $R^{a'}$ and $R^{b'}$ are independently selected from CN or $CF_3$.

According to an embodiment in formula (II) $R^1$, $R^2$, and $R^3$, if present, are independently selected from H, D, CN, or $CF_3$, and $R^a$ and $R^b$ are independently selected from $CF_3$ or CN; and wherein in formula (III), $R^{1'}$, $R^{2'}$, $R^{3'}$ and $R^{4'}$, if present, are independently selected from H, D, CN, or $CF_3$, and $R^{a'}$ is selected from CN or $CF_3$.

According to an embodiment in formula (II) $R^1$, $R^2$, and $R^3$, if present, are independently selected from H, D, CN, or $CF_3$, and $R^a$ and $R^b$ are independently selected from $CF_3$ or CN; and wherein in formula (IIIb) $R^{1'}$, $R^{2'}$, $R^{3'}$ and $R^{4'}$ are independently selected from H, D, CN, or $CF_3$, and $R^{a'}$ is selected from CN or $CF_3$.

According to an embodiment in formula (II) $R^1$, $R^2$, and $R^3$, if present, are independently selected from H, D, CN, or $CF_3$, and $R^a$ and $R^b$ are independently selected from $CF_3$ or CN; and wherein in formula (IIIc) $R^{1'}$, $R^{2'}$, and $R^{3'}$ are independently selected from H, D, CN, or $CF_3$, and $R^{a'}$ and $R^{b'}$ are independently selected from CN or $CF_3$.

According to an embodiment when in formula (II) one or two of X is N then in formula (III) none of the $X^{1'}$ to $X^{4'}$ is N.

According to an embodiment when in formula (II) one to three of X is N then in formula (III) none of the $X^{1'}$ to $X^{4'}$ is N.

According to an embodiment when in formula (II) one to three of X is N then $A^3$ is selected from formula (III), and none of the $X^{1'}$ to $X^{4'}$ in formula (III) is N, or when in formula (III) one to four of X is N then $A^3$ is selected from formula (II) and none of the $X^1$ to $X^3$ in formula (II) is N.

According to an embodiment when in formula (II) one or two of X is N then in formula (III) none of the $X^{1'}$ to $X^{4'}$ is N and only one of $R^{1'}$, $R^{2'}$, $R^{3'}$ and $R^{4'}$ is H or D.

According to an embodiment when in formula (II) one to three of X is N then in formula (III) none of the $X^{1'}$ to $X^{4'}$ is N and only one of $R^1$, $R^{2'}$, $R^{3'}$ and $R^{4'}$ is H or D.

According to an embodiment when in formula (II) one to three of X is N then $A^3$ is selected from formula (III), none of the $X^{1'}$ to $X^{4'}$ in formula (III) is N and only one of $R^1$, $R^{2'}$, $R^{3'}$ and $R^{4'}$ is H or D, or when in formula (III) one to four of X is N then $A^3$ is selected from formula (II) and none of the $X^1$ to $X^3$ in formula (II) is N.

According to an embodiment in formula (II) and/or (III) if $X^n$ is N then $X^{n+1}$ is $CR^{n+1}$ with n=1 to 2, and $X^{n-1}$ is $CR^{n-1}$ with n=2 to 3, in other words neither the moiety of formula (II) or formula (III) has a pyrazidine core or 1,2,3-triazine core.

The thermal stability of the compounds of formula (I) can be further increased.

According to an embodiment formula (II) is selected from formula (IIb)

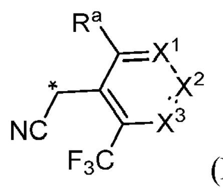

(IIb).

According to an embodiment, formula (II) is selected from B1 to B24:

| | | | |
|---|---|---|---|
| 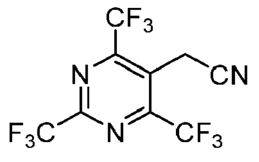 | 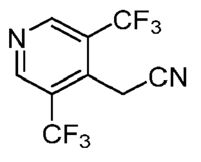 | 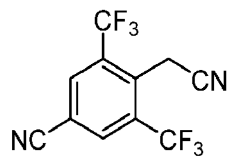 | 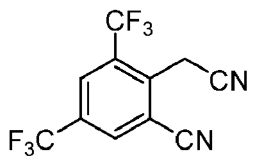 |
| B1 | B2 | B3 | B4 |
| 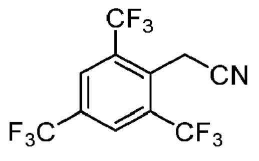 | 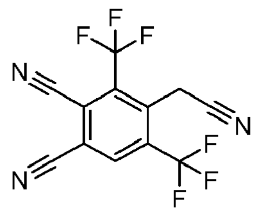 | 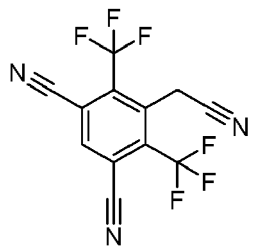 | 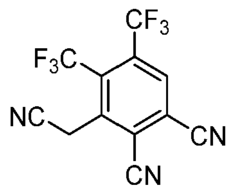 |
| B5 | B6 | B7 | B8 |
| 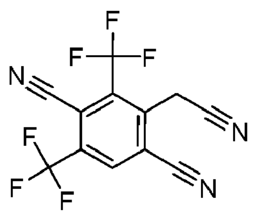 | 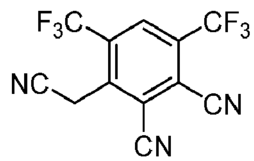 | 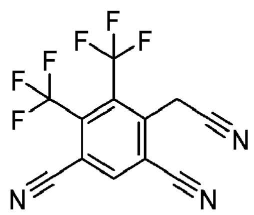 | 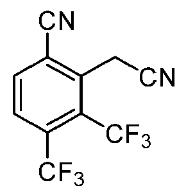 |
| B9 | B10 | B11 | B12 |
| 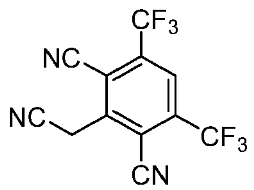 | 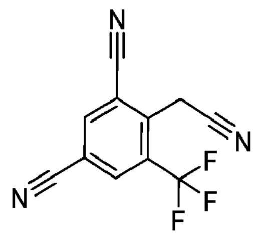 | 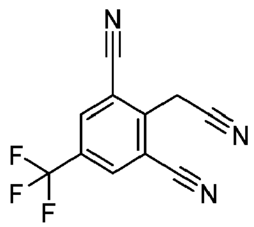 | 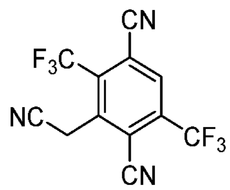 |
| B13 | B14 | B15 | B16 |
| 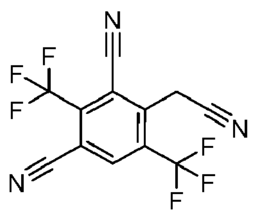 | 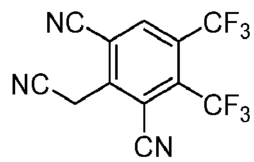 | 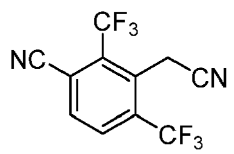 | 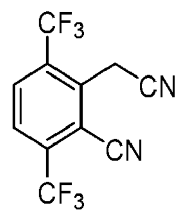 |
| B17 | B18 | B19 | B20 |
| 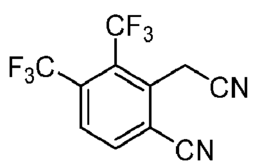 | 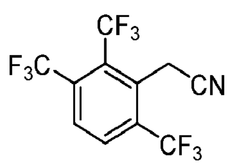 | 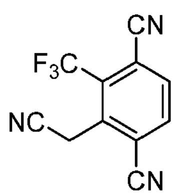 | 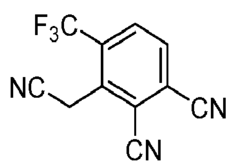 |
| B21 | B22 | B23 | B24 |

According to a preferred embodiment, formula (II) is selected from B1 to B23.

According to a more preferred embodiment, formula (II) is selected from B1 to 12.

According to an embodiment, wherein formula (III) is selected from C1 to C56:

| | | | |
|---|---|---|---|
| 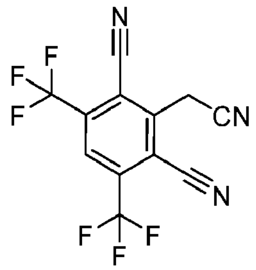 | 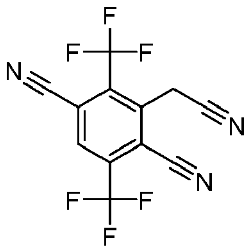 | 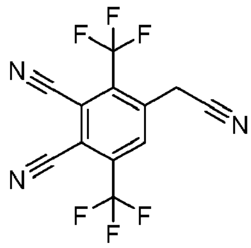 | 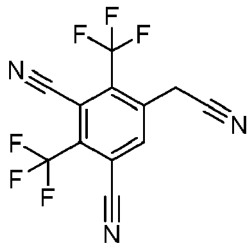 |
| C1 | C2 | C3 | C4 |
| 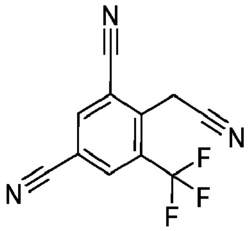 | 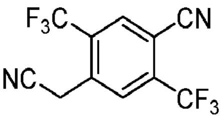 | 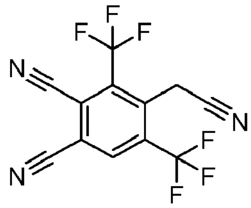 | 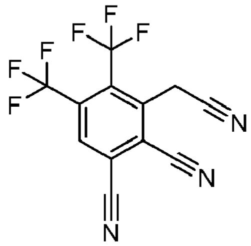 |
| C5 | C6 | C7 | C8 |
| 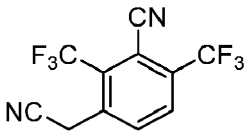 | 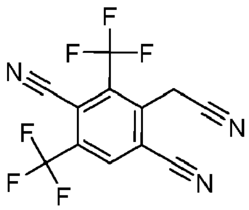 | 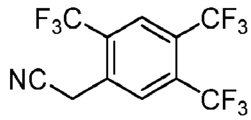 | 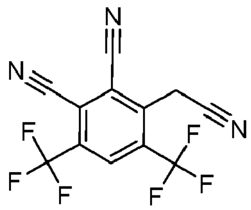 |
| C9 | C10 | C11 | C12 |
| 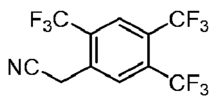 | 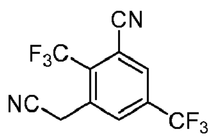 | 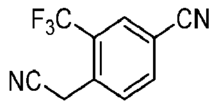 | 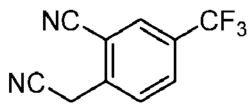 |
| C13 | C14 | C15 | C16 |
| 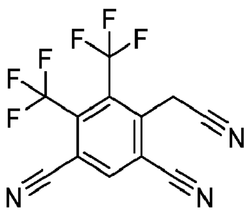 | 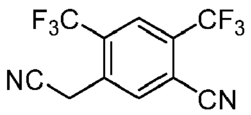 | 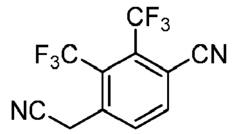 | 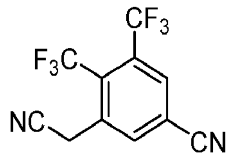 |
| C17 | C18 | C19 | C20 |
| 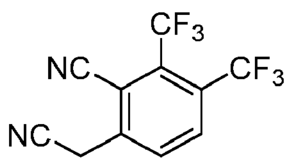 | 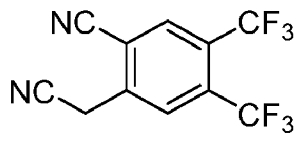 | | |
| C21 | C22 | | |

(continued)

| | | | |
|---|---|---|---|
| 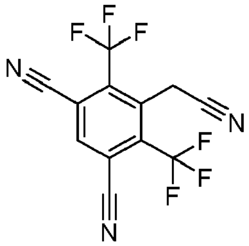 | 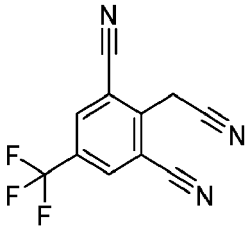 | 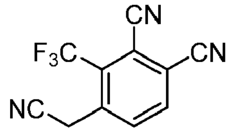 | 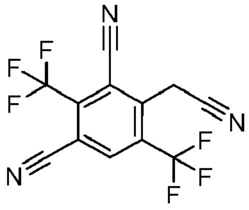 |
| C23 | C24 | C25 | C26 |
| 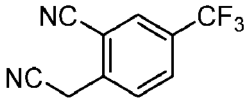 | 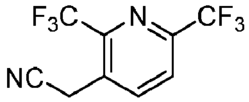 | 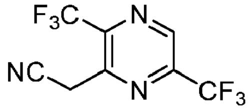 | 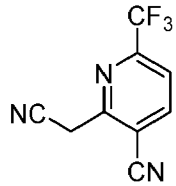 |
| C27 | C28 | C29 | C30 |
| 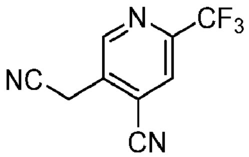 | 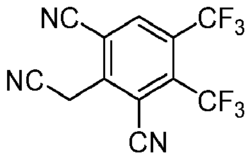 | 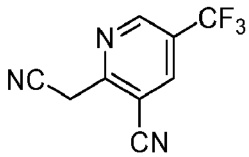 | |
| C31 | C32 | C33 | |
| 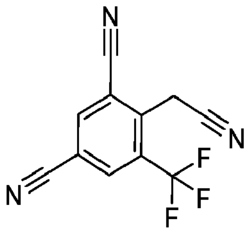 | 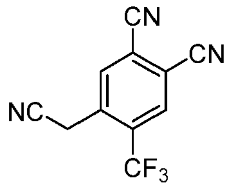 | 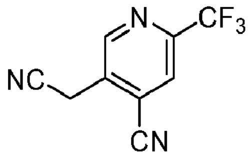 | 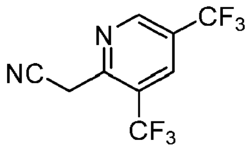 |
| C34 | C35 | C36 | C37 |
| 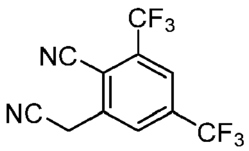 | 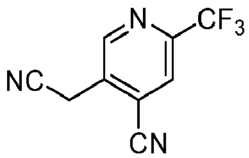 | 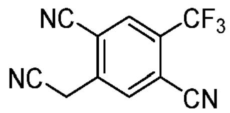 | 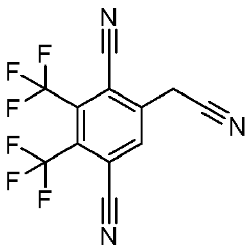 |
| C38 | C39 | C40 | C41 |
| 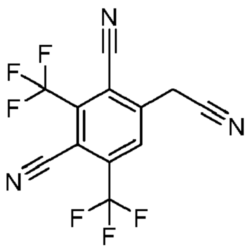 | 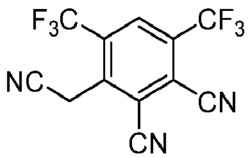 | 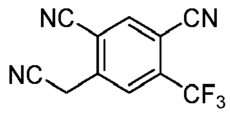 | 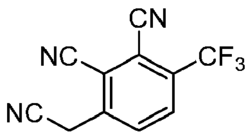 |
| C42 | C43 | C44 | C45 |

(continued)

| | | | |
|---|---|---|---|
| 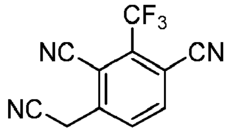 | 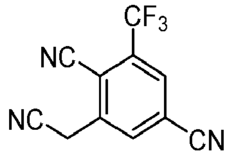 | 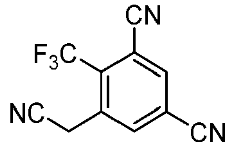 | 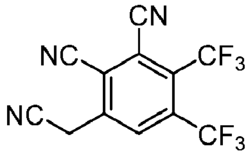 |
| C46 | C47 | C48 | C49 |
| 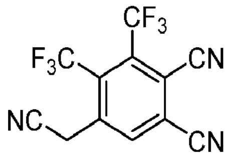 | 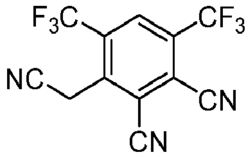 | 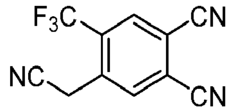 | 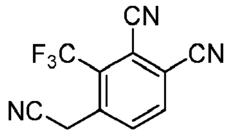 |
| C50 | C51 | C52 | C53 |
| 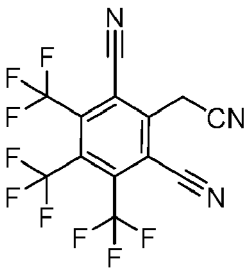 | 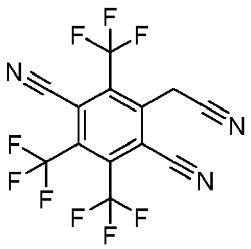 | 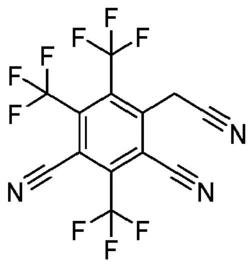 | |
| C54 | C55 | C56 | |

According to a preferred embodiment formula (III) is selected from to C53.

According to a more preferred embodiment formula (III) is selected from C1 to C33.

According to a most preferred embodiment formula (III) is selected from C1 to C22 or C26.

According to an embodiment formula (II) is selected B1 to B24, and formula (III) is selected from C1 to C33.

According to an embodiment, the compound of formula (I) is selected from A1 to A351:

| | $A^1$ | $A^2$ | A3 |
|---|---|---|---|
| A1 | 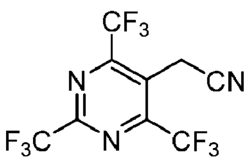 | 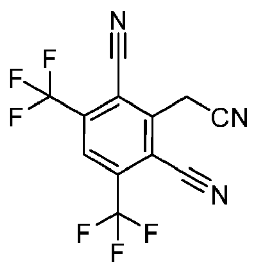 | 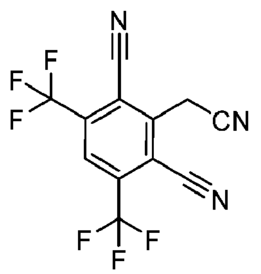 |
| A2 | 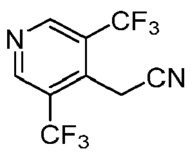 | 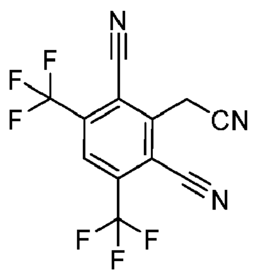 | 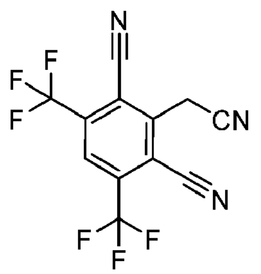 |

(continued)

| | $A^1$ | $A^2$ | A3 |
|---|---|---|---|
| A3 | 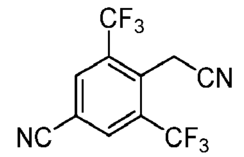 | 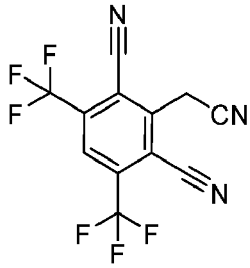 | 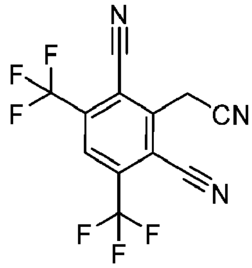 |
| A4 | 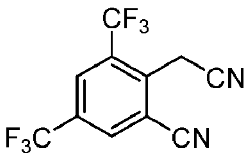 | 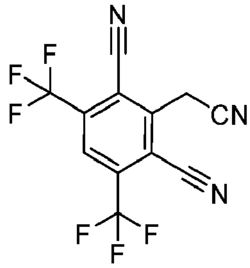 | 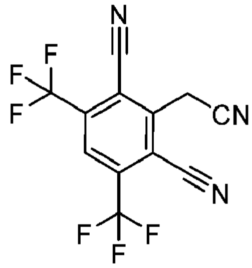 |
| A5 | 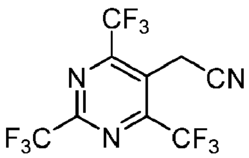 | 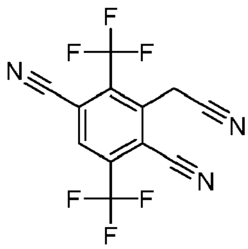 | 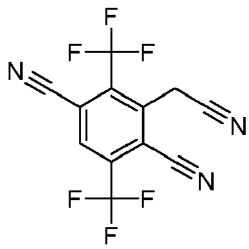 |
| A6 | 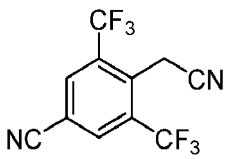 | 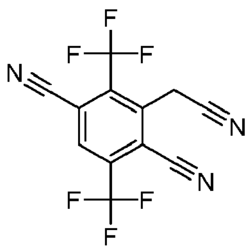 | 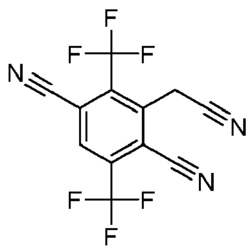 |
| A7 | 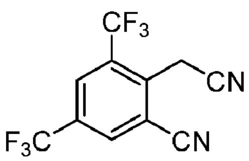 | 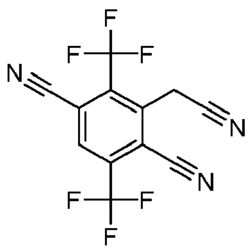 | 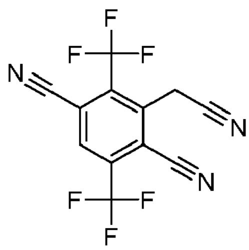 |
| A8 | 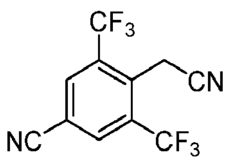 | 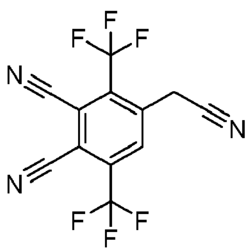 | 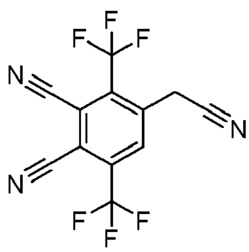 |

(continued)

| | $A^1$ | $A^2$ | A3 |
|---|---|---|---|
| A9 | 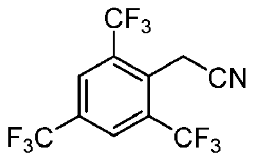 | 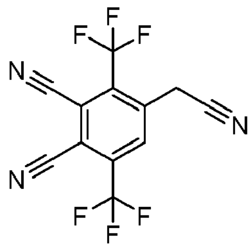 | 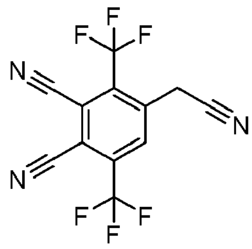 |
| A10 | 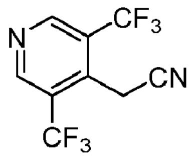 | 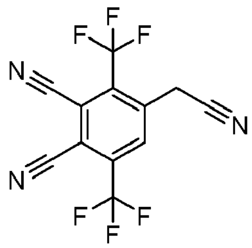 | 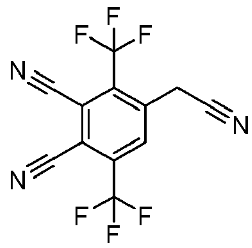 |
| A11 | 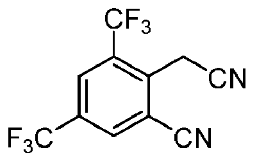 | 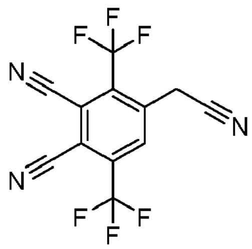 | 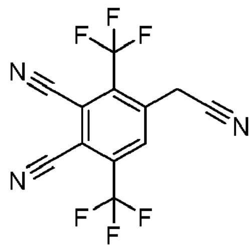 |
| A12 | 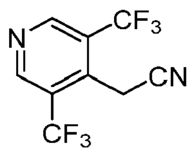 | 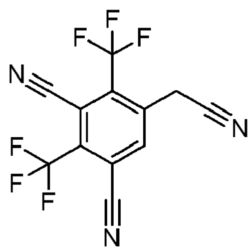 | 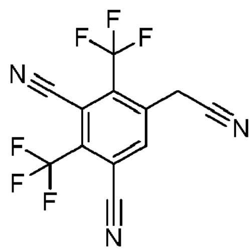 |
| A13 | 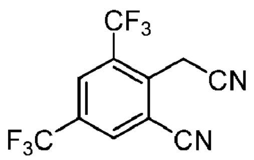 | 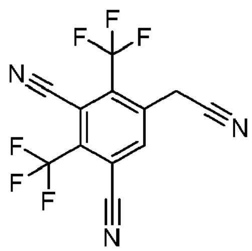 | 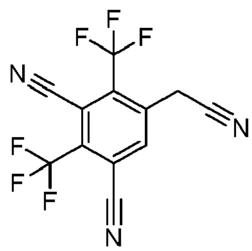 |
| A14 | 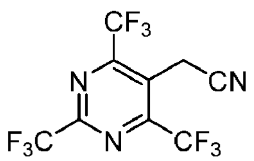 | 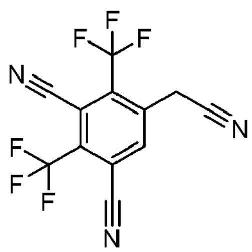 | 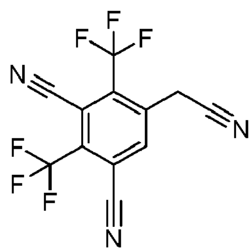 |

(continued)

| | A[1] | A[2] | A3 |
|---|---|---|---|
| A15 | 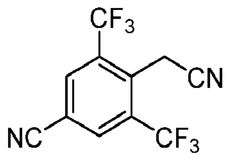 | 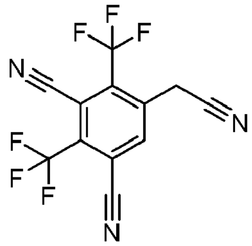 | 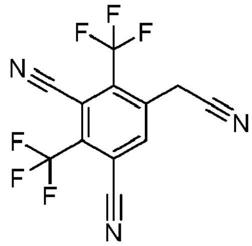 |
| A16 | 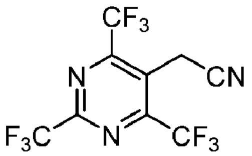 | 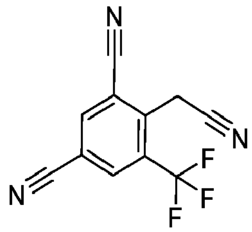 | 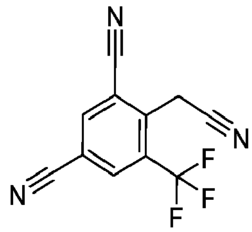 |
| A17 | 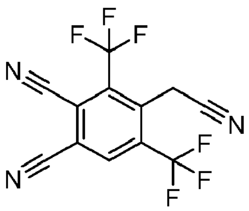 | 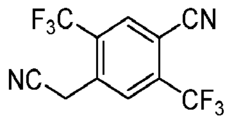 | 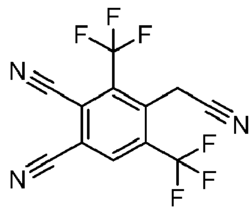 |
| A18 | 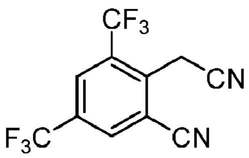 | 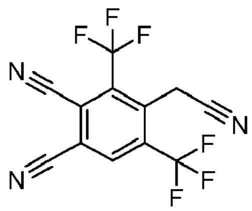 | 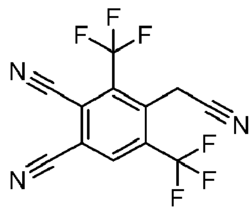 |
| A19 | 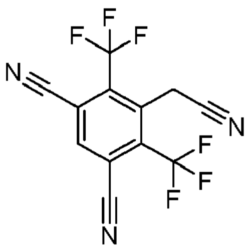 | 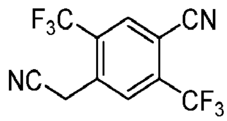 | 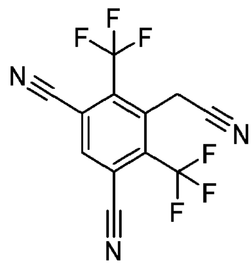 |
| A20 | 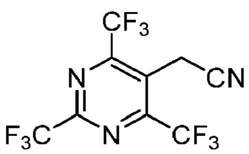 | 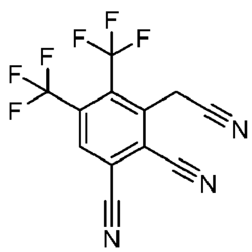 | 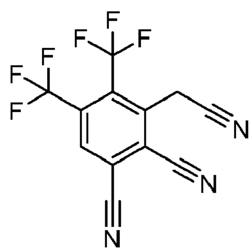 |

(continued)

| | $A^1$ | $A^2$ | A3 |
|---|---|---|---|
| A21 | 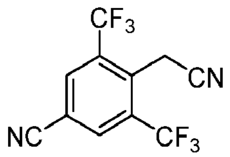 | 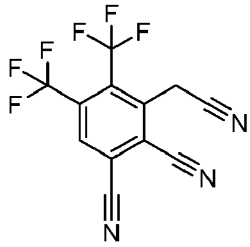 | 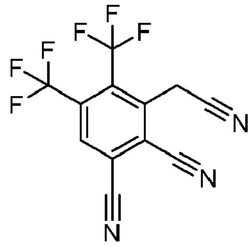 |
| A22 | 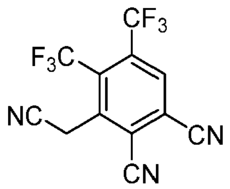 | 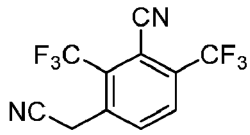 | 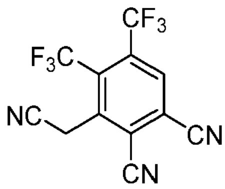 |
| A23 | 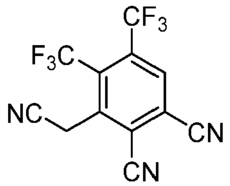 | 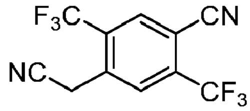 | 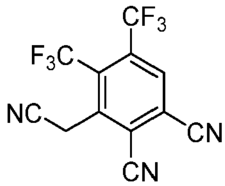 |
| A24 | 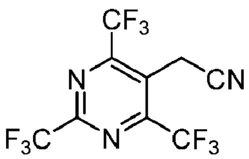 | 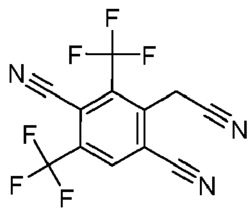 | 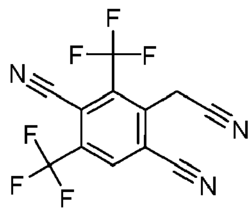 |
| A25 | 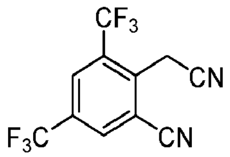 | 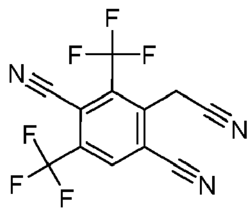 | 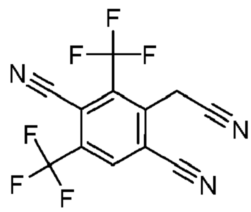 |
| A26 | 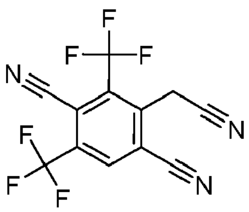 | 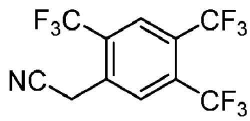 | 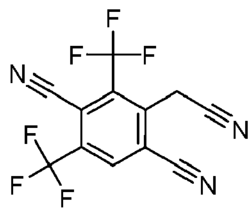 |
| A27 | 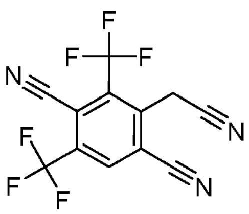 | 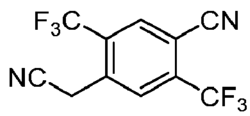 | 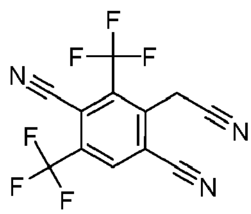 |

(continued)

| | $A^1$ | $A^2$ | A3 |
|---|---|---|---|
| A28 | 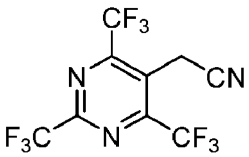 | 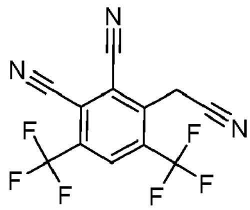 | 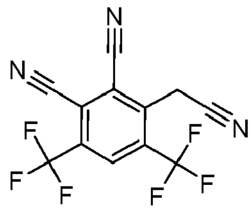 |
| A29 | 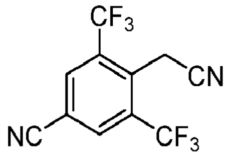 | 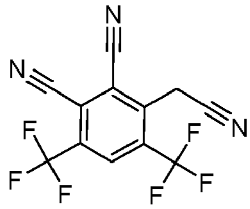 | 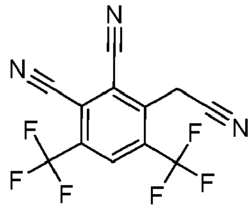 |
| A30 | 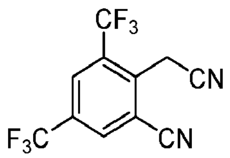 | 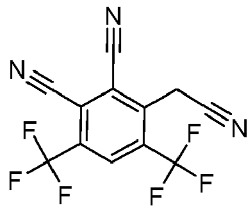 | 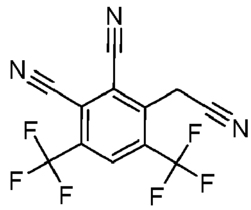 |
| A31 | 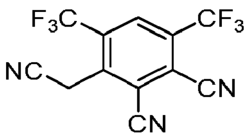 | 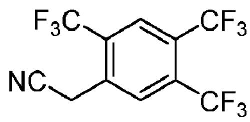 | 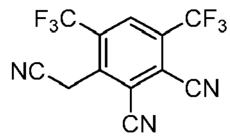 |
| A32 | 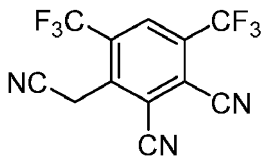 | 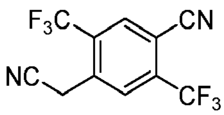 | 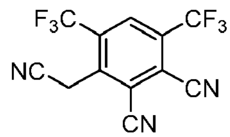 |
| A33 | 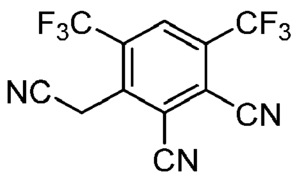 | 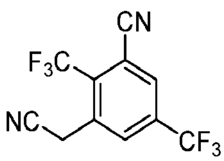 | 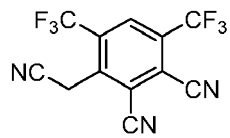 |
| A34 | 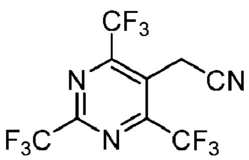 | 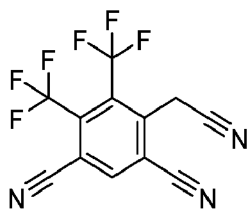 | 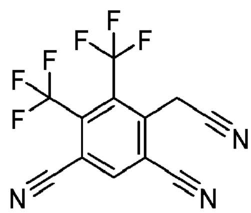 |
| A35 | 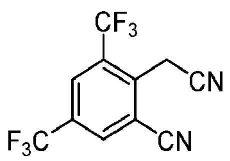 | 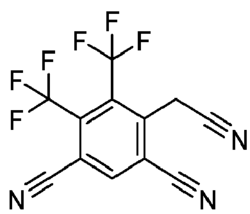 | 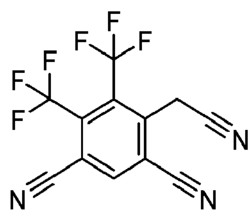 |

(continued)

| | A[1] | A[2] | A3 |
|---|---|---|---|
| A36 | 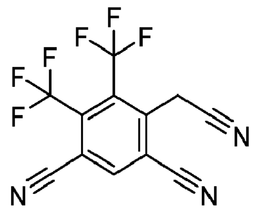 | 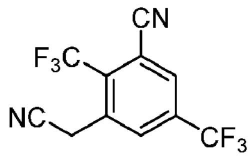 | 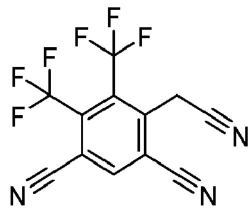 |
| A37 | 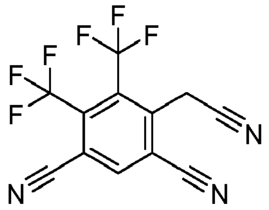 | 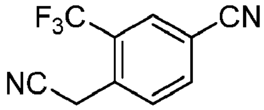 | 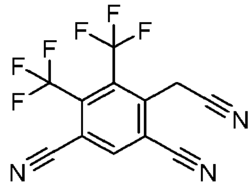 |
| A38 | 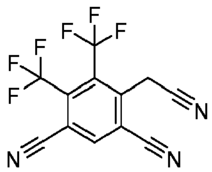 | 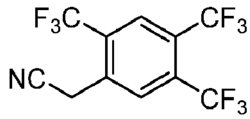 | 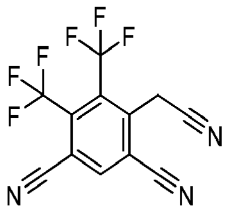 |
| A39 | 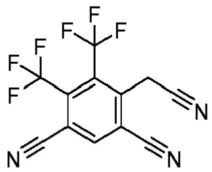 | 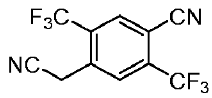 | 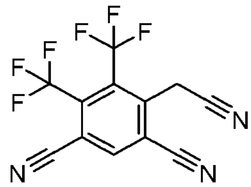 |
| A40 | 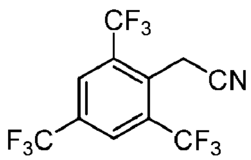 | 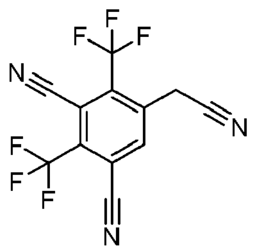 | 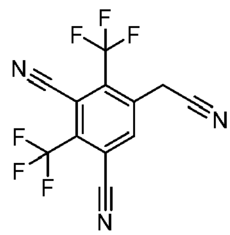 |
| A41 | 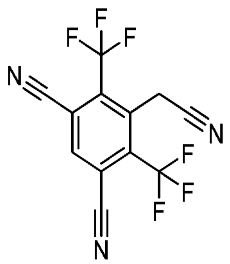 | 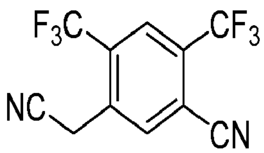 | 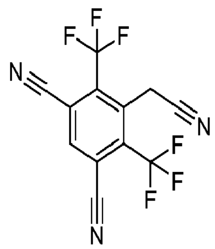 |

(continued)

| | $A^1$ | $A^2$ | A3 |
|---|---|---|---|
| A42 | 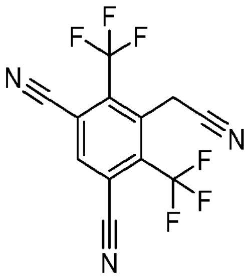 | 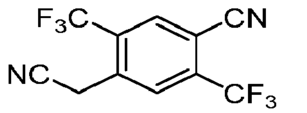 | 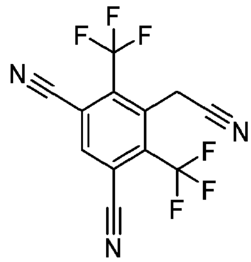 |
| A43 | 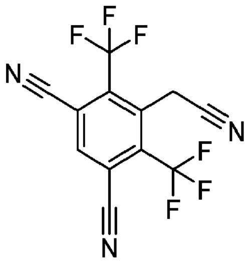 | 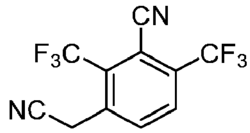 | 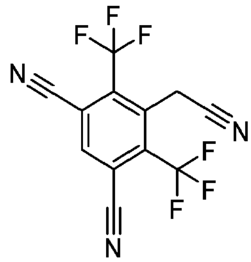 |
| A44 | 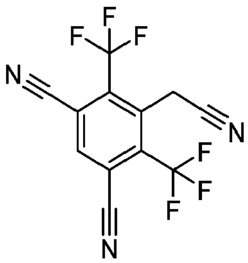 | 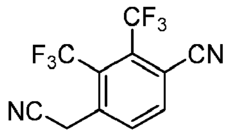 | 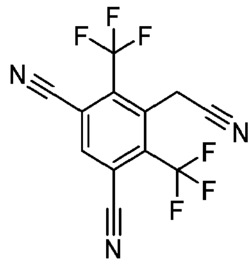 |
| A45 | 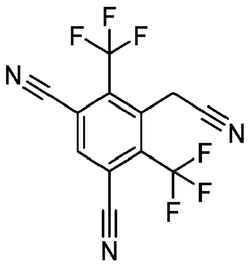 | 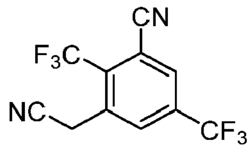 | 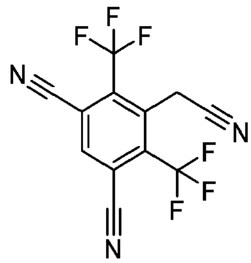 |
| A46 | 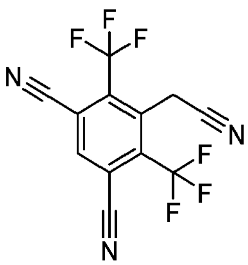 | 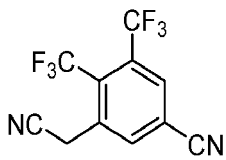 | 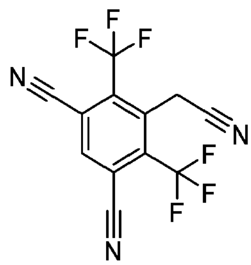 |
| A47 | 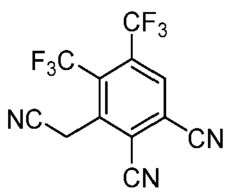 | 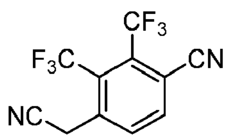 | 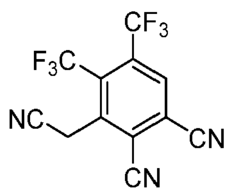 |

(continued)

| | $A^1$ | $A^2$ | A3 |
|---|---|---|---|
| A48 | 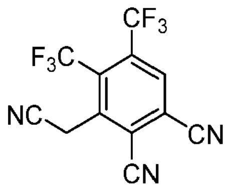 | 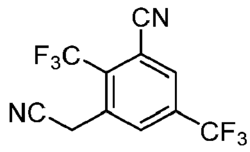 | 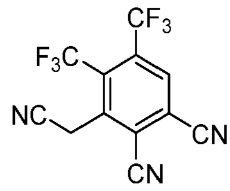 |
| A49 | 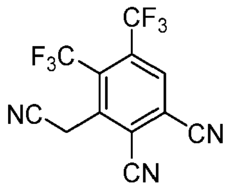 | 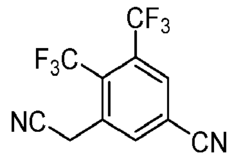 | 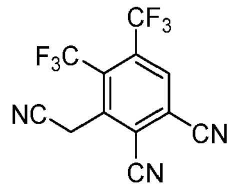 |
| A50 | 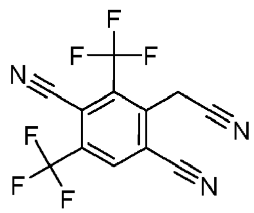 | 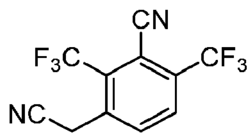 | 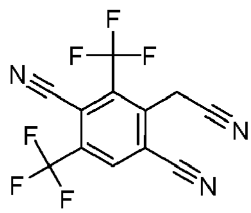 |
| A51 | 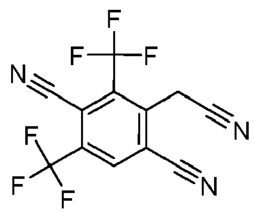 | 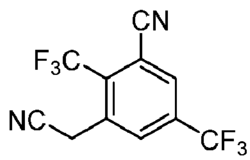 | 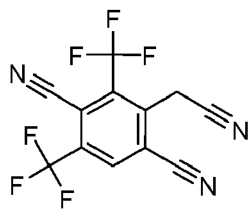 |
| A52 | 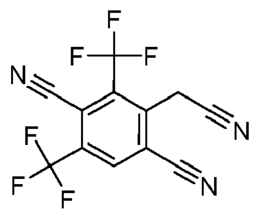 | 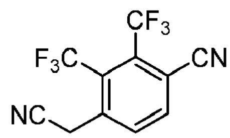 | 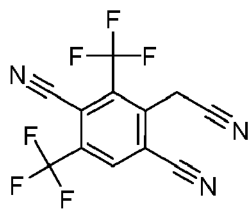 |
| A53 | 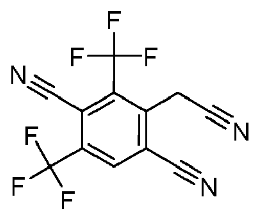 | 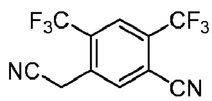 | 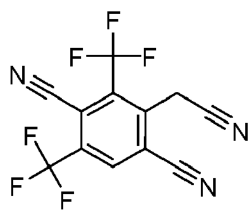 |
| A54 | 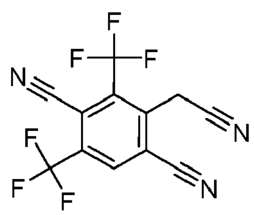 | 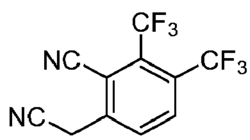 | 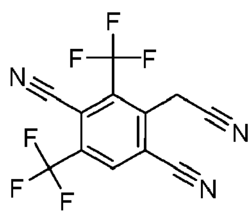 |

(continued)

| | $A^1$ | $A^2$ | A3 |
|---|---|---|---|
| A55 | 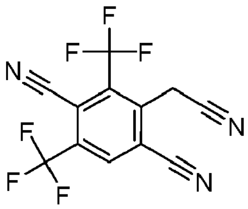 | 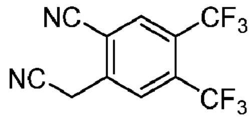 | 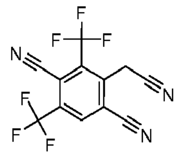 |
| A56 | 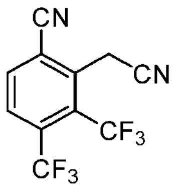 | 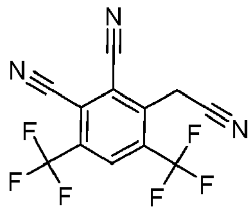 | 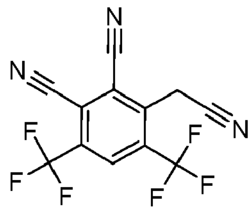 |
| A57 | 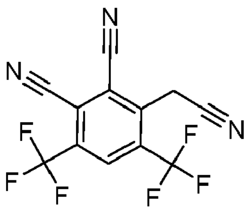 | 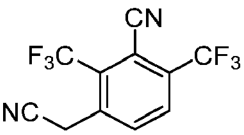 | 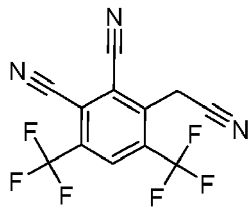 |
| A58 | 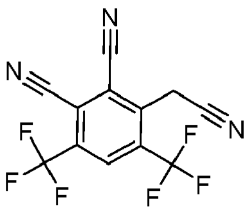 | 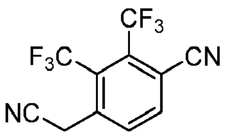 | 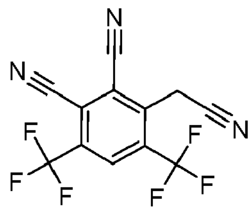 |
| A59 | 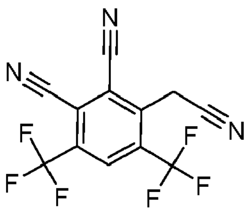 | 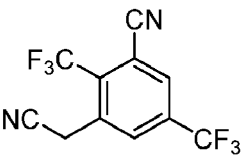 | 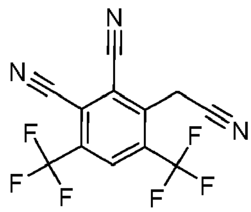 |
| A60 | 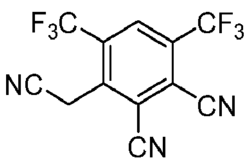 | 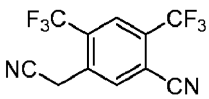 | 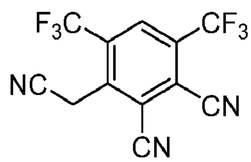 |
| A61 | 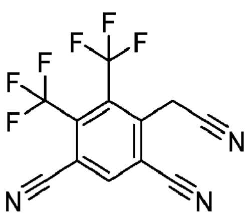 | 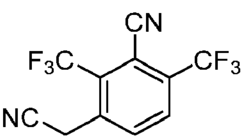 | 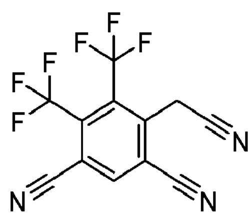 |

(continued)

| | $A^1$ | $A^2$ | A3 |
|---|---|---|---|
| A62 | 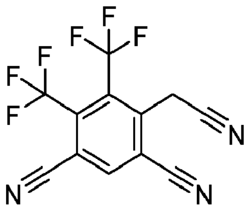 | 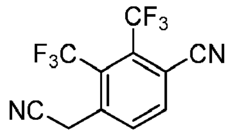 | 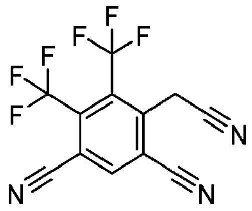 |
| A63 | 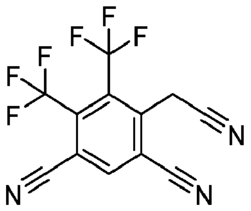 | 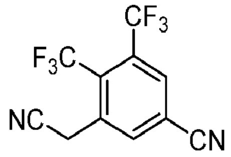 | 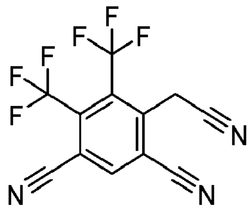 |
| A64 | 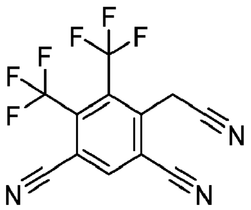 | 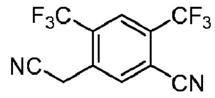 | 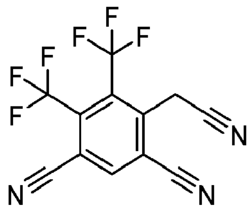 |
| A65 | 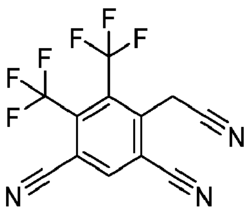 | 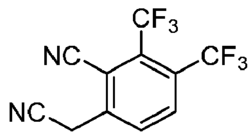 | 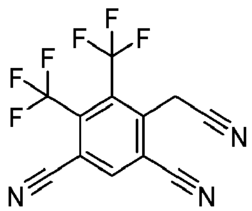 |
| A66 | 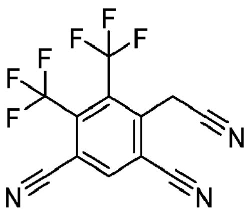 | 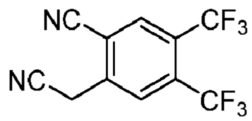 | 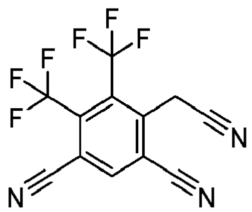 |
| A67 | 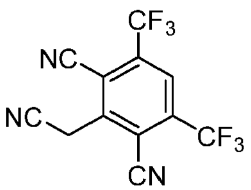 | 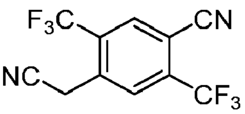 | 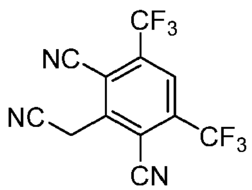 |
| A68 | 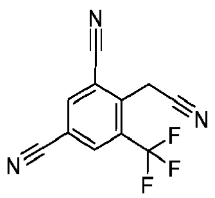 | 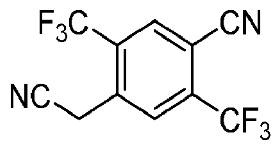 | 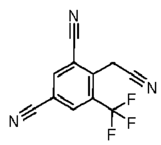 |

(continued)

| | $A^1$ | $A^2$ | A3 |
|---|---|---|---|
| A69 | 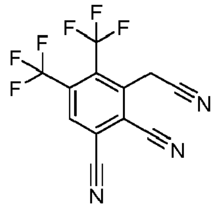 | 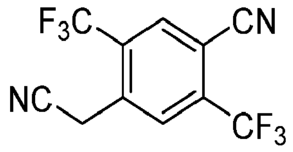 | 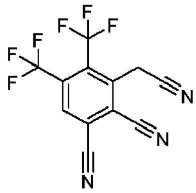 |
| A70 | 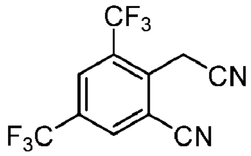 | 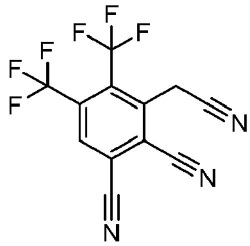 | 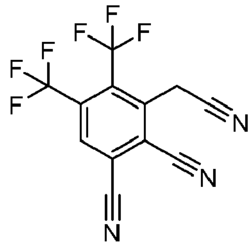 |
| A71 | 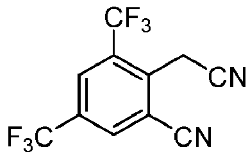 | 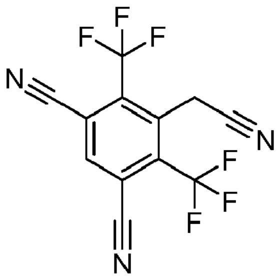 | 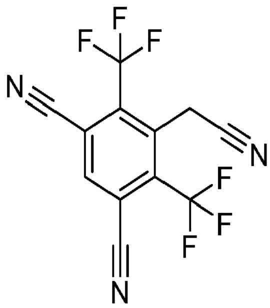 |
| A72 | 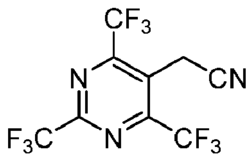 | 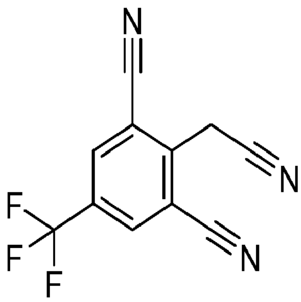 | 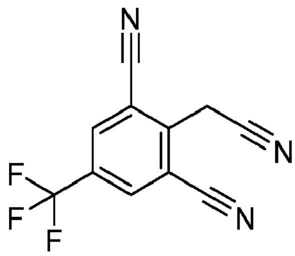 |
| A73 | 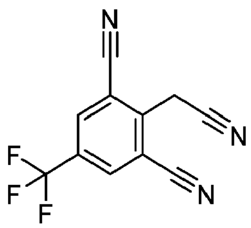 | 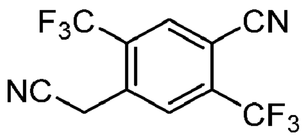 | 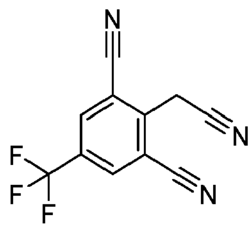 |
| A74 | 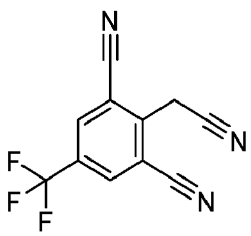 | 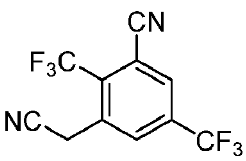 | 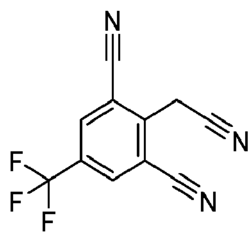 |

(continued)

| | A[1] | A[2] | A3 |
|---|---|---|---|
| A75 | 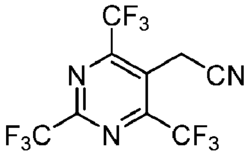 | 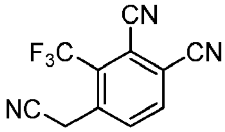 | 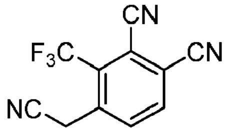 |
| A76 | 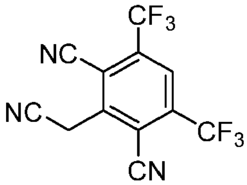 | 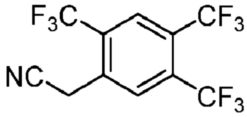 | 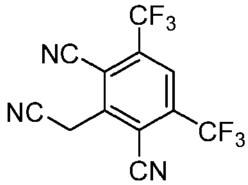 |
| A77 | 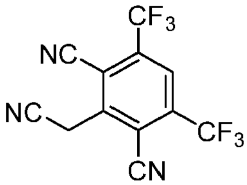 | 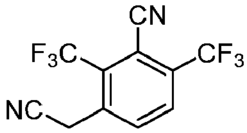 | 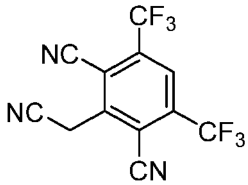 |
| A78 | 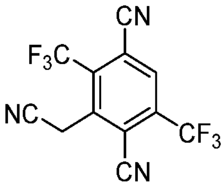 | 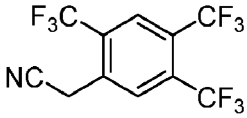 | 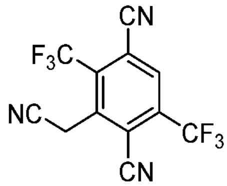 |
| A79 | 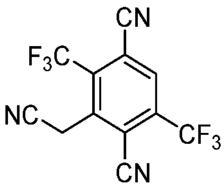 | 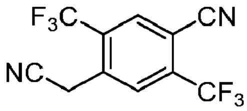 | 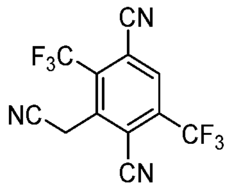 |
| A80 | 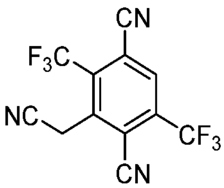 | 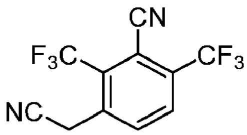 | 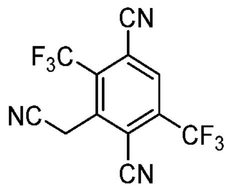 |
| A81 | 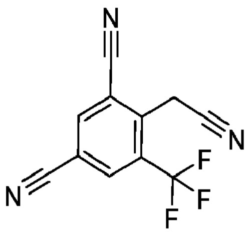 | 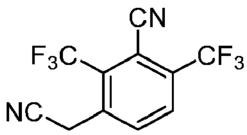 | 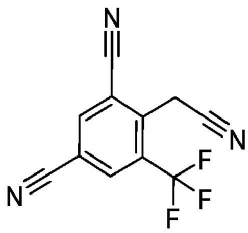 |
| A82 | 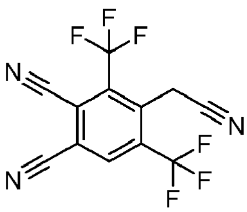 | 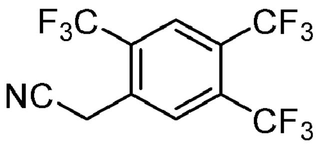 | 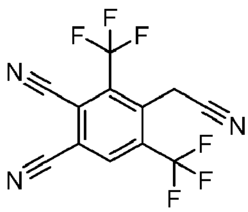 |

(continued)

| | $A^1$ | $A^2$ | A3 |
|---|---|---|---|
| A83 | 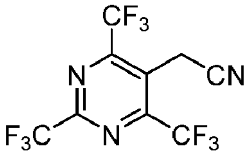 | 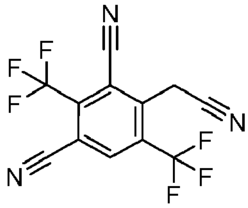 | 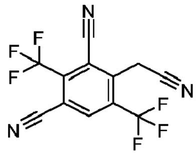 |
| A84 | 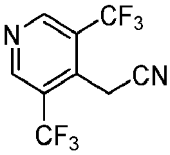 | 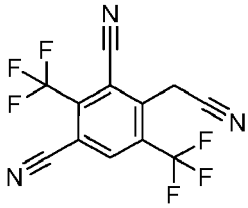 | 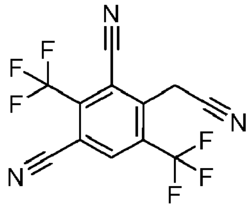 |
| A85 | 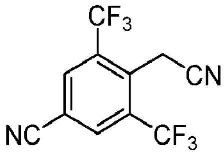 | 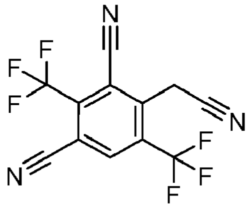 | 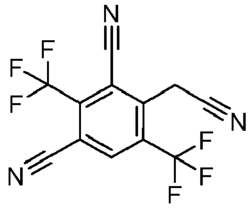 |
| A86 | 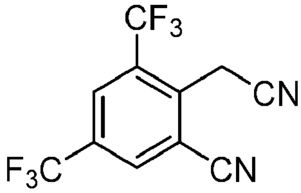 | 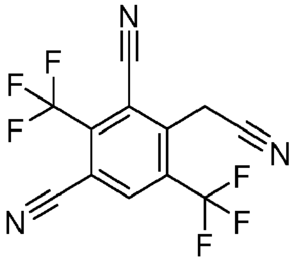 | 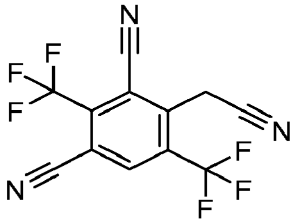 |
| A87 | 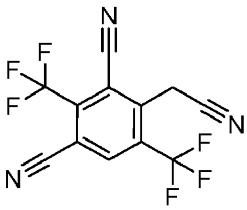 | 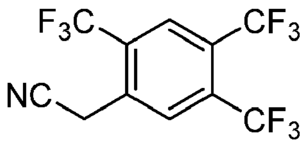 | 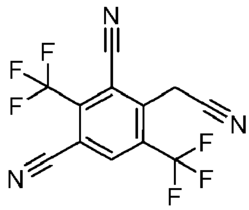 |
| A88 | 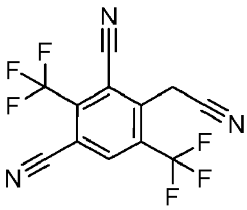 | 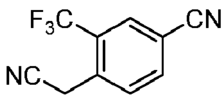 | 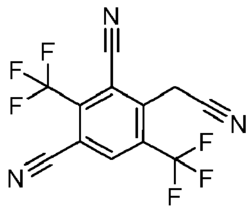 |
| A89 | 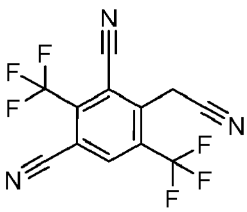 | 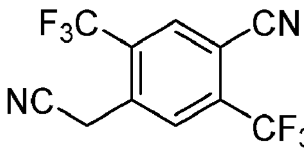 | 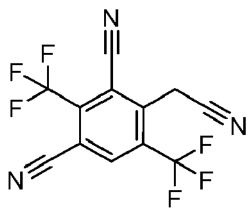 |

(continued)

| | $A^1$ | $A^2$ | A3 |
|---|---|---|---|
| A90 | 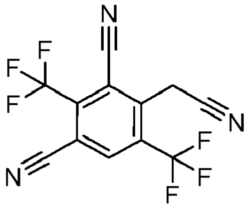 | 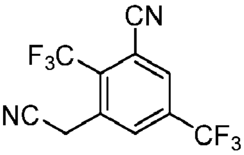 | 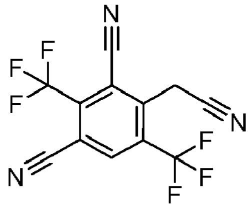 |
| A91 | 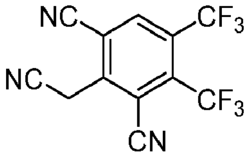 | 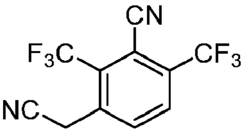 | 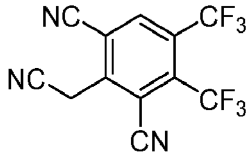 |
| A92 | 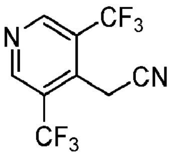 | 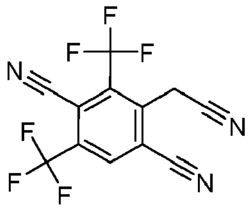 | 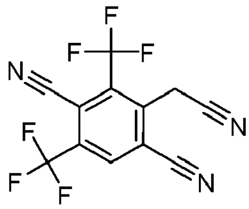 |
| A93 | 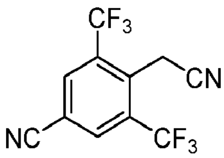 | 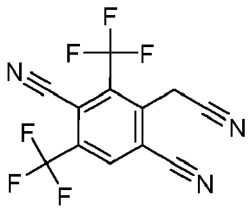 | 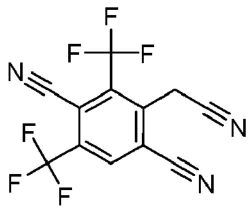 |
| A94 | 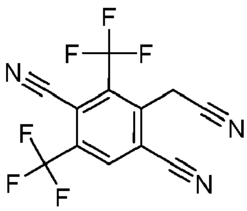 | 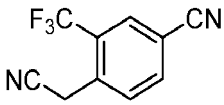 | 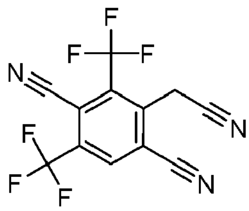 |
| A95 | 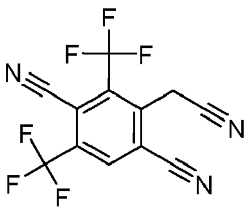 | 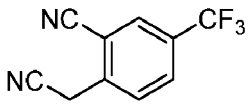 | 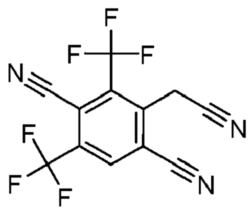 |
| A96 | 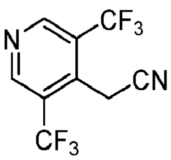 | 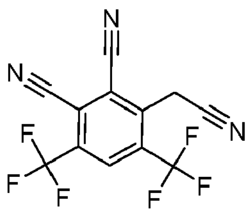 | 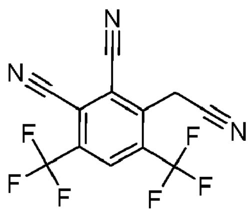 |

(continued)

| | A[1] | A[2] | A3 |
|---|---|---|---|
| A97 | 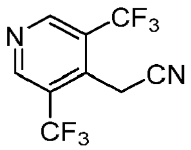 | 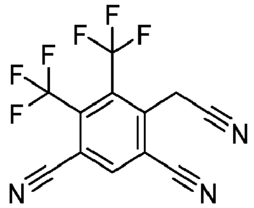 | 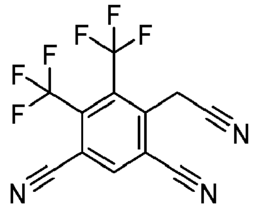 |
| A98 | 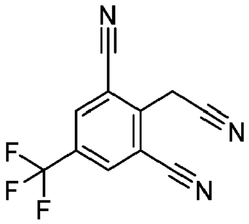 | 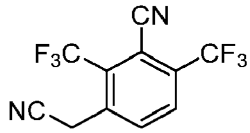 | 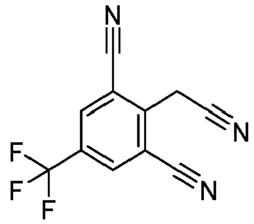 |
| A99 | 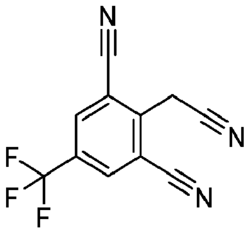 | 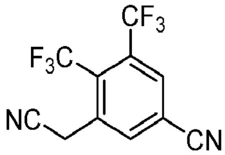 | 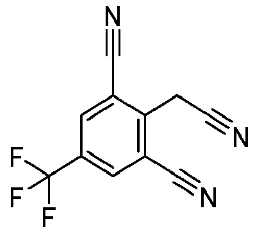 |
| A100 | 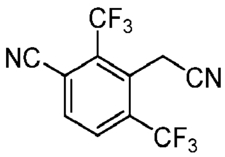 | 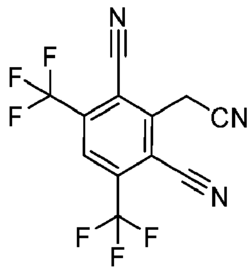 | 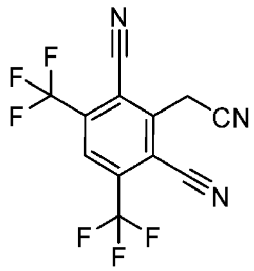 |
| A101 | 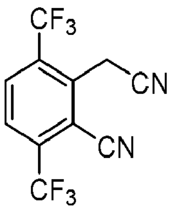 | 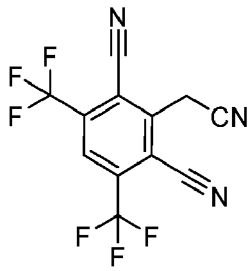 | 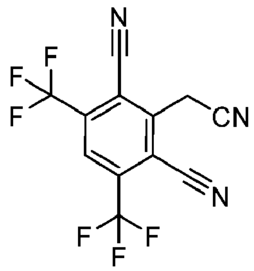 |
| A102 | 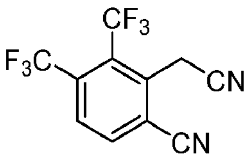 | 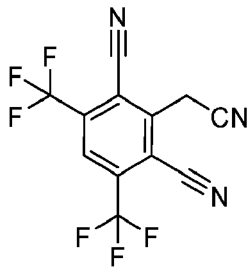 | 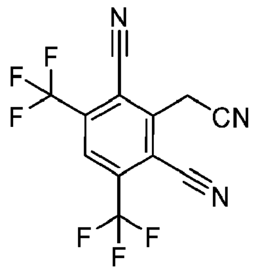 |

(continued)

| | $A^1$ | $A^2$ | A3 |
|---|---|---|---|
| A103 | 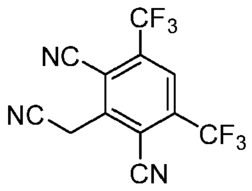 | 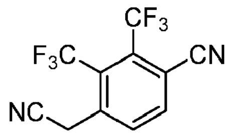 | 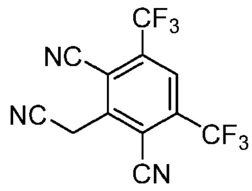 |
| A104 | 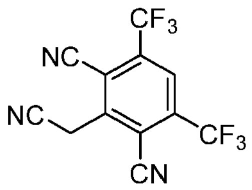 | 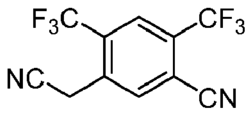 | 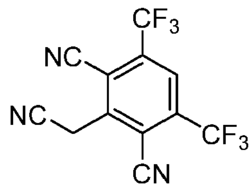 |
| A105 | 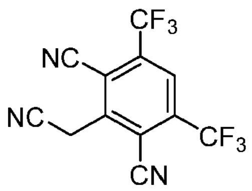 | 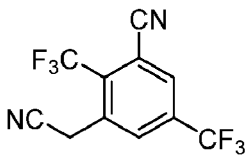 | 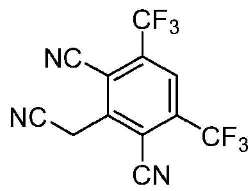 |
| A106 | 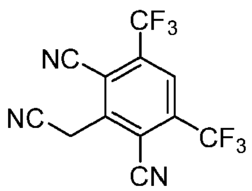 | 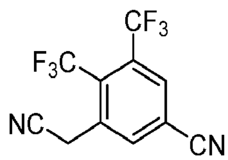 | 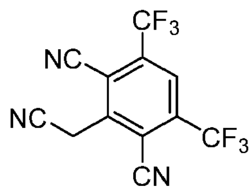 |
| A107 | 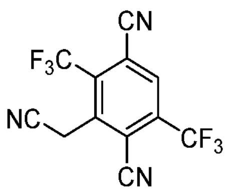 | 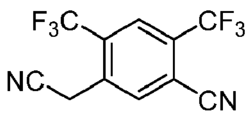 | 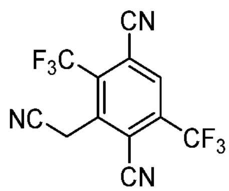 |
| A108 | 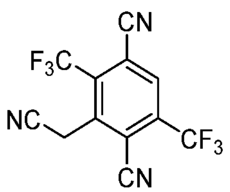 | 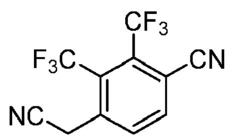 | 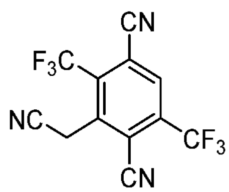 |
| A109 | 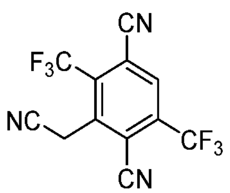 | 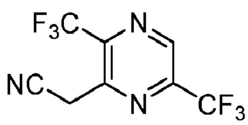 | 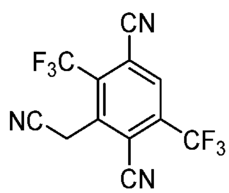 |
| A110 | 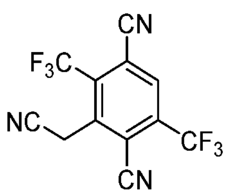 | 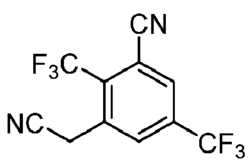 | 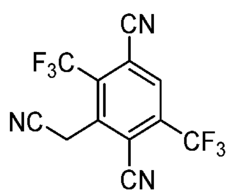 |

(continued)

| | A¹ | A² | A3 |
|---|---|---|---|
| A111 | 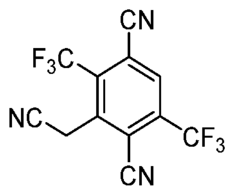 | 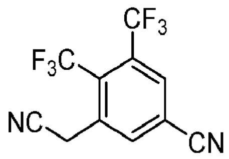 | 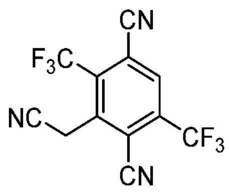 |
| A112 | 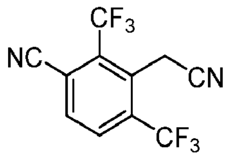 | 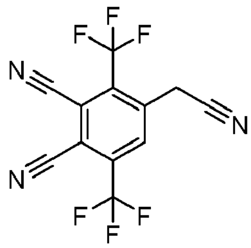 | 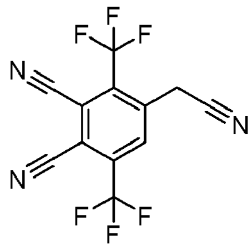 |
| A113 | 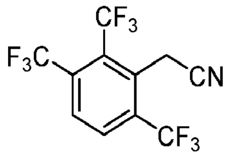 | 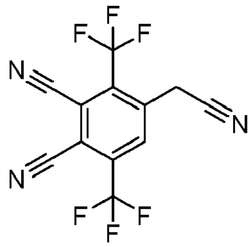 | 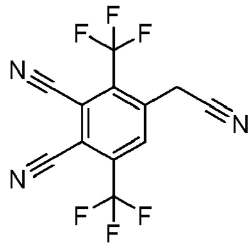 |
| A114 | 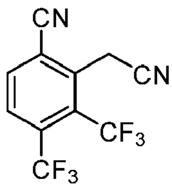 | 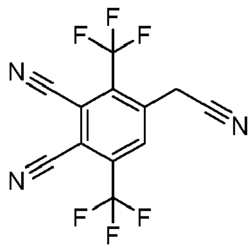 | 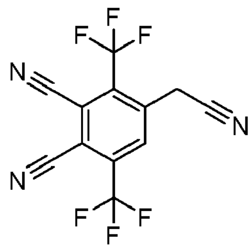 |
| A115 | 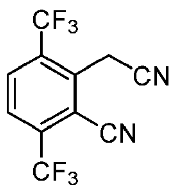 | 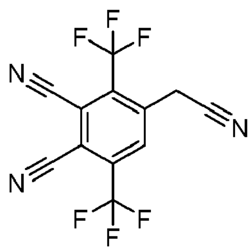 | 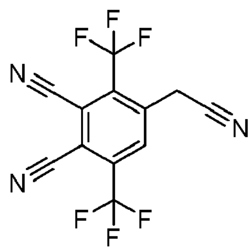 |
| A116 | 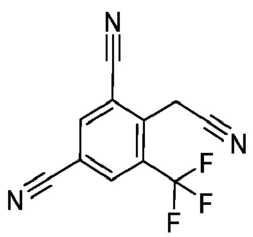 | 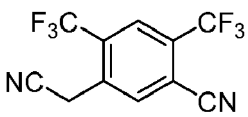 | 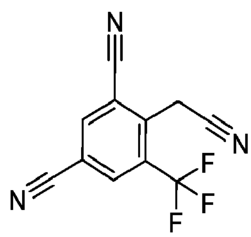 |

(continued)

| | $A^1$ | $A^2$ | A3 |
|---|---|---|---|
| A117 | 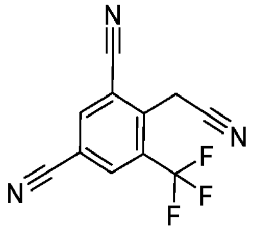 | 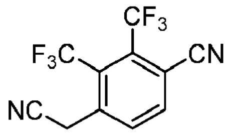 | 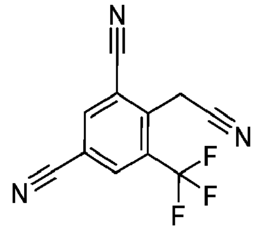 |
| A118 | 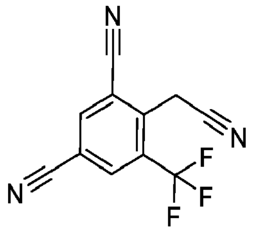 | 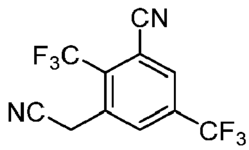 | 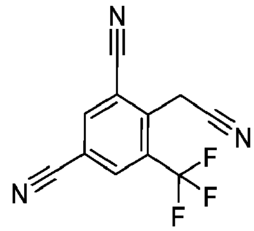 |
| A119 | 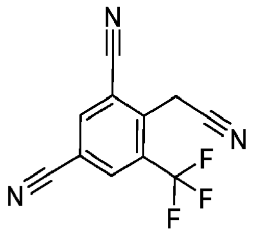 | 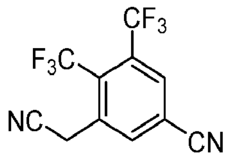 | 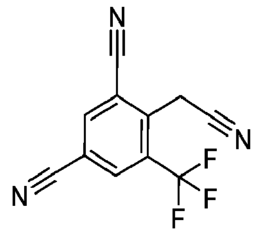 |
| A120 | 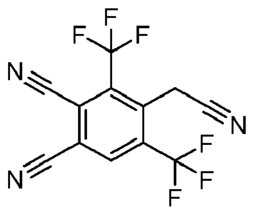 | 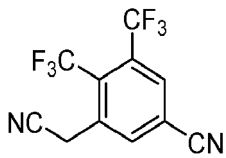 | 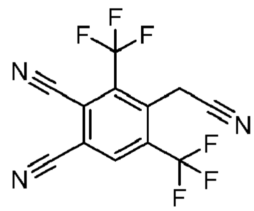 |
| A121 | 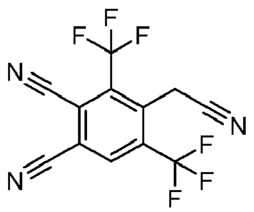 | 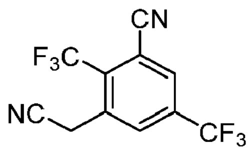 | 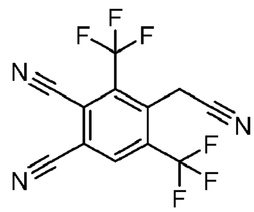 |
| A122 | 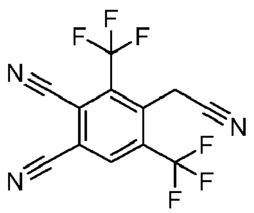 | 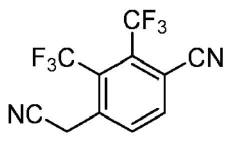 | 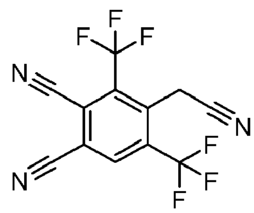 |
| A123 | 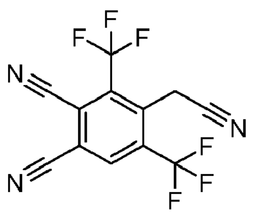 | 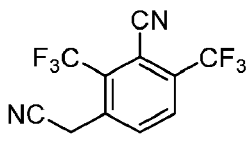 | 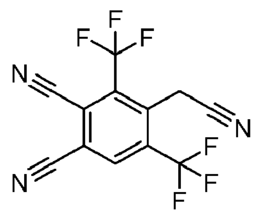 |

(continued)

| | A[1] | A[2] | A3 |
|---|---|---|---|
| A124 | 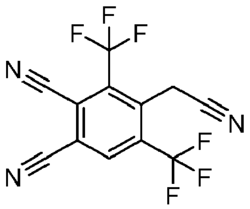 | 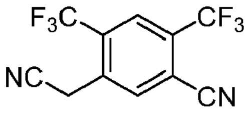 | 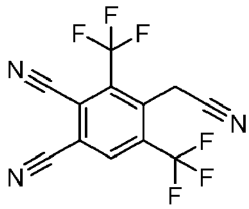 |
| A125 | 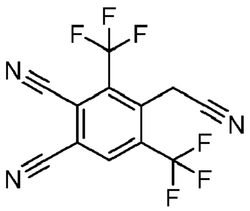 | 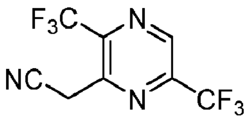 | 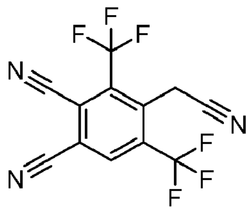 |
| A126 | 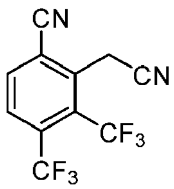 | 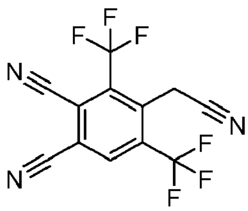 | 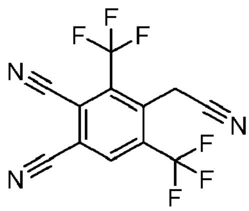 |
| A127 | 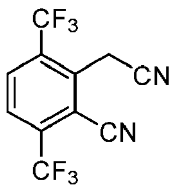 | 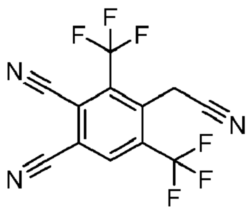 | 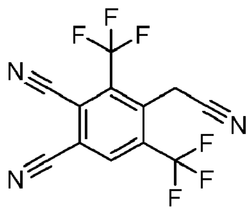 |
| A128 | 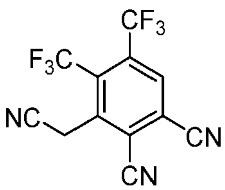 | 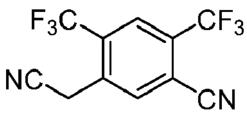 | 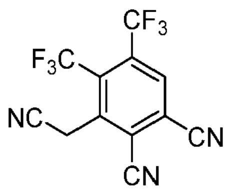 |
| A129 | 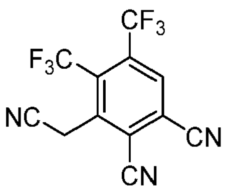 | 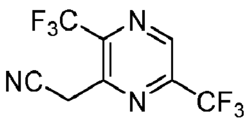 | 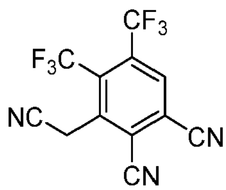 |
| A130 | 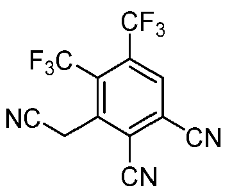 | 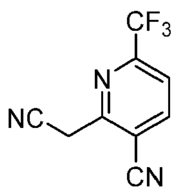 | 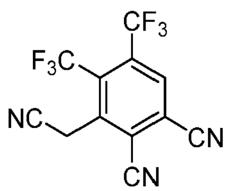 |

(continued)

| | $A^1$ | $A^2$ | A3 |
|---|---|---|---|
| A131 | 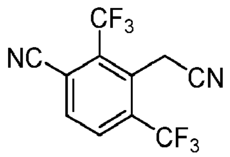 | 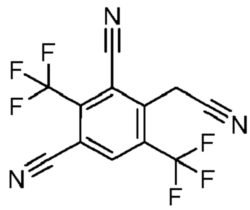 | 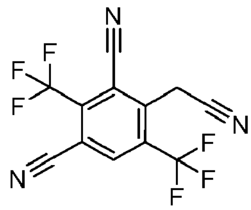 |
| A132 | 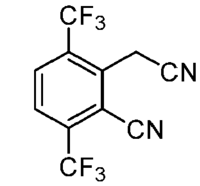 | 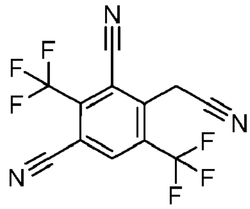 | 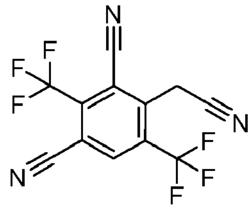 |
| A133 | 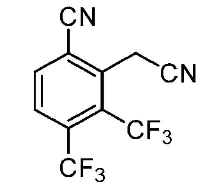 | 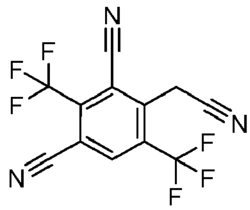 | 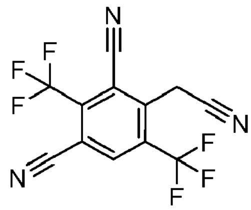 |
| A134 | 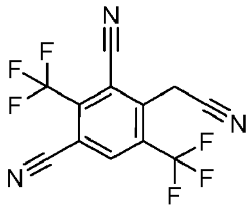 | 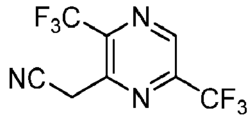 | 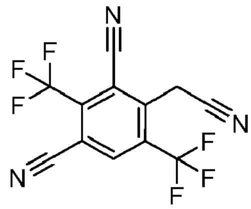 |
| A135 | 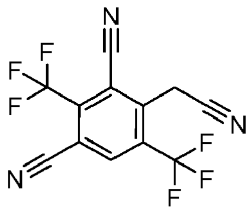 | 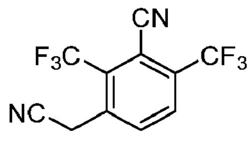 | 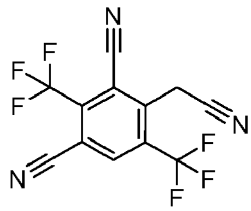 |
| A136 | 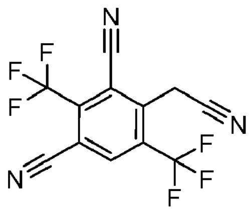 | 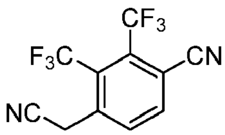 | 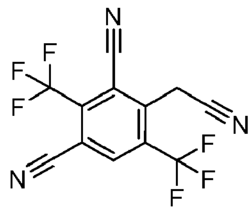 |
| A137 | 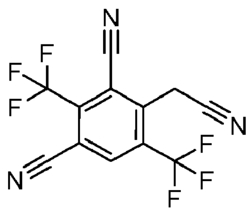 | 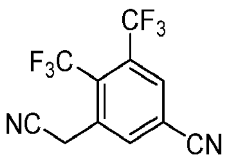 | 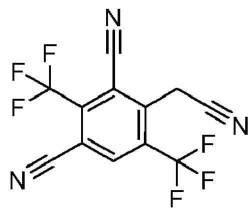 |

(continued)

| | A1 | A2 | A3 |
|---|---|---|---|
| A138 | 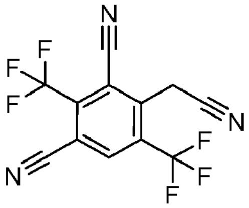 | 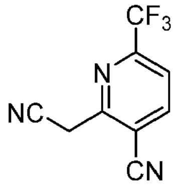 | 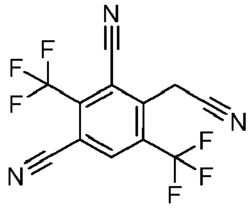 |
| A139 | 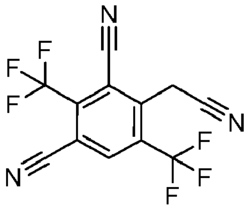 | 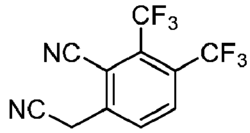 | 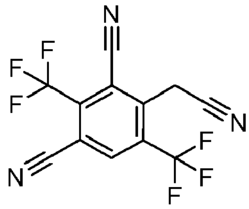 |
| A140 | 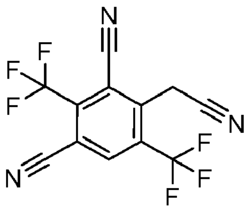 | 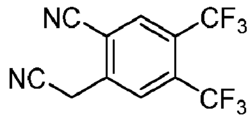 | 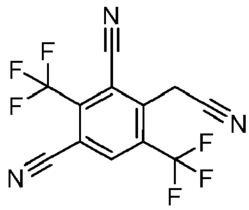 |
| A141 | 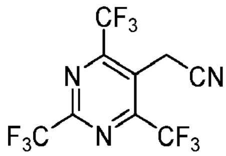 | 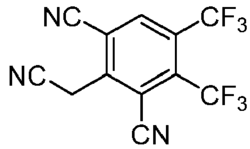 | 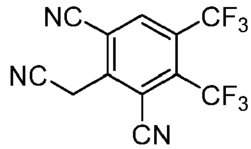 |
| A142 | 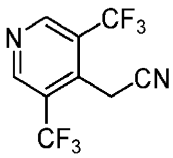 | 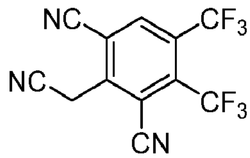 | 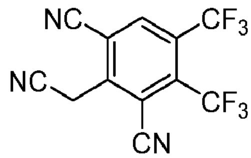 |
| A143 | 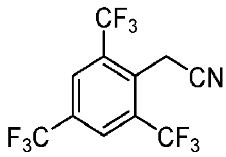 | 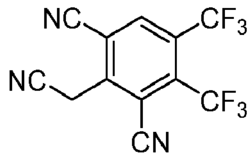 | 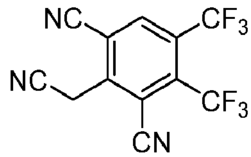 |
| A144 | 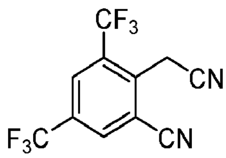 | 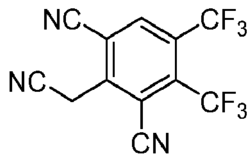 | 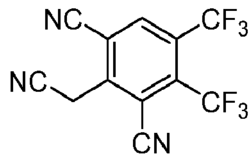 |
| A145 | 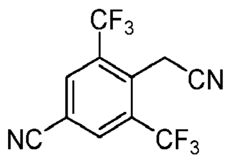 | 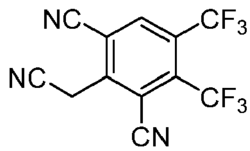 | 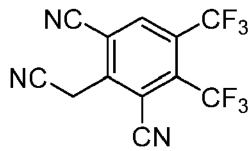 |

(continued)

| | $A^1$ | $A^2$ | A3 |
|---|---|---|---|
| A146 | 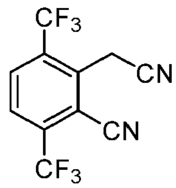 | 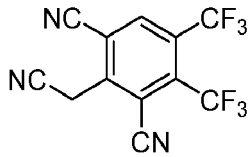 | 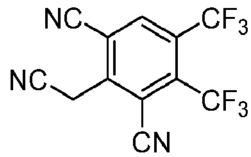 |
| A147 | 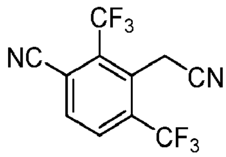 | 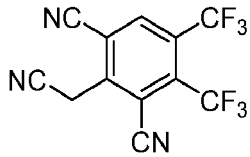 | 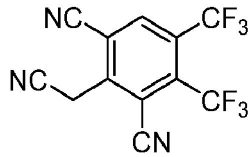 |
| A148 | 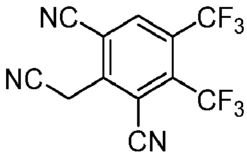 | 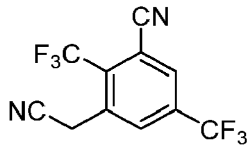 | 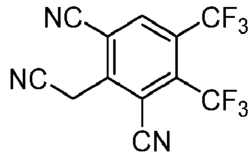 |
| A149 | 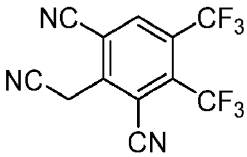 | 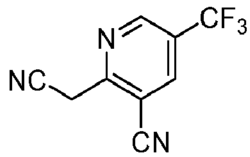 | 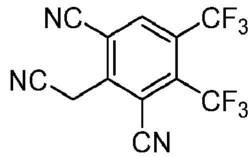 |
| A150 | 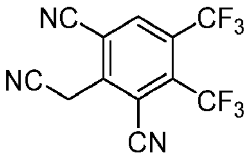 | 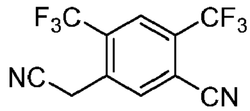 | 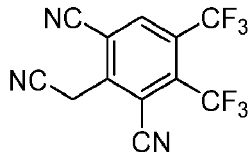 |
| A151 | 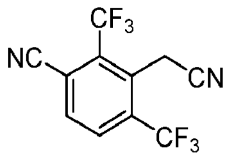 | 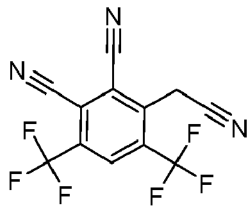 | 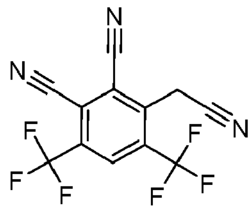 |
| A152 | 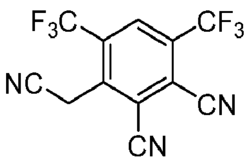 | 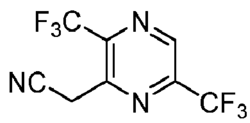 | 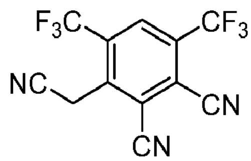 |
| A153 | 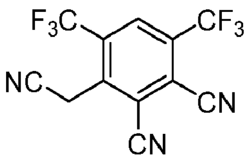 | 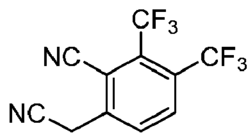 | 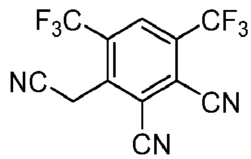 |
| A154 | 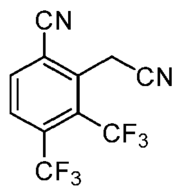 | 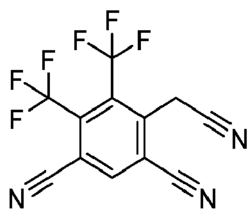 | 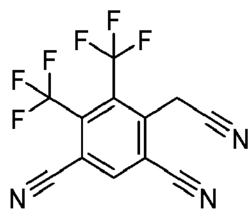 |

(continued)

| | A1 | A2 | A3 |
|---|---|---|---|
| A155 | 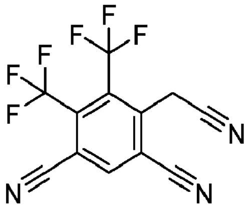 | 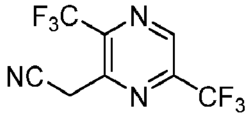 | 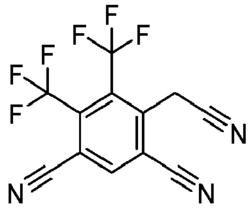 |
| A156 | 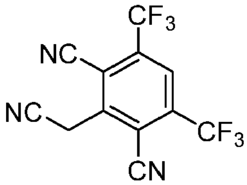 | 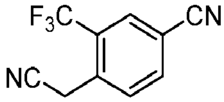 | 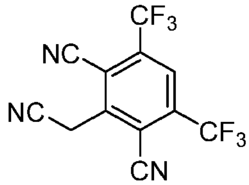 |
| A157 | 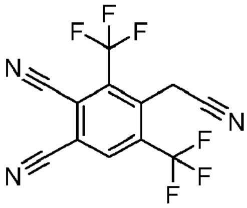 | 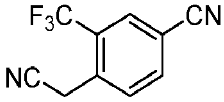 | 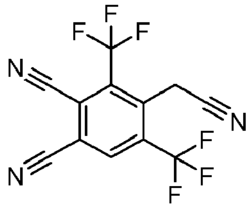 |
| A158 | 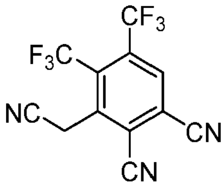 | 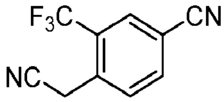 | 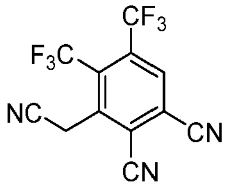 |
| A159 | 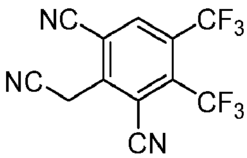 | 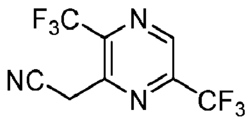 | 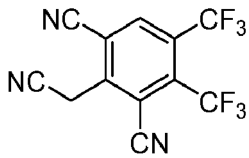 |
| A160 | 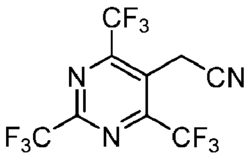 | 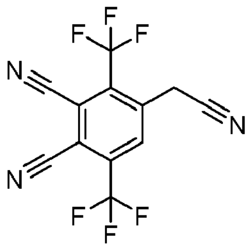 | 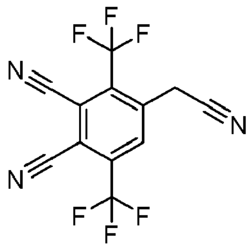 |
| A161 | 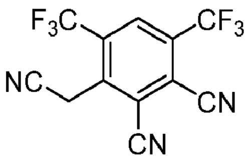 | 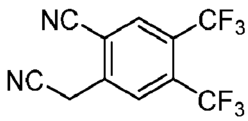 | 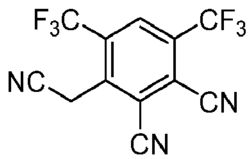 |

(continued)

| | $A^1$ | $A^2$ | A3 |
|---|---|---|---|
| A162 | 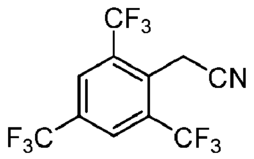 | 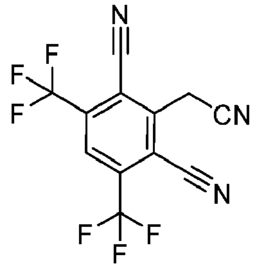 | 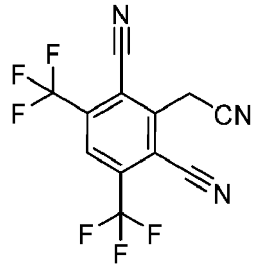 |
| A163 | 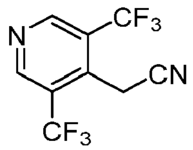 | 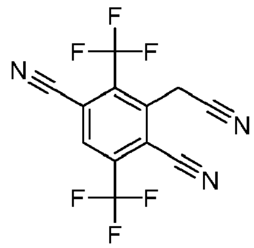 | 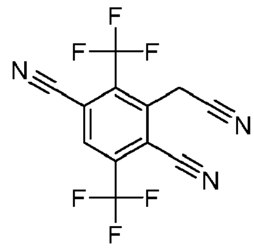 |
| A164 | 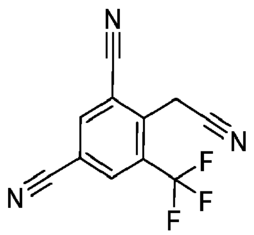 | 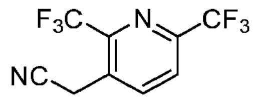 | 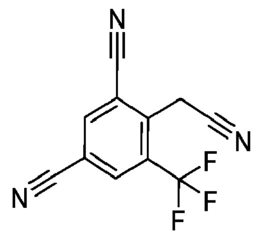 |
| A165 | 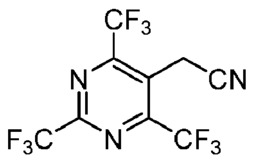 | 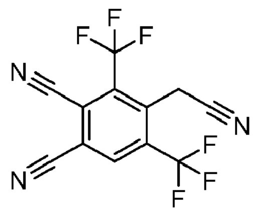 | 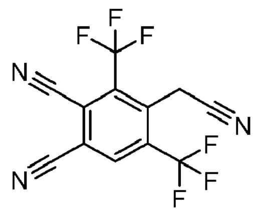 |
| A166 | 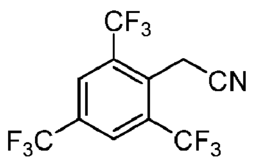 | 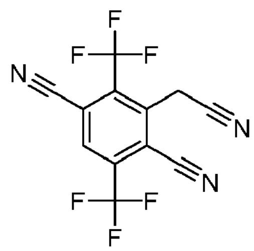 | 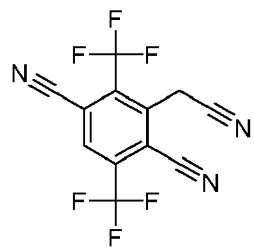 |
| A167 | 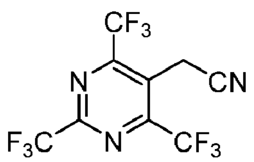 | 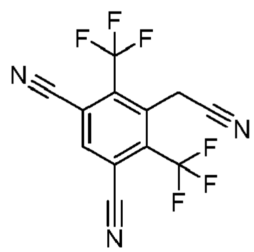 | 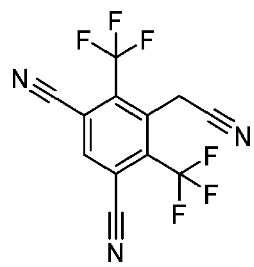 |

(continued)

| | $A^1$ | $A^2$ | A3 |
|---|---|---|---|
| A168 | 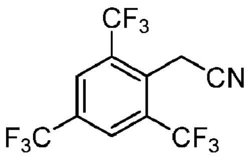 | 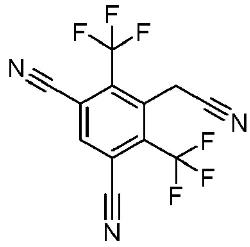 | 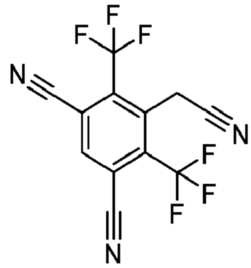 |
| A169 | 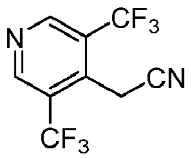 | 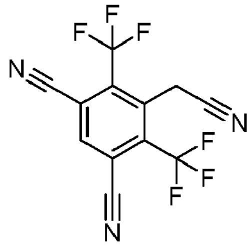 | 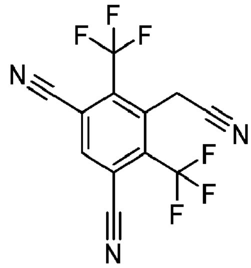 |
| A170 | 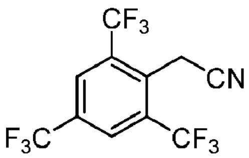 | 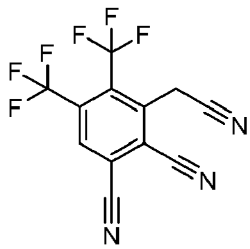 | 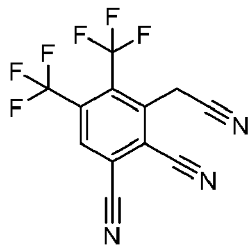 |
| A171 | 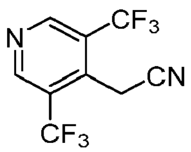 | 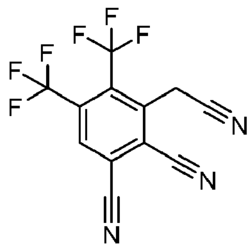 | 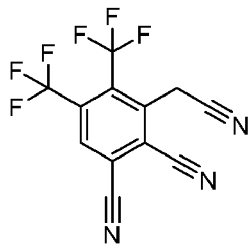 |
| A172 | 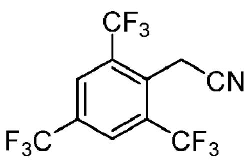 | 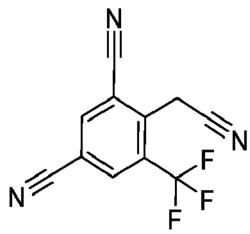 | 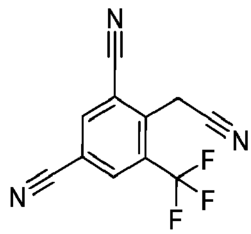 |
| A173 | 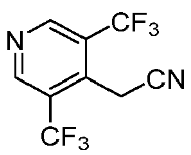 | 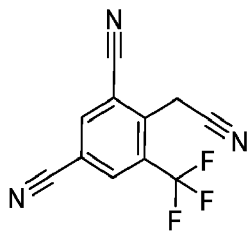 | 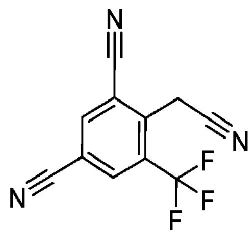 |

(continued)

| | $A^1$ | $A^2$ | A3 |
|---|---|---|---|
| A174 | 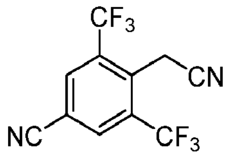 | 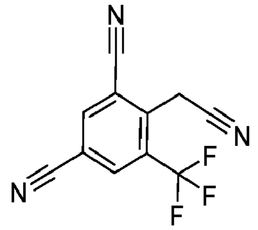 | 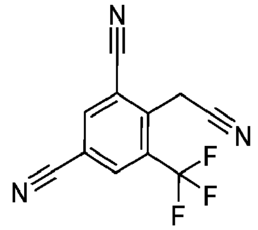 |
| A175 | 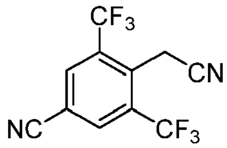 | 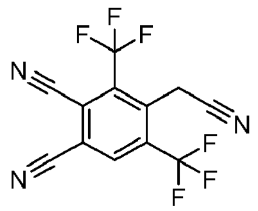 | 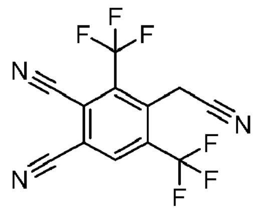 |
| A176 | 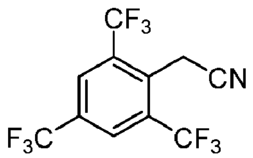 | 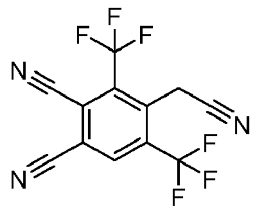 | 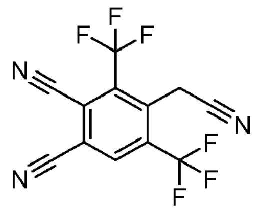 |
| A177 | 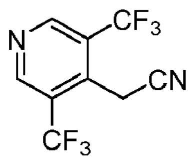 | 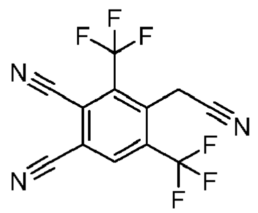 | 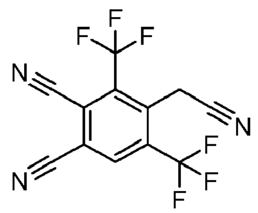 |
| A178 | 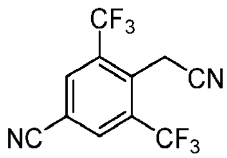 | 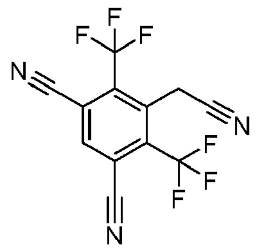 | 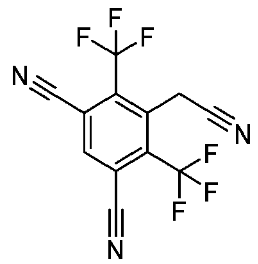 |
| A179 | 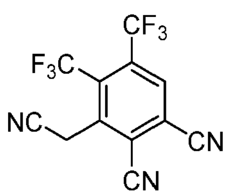 | 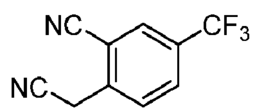 | 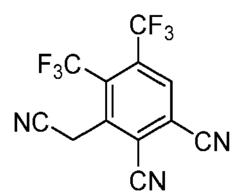 |
| A180 | 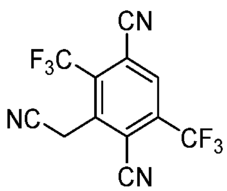 | 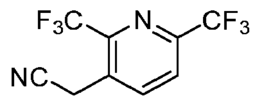 | 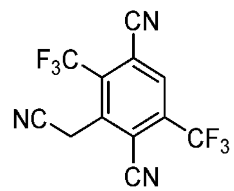 |

(continued)

| | A$^1$ | A$^2$ | A3 |
|---|---|---|---|
| A181 | 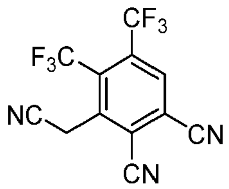 | 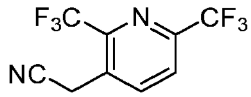 | 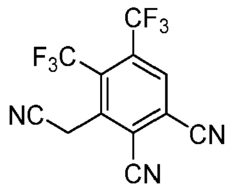 |
| A182 | 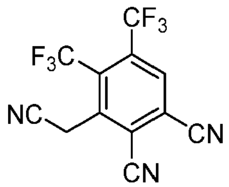 | 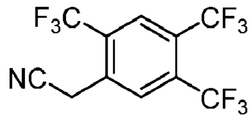 | 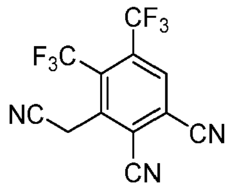 |
| A183 | 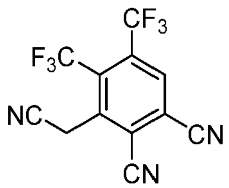 | 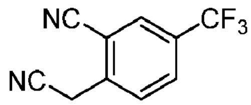 | 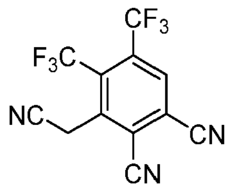 |
| A184 | 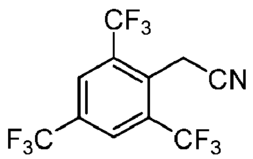 | 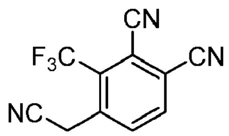 | 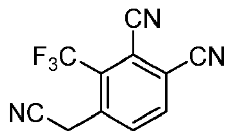 |
| A185 | 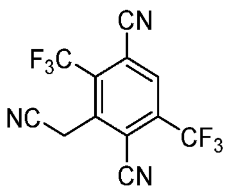 | 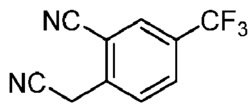 | 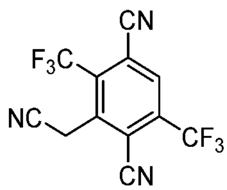 |
| A186 | 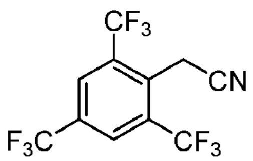 | 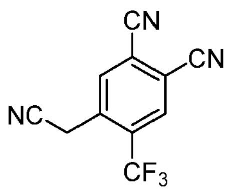 | 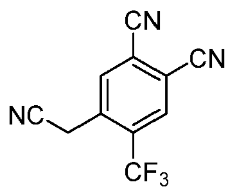 |
| A187 | 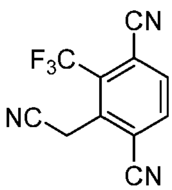 | 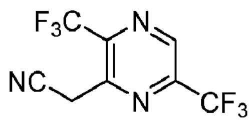 | 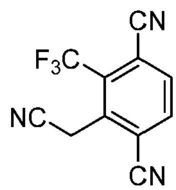 |
| A188 | 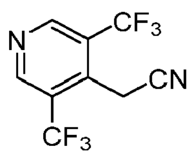 | 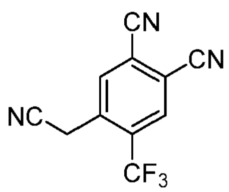 | 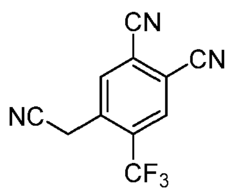 |

(continued)

| | $A^1$ | $A^2$ | A3 |
|---|---|---|---|
| A189 | 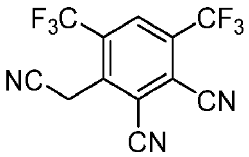 | 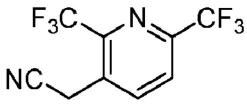 | 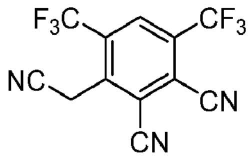 |
| A190 | 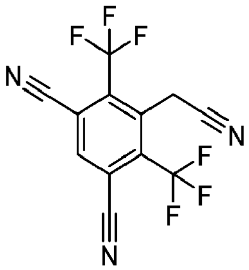 | 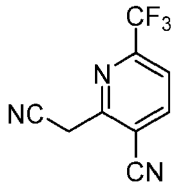 | 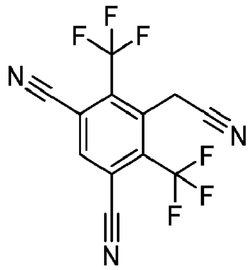 |
| A191 | 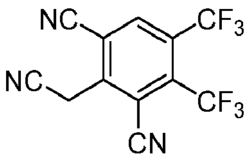 | 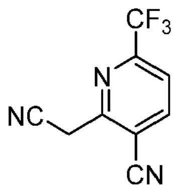 | 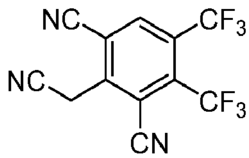 |
| A192 | 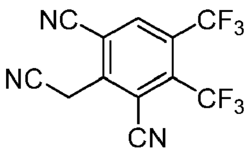 | 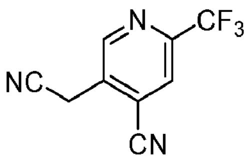 | 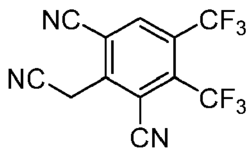 |
| A193 | 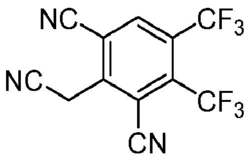 | 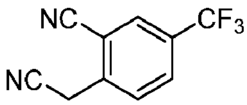 | 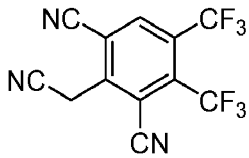 |
| A194 | 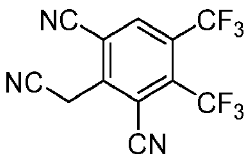 | 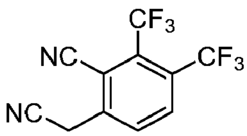 | 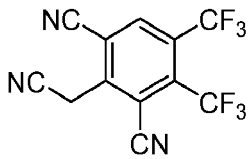 |
| A195 | 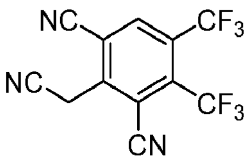 | 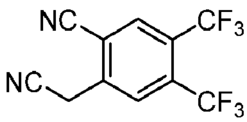 | 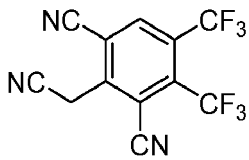 |
| A196 | 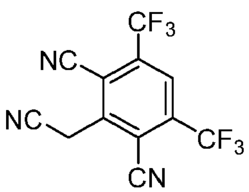 | 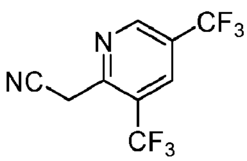 | 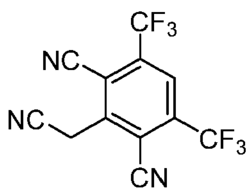 |

(continued)

| | $A^1$ | $A^2$ | A3 |
|---|---|---|---|
| A197 | 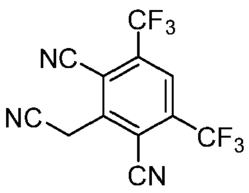 | 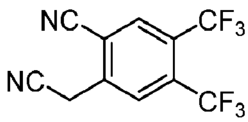 | 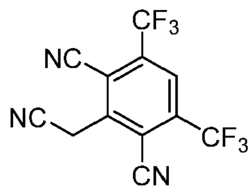 |
| A198 | 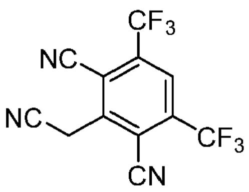 | 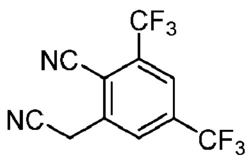 | 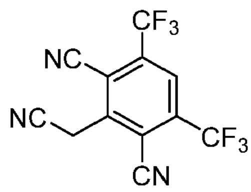 |
| A200 | 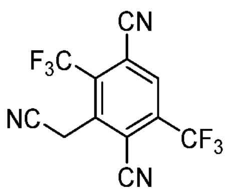 | 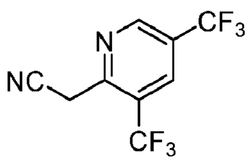 | 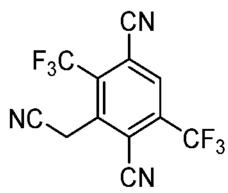 |
| A201 | 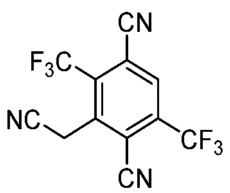 | 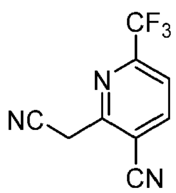 | 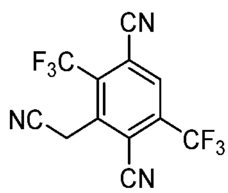 |
| A202 | 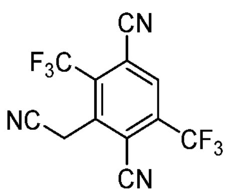 | 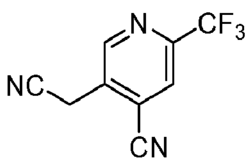 | 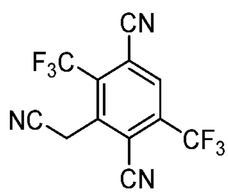 |
| A203 | 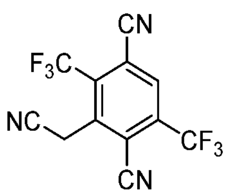 | 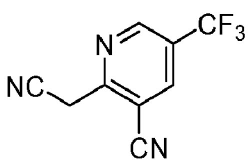 | 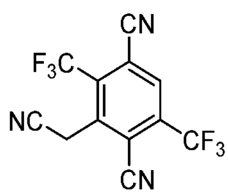 |
| A204 | 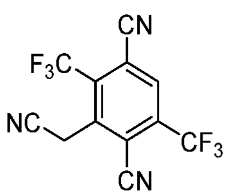 | 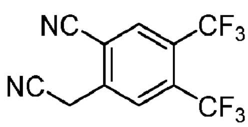 | 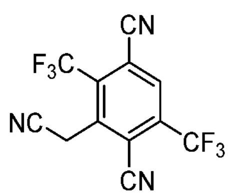 |
| A205 | 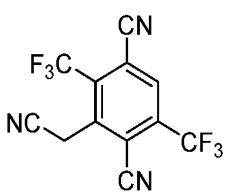 | 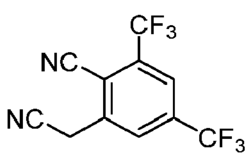 | 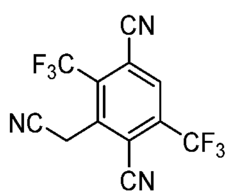 |

(continued)

| | $A^1$ | $A^2$ | A3 |
|---|---|---|---|
| A206 | 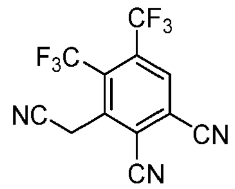 | 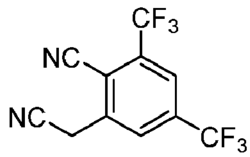 | 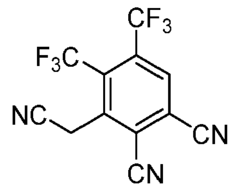 |
| A207 | 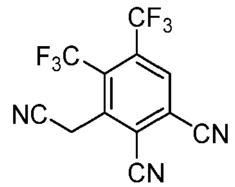 | 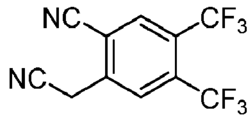 | 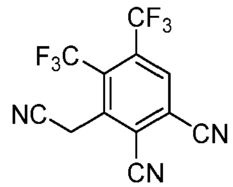 |
| A208 | 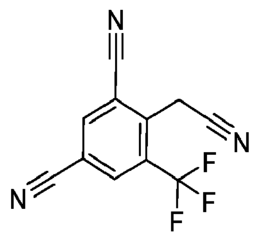 | 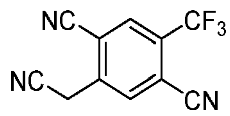 | 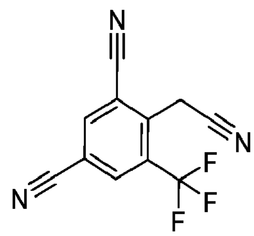 |
| A209 | 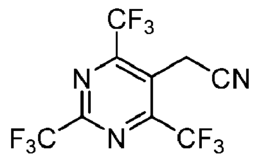 | 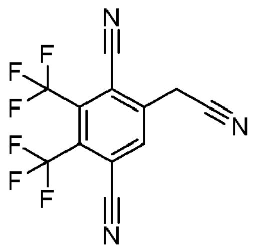 | 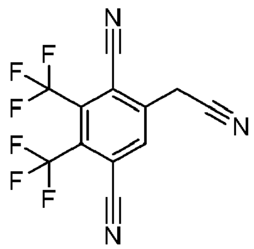 |
| A210 | 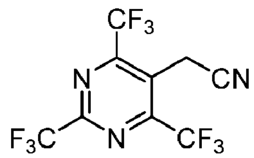 | 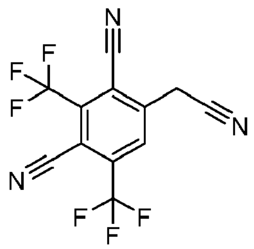 | 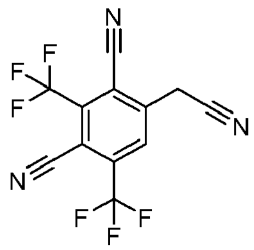 |
| A211 | 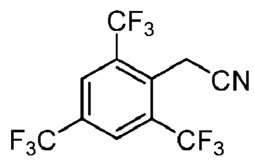 | 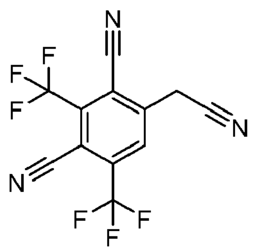 | 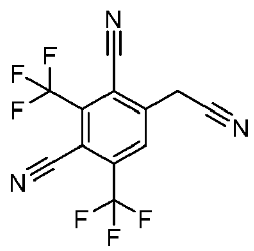 |
| A212 | 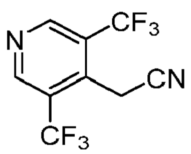 | 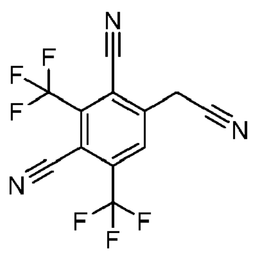 | 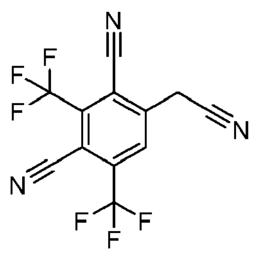 |

(continued)

| | A¹ | A² | A3 |
|---|---|---|---|
| A213 | 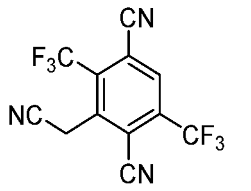 | 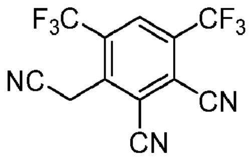 | 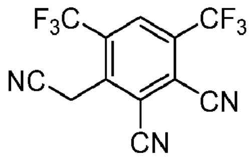 |
| A214 | 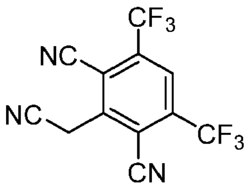 | 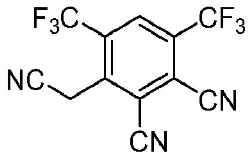 | 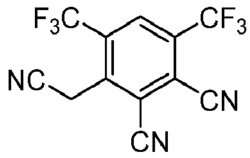 |
| A215 | 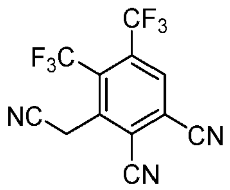 | 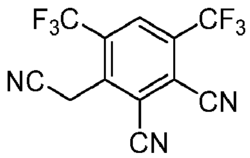 | 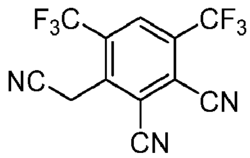 |
| A216 | 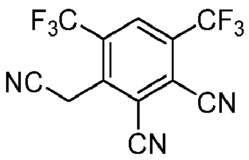 | 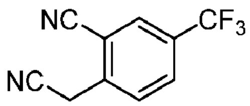 | 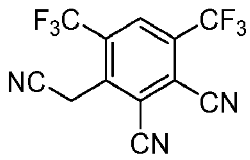 |
| A217 | 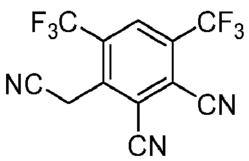 | 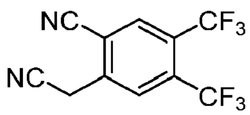 | 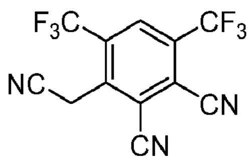 |
| A218 | 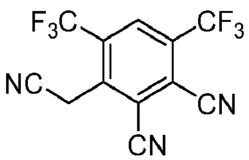 | 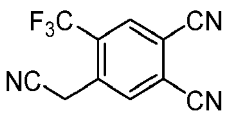 | 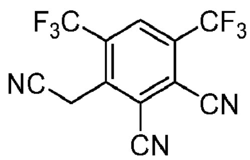 |
| A219 | 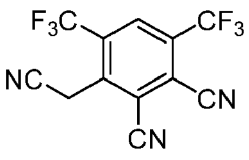 | 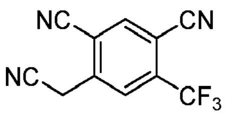 | 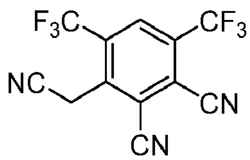 |
| A220 | 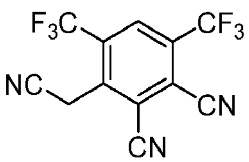 | 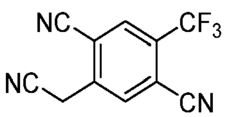 | 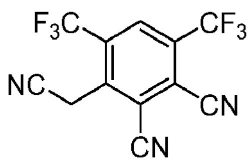 |
| A221 | 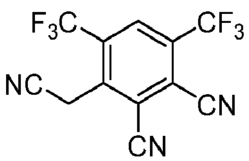 | 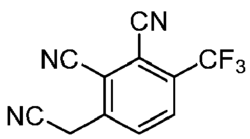 | 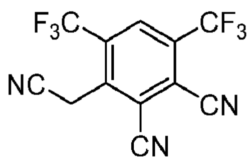 |

(continued)

| | A1 | A2 | A3 |
|---|---|---|---|
| A222 | 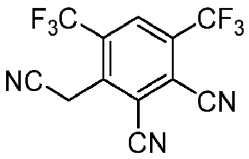 | 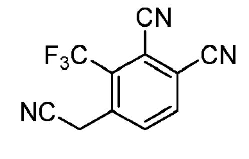 | 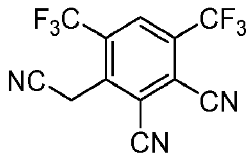 |
| A223 | 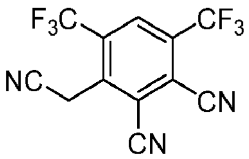 | 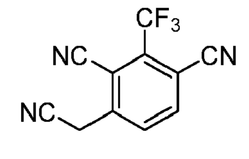 | 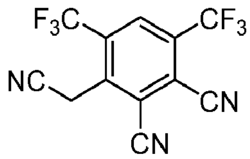 |
| A224 | 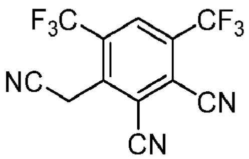 | 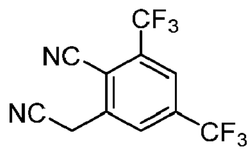 | 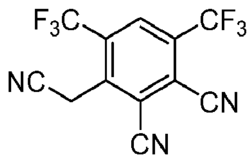 |
| A225 | 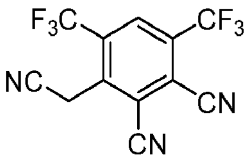 | 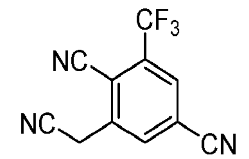 | 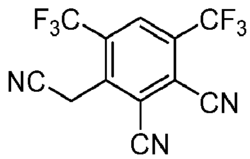 |
| A226 | 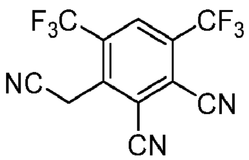 | 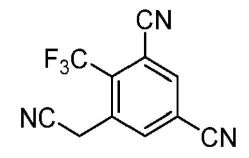 | 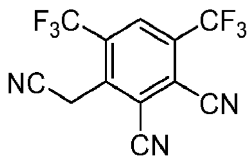 |
| A227 | 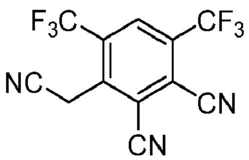 | 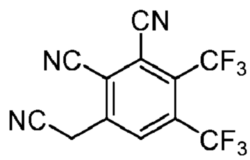 | 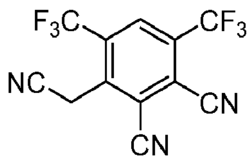 |
| A228 | 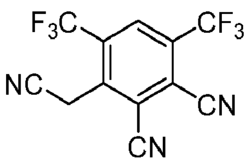 | 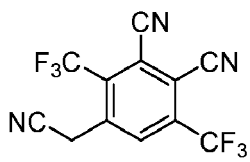 | 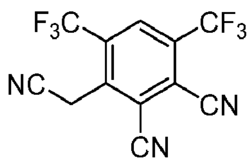 |
| A229 | 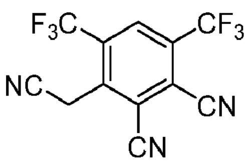 | 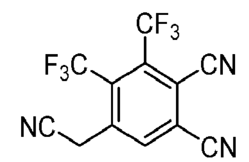 | 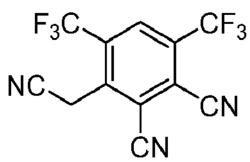 |
| A230 | 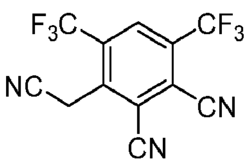 | 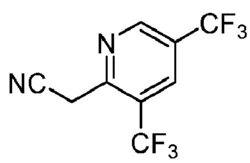 | 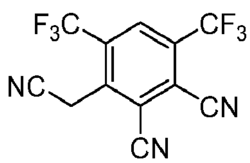 |

(continued)

| | A[1] | A[2] | A3 |
|---|---|---|---|
| A231 | 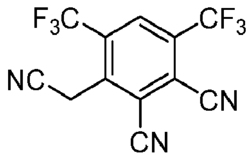 | 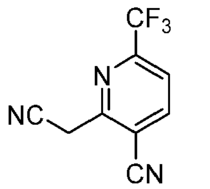 | 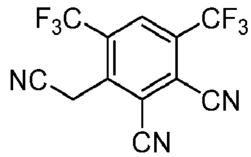 |
| A232 | 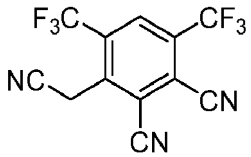 | 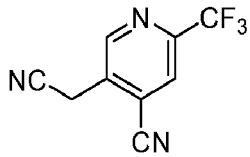 | 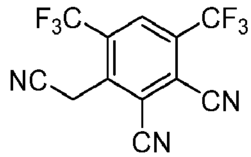 |
| A233 | 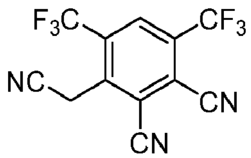 | 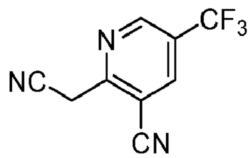 | 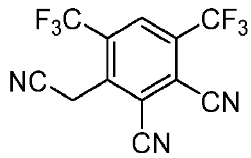 |
| A234 | 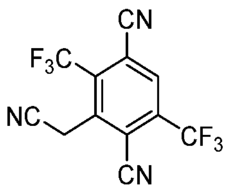 | 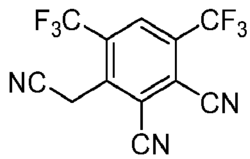 | 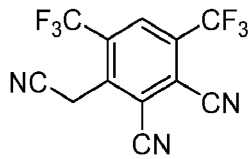 |
| A235 | 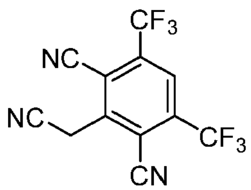 | 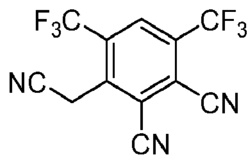 | 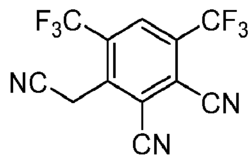 |
| A236 | 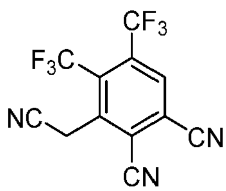 | 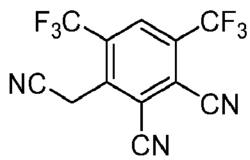 | 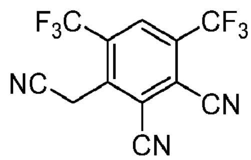 |
| A237 | 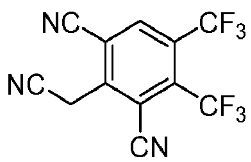 | 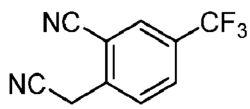 | 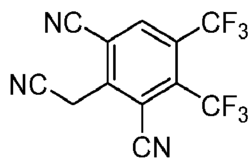 |
| A238 | 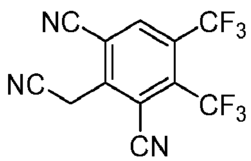 | 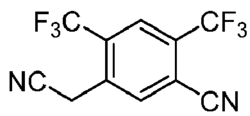 | 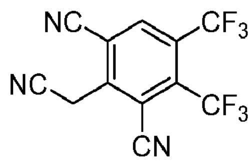 |
| A239 | 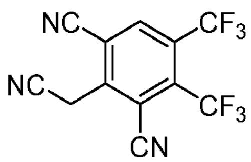 | 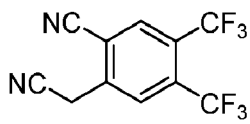 | 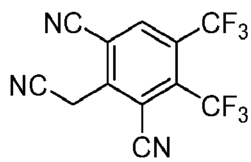 |

(continued)

| | A1 | A2 | A3 |
|---|---|---|---|
| A240 | 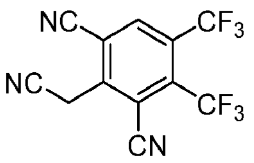 | 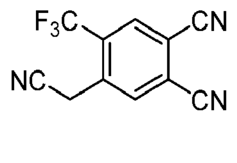 | 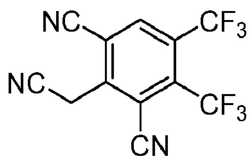 |
| A241 | 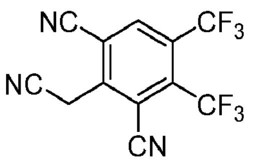 | 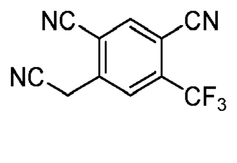 | 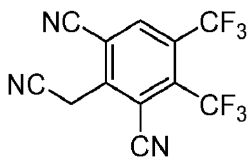 |
| A242 | 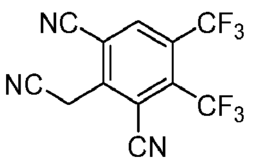 | 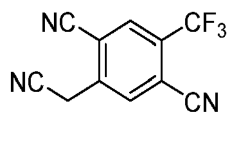 | 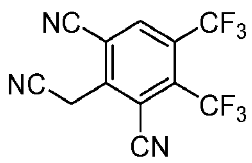 |
| A243 | 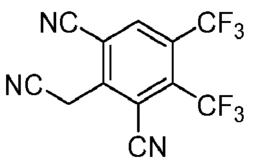 | 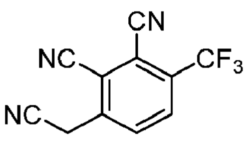 | 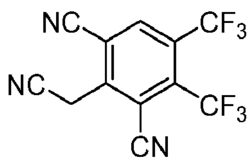 |
| A244 | 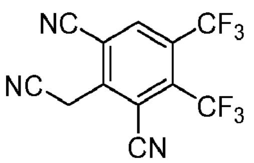 | 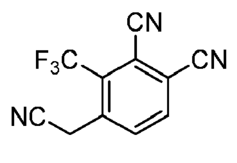 | 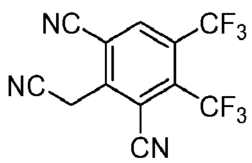 |
| A245 | 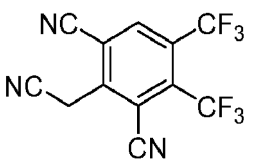 | 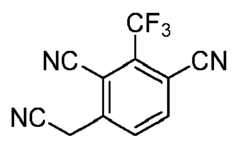 | 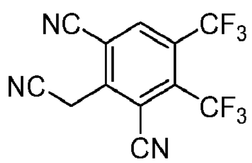 |
| A246 | 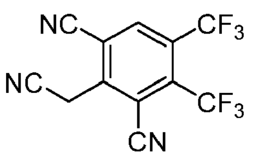 | 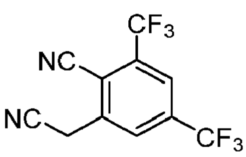 | 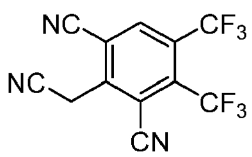 |
| A247 | 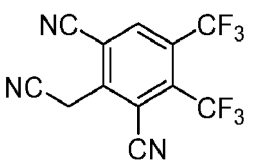 | 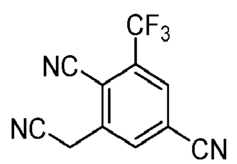 | 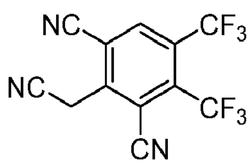 |
| A248 | 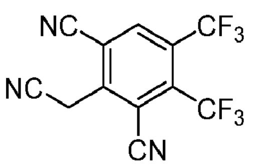 | 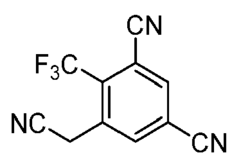 | 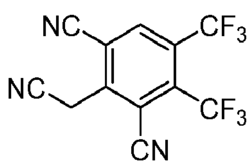 |

(continued)

| | A1 | A2 | A3 |
|---|---|---|---|
| A249 | 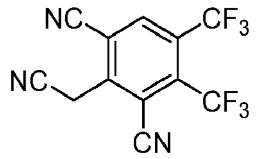 | 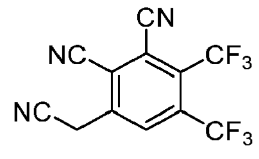 | 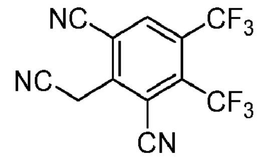 |
| A250 | 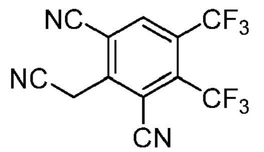 | 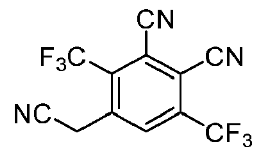 | 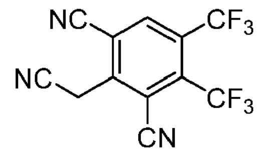 |
| A251 | 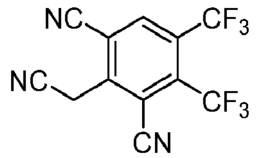 | 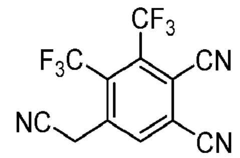 | 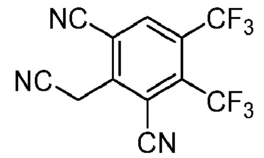 |
| A252 | 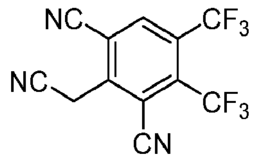 | 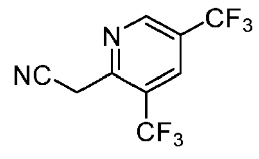 | 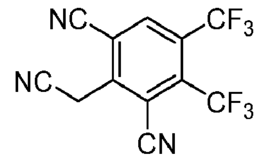 |
| A253 | 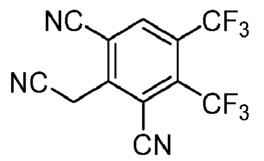 | 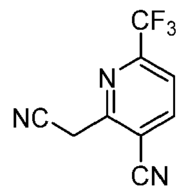 | 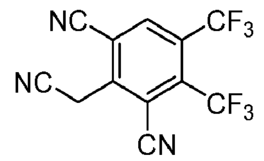 |
| A254 | 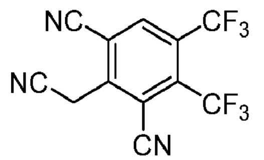 | 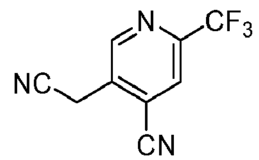 | 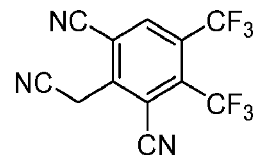 |
| A255 | 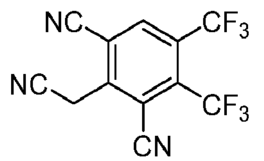 | 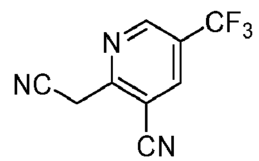 | 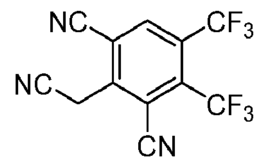 |
| A256 | 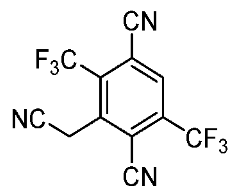 | 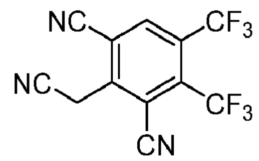 | 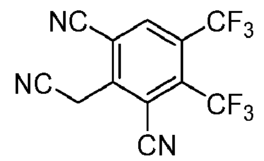 |
| A257 | 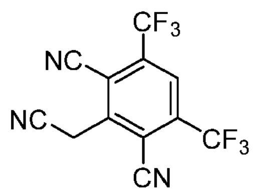 | 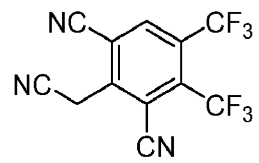 | 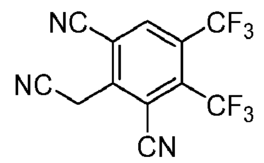 |

(continued)

| | A1 | A2 | A3 |
|---|---|---|---|
| A258 | 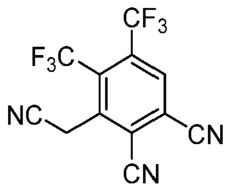 | 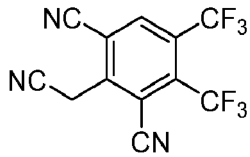 | 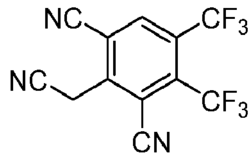 |
| A259 | 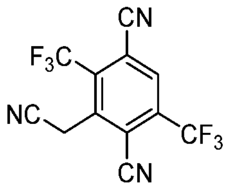 | 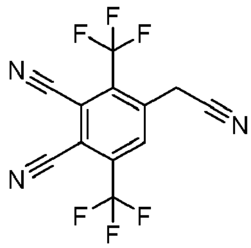 | 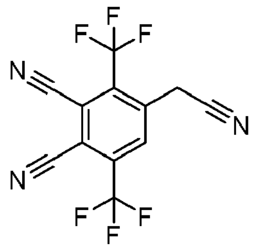 |
| A260 | 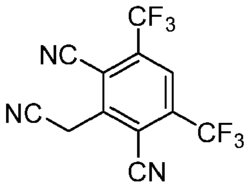 | 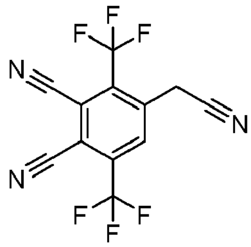 | 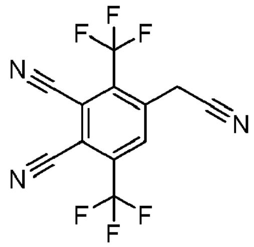 |
| A261 | 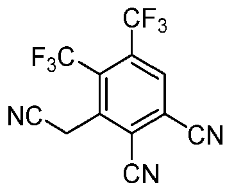 | 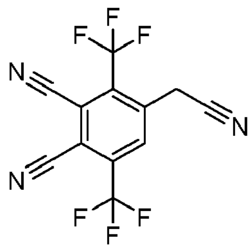 | 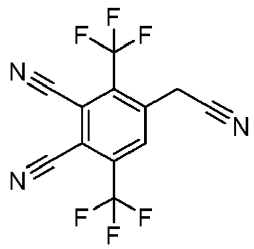 |
| A262 | 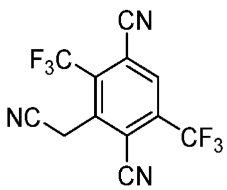 | 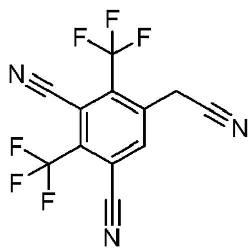 | 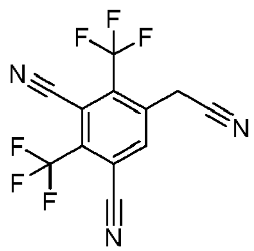 |
| A263 | 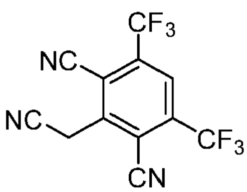 | 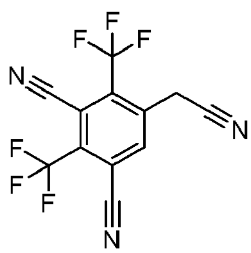 | 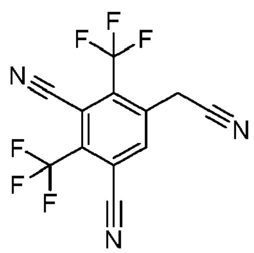 |

(continued)

| | A¹ | A² | A3 |
|---|---|---|---|
| A264 | 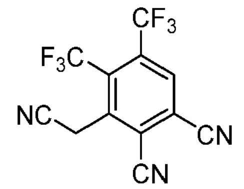 | 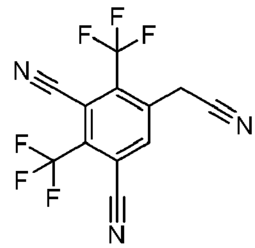 | 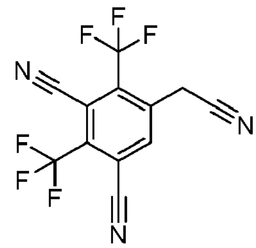 |
| A265 | 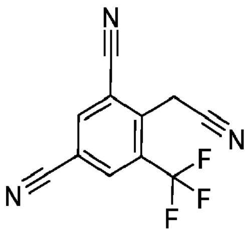 | 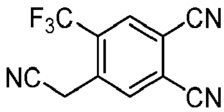 | 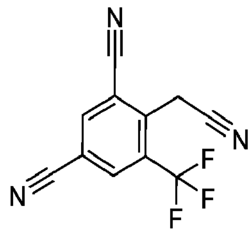 |
| A266 | 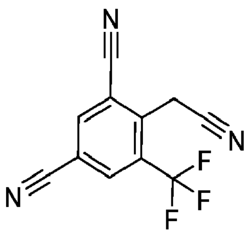 | 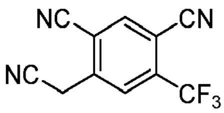 | 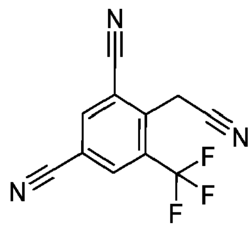 |
| A267 | 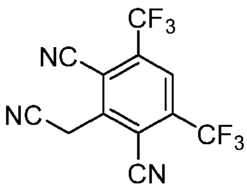 | 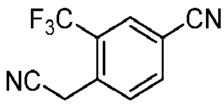 | 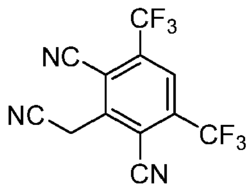 |
| A268 | 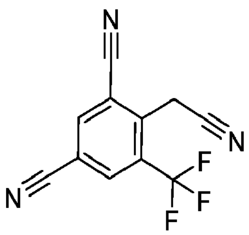 | 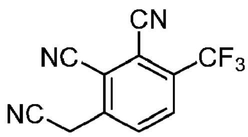 | 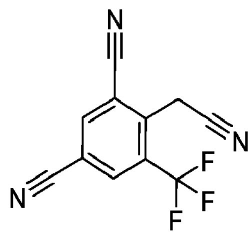 |
| A269 | 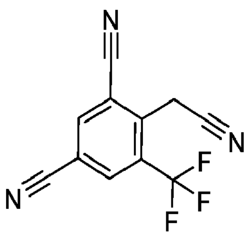 | 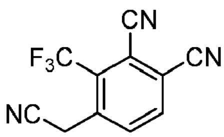 | 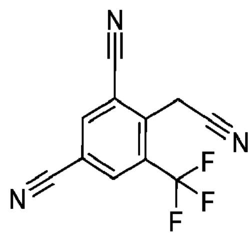 |
| A270 | 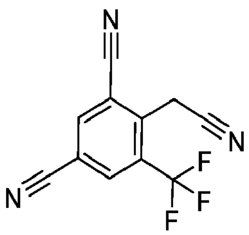 | 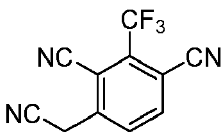 | 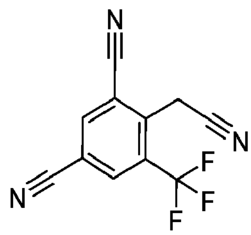 |

(continued)

| | A[1] | A[2] | A3 |
|---|---|---|---|
| A271 | 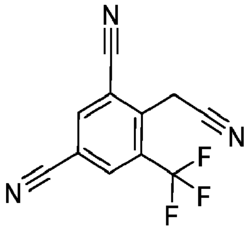 | 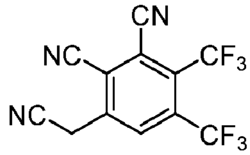 | 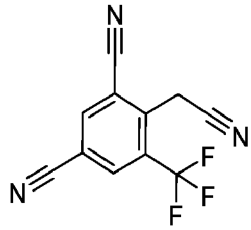 |
| A272 | 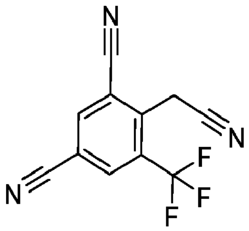 | 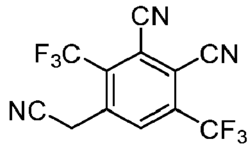 | 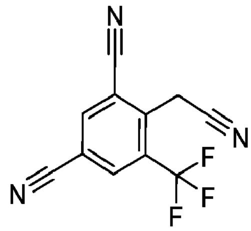 |
| A273 | 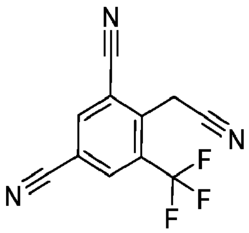 | 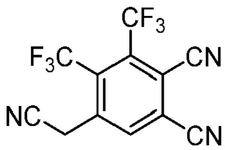 | 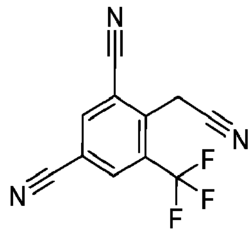 |
| A274 | 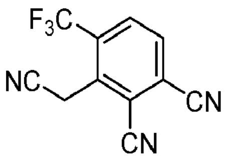 | 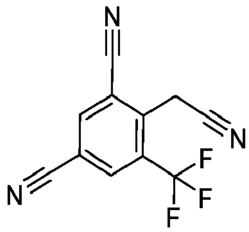 | 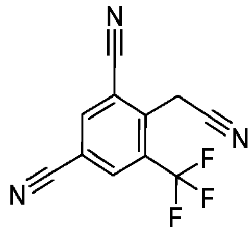 |
| A275 | 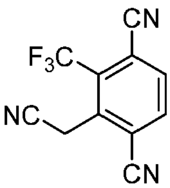 | 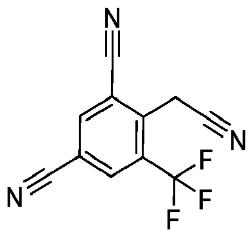 | 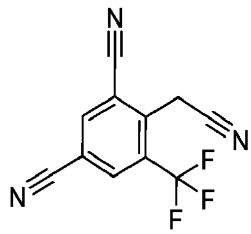 |
| A276 | 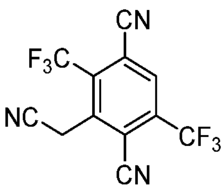 | 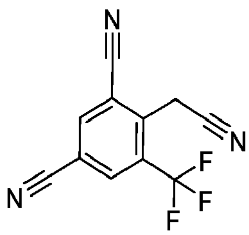 | 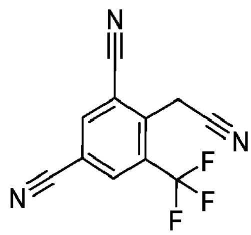 |

(continued)

| | A[1] | A[2] | A3 |
|---|---|---|---|
| A277 | 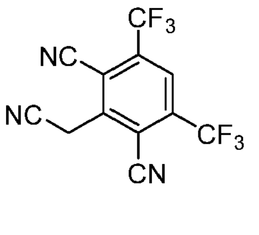 | 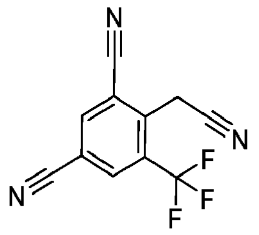 | 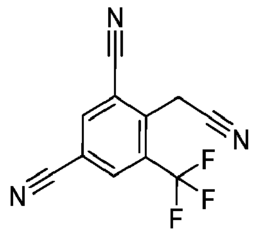 |
| A278 | 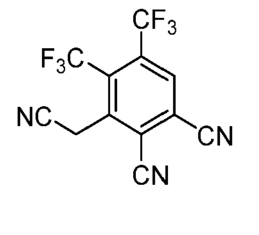 | 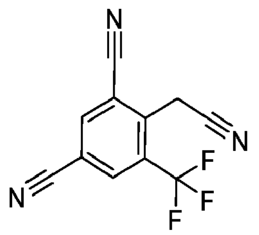 | 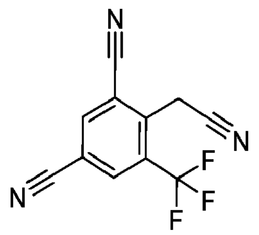 |
| A279 | 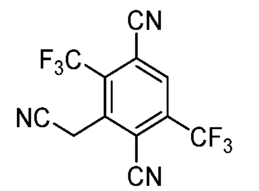 | 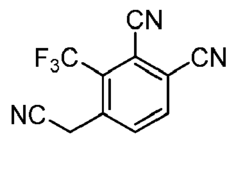 | 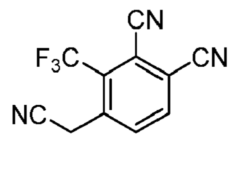 |
| A280 | 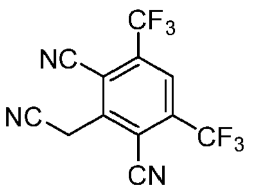 | 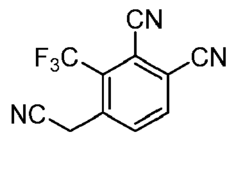 | 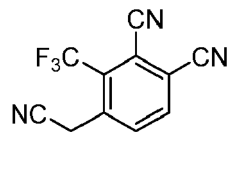 |
| A281 | 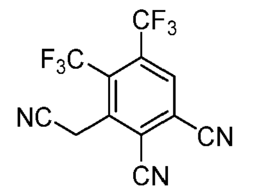 | 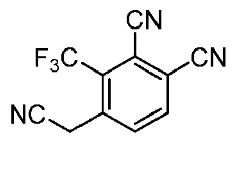 | 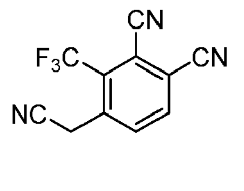 |
| A282 | 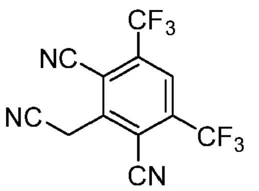 | 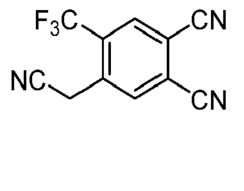 | 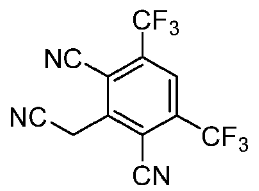 |
| A283 | 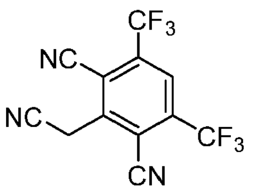 | 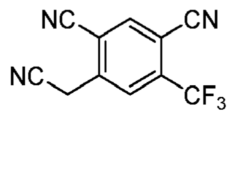 | 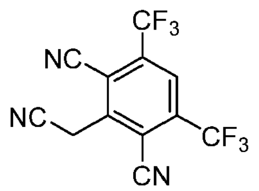 |

(continued)

| | $A^1$ | $A^2$ | A3 |
|---|---|---|---|
| A284 | 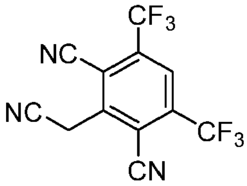 | 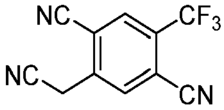 | 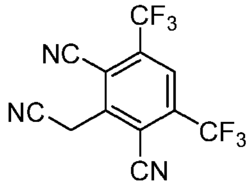 |
| A285 | 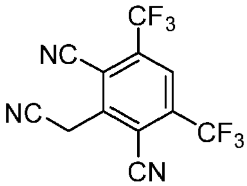 | 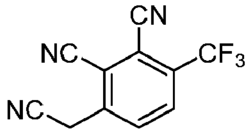 | 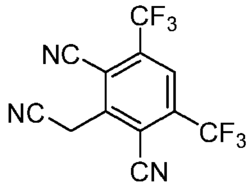 |
| A286 | 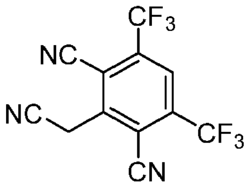 | 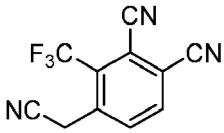 | 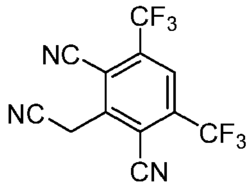 |
| A287 | 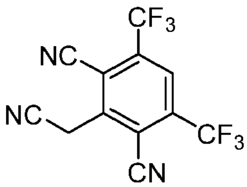 | 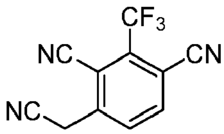 | 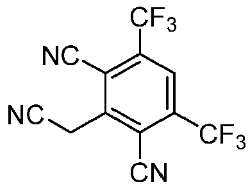 |
| A288 | 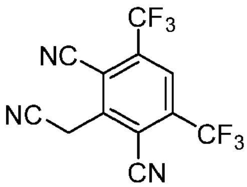 | 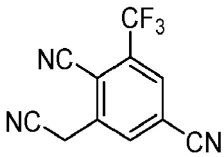 | 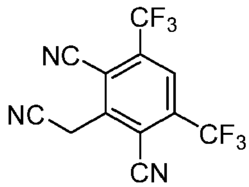 |
| A289 | 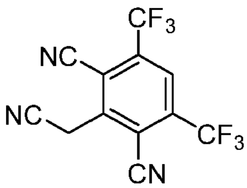 | 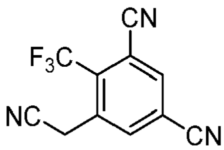 | 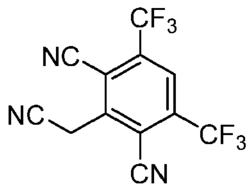 |
| A290 | 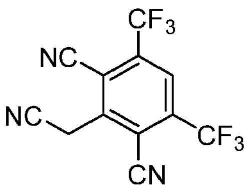 | 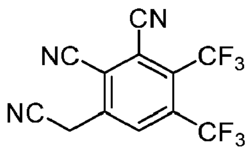 | 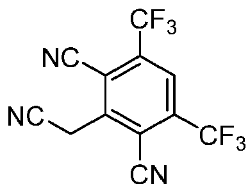 |
| A291 | 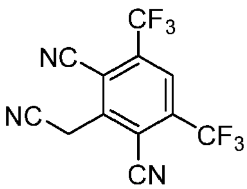 | 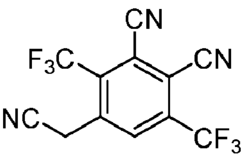 | 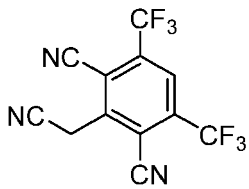 |

(continued)

| | $A^1$ | $A^2$ | A3 |
|---|---|---|---|
| A292 | 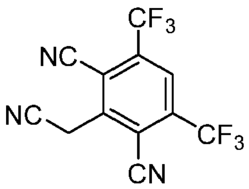 | 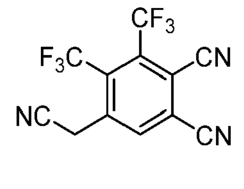 | 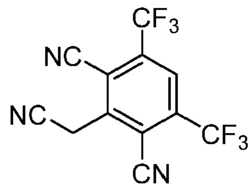 |
| A293 | 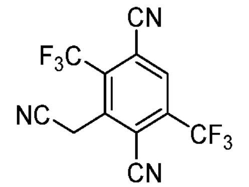 | 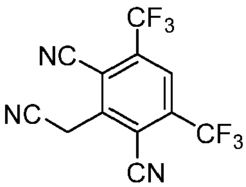 | 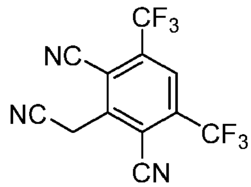 |
| A294 | 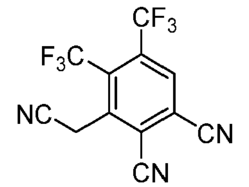 | 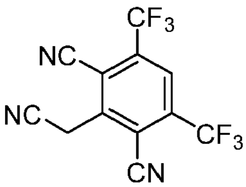 | 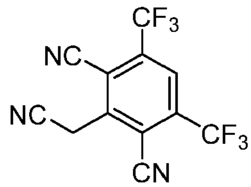 |
| A295 | 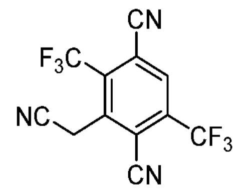 | 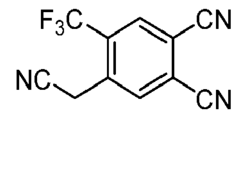 | 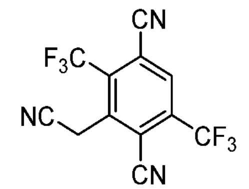 |
| A296 | 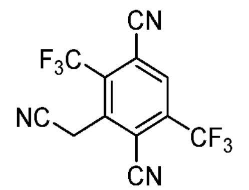 | 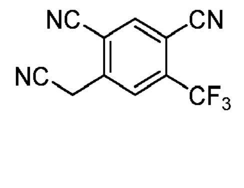 | 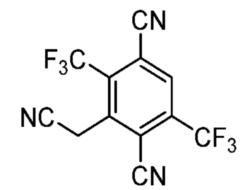 |
| A297 | 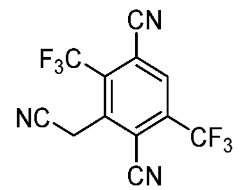 | 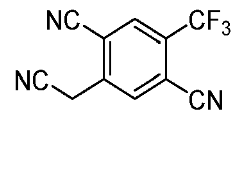 | 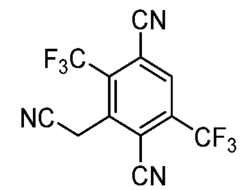 |
| A298 | 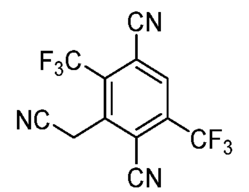 | 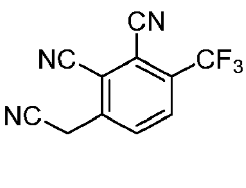 | 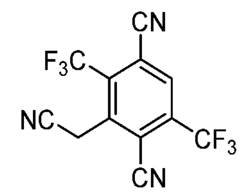 |
| A299 | 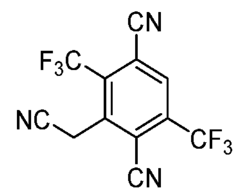 | 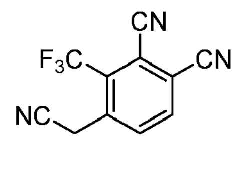 | 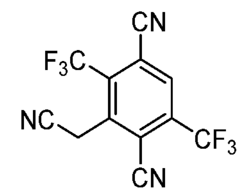 |

(continued)

| | A1 | A2 | A3 |
|---|---|---|---|
| A300 | 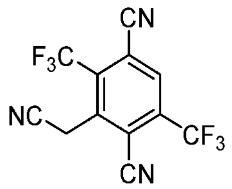 | 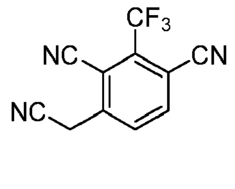 | 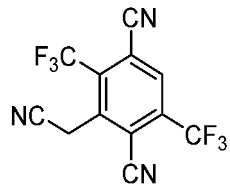 |
| A301 | 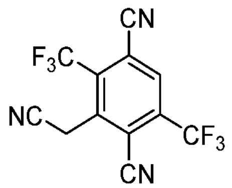 | 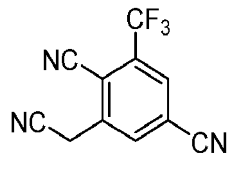 | 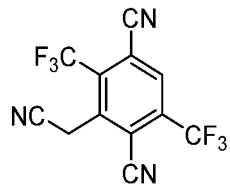 |
| A302 | 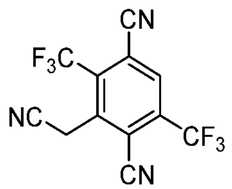 | 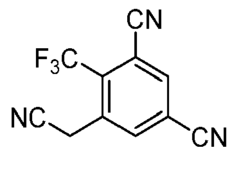 | 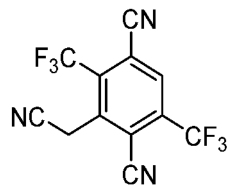 |
| A303 | 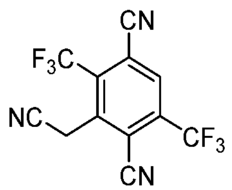 | 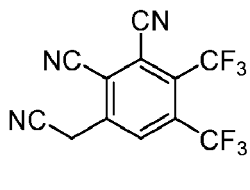 | 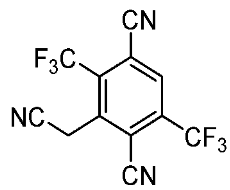 |
| A304 | 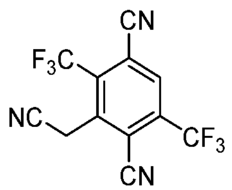 | 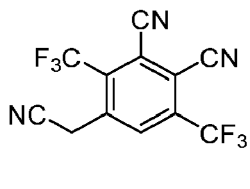 | 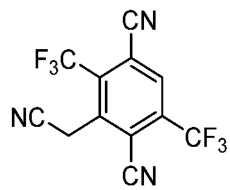 |
| A305 | 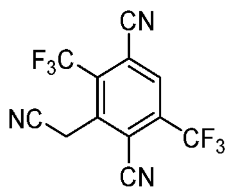 | 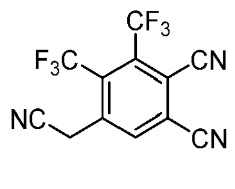 | 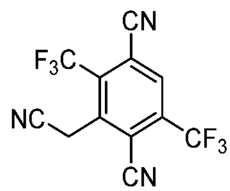 |
| A306 | 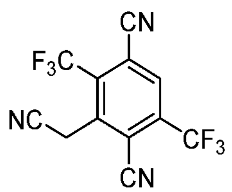 | 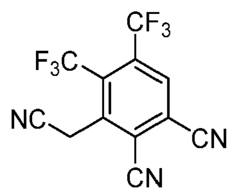 | 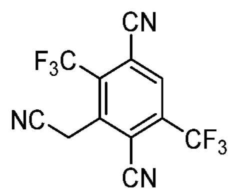 |
| A307 | 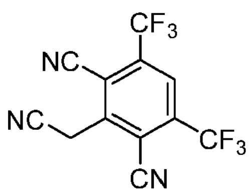 | 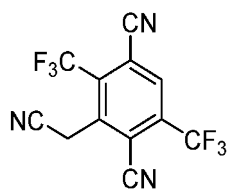 | 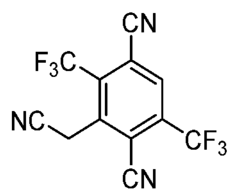 |

(continued)

| | A¹ | A² | A3 |
|---|---|---|---|
| A308 | 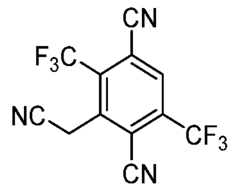 | 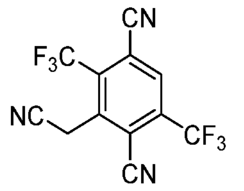 | 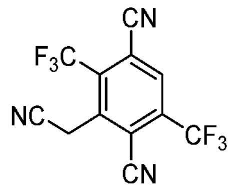 |
| A309 | 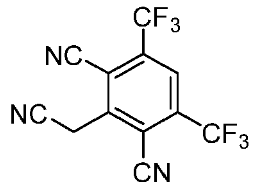 | 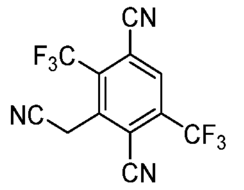 | 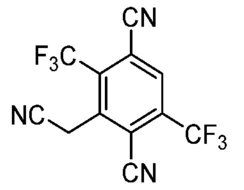 |
| A310 | 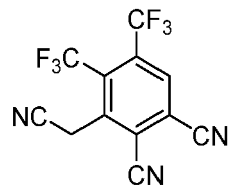 | 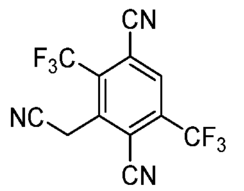 | 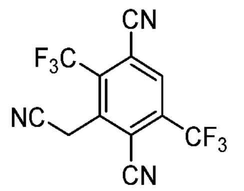 |
| A311 | 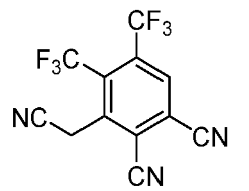 | 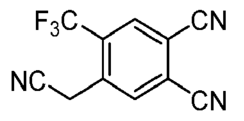 | 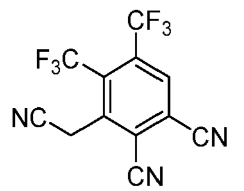 |
| A312 | 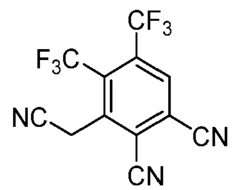 | 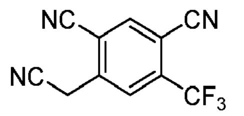 | 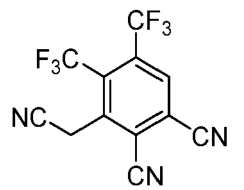 |
| A313 | 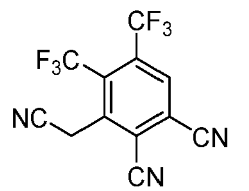 | 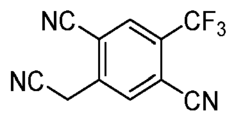 | 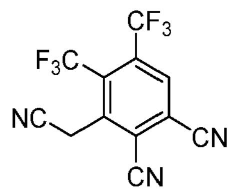 |
| A314 | 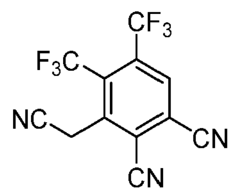 | 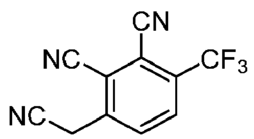 | 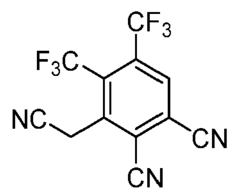 |
| A315 | 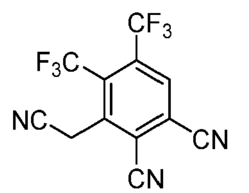 | 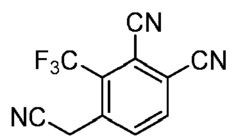 | 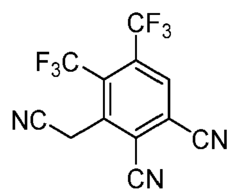 |

(continued)

| | $A^1$ | $A^2$ | A3 |
|---|---|---|---|
| A316 | 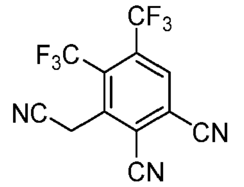 | 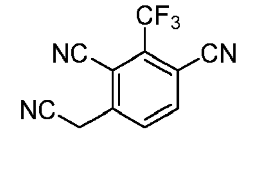 | 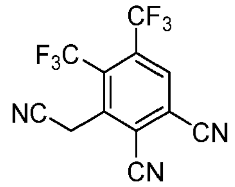 |
| A317 | 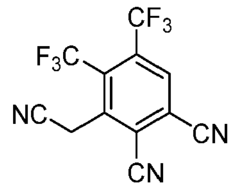 | 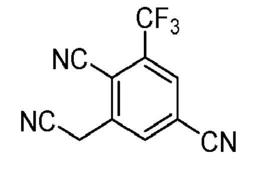 | 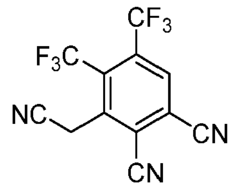 |
| A318 | 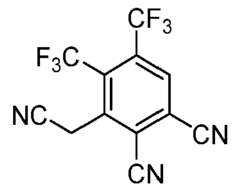 | 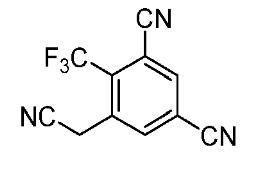 | 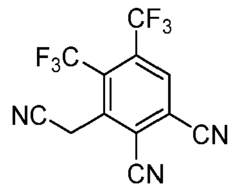 |
| A319 | 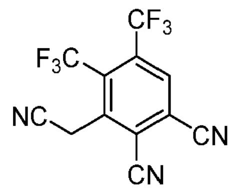 | 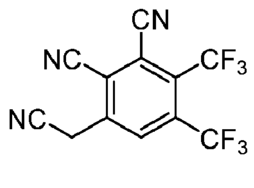 | 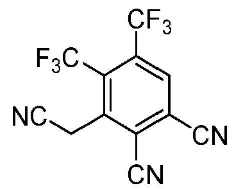 |
| A320 | 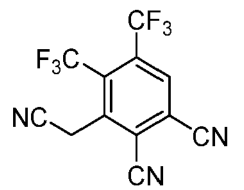 | 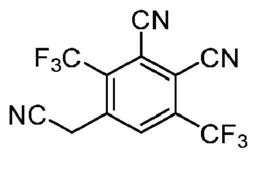 | 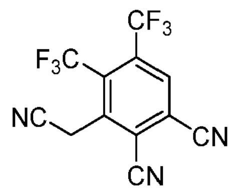 |
| A321 | 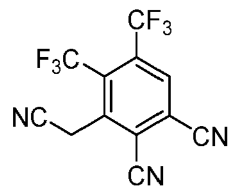 | 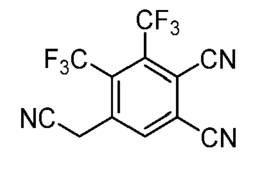 | 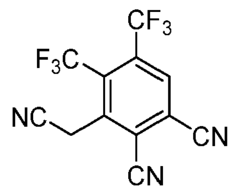 |
| A322 | 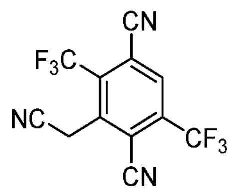 | 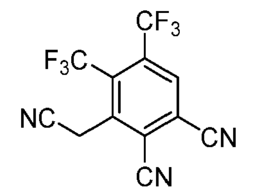 | 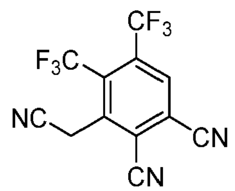 |
| A323 | 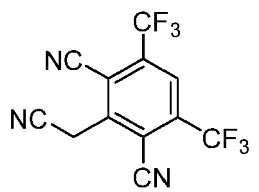 | 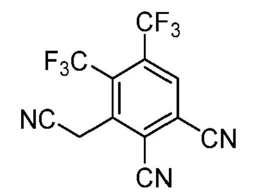 | 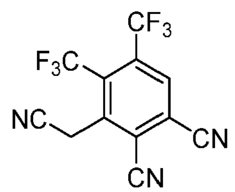 |

(continued)

| | A[1] | A[2] | A3 |
|---|---|---|---|
| A324 | 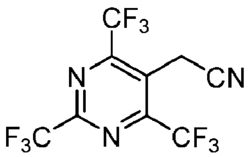 | 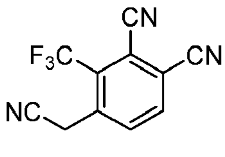 | 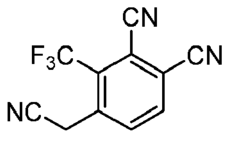 |
| A325 | 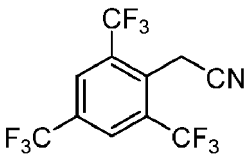 | 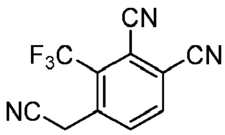 | 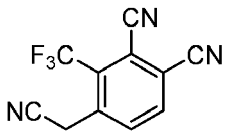 |
| A326 | 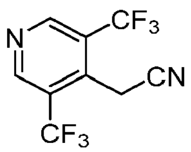 | 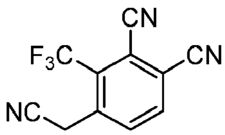 | 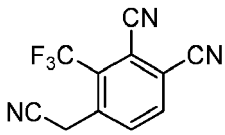 |
| A327 | 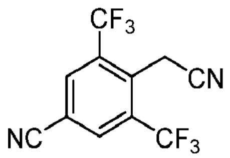 | 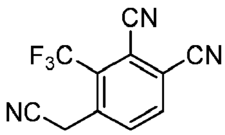 | 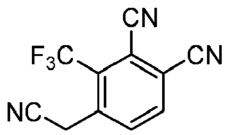 |
| A328 | 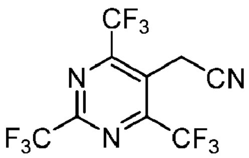 | 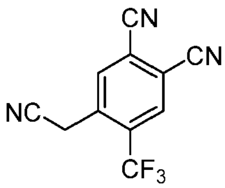 | 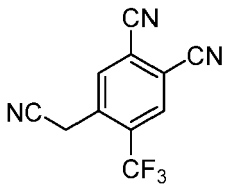 |
| A329 | 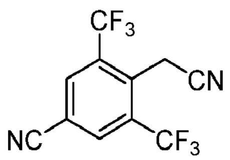 | 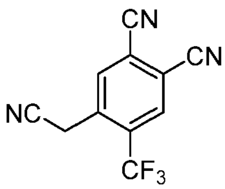 | 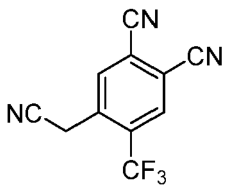 |
| A330 | 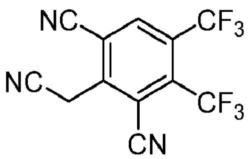 | 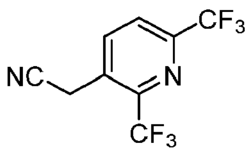 | 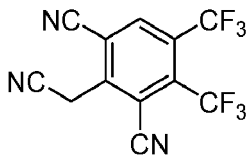 |
| A331 | 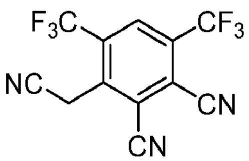 | 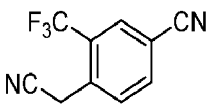 | 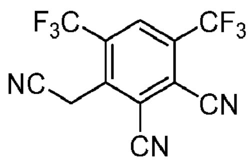 |
| A332 | 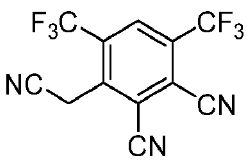 | 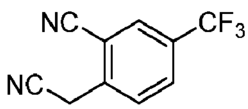 | 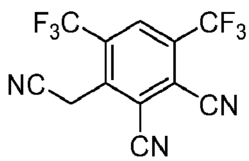 |

(continued)

| | $A^1$ | $A^2$ | A3 |
|---|---|---|---|
| A333 | 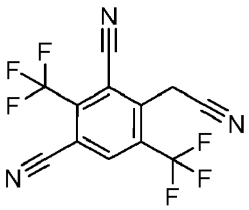 | 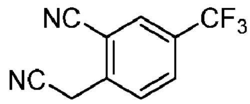 | 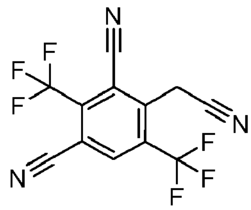 |
| A334 | 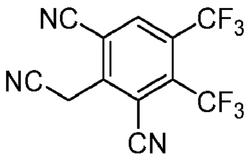 | 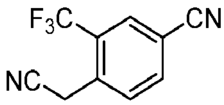 | 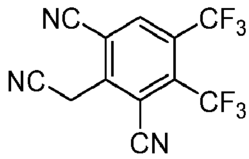 |
| A335 | 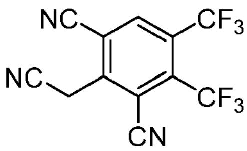 | 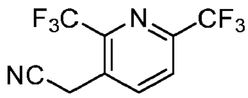 | 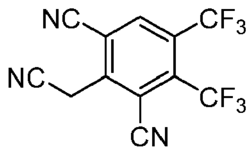 |
| A336 | 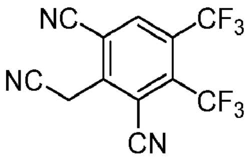 | 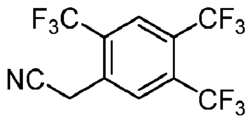 | 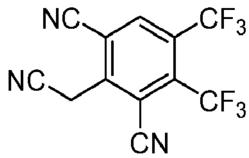 |
| A337 | 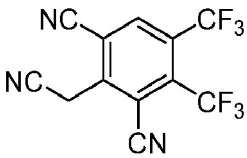 | 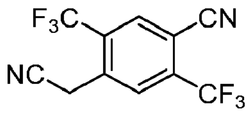 | 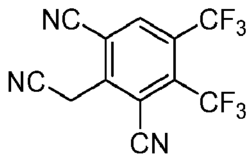 |
| A338 | 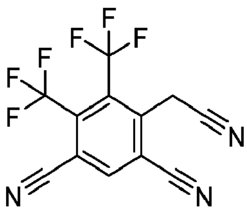 | 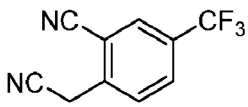 | 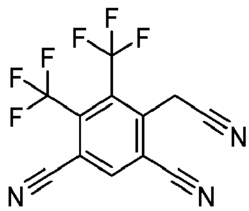 |
| A339 | 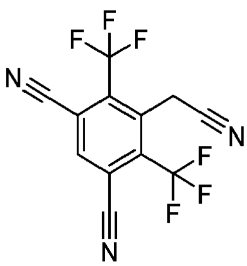 | 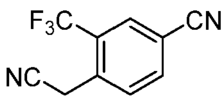 | 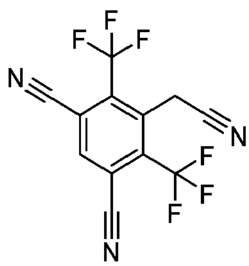 |
| A340 | 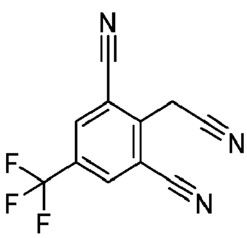 | 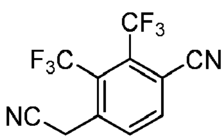 | 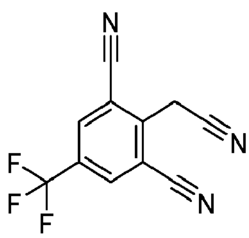 |

(continued)

| | $A^1$ | $A^2$ | A3 |
|---|---|---|---|
| A341 | 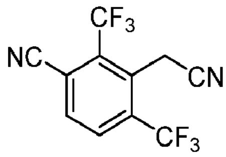 | 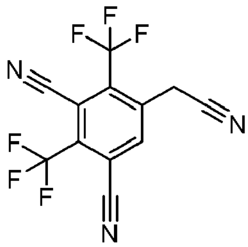 | 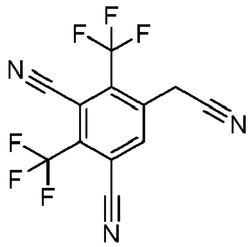 |
| A342 | 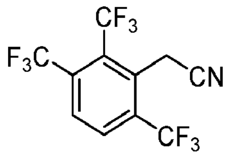 | 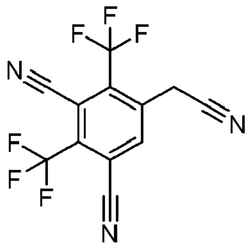 | 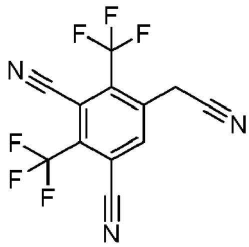 |
| A343 | 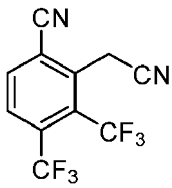 | 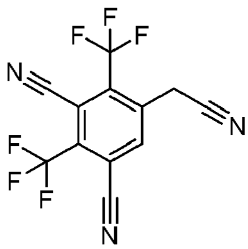 | 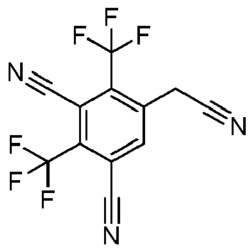 |
| A344 | 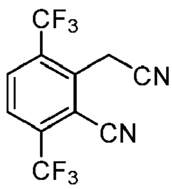 | 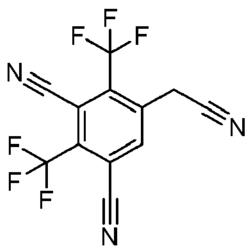 | 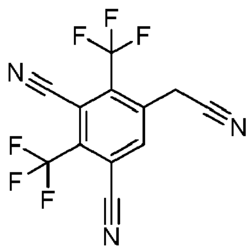 |
| A345 | 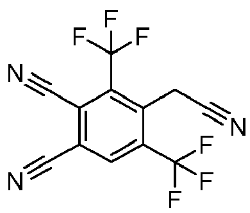 | 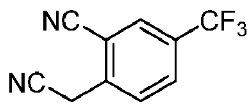 | 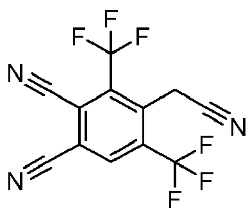 |
| A346 | 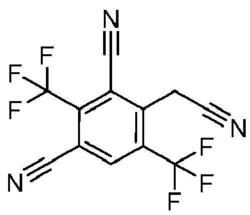 | 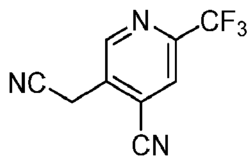 | 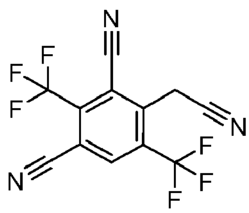 |
| A347 | 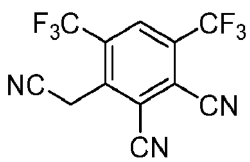 | 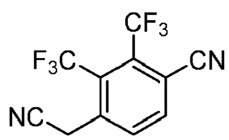 | 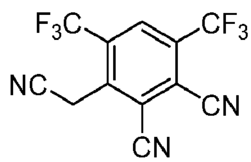 |

(continued)

| | A1 | A2 | A3 |
|---|---|---|---|
| A348 | 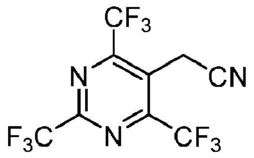 | 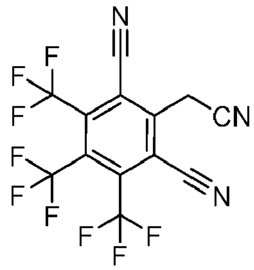 | 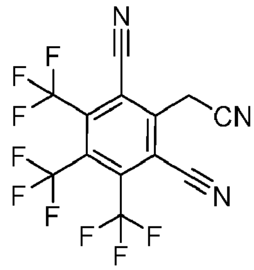 |
| A349 | 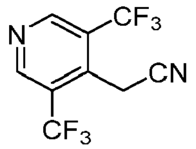 | 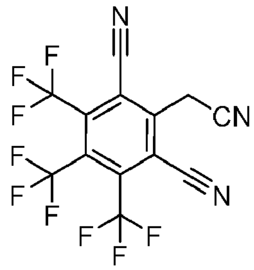 | 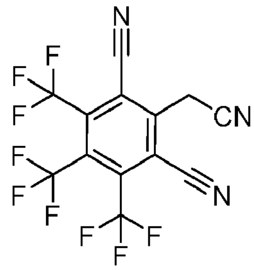 |
| A350 | 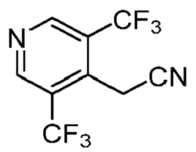 | 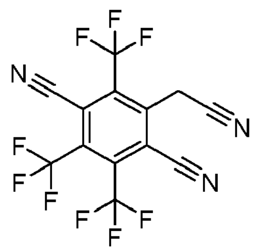 | 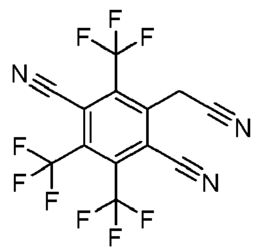 |
| A351 | 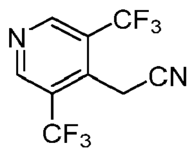 | 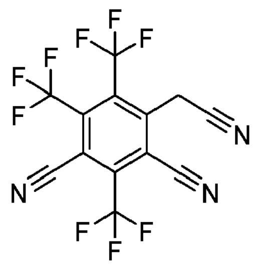 | 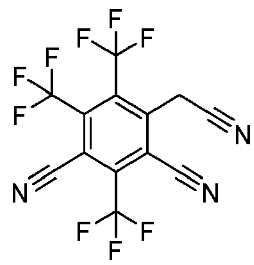 |

According to a preferred embodiment, the compound of formula (I) is selected from A1 to A347.

According to a more preferred embodiment, the compound of formula (I) is selected from A1 to A179.

According to a most preferred embodiment, the compound of formula (I) is selected from A1 to A 69.

The present invention furthermore relates to a composition comprising a compound of formula (IV) and at least one compound of formula (IVa) to (IVd)

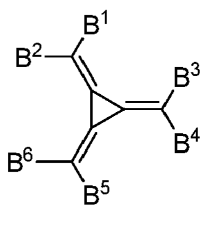

(IVa)

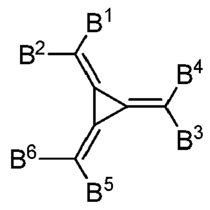

(IVb)

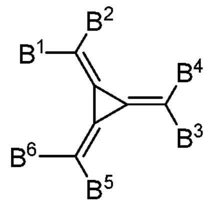

(IVc)

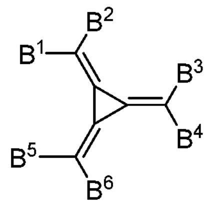

(IVd).

The present invention furthermore relates to an organic semiconductor layer, whereby the organic semiconductor layer comprises a compound according to the present invention or a composition according to the present invention.

In case the organic semiconductor layer comprises a composition according to the invention, throughout this application text the term "compound of formula (I)" shall also intend to include the composition as described above.

According to one embodiment of the present invention the organic semiconductor layer and/or the compound of formula (I) are non-emissive.

In the context of the present specification the term "essentially non-emissive" or "non-emissive" means that the contribution of the compound or layer to the visible emission spectrum from the device is less than 10%, preferably less than 5 % relative to the visible emission spectrum. The visible emission spectrum is an emission spectrum with a wavelength of about ≥ 380 nm to about ≤ 780 nm.

According to one embodiment of the present invention, the organic semiconductor layer is arranged between an anode and an emission layer. Particularly, according to on embodiment of the present invention, the organic semiconductor layer is a hole injection layer.

According to one embodiment of the present invention, the organic semiconductor layer is arranged between a cathode and an emission layer. Particularly, according to one embodiment of the invention, the organic semiconductor layer is a charge generation layer, preferably a p-type charge generation layer.

According to one embodiment of the invention, the at least one organic semiconductor layer further comprises a substantially covalent matrix compound.

According to one embodiment of the present invention, the p-type charge generation layer comprises a substantially covalent matrix compound.

According to one embodiment of the present invention, the hole injection layer comprises a substantially covalent matrix compound.

<u>Substantially covalent matrix compound</u>

The organic semiconductor layer may further comprises a substantially covalent matrix compound. According to one embodiment the substantially covalent matrix compound may be selected from at least one organic compound. The substantially covalent matrix may consists substantially from covalently bound C, H, O, N, S, which optionally comprise in

addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

Compound of formula (VI) or a compound of formula (VII)

According to another aspect of the present invention, the at least one matrix compound, also referred to as "substantially covalent matrix compound", may comprises at least one arylamine compound, diarylamine compound, triarylamine compound, a compound of formula (VI) or a compound of formula (VII)

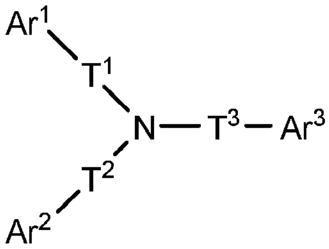
(VI),
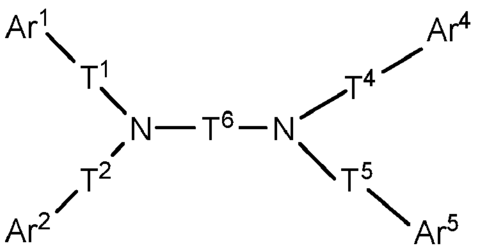
(VII),

wherein:

$T^1$, $T^2$, $T^3$, $T^4$ and $T^5$ are independently selected from a single bond, phenylene, biphenylene, terphenylene or naphthenylene, preferably a single bond or phenylene;

$T^6$ is phenylene, biphenylene, terphenylene or naphthenylene;

$Ar^1$, $Ar^2$, $Ar^3$, $Ar^4$ and $Ar^5$ are independently selected from substituted or unsubstituted $C_6$ to $C_{20}$ aryl, or substituted or unsubstituted $C_3$ to $C_{20}$ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;

wherein

the substituents of $Ar^1$, $Ar^2$, $Ar^3$, $Ar^4$ and $Ar^5$ are selected the same or different from the group comprising H, D, F, C(-O)

$R^2$, CN, $Si(R^2)_3$, $P(-O)(R^2)_2$, $OR^2$, $S(-O)R^2$, $S(-O)_2R^2$, substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted $C_6$ to Cis aryl, unsubstituted $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,

wherein $R^2$ may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, $C_6$ to Cis aryl or $C_3$ to Cis heteroaryl.

According to an embodiment wherein $T^1$, $T^2$, $T^3$, $T^4$ and $T^5$ may be independently selected from a single bond, phenylene, biphenylene or terphenylene. According to an embodiment wherein $T^1$, $T^2$, $T^3$, $T^4$ and $T^5$ may be independently selected from phenylene, biphenylene or terphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and two of $T^1$, $T^2$, $T^3$, $T^4$ and $T^5$ are a single bond.

According to an embodiment wherein $T^1$, $T^2$ and $T^3$ may be independently selected from phenylene and one of $T^1$, $T^2$ and $T^3$ are a single bond. According to an embodiment wherein $T^1$, $T^2$ and $T^3$ may be independently selected from phenylene and two of $T^1$, $T^2$ and $T^3$ are a single bond.

According to an embodiment wherein $T^6$ may be phenylene, biphenylene, terphenylene. According to an embodiment wherein $T^6$ may be phenylene. According to an embodiment wherein $T^6$ may be biphenylene. According to an embodiment wherein $T^6$ may be terphenylene.

According to an embodiment wherein $Ar^1$, $Ar^2$, $Ar^3$, $Ar^4$ and $Ar^5$ may be independently selected from D1 to D16:

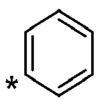 (D1), 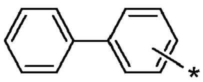 (D2), 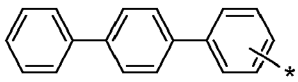 (D3), 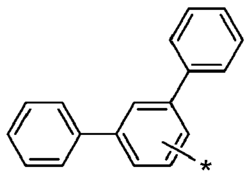 (D4),

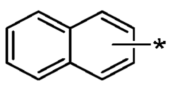 (D5), 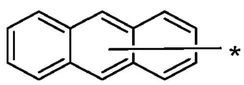 (D6), 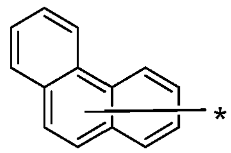 (D7), 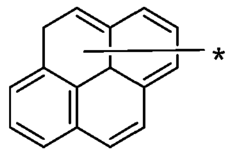 (D8),

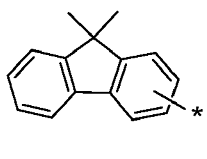 (D9), 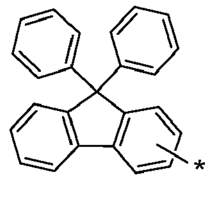 (D10), 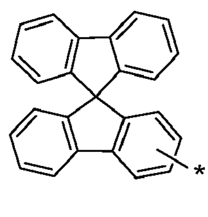 (D11),

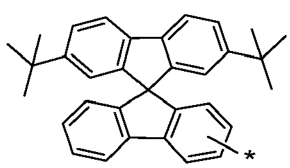 (D12), 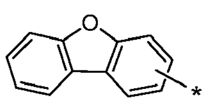 (D13), 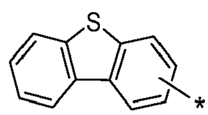 (D14),

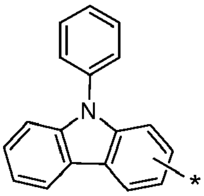 (D15), 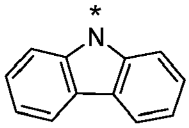 (D16),

wherein the asterix "*" denotes the binding position.

According to an embodiment $Ar^1$, $Ar^2$, $Ar^3$, $Ar^4$ and $Ar^5$ may be independently selected from D1 to D15; alternatively selected from D1 to D10 and D13 to D15.

According to an embodiment $Ar^1$, $Ar^2$, $Ar^3$, $Ar^4$ and $Ar^5$ may be independently selected from the group consisting of D1, D2, D5, D7, D9, D10, D13 to D16.

The rate onset temperature may be in a range particularly suited to mass production, when $Ar^1$, $Ar^2$, $Ar^3$, $Ar^4$ and $Ar^5$ are selected in this range.

The "matrix compound of formula (VI) or formula (VII)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (VI) or formula (VII) are selected from F1 to F21:

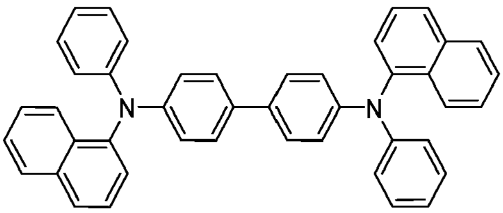 (F1), 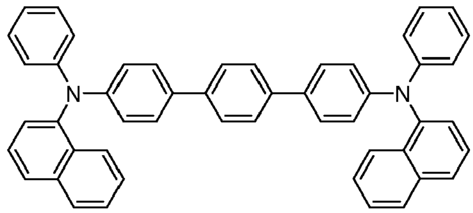 (F2),

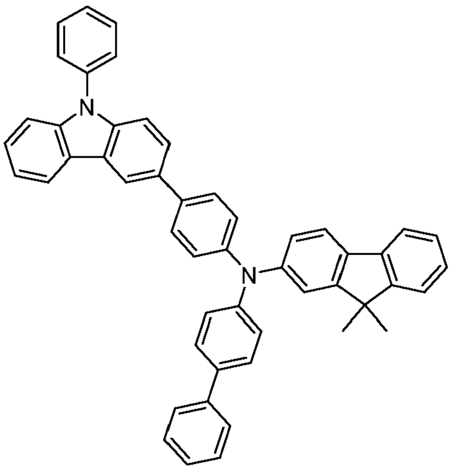 (F3), 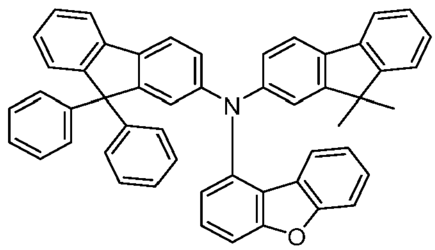 (F4),

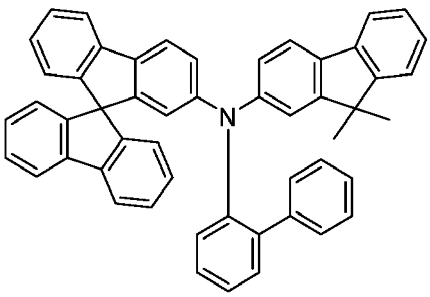 (F5), 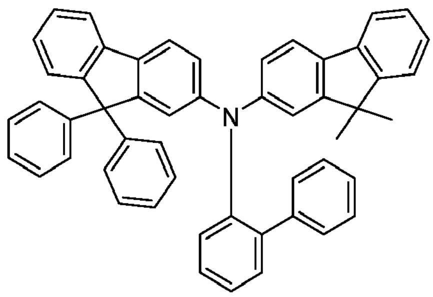 (F6),

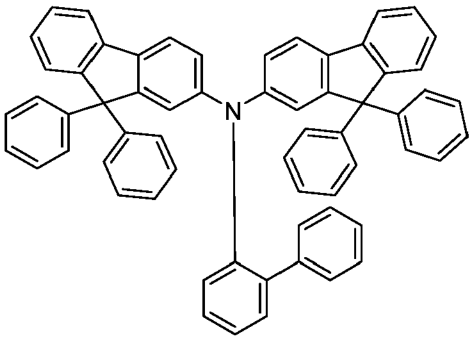
(F7),
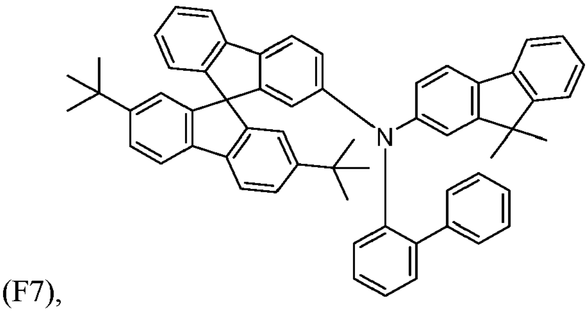
(F8),
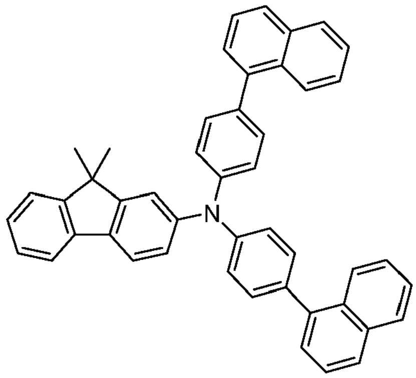
(F9),
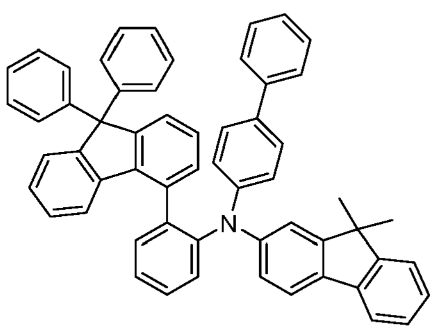
(F10),
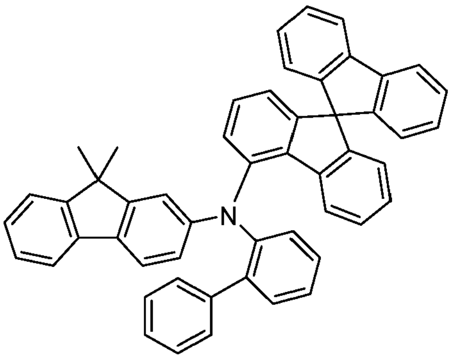
(F11),
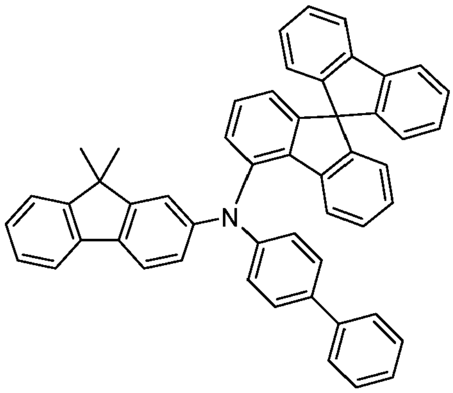
(F12),
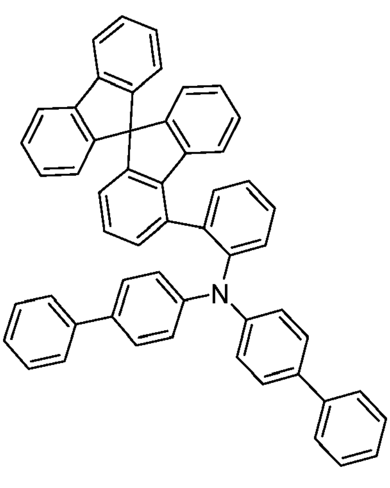
(F13),
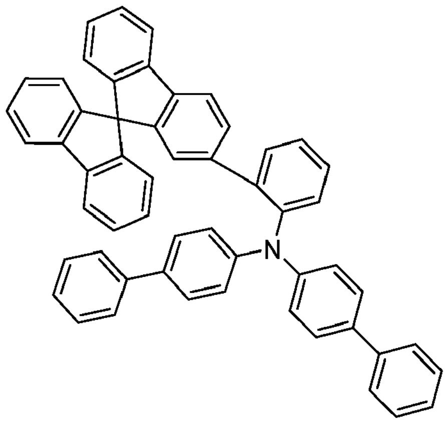
(F14),

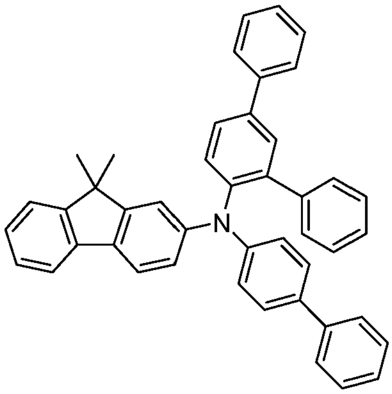 (F15),

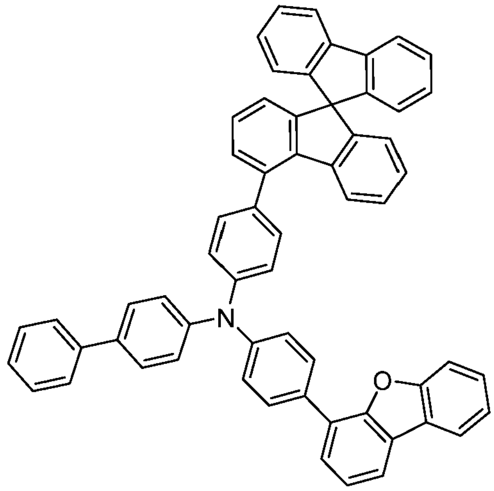 (F16),

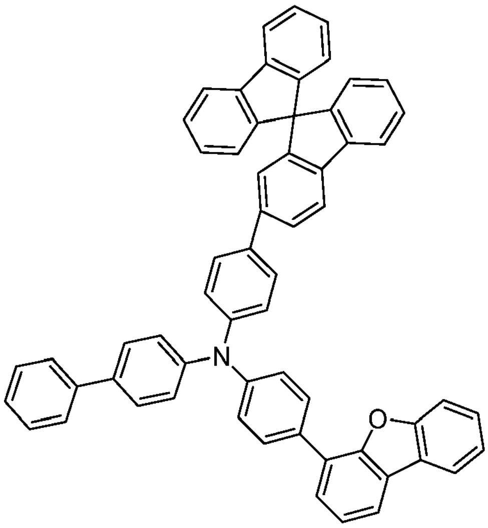 (F17)

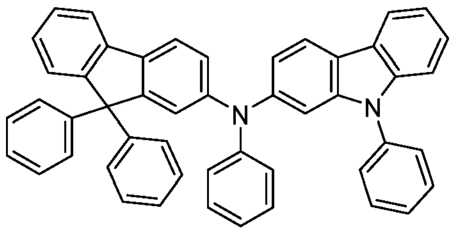 (F18)

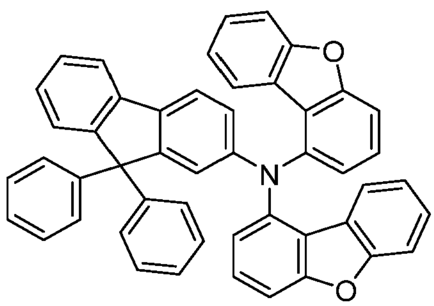 (F19),

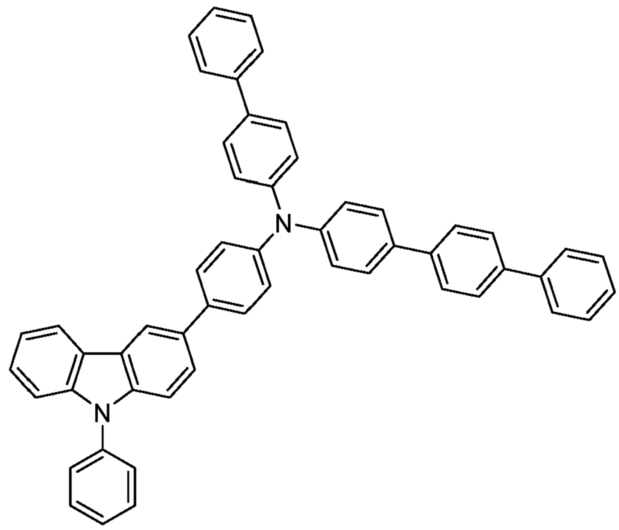 (F20)

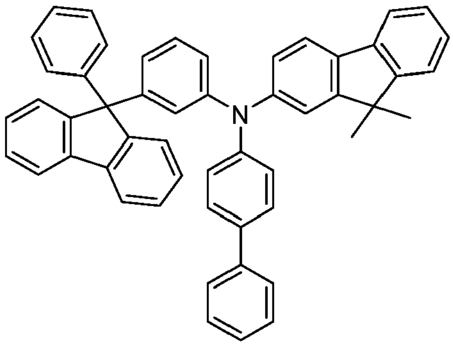

(F21).

The present invention furthermore relates to an organic electronic device comprising at least one organic semiconductor layer according to the present invention.

According to an embodiment, the organic electronic device comprises an anode layer, a cathode layer, and at least one organic semiconductor layer according to the present invention, wherein the organic semiconductor layer is arranged between the anode layer and the cathode layer.

According to an embodiment, the organic electronic device comprises an anode layer, a cathode layer, and at least one organic semiconductor layer, wherein the organic semiconductor layer is arranged between the anode layer and the cathode layer, wherein the organic semiconductor layer is a hole injection layer or a p-type charge generation layer.

According to an embodiment, the organic electronic device comprises an anode layer, a cathode layer, and at least one organic semiconductor layer, wherein the organic semiconductor layer is arranged between the anode layer and the cathode layer, wherein the at least one organic semiconductor layer is a hole injection layer and/or a p-type charge generation layer.

According to an embodiment, the organic electronic device comprises an anode layer, a cathode layer, and at least one organic semiconductor layer, wherein the organic semiconductor layer is arranged between the anode layer and the cathode layer, wherein the at least one organic semiconductor layer is a hole injection layer and a p-type charge generation layer.

According to an embodiment, wherein the organic electronic device is an electroluminescent device, an organic electroluminescent device, an organic light emitting diode (OLED), a light emitting device, thin film transistor, a battery, an organic photovoltaic cell (OPV), or an organic solar cell, preferably an organic electroluminescent device, an organic light emitting diode (OLED), a light emitting device, more preferably an organic electroluminescent device or an organic light emitting diode (OLED).

According to an embodiment, the organic electronic device is an organic electroluminescent device. All specifications and embodiments described in the context of the organic electronic device throughout this application text may *mutatis mutandis* apply for an organic electroluminescent device, too.

According to an embodiment, the organic semiconductor layer cormprising the compound of formula (I) in the organic electronic device is a hole injection layer.

According to an embodiment the organic semiconductor layer comprising the compound of formula (I) in the organic electronic device is a hole injection layer, wherein the hole injection layer is adjacent to the anode layer.

According to an embodiment, the organic electronic device further comprises a hole injection layer.

According to an embodiment, the organic electronic device further comprises a hole injection layer.

According to an embodiment, the organic electronic device further comprises a hole injection layer, wherein the hole injection layer is located between the anode layer and a hole transport layer.

According to an embodiment the organic semiconductor layer comprising the compound of formula (I) in the organic electronic device, is a hole injection layer, wherein the hole injection layer is in direct contact to the anode layer.

According to an embodiment, the organic electronic device further comprises a hole injection layer, wherein the hole injection layer is in direct contact to the hole transport layer.

According to an embodiment, the organic electronic device further comprises a hole injection layer, wherein the hole injection layer is located both in direct contact between the anode layer and a hole transport layer.

According to one embodiment, wherein the compound of formula (I) is present in hole injection layer in an amount of ≤99.9 wt.-% based on the total weight of the hole injection layer, preferably ≤99 wt.-%, more preferably ≤95 wt.-%, more preferably ≤90 wt.-%, more preferably ≤80 wt.-%, more preferably ≤70 wt.-%, more preferably ≤60 wt.-%, more preferably ≤50 wt.-%, more preferably ≤40 wt.-%, more preferably ≤30 wt.-%, more preferably ≤20 wt.-%, more preferably ≤ 10 wt.-%, more preferably ≤ 5 wt.-%, more preferably ≤ 3.0 wt.-%, more preferably ≤ 2.75 wt.-%, more preferably ≤ 2.5 wt.-%, more preferably ≤ 2.25 wt.-%, and most preferably ≤ 2.0 wt.-%, based on the total weight of the hole injection layer.

According to one embodiment, wherein the hole transport matrix compound is present in the hole injection layer in

an amount of ≥0.1 wt.-%, preferably ≥1 wt.-%, more preferably ≥5 wt.%, more preferably ≥10 wt.-%, more preferably ≥20 wt.-%, more preferably ≥30 wt.-%, more preferably ≥40 wt.-%, more preferably ≥50 wt.-%, more preferably ≥60 wt.-%, more preferably ≥70 wt.-%, more preferably ≥80 wt.-%, more preferably ≥90 wt.-%, more preferably ≥95 wt.-%, more preferably ≥97.0 wt.-%, more preferably ≥97.25 wt.-%, more preferably ≥97.5 wt.-%, more preferably ≥97.75 wt.-% and most preferably ≥98.0 wt.-%, based on the total weight of the hole injection layer.

According to one embodiment, wherein the compound of formula (I) is present in hole injection layer in an amount of ≤99.9 wt.-% based on the total weight of the hole injection layer, preferably ≤99 wt.-%, more preferably ≤95 wt.-%, more preferably ≤90 wt.-%, more preferably ≤80 wt.-%, more preferably ≤70 wt.-%, more preferably ≤60 wt.-%, more preferably ≤50 wt.-%, more preferably ≤40 wt.-%, more preferably ≤30 wt.-%, more preferably ≤20 wt.-%, more preferably ≤ 10 wt.-%, more preferably ≤ 5 wt.-%, more preferably ≤ 3.0 wt.-%, more preferably ≤ 2.75 wt.-%, more preferably ≤ 2.5 wt.-%, more preferably ≤ 2.25 wt.-%, and most preferably ≤ 2.0 wt.-%, based on the total weight of the hole injection layer, and wherein the hole transport matrix compound is present in the hole injection layer in an amount of ≥0.1 wt.-%, preferably ≥1 wt.-%, more preferably ≥5 wt.-%, more preferably ≥10 wt.-%, more preferably ≥20 wt.-%, more preferably ≥30 wt.-%, more preferably ≥40 wt.-%, more preferably ≥50 wt.-%, more preferably ≥60 wt.-%, more preferably ≥70 wt.-%, more preferably ≥80 wt.-%, more preferably ≥90 wt.-%, more preferably ≥95 wt.-%, more preferably ≥97.0 wt.-%, more preferably ≥97.25 wt.-%, more preferably ≥97.5 wt.-%, more preferably ≥97.75 wt.-% and most preferably ≥98.0 wt.-%, based on the total weight of the hole injection layer.

The hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto.

The p-type dopant may be a radialene compound, preferably according to formula (I) or for example 2,2',2"-(cyclopropane-1,2,3-triylidene)tris(2-(p-cyanotetrafluorophenyl)acetonitrile) (CC3).

The p-type dopant concentrations can be selected from 1 to 20 wt.-%, more preferably from 3 wt.-% to 10 wt.-%.

The p-type dopant concentrations can be selected from 1 to 20 vol.-%, more preferably from 3 vol.-% to 10 vol.-%.

According to an embodiment the organic electronic device comprises a substrate, an anode layer formed on the substrate, the organic semiconductor layer comprising the compound of formula (I), wherein the organic semiconductor layer is a hole injection layer, an emission layer and a cathode layer, wherein the hole injection layer, and emission layer are arranged between the anode layer and the cathode layer, wherein the hole injection layer is arranged between the anode layer and emission layer.

According to an embodiment the organic electronic device comprises a substrate, an anode layer formed on the substrate, the organic semiconductor layer comprising the compound of formula (I), wherein the organic semiconductor layer is a hole injection layer, an emission layer and a cathode layer, wherein the hole injection layer, and emission layer are arranged between the anode layer and the cathode layer, wherein the hole injection layer is arranged between the anode layer and emission layer, wherein the hole injection layer is adjacent to the anode layer.

According to an embodiment the organic electronic device comprises a substrate, an anode layer formed on the substrate, the organic semiconductor layer comprising the compound of formula (I), wherein the organic semiconductor layer is a hole injection layer, an emission layer and a cathode layer, wherein the hole injection layer, and emission layer are arranged between the anode layer and the cathode layer, wherein the hole injection layer is arranged between the anode layer and emission layer, wherein the hole injection layer is in direct contact to the anode layer.

According to an embodiment the organic electronic device comprises a substrate, an anode layer formed on the substrate, the organic semiconductor layer comprising the compound of formula (I), wherein the organic semiconductor layer is a hole injection layer, a hole transport layer, an emission layer and a cathode layer, wherein the hole injection layer, hole transport layer and emission layer are arranged between the anode layer and the cathode layer, wherein the hole injection layer and the hole transport layer are arranged between the anode layer and emission layer, wherein the hole

injection layer is closer to the anode layer than the hole transport layer.

According to an embodiment the organic electronic device comprises a substrate, an anode layer formed on the substrate, the organic semiconductor layer comprising the compound of formula (I), wherein the organic semiconductor layer is a hole injection layer, a hole transport layer, an emission layer and a cathode layer, wherein the hole injection layer, hole transport layer and emission layer are arranged between the anode layer and the cathode layer, wherein the hole injection layer and the hole transport layer are arranged between the anode layer and emission layer, wherein the hole injection layer is closer to the anode layer than the hole transport layer, wherein the hole injection layer is adjacent to the anode layer, and wherein the hole injection layer is adjacent to the hole transport layer.

According to an embodiment the organic electronic device comprises a substrate, an anode layer formed on the substrate, an organic semiconductor layer comprising the compound of formula (I),wherein the organic semiconductor layer is a hole injection layer, a hole transport layer, an emission layer and a cathode layer, hole injection layer and the hole transport layer are arranged between the anode layer and emission layer, wherein the hole injection layer and the hole transport layer are arranged between the anode layer and emission layer, wherein the hole injection layer is closer to the anode layer than the hole transport layer, wherein the hole injection layer is in direct contact to the anode layer, and wherein the hole injection layer is adjacent to the hole transport layer.

According to an embodiment the organic electronic device comprises a substrate, an anode layer formed on the substrate, the organic semiconductor layer comprising the compound of formula (I), wherein the organic semiconductor layer is a hole injection layer, a hole transport layer, an emission layer and a cathode layer, hole injection layer and the hole transport layer are arranged between the anode layer and emission layer, wherein the hole injection layer and the hole transport layer are arranged between the anode layer and emission layer, wherein the hole injection layer is closer to the anode layer than the hole transport layer, wherein the hole injection layer is in direct contact to the anode layer, and wherein the hole injection layer is direct contact to the hole transport layer.

According to an embodiment the organic electronic device comprises a substrate, an anode layer formed on the substrate, the organic semiconductor layer comprising the compound of formula (I), wherein the organic semiconductor layer is a hole injection layer, a hole transport layer, an optional electron blocking layer, an emission layer, an optional hole blocking layer, an electron transport layer, an optional electron injection layer and a cathode layer.

According to an embodiment the organic electronic device comprises a substrate, an anode layer formed on the substrate, the organic semiconductor layer comprising the compound of formula (I), wherein the organic semiconductor layer is a hole injection layer, wherein the hole injection layer is in direct contact to the anode layer, a hole transport layer, an optional electron blocking layer, an emission layer, an optional hole blocking layer, an electron transport layer, an optional electron injection layer and a cathode layer.

According to an embodiment the organic electronic device comprises a substrate, an anode layer formed on the substrate, the organic semiconductor layer comprising the compound of formula (I), wherein the organic semiconductor layer is a hole injection layer, wherein the hole injection layer is in direct contact to the anode layer, a hole transport layer, wherein the hole transport layer is in direct contact to the hole injection layer, an optional electron blocking layer, an emission layer, an optional hole blocking layer, an electron transport layer, an optional electron injection layer and a cathode layer.

According to an embodiment the organic electronic device comprises a substrate, an anode layer formed on the substrate, the organic semiconductor layer comprising the compound of formula (I), wherein the organic semiconductor layer is a hole injection layer, a hole transport layer, an electron blocking layer, an emission layer, an optional hole blocking layer, an electron transport layer, an optional electron injection layer and a cathode layer.

According to an embodiment the organic electronic device comprises a substrate, an anode layer formed on the substrate, the organic semiconductor layer comprising the compound of formula (I), wherein the organic semiconductor layer is a hole injection layer, wherein the hole injection layer is in direct contact to the anode layer, a hole transport layer, an electron blocking layer, an emission layer, an optional hole blocking layer, an electron transport layer, an optional electron injection layer and a cathode layer.

According to an embodiment the organic electronic device comprises a substrate, an anode layer formed on the substrate, the organic semiconductor layer comprising the compound of formula (I), wherein the the organic semiconductor layer is a hole injection layer, wherein the hole injection layer is in direct contact to the anode layer, a hole transport layer, wherein the hole transport layer is in direct contact to the hole injection layer, an electron blocking layer, an emission layer, an optional hole blocking layer, an electron transport layer, an optional electron injection layer and a cathode layer.

According to an embodiment the organic electronic device comprises a substrate, an anode layer formed on the substrate, the organic semiconductor layer comprising the compound of formula (I),wherein the organic semiconductor layer is hole injection layer, a hole transport layer, an electron blocking layer, an emission layer, an optional hole blocking layer, an electron transport layer, an electron injection layer and a cathode layer.

According to an embodiment the organic electronic device comprises a substrate, an anode layer formed on the substrate, a hole injection layer comprising a compound of formula (I), hole injection layer is in direct contact to the anode layer, a hole transport layer, an electron blocking layer, an emission layer, an optional hole blocking layer, an electron

transport layer, an electron injection layer and a cathode layer.

According to an embodiment the organic electronic device comprises a substrate, an anode layer formed on the substrate, a hole injection layer comprising a compound of formula (I), hole injection layer is in direct contact to the anode layer, a hole transport layer, wherein the hole transport layer is in direct contact to the hole injection layer, an electron blocking layer, an emission layer, an optional hole blocking layer, an electron transport layer, an electron injection layer and a cathode layer.

According to an embodiment the organic electronic device comprises a substrate, an anode layer formed on the substrate, a hole injection layer comprising a compound of formula (I), a hole transport layer, an electron blocking layer, an emission layer, a hole blocking layer, an electron transport layer, an electron injection layer and a cathode layer.

According to an embodiment the organic electronic device comprises a substrate, an anode layer formed on the substrate, a hole injection layer comprising a compound of formula (I), hole injection layer is in direct contact to the anode layer, a hole transport layer, an electron blocking layer, an emission layer, a hole blocking layer, an electron transport layer, an electron injection layer and a cathode layer.

According to an embodiment the organic electronic device comprises a substrate, an anode layer formed on the substrate, a hole injection layer comprising a compound of formula (I), a hole transport layer, an electron blocking layer, an emission layer, a hole blocking layer, an electron transport layer, an electron injection layer and a cathode layer.

According to an embodiment the organic electronic device comprises a substrate, an anode layer formed on the substrate, a hole injection layer comprising a compound of formula (I), wherein the hole injection layer is in direct contact to the anode layer, a hole transport layer, wherein the hole transport layer is in direct contact to the hole injection layer, an electron blocking layer, an emission layer, a hole blocking layer, an electron transport layer, an electron injection layer and a cathode layer.

According to an embodiment the organic electronic device comprises a substrate, an anode layer formed on the substrate, a hole injection layer comprising a compound of formula (I), wherein the hole injection layer is in direct contact to the anode layer, a hole transport layer, wherein the hole transport layer is in direct contact to the hole injection layer, an electron blocking layer, wherein the electron blocking layer is in direct contact to the hole transport layer, an emission layer, wherein the emission layer is in direct contact to the electron blocking layer, a hole blocking layer, wherein the hole blocking layer is in direct contact to the emission layer, an electron transport layer, wherein the electron transport layer is in direct contact to the hole blocking layer, an electron injection layer, wherein the electron injection layer is in direct contact to electron transport layer, and a cathode layer.

*p-type charge generation layer*

According to an embodiment, the p-type charge generation layer further comprises a hole transport matrix compound.

According to an embodiment, the compound of formula (I) is present in the p-type charge generation layer in an amount of ≤99.9 wt.-% based on the total weight of the p-type charge generation layer, preferably ≤99 wt.-%, more preferably ≤95 wt.-%, more preferably ≤90 wt.-%, more preferably ≤80 wt.-%, more preferably ≤70 wt.-%, more preferably ≤60 wt.-%, more preferably ≤50 wt.-%, more preferably ≤40 wt.-%, more preferably ≤30 wt.-%, more preferably ≤20 wt.-%, more preferably ≤ 10 wt.-%, more preferably ≤ 5 wt.-%.

According to an embodiment, the hole transport matrix compound is present in the p-type charge generation layer in an amount of ≥0. 1 wt.-% based on the total weight of the p-type charge generation layer, preferably ≥1 wt.-%, more preferably ≥5 wt.-%, more preferably ≥10 wt.-%, more preferably ≥20 wt.-%, more preferably ≥30 wt.-%, more preferably ≥40 wt.-%, more preferably ≥50 wt.-%, more preferably ≥60 wt.-%, more preferably ≥70 wt.-%, more preferably ≥80 wt.-%, more preferably ≥90 wt.-%, more preferably ≥95 wt.-%.

According to an embodiment, the compound of formula (I) is present in the p-type charge generation layer in an amount of ≤99.9 wt.-% based on the total weight of the p-type charge generation layer, preferably ≤99 wt.-%, more preferably ≤95 wt.-%, more preferably ≤90 wt.-%, more preferably ≤80 wt.-%, more preferably ≤70 wt.-%, more preferably ≤60 wt.-%, more preferably ≤50 wt.-%, more preferably ≤40 wt.-%, more preferably ≤30 wt.-%, more preferably ≤20 wt.-%, more preferably ≤ 10 wt.-%, more preferably ≤ 5 wt.-%, and the hole transport matrix compound is present in the p-type charge generation layer in an amount of ≥0.1 wt.-% based on the total weight of the p-type charge generation layer, preferably ≥1 wt.-%, more preferably ≥5 wt.-%, more preferably ≥10 wt.-%, more preferably ≥20 wt.-%, more preferably ≥30 wt.-%, more preferably ≥40 wt.-%, more preferably ≥50 wt.-%, more preferably ≥60 wt.-%, more preferably ≥70 wt.-%, more preferably ≥80 wt.-%, more preferably ≥90 wt.-%, more preferably ≥95 wt.-%.

According to an embodiment the organic semiconductor layer comprising the compound of formula (I) is a p-type charge generation layer, and the organic electronic device further comprises a first emission layer, and a second emission layer, wherein the organic semiconductor layer is arranged between the first emission layer and the second emission layer.

According to an embodiment the organic semiconductor layer comprising the compound of formula (I) is a p-type charge generation layer, and the organic electronic device further comprises an n-type charge generation layer, a first

emission layer, and a second emission layer, wherein the p-type charge generation layer and the n-type charge generation layer is arranged between the first emission layer and the second emission layer.

According to an embodiment the organic semiconductor layer comprising the compound of formula (I) is a p-type charge generation layer, and the organic electronic device further comprises an n-type charge generation layer, a first emission layer, and a second emission layer, wherein the p-type charge generation layer and n-type charge generation layer is arranged between the first emission layer and the second emission layer,
wherein the n-type charge generation layer is arranged closer to the anode layer than the organic semiconductor layer.

According to an embodiment the organic semiconductor layer comprising the compound of formula (I) is a p-type charge generation layer, and the organic electronic device further comprises an electron transport layer, an n-type charge generation layer, a first emission layer, and a second emission layer, wherein the p-type charge generation layer and the n-type charge generation layer is arranged between the first emission layer and the second emission layer,
wherein the electron transport layer is arranged between the first emission layer and the second emission layer, wherein the electron transport layer is in direct contact to the n-type charge generation layer.

According to an embodiment the organic semiconductor layer comprising the compound of formula (I) is a p-type charge generation layer, and the organic electronic device further comprises a hole transport layer, an n-type charge generation layer, a first emission layer, and a second emission layer,

wherein the p-type charge generation layer and the n-type charge generation layer is arranged between the first emission layer and the second emission layer,
wherein the hole transport layer is arranged between the first emission layer and the second emission layer.

According to an embodiment the organic semiconductor layer comprising the compound of formula (I) is a p-type charge generation layer, and the organic electronic device further comprises a hole transport layer, a first emission layer, and a second emission layer, wherein the p-type charge generation layer is arranged between the first emission layer and the second emission layer,
wherein the hole transport layer is arranged between the first emission layer and the second emission layer, wherein the hole transport layer is in direct contact to the p-type charge generation layer.

According to an embodiment the organic semiconductor layer comprising the compound of formula (I) is a p-type charge generation layer, and the organic electronic device further comprises an electron transport layer, a hole transport layer, a first emission layer, and a second emission layer,

wherein the p-type charge generation layer and the n-type charge generation layer is arranged between the first emission layer and the second emission layer,
wherein the electron transport layer is arranged between the first emission layer and the second emission layer, and wherein the hole transport layer is arranged between the first emission layer and the second emission layer.

According to an embodiment the organic semiconductor layer comprising the compound of formula (I) is a p-type charge generation layer, and the organic electronic device further comprises an electron transport layer, a hole transport layer, an n-type charge generation layer, a first emission layer, and a second emission layer, wherein the p-type charge generation layer and the n-type charge generation layer is arranged between the first emission layer and the second emission layer,
wherein the electron transport layer and the hole transport layer are arranged between the first emission layer and the second emission layer, wherein the electron transport layer is in direct contact to the n-type charge generation layer, and wherein the hole transport layer is in direct contact to the p-type charge generation layer.

According to an embodiment the organic semiconductor layer comprising the compound of formula (I) is a p-type charge generation layer, and the organic electronic device further comprises an n-type charge generation layer, a first emission layer, and a second emission layer, wherein the p-type charge generation layer is in direct contact to the n-type charge generation layer, wherein the p-type charge generation layer and the n-type charge generation layer are arranged between the first emission layer and the second emission layer.

According to an embodiment the organic semiconductor layer comprising a compound of formula (I) is a p-type charge generation layer, and the organic electronic device further comprises an electron transport layer, a hole transport layer, an n-type charge generation layer, a first emission layer, and a second emission layer, wherein the electron transport layer, and the hole transport layer are arranged between the first emission layer and the second emission layer, wherein the electron transport layer is in direct contact to the n-type charge generation layer, wherein the hole transport layer is in direct contact to the p-type charge generation layer, wherein the p-type charge generation layer is in direct contact to the n-type charge generation layer, wherein the p-type charge generation layer and the n-type charge generation layer are arranged between the first emission layer and the second emission layer.

According to an embodiment, the organic electronic device may comprise a substrate, an anode layer, a hole

injection layer comprising a compound of formula (I), a first hole transport layer, a first electron blocking layer, a first emission layer, an optional first hole blocking layer, a first electron transport layer, an n-type charge generation layer, a p-type charge generation layer, a second hole transport layer, a second electron blocking layer, second emission layer, a cathode layer, wherein between the second emission layer and the cathode layer an optional second hole blocking layer, an optional second electron transport layer and/or an optional electron injection layer are arranged.

According to an embodiment, the organic electronic device may comprise a substrate, an anode layer, a hole injection layer, a first hole transport layer, a first electron blocking layer, a first emission layer, an optional first hole blocking layer, a first electron transport layer, an n-type charge generation layer, a p-type charge generation layer comprising a compound of formula (I), a second hole transport layer, a second electron blocking layer, second emission layer, a cathode layer, wherein between the second emission layer and the cathode layer an optional second hole blocking layer, an optional second electron transport layer and/or an optional electron injection layer are arranged.

According to an embodiment, the organic electronic device may comprise a substrate, an anode layer, a hole injection layer comprising a compound of formula (I), a first hole transport layer, a first electron blocking layer, a first emission layer, an optional first hole blocking layer, a first electron transport layer, an n-type charge generation layer, a p-type charge generation layer comprising a compound of formula (I), a second hole transport layer, a second electron blocking layer, second emission layer, a cathode layer, wherein between the second emission layer and the cathode layer an optional second hole blocking layer, an optional second electron transport layer and/or an optional electron injection layer are arranged, wherein the compound of formula (I) in the hole injection layer and the compound of formula (I) in the p-type charge generation layer can be the same or different.

According to an embodiment, the organic electronic device may comprise a layer structure of a substrate that is adjacent arranged to an anode layer, a hole injection layer comprising a compound of formula (I), wherein the hole injection layer is arranged adjacent to the anode layer, a first hole transport layer, wherein the first hole transport layer is arranged adjacent to the hole injection layer, a first electron blocking layer, wherein the first electron blocking layer is arranged adjacent to the first hole transport layer, a first emission layer, wherein the first emission layer is arranged adjacent to the first electron blocking layer, an optional first hole blocking layer, wherein the optional first hole blocking layer is arranged adjacent to the first emission layer, a first electron transport layer, wherein the first electron transport layer is arranged adjacent to the first emission layer or adjacent to the optional first hole blocking layer, an n-type charge generation layer, wherein the n-type charge generation layer is arranged adjacent to the first electron transport layer, a p-type charge generation layer, wherein the p-type charge generation layer is arranged adjacent to the n-type charge generation layer, a second hole transport layer, wherein the second hole transport layer is arranged adjacent to the p-type charge generation layer, a second electron blocking layer, wherein the second electron blocking layer is arranged adjacent to the second hole transport layer, a second emission layer, wherein the second emission layer is arranged adjacent to the second electron blocking layer, a cathode layer, wherein between the second emission layer and the cathode layer an optional second hole blocking layer, optional second electron transport layer and/or an optional electron injection layer are arranged.

According to an embodiment, the organic electronic device may comprise a layer structure of a substrate that is adjacent arranged to an anode layer, a hole injection layer, wherein the hole injection layer is arranged adjacent to the anode layer, a first hole transport layer, wherein the first hole transport layer is arranged adjacent to the hole injection layer, a first electron blocking layer, wherein the first electron blocking layer is arranged adjacent to the first hole transport layer, a first emission layer, wherein the first emission layer is arranged adjacent to the first electron blocking layer, an optional first hole blocking layer, wherein the optional first hole blocking layer is arranged adjacent to the first emission layer, a first electron transport layer, wherein the first electron transport layer is arranged adjacent to the first emission layer or adjacent to the optional first hole blocking layer, an n-type charge generation layer, wherein the n-type charge generation layer is arranged adjacent to the first electron transport layer, a p-type charge generation layer comprising a compound of formula (I), wherein the p-type charge generation layer is arranged adjacent to the n-type charge generation layer, a second hole transport layer, wherein the second hole transport layer is arranged adjacent to the p-type charge generation layer, a second electron blocking layer, wherein the second electron blocking layer is arranged adjacent to the second hole transport layer, a second emission layer, wherein the second emission layer is arranged adjacent to the second electron blocking layer, a cathode layer, wherein between the second emission layer and the cathode layer an optional second hole blocking layer, optional second electron transport layer and/or an optional electron injection layer are arranged.

According to an embodiment, the organic electronic device may comprise a layer structure of a substrate that is adjacent arranged to an anode layer, a hole injection layer comprising a compound of formula (I), wherein the hole injection layer is arranged adjacent to the anode layer, a first hole transport layer, wherein the first hole transport layer is arranged adjacent to the hole injection layer, a first electron blocking layer, wherein the first electron blocking layer is arranged adjacent to the first hole transport layer, a first emission layer, wherein the first emission layer is arranged adjacent to the first electron blocking layer, an optional first hole blocking layer, wherein the optional first hole blocking layer is arranged adjacent to the first emission layer, a first electron transport layer, wherein the first electron transport layer is arranged adjacent to the first emission layer or adjacent to the optional first hole blocking layer, an n-type charge generation layer, wherein the n-type charge generation layer is arranged adjacent to the first electron transport layer, a p-type charge

generation layer comprising a compound of formula (I), wherein the p-type charge generation layer is arranged adjacent to the n-type charge generation layer, a second hole transport layer, wherein the second hole transport layer is arranged adjacent to the p-type charge generation layer, a second electron blocking layer, wherein the second electron blocking layer is arranged adjacent to the second hole transport layer, a second emission layer, wherein the second emission layer is arranged adjacent to the second electron blocking layer, a cathode layer, wherein between the second emission layer and the cathode layer an optional second hole blocking layer, optional second electron transport layer and/or an optional electron injection layer are arranged, wherein the compound of formula (I) in the hole injection layer and the compound of formula (I) in the p-type charge generation layer can be the same or different.

According to an embodiment, the organic electronic device may comprise a layer structure of a substrate that is adjacent arranged to an anode layer, a hole injection layer comprising a compound of formula (I), wherein the hole injection layer is arranged adjacent to the anode layer, a first hole transport layer, wherein the first hole transport layer is arranged adjacent to the hole injection layer, a first electron blocking layer, wherein the first electron blocking layer is arranged adjacent to the first hole transport layer, a first emission layer, wherein the first emission layer is arranged adjacent to the first electron blocking layer, an optional first hole blocking layer, wherein the optional first hole blocking layer is arranged adjacent to the first emission layer, a first electron transport layer, wherein the first electron transport layer is arranged adjacent to the first emission layer or adjacent to the optional first hole blocking layer, an n-type charge generation layer, wherein the n-type charge generation layer is arranged adjacent to the first electron transport layer, a p-type charge generation layer, wherein the p-type charge generation layer is arranged adjacent to the n-type charge generation layer, a second hole transport layer, wherein the second hole transport layer is arranged adjacent to the p-type charge generation layer, a second electron blocking layer, wherein the second electron blocking layer is arranged adjacent to the second hole transport layer, a second emission layer, wherein the second emission layer is arranged adjacent to the second electron blocking layer, an optional second hole blocking layer, wherein the optional second hole blocking layer is arranged adjacent to the second emission layer, a second electron transport layer, wherein the second electron transport layer is arranged adjacent to the second emission layer or arranged adjacent to the optional second hole blocking layer, an electron injection layer, wherein the electron injection layer is arranged adjacent to the second electron transport layer, a cathode layer.

According to an embodiment, the organic electronic device may comprise a layer structure of a substrate that is adjacent arranged to an anode layer, a hole injection layer, wherein the hole injection layer is arranged adjacent to the anode layer, a first hole transport layer, wherein the first hole transport layer is arranged adjacent to the hole injection layer, a first electron blocking layer, wherein the first electron blocking layer is arranged adjacent to the first hole transport layer, a first emission layer, wherein the first emission layer is arranged adjacent to the first electron blocking layer, an optional first hole blocking layer, wherein the optional first hole blocking layer is arranged adjacent to the first emission layer, a first electron transport layer, wherein the first electron transport layer is arranged adjacent to the first emission layer or adjacent to the optional first hole blocking layer, an n-type charge generation layer, wherein the n-type charge generation layer is arranged adjacent to the first electron transport layer, a p-type charge generation layer comprising a compound of formula (I), wherein the p-type charge generation layer is arranged adjacent to the n-type charge generation layer, a second hole transport layer, wherein the second hole transport layer is arranged adjacent to the p-type charge generation layer, a second electron blocking layer, wherein the second electron blocking layer is arranged adjacent to the second hole transport layer, a second emission layer, wherein the second emission layer is arranged adjacent to the second electron blocking layer, an optional second hole blocking layer, wherein the optional second hole blocking layer is arranged adjacent to the second emission layer, a second electron transport layer, wherein the second electron transport layer is arranged adjacent to the second emission layer or arranged adjacent to the optional second hole blocking layer, an electron injection layer, wherein the electron injection layer is arranged adjacent to the second electron transport layer, a cathode layer.

According to an embodiment, the organic electronic device may comprise a layer structure of a substrate that is adjacent arranged to an anode layer, a hole injection layer comprising a compound of formula (I), wherein the hole injection layer is arranged adjacent to the anode layer, a first hole transport layer, wherein the first hole transport layer is arranged adjacent to the hole injection layer, a first electron blocking layer, wherein the first electron blocking layer is arranged adjacent to the first hole transport layer, a first emission layer, wherein the first emission layer is arranged adjacent to the first electron blocking layer, an optional first hole blocking layer, wherein the optional first hole blocking layer is arranged adjacent to the first emission layer, a first electron transport layer, wherein the first electron transport layer is arranged adjacent to the first emission layer or adjacent to the optional first hole blocking layer, an n-type charge generation layer, wherein the n-type charge generation layer is arranged adjacent to the first electron transport layer, a p-type charge generation layer comprising a compound of formula (I), wherein the p-type charge generation layer is arranged adjacent to the n-type charge generation layer, a second hole transport layer, wherein the second hole transport layer is arranged adjacent to the p-type charge generation layer, a second electron blocking layer, wherein the second electron blocking layer is arranged adjacent to the second hole transport layer, a second emission layer, wherein the second emission layer is arranged adjacent to the second electron blocking layer, an optional second hole blocking layer, wherein the optional

second hole blocking layer is arranged adjacent to the second emission layer, a second electron transport layer, wherein the second electron transport layer is arranged adjacent to the second emission layer or arranged adjacent to the optional second hole blocking layer, an electron injection layer, wherein the electron injection layer is in direct contact to the second electron transport layer, a cathode layer, wherein the compound of formula (I) in the hole injection layer and the compound of formula (I) in the p-type charge generation layer can be the same or different.

According to an embodiment, the organic electronic device may comprise a layer structure of a substrate that is adjacent arranged to an anode layer, a hole injection layer comprising a compound of formula (I), wherein the hole injection layer is in direct contact to the anode layer, a first hole transport layer, wherein the first hole transport layer is in direct contact to the hole injection layer, a first electron blocking layer, wherein the first electron blocking layer is in direct contact to the first hole transport layer, a first emission layer, wherein the first emission layer is in direct contact to the first electron blocking layer, an optional first hole blocking layer, wherein the optional first hole blocking layer is in direct contact to the first emission layer, a first electron transport layer, wherein the first electron transport layer is in direct contact to the first emission layer or in direct contact to the optional first hole blocking layer, an n-type charge generation layer, wherein the n-type charge generation layer is in direct contact to the first electron transport layer, a p-type charge generation layer, wherein the p-type charge generation layer is in direct contact to the n-type charge generation layer, a second hole transport layer, wherein the second hole transport layer is in direct contact to the p-type charge generation layer, a second electron blocking layer, wherein the second electron blocking layer is in direct contact to the second hole transport layer, a second emission layer, wherein the second emission layer is in direct contact to the second electron blocking layer, a cathode layer, wherein between the second emission layer and the cathode layer an optional second hole blocking layer, optional second electron transport layer and/or an optional electron injection layer are arranged.

According to an embodiment, the organic electronic device may comprise a layer structure of a substrate that is adjacent arranged to an anode layer, a hole injection layer, wherein the hole injection layer is in direct contact to the anode layer, a first hole transport layer, wherein the first hole transport layer is in direct contact to the hole injection layer, a first electron blocking layer, wherein the first electron blocking layer is in direct contact to the first hole transport layer, a first emission layer, wherein the first emission layer is in direct contact to the first electron blocking layer, an optional first hole blocking layer, wherein the optional first hole blocking layer is in direct contact to the first emission layer, a first electron transport layer, wherein the first electron transport layer is in direct contact to the first emission layer or in direct contact to the optional first hole blocking layer, an n-type charge generation layer, wherein the n-type charge generation layer is in direct contact to the first electron transport layer, a p-type charge generation layer comprising a compound of formula (I), wherein the p-type charge generation layer is in direct contact to the n-type charge generation layer, a second hole transport layer, wherein the second hole transport layer is in direct contact to the p-type charge generation layer, a second electron blocking layer, wherein the second electron blocking layer is in direct contact to the second hole transport layer, a second emission layer, wherein the second emission layer is in direct contact to the second electron blocking layer, a cathode layer, wherein between the second emission layer and the cathode layer an optional second hole blocking layer, optional second electron transport layer and/or an optional electron injection layer are arranged.

According to an embodiment, the organic electronic device may comprise a layer structure of a substrate that is adjacent arranged to an anode layer, a hole injection layer comprising a compound of formula (I), wherein the hole injection layer is in direct contact to the anode layer, a first hole transport layer, wherein the first hole transport layer is in direct contact to the hole injection layer, a first electron blocking layer, wherein the first electron blocking layer is in direct contact to the first hole transport layer, a first emission layer, wherein the first emission layer is in direct contact to the first electron blocking layer, an optional first hole blocking layer, wherein the optional first hole blocking layer is in direct contact to the first emission layer, a first electron transport layer, wherein the first electron transport layer is in direct contact to the first emission layer or in direct contact to the optional first hole blocking layer, an n-type charge generation layer, wherein the n-type charge generation layer is in direct contact to the first electron transport layer, a p-type charge generation layer comprising a compound of formula (I), wherein the p-type charge generation layer is in direct contact to the n-type charge generation layer, a second hole transport layer, wherein the second hole transport layer is in direct contact to the p-type charge generation layer, a second electron blocking layer, wherein the second electron blocking layer is in direct contact to the second hole transport layer, a second emission layer, wherein the second emission layer is in direct contact to the second electron blocking layer, a cathode layer, wherein between the second emission layer and the cathode layer an optional second hole blocking layer, optional second electron transport layer and/or an optional electron injection layer are arranged, wherein the compound of formula (I) in the hole injection layer and the compound of formula (I) in the p-type charge generation layer can be the same or different.

According to an embodiment, the organic electronic device may comprise a layer structure of a substrate that is adjacent arranged to an anode layer, a hole injection layer comprising a compound of formula (I), wherein the hole injection layer is in direct contact to the anode layer, a first hole transport layer, wherein the first hole transport layer is in direct contact to the hole injection layer, a first electron blocking layer, wherein the first electron blocking layer is in direct contact to the first hole transport layer, a first emission layer, wherein the first emission layer is in direct contact to the first electron blocking layer, an optional first hole blocking layer, wherein the optional first hole blocking layer is in direct contact to the first

emission layer, a first electron transport layer, wherein the first electron transport layer is in direct contact to the first emission layer or in direct contact the optional first hole blocking layer, an n-type charge generation layer, wherein the n-type charge generation layer is in direct contact to the first electron transport layer, a p-type charge generation layer, wherein the p-type charge generation layer is in direct contact to the n-type charge generation layer, a second hole transport layer, wherein the second hole transport layer is in direct contact to the p-type charge generation layer, a second electron blocking layer, wherein the second electron blocking layer is in direct contact to the second hole transport layer, a second emission layer, wherein the second emission layer is in direct contact to the second electron blocking layer, an optional second hole blocking layer, wherein the optional second hole blocking layer is arranged in direct contact the second emission layer, a second electron transport layer, wherein the second electron transport layer is in direct contact to the second emission layer or in direct contact to the optional second hole blocking layer, an electron injection layer, wherein the electron injection layer is in direct contact to the second electron transport layer, a cathode layer.

According to an embodiment, the organic electronic device may comprise a layer structure of a substrate that is adjacent arranged to an anode layer, a hole injection layer, wherein the hole injection layer is in direct contact to the anode layer, a first hole transport layer, wherein the first hole transport layer is in direct contact to the hole injection layer, a first electron blocking layer, wherein the first electron blocking layer is in direct contact to the first hole transport layer, a first emission layer, wherein the first emission layer is in direct contact to the first electron blocking layer, an optional first hole blocking layer, wherein the optional first hole blocking layer is in direct contact to the first emission layer, a first electron transport layer, wherein the first electron transport layer is in direct contact to the first emission layer or in direct contact the optional first hole blocking layer, an n-type charge generation layer, wherein the n-type charge generation layer is in direct contact to the first electron transport layer, a p-type charge generation layer comprising a compound of formula (I), wherein the p-type charge generation layer is in direct contact to the n-type charge generation layer, a second hole transport layer, wherein the second hole transport layer is in direct contact to the p-type charge generation layer, a second electron blocking layer, wherein the second electron blocking layer is in direct contact to the second hole transport layer, a second emission layer, wherein the second emission layer is in direct contact to the second electron blocking layer, an optional second hole blocking layer, wherein the optional second hole blocking layer is arranged in direct contact the second emission layer, a second electron transport layer, wherein the second electron transport layer is in direct contact to the second emission layer or in direct contact to the optional second hole blocking layer, an electron injection layer, wherein the electron injection layer is in direct contact to the second electron transport layer, a cathode layer.

According to an embodiment, the organic electronic device may comprise a layer structure of a substrate that is adjacent arranged to an anode layer, a hole injection layer comprising a compound of formula (I), wherein the hole injection layer is in direct contact to the anode layer, a first hole transport layer, wherein the first hole transport layer is in direct contact to the hole injection layer, a first electron blocking layer, wherein the first electron blocking layer is in direct contact to the first hole transport layer, a first emission layer, wherein the first emission layer is in direct contact to the first electron blocking layer, an optional first hole blocking layer, wherein the optional first hole blocking layer is in direct contact to the first emission layer, a first electron transport layer, wherein the first electron transport layer is in direct contact to the first emission layer or in direct contact the optional first hole blocking layer, an n-type charge generation layer, wherein the n-type charge generation layer is in direct contact to the first electron transport layer, a p-type charge generation layer comprising a compound of formula (I), wherein the p-type charge generation layer is in direct contact to the n-type charge generation layer, a second hole transport layer, wherein the second hole transport layer is in direct contact to the p-type charge generation layer, a second electron blocking layer, wherein the second electron blocking layer is in direct contact to the second hole transport layer, a second emission layer, wherein the second emission layer is in direct contact to the second electron blocking layer, an optional second hole blocking layer, wherein the optional second hole blocking layer is arranged in direct contact the second emission layer, a second electron transport layer, wherein the second electron transport layer is in direct contact to the second emission layer or in direct contact to the optional second hole blocking layer, an electron injection layer, wherein the electron injection layer is in direct contact to the second electron transport layer, a cathode layer, wherein the compound of formula (I) in the hole injection layer and the compound of formula (I) in the p-type charge generation layer can be the same or different.

*n-type charge generation layer*

According to an embodiment, the n-type charge generation layer comprises a metal.

According to an embodiment, the n-type charge generation layer comprises an electron transport matrix compound.

According to an embodiment, the n-type charge generation layer comprises a metal, and an electron transport matrix compound.

According to an embodiment, the electron transport matrix compound is an organic electron transport matrix compound.

According to an embodiment, the electron transport matrix compound is a substantially covalent matrix

compound.

According to an embodiment, the electron transport matrix compound is a substantially covalent organic matrix compound.

According to an embodiment, the n-type charge generation layer comprises a metal and an electron transport matrix compound.

According to an embodiment, the electron transport material comprises at least one $C_2$ to $C_{24}$ N-heteroaryl or P=X group, with X being O, P, Se, with P=O especially preferred.

According to an embodiment of the present invention, the at least one $C_2$ to $C_{24}$ N-heteroaryl may be selected from a compound comprising at least one azine group, preferably at least two azine groups, also preferred three azine groups.

According to an embodiment of the present invention, the electron transport matrix compound comprises at least one group selected from the list consisting of pyridine, pyrimidine, triazine, imidazole, benzimidazole, benzoxazole, quinone, benzoquinone, imidazo[1,5-*a*]pyridine, quinoxaline, benzoquinoxaline, acridine, phenanthroline, benzoacridine, dibenzoacridine phosphine oxide, terpyridine.

According to an embodiment of the present invention, the electron transport matrix compound comprises at least one phenanthroline group, preferably two phenanthroline groups, one or more pyridine groups, one or more pyrimidine groups, one or more triazine groups, one or more imidazo[1,5-*a*]pyridine groups, or one or more phosphine oxide groups.

According to an embodiment of the present invention, the electron transport matrix compound comprises at least one phenanthroline group, preferably two phenanthroline groups, one or more pyridine groups, one or more pyrimidine groups, or one or more phosphine oxide groups.

According to an embodiment of the present invention, the electron transport matrix compound comprises at least one phenanthroline group, preferably two phenanthroline groups, a pyridine group, a pyrimidine groups, or a phosphine oxide group.

According to an embodiment of the present invention, the electron transport matrix compound comprises at least one phenanthroline group, preferably two phenanthroline groups, one or more pyridine groups, one or more pyrimidine groups, one or more triazine groups.

According to an embodiment of the present invention, the electron transport matrix compound is selected from the group comprising 2,2'-(1,3-Phenylene)bis[9-phenyl-1,10-phenanthroline], (3-(10-(3-(2,6-diphenylpyrimidin-4-yl)phenyl)anthracen-9-yl)phenyl)dimethylphosphine oxide, 3-(3-(9,10-diphenylanthracen-2-yl)phenyl)-1-(pyridin-2-yl)imidazo [1,5-a]pyridine, 7-(3-(1,10-phenanthrolin-2-yl)phenyl)dibenzo[c,h]acridine, 7-(3-([2,2':6',2''-terpyridin]-4'-yl)phenyl)dibenzo[c,h]acridine, 4'-(4'-(4,6-diphenyl-1,3,5-triazin-2-yl)-[1,1'-biphenyl]-4-yl)-2,2':6',2''-terpyridine, 4'-(4-(fluoranthen-3-yl)phenyl)-2,2':6',2''-terpyridine, or 3-(9,10-di-2-naphthalenyl-2-anthracenyl)phenyl] dimethyl phosphine oxide.

According to an embodiment of the present invention, the electron transport matrix compound comprises at least one phenanthroline group, preferably two phenanthroline groups.

According to an embodiment, the metal is a metal dopant.

According to an embodiment, the metal is selected from a metal with an electronegativity of ≤ 1.4 eV by Pauling scale or a metal alloy comprising a metal with an electronegativity of ≤ 1.4 eV by Pauling scale.

According to an embodiment, the metal is selected from a metal with an electronegativity of ≤ 1.35 eV by Pauling scale or a metal alloy comprising a metal with an electronegativity of ≤ 1.35 eV by Pauling scale.

According to an embodiment, the metal is selected from the group consisting of Li, Na, K, Rb, Cs, Mg, Ca, Sr, Ba, Sm, Eu and Yb or a metal alloy comprising a metal selected from the group consisting of Li, Na, K, Rb, Cs, Mg, Ca, Sr, Ba, Sm, Eu and Yb.

According to an embodiment, the metal is selected from the group consisting of Li, Na, K, Cs, Mg, Ca, Ba, Sm, Eu and Yb or a metal alloy comprising a metal selected from the group consisting of Li, Na, K, Cs, Mg, Ca, Ba, Sm, Eu and Yb.

According to an embodiment, the metal is a metal selected from the group consisting of Li, Mg and Yb or a metal alloy comprising a metal selected from the group consisting of Li, Mg and Yb.

According to an embodiment, the metal is selected from the group consisting of Li and Yb or a metal alloy comprising a metal selected from the group consisting of Li, and Yb.

According to an embodiment, the metal is Yb or a metal alloy comprising a metal selected from the group consisting of Li and Yb.

According to an embodiment, the metal is Yb.

According to an embodiment, the metal is present in the n-type charge generation layer in an amount of ≤ 99.9 vol% based on the total volume of the layer, preferably ≤ 99 vol%, more preferably ≤ 95 vol%, more preferably ≤ 90 vol%, more preferably ≤80 vol%, more preferably ≤ 70 vol%, more preferably ≤ 60 vol% , more preferably ≤ 50 vol%, more preferably ≤ 40 vol%, more preferably ≤ 30 vol%, more preferably ≤ 20 vol%, more preferably ≤ 10 vol%, more preferably ≤ 5 vol% , more preferably ≤ 3.0 vol%, more preferably ≤ 2 vol%, more preferably ≤ 1.5 vol%, more preferably ≤ 1.25 vol%, and most preferably ≤ 1.0 vol%.

According to an embodiment, the electron transport matrix compound is present in the n-type charge generation

layer in an amount of ≥ 0.1 vol% based on the total volume of the layer, preferably ≥ 1 vol%, more preferably ≥ 5 vol%, more preferably ≥ 10 vol%, more preferably ≥ 20 vol%, more preferably ≥3 0 vol%, more preferably ≥ 40 vol%, more preferably ≥ 50 vol%, more preferably ≥ 60 vol%, more preferably ≥ 70 vol%, more preferably ≥ 80 vol%, more preferably ≥ 90 vol%, more preferably ≥ 95 vol%, more preferably ≥ 97.0 vol%, more preferably ≥ 98 vol%, more preferably ≥ 98.25 vol%, more preferably ≥ 98.5 vol%, more preferably ≥ 98.75 vol% and most preferably ≥ 99.0 vol%.

According to an embodiment, the metal is present in the n-type charge generation layer in an amount of ≤ 99.9 vol% based on the total volume of the layer, preferably ≤ 99 vol%, more preferably ≤ 95 vol%, more preferably ≤ 90 vol%, more preferably ≤80 vol%, more preferably ≤ 70 vol%, more preferably ≤ 60 vol% , more preferably ≤ 50 vol%, more preferably ≤ 40 vol%, more preferably ≤ 30 vol%, more preferably ≤ 20 vol%, more preferably ≤ 10 vol%, more preferably ≤ 5 vol% , more preferably ≤ 3.0 vol%, more preferably ≤ 2 vol%, more preferably ≤ 1.5 vol%, more preferably ≤ 1.25 vol%, and most preferably ≤ 1.0 vol%, and electron transport matrix compound is present in the n-type charge generation layer in an amount of ≥ 0.1 vol% based on the total volume of the layer, preferably ≥ 1 vol%, more preferably ≥ 5 vol%, more preferably ≥ 10 vol%, more preferably ≥ 20 vol%, more preferably ≥3 0 vol%, more preferably ≥ 40 vol%, more preferably ≥ 50 vol%, more preferably ≥ 60 vol%, more preferably ≥ 70 vol%, more preferably ≥ 80 vol%, more preferably ≥ 90 vol%, more preferably ≥ 95 vol%, more preferably ≥ 97.0 vol%, more preferably ≥ 98 vol%, more preferably ≥ 98.25 vol%, more preferably ≥ 98.5 vol%, more preferably ≥ 98.75 vol% and most preferably ≥ 99.0 vol%.

According to one embodiment of the present invention, the organic electronic device further comprises at least one photoactive layer, wherein the at least one photoactive layer is arranged between the anode layer and the cathode layer.

According to one embodiment of the present invention, the photoactive layer is a light emitting layer.

According to one embodiment of the present invention, the organic electronic device further comprises a charge generation layer, wherein the charge generation layer comprises a p-type charge generation layer and a n-type charge generation layer, wherein the p-type charge generation layer is an organic semiconductor layer according to the present invention.

According to one embodiment of the present invention, the charge generation layer is arranges between the photoactive layer and the cathode layer.

According to one embodiment of the present invention, the organic electronic device is an electroluminescent device, preferably an organic light emitting diode.

According to one embodiment of the present invention, the organic electronic device further comprises a substrate.

According to one embodiment of the present invention, the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide; and
- the second anode sub-layer is arranged closer to the hole injection layer.

According to one embodiment of the present invention, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide or indium zinc oxide, more preferred indium tin oxide.

According to one embodiment of the present invention, the second anode sub-layer may has a thickness in the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to one embodiment of the present invention, anode layer of the organic electronic device comprises in addition a third anode sub-layer comprising a transparent conductive oxide, wherein the third anode sub-layer is arranged between the substrate and the first anode sub-layer.

According to one embodiment of the present invention, the third anode sub-layer comprises a transparent oxide, preferably from the group selected from the group comprising indium tin oxide or indium zinc oxide, more preferred indium tin oxide.

According to one embodiment of the present invention, the third anode sub-layer may has a thickness in the

range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the anode layer comprises a first anode sub-layer comprising of Ag, a second anode sub-layer comprising of transparent conductive oxide, preferably ITO, and a third anode sub-layer comprising of transparent conductive oxide, preferably ITO; wherein the first anode sub-layer is arranged between the second and the third anode sub-layer.

According to one embodiment of the present invention, the hole injection layer is in direct contact with the anode layer.

According to one embodiment of the present invention, the hole injection layer is in direct contact with the anode layer and the anode layer is in direct contact with the substrate, wherein the substrate is selected from a glass substrate, a plastic substrate, a metal substrate or a backplane.

The present invention furthermore relates to a display device comprising an organic electronic device according to the present invention.

## Further layers

In accordance with the invention, the organic electronic device may comprise, besides the layers already mentioned above, further layers. Exemplary embodiments of respective layers are described in the following:

### *Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a backplane.

### *Anode layer*

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

### *Hole transport layer*

According to one embodiment of the present invention, the organic electronic device comprises a hole transport layer, wherein the hole transport layer is arranged between the hole injection layer and the at least one first emission layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise a substantially covalent matrix compound as described above.

According to one embodiment of the present invention, the hole transport layer may comprise a compound of formula (VI) or (VII) as described above.

According to one embodiment of the present invention, the hole injection layer and the hole transport layer

comprises the same substantially covalent matrix compound as described above.

According to one embodiment of the present invention, the hole injection layer and the hole transport layer comprises the same compound of formula (VI) or (VII) as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EMI,.

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4''-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp) 3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

*Electron transport layer (ETL)*

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIl, is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

*Electron injection layer (EIL)*

An optional EIL, which may facilitates injection of electrons from the cathode, may be formed on the ETL, preferably directly on the electron transport layer. Examples of materials for forming the EIl, include lithium 8-hydroxyquinolinolate (LiQ), LiF, NaCl, CsF, Li2O, BaO, Ca, Ba, Yb, Mg which are known in the art. Deposition and coating conditions for forming the EIl, are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIl, is within this range, the EIl, may have satisfactory electron-injecting properties, without a substantial penalty in driving voltage.

*Cathode layer*

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

According to a preferred embodiment of the present invention, the cathode is transparent.

It is to be understood that the cathode layer is not part of an electron injection layer or the electron transport layer.

*Organic light-emitting diode (OLED)*

The organic electronic device according to the invention may be an organic light-emitting device.

According to one aspect of the present invention, there is provided an organic light-emitting diode (OLED) comprising: a substrate; an anode electrode formed on the substrate; a hole injection layer comprising a compound of formula (I), a hole transport layer, an emission layer, an electron transport layer and a cathode electrode.

According to another aspect of the present invention, there is provided an OLED comprising: a substrate; an anode electrode formed on the substrate; a hole injection layer comprising a compound of formula (I), a hole transport layer, an electron blocking layer, an emission layer, a hole blocking layer, an electron transport layer and a cathode

electrode.

According to another aspect of the present invention, there is provided an OLED comprising: a substrate; an anode electrode formed on the substrate; a hole injection layer comprising a compound of formula (I), a hole transport layer, an electron blocking layer, an emission layer, a hole blocking layer, an electron transport layer, an electron injection layer, and a cathode electrode.

According to various embodiments of the present invention, there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The organic semiconductor layer according to the invention may be the first hole injection layer and/or the p-type charge generation layer.

Organic electronic device

The organic electronic device according to the invention may be a light emitting device, or a photovoltaic cell, and preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of formula (I) according to the invention, and
- a second deposition source to release the substantially covalent matrix compound;

the method comprising the steps of forming the hole injection layer and/or p-type charge generation layer; whereby for an organic light-emitting diode (OLED):

- the hole injection layer and/or p-type charge generation layer is formed by releasing the compound of formula (I) according to the invention from the first deposition source and the substantially covalent matrix compound from the second deposition source.

According to various embodiments of the present invention, the method may further include forming on the anode electrode, at least one layer selected from the group consisting of forming a hole transport layer or forming a hole blocking layer, and an emission layer between the anode electrode and the first electron transport layer.

According to various embodiments of the present invention, the method may further include the steps for forming an organic light-emitting diode (OLED), wherein

- on a substrate an anode electrode is formed,
- on the anode electrode a hole injection layer comprising a compound of formula (I) is formed,
- on the hole injection layer comprising a compound of formula (I) a hole transport layer is formed,
- on the hole transport layer an emission layer is formed,

- on the emission layer an electron transport layer is formed, optionally a hole blocking layer is formed on the emission layer,
- and finally a cathode electrode is formed,
- optional a hole blocking layer is formed in that order between the first anode electrode and the emission layer,
- optional an electron injection layer is formed between the electron transport layer and the cathode electrode.

According to various embodiments, the OLED may have the following layer structure, wherein the layers having the following order:
anode, hole injection layer comprising a compound of formula (I) according to the invention, first hole transport layer, second hole transport layer, emission layer, optional hole blocking layer, electron transport layer, optional electron injection layer, and cathode.
According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.
Hereinafter, the embodiments are illustrated in more detail with reference to examples. However, the present disclosure is not limited to the following examples. Reference will now be made in detail to the exemplary aspects.

**Description of the Drawings**

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.
Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

Fig. 1 is a schematic sectional view of an OLED comprising an organic semiconductor layer, according to an exemplary embodiment of the present invention.
Fig. 2 is a schematic sectional view of a stacked OLED 100 comprising a hole injection layer as organic semiconductor layer, according to another exemplary embodiment of the present invention.
Fig. 3 is a schematic sectional view of a stacked OLED 100 comprising a hole injection layer as organic semiconductor layer, according to another exemplary embodiment of the present invention.
Fig. 4 is a schematic sectional view of an OLED comprising an organic semiconductor layer which is hole injection layer and an organic semiconductor layer which is charge generation layer, according to an exemplary embodiment of the present invention.
Fig. 5 is a schematic sectional view of an OLED comprising a charge generation layer, according to an exemplary embodiment of the present invention.
Fig. 6 is a schematic sectional view of a stacked OLED comprising a charge generation layer, according to an exemplary embodiment of the present invention.

Hereinafter, the figures are illustrated in more detail with reference to examples. However, the present disclosure is not limited to the following figures.
Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.
Fig. 1 is a schematic sectional view of an OLED 100, according to one exemplary embodiment of the present invention.
Referring to Fig. 1 the OLED 100 includes a substrate 110, an anode layer 120, an organic semiconductor layer 125, and a cathode layer 190. The organic semiconductor layer may comprise a compound of Formula (I).
Fig. 2 is a schematic sectional view of a stacked OLED 100, according to another exemplary embodiment of the present invention.
Referring to Fig. 2 the OLED 100 includes a substrate 110, an anode layer 120, a hole injection layer (HIL) 130

which may comprise a compound of Formula (I), a first hole transport layer (HTL) 140, a first emission layer (EML) 150, and a cathode layer 190.

Fig. 3 is a schematic sectional view of a stacked OLED 100, according to another exemplary embodiment of the present invention.

Referring to Fig. 3 the OLED 100 includes a substrate 110, an anode layer 120, a hole injection layer (HIL) 130 which may comprise a compound of Formula (I), a first hole transport layer (HTL) 140, a first electron blocking layer (EBL) 145, a first emission layer (EML) 150, a first hole blocking layer (HBL) 155, a first electron transport layer (ETL) 160, an electron injection layer (EIL) 180 and a cathode layer 190.

Fig. 4 is a schematic sectional view of an OLED 100, according to one exemplary embodiment of the present invention.

Referring to Fig. 4 the OLED 100 includes a substrate 110, an anode layer 120, a hole injection layer (HIL) 130 which may comprise a compound of Formula (I), an emission layer (EML) 150, an n-type charge generation layer (n-CGL) 185, a p-type charge generation layer (p-GCL) 135 which may comprise a compound of formula (I), a second emission layer 151 and a cathode layer 190.

Fig. 5 is a schematic sectional view of an OLED 100, according to one exemplary embodiment of the present invention.

Referring to Fig. 5 the OLED 100 includes a substrate 110, an anode layer 120, a hole injection layer (HIL) 130, a first hole transport layer (HTL1) 140, an electron blocking layer (EBL) 145, an emission layer (EML) 150, a hole blocking layer (HBL) 155, an electron transport layer (ETL) 160, an n-type charge generation layer (n-CGL) 185, a p-type charge generation layer (p-GCL) 135 which may comprise a compound of formula (I), a second hole transport layer (HTL2) 141, and electron injection layer (EIL) 180 and a cathode layer 190. The HIL may comprise a compound of Formula (I).

Fig. 6 is a schematic sectional view of a stacked OLED 100, according to another exemplary embodiment of the present invention. Fig. 6 differs from Fig. 5 in that the OLED 100 of Fig. 5 further comprises a second emission layer.

Referring to Fig. 6 the OLED 100 includes a substrate 110, an anode layer 120, a hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-CGL) 185, a p-type charge generation layer (p-GCL) 135 which may comprise compound of Formula (I), a second hole transport layer (HTL) 141, a second electron blocking layer (EBL) 146, a second emission layer (EML) 151, a second hole blocking layer (EBL) 156, a second electron transport layer (ETL) 161, an electron injection layer (EIL) 180 and a cathode layer 190. The HIL may comprise a compound of Formula (I).

In the description above the method of manufacture an OLED 100 of the present invention is started with a substrate 110 onto which an anode layer 120 is formed, on the anode layer 120, a hole injection layer 130, a first hole transport layer 140, optional a first electron blocking layer 145, a first emission layer 150, optional a first hole blocking layer 155, optional at least one first electron transport layer 160, an n-CGL 185, a p-CGL 135, a second hole transport layer 141, optional a second electron blocking layer 146, a second emission layer 151, an optional second hole blocking layer 156, an optional at least one second electron transport layer 161, an optional electron injection layer (EIL) 180 and a cathode layer 190 are formed, in that order or the other way around.

While not shown in Fig. 1 to 6, a sealing and/or capping layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

**Detailed description**

The invention is furthermore illustrated by the following examples which are illustrative only and non-binding. Compounds of formula (I) may be prepared as described in EP2180029A1 and WO2016097017A1. Additional synthetic procedures for selected precursors to be used for preparing compounds according to the present invention are inter alia the following:

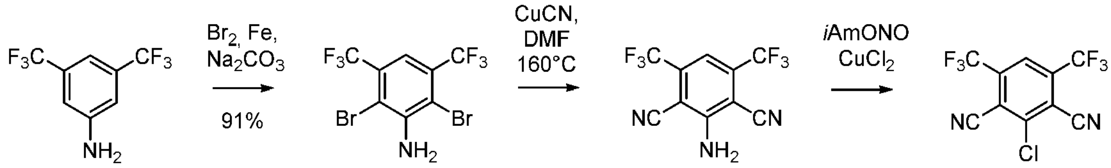


Intermediate 1a Intermediate 2a Intermediate 3a

Intermediate 1a

Bromine (36 ml, 112 g, 1.42 mol) in dichloromethane (DCM) (250 ml) was added portionwise to a stirred solution of 3,5-bis(trifluoromethyl)aniline (50 g, 214 mmol), sodium carbonate (25 g, 235 mmol) and iron powder (500 mg, 9.0 mmol) in DCM (250 ml). The mixture was refluxed 24 h. After cooling, DCM (250 ml) was added, and the solution was washed with 10% $Na_2S_2O_3 \cdot 5H_2O$ (2 x 200 ml) and brine (200 ml), dried over $MgSO_4$ and concentrated in vacuo to afford the title compound as a pale-yellow solid (75 g, 91 %) which was purified by vacuum distillation, or used for the next step without further purification.
M.p. 52-53°C. B.p. 105°C / 1 mmHg.

Intermediate 2a

2,6-Dibromo-3,5-bis(trifluoromethyl)aniline (20 g, 52 mmol) was added to the copper cyanide (20 g, 223 mmol) in N,N-dimethylformamide (DMF) (100 ml) and heated while stirring for 12 h at 160°C. After cooling, the reaction mixture was poured into saturated sodium carbonate solution (300 ml), the obtained precipitate filtered and washed on filter with diethyl ether (3 x 150 ml). The obtained water-ethereal filtrate was extracted with additional amount of diethyl ether (3 x 150 ml), washed with brine (200 ml), dried over magnesium sulfate, filtrated, and evaporated. Dichloromethane was added to the residue resulting in the precipitation of the product, which was then separated by filtration providing **2** in 80% purity as carrot colored solid. For additional purification this solid was sublimed (160°C/1 mmHg).
Colorless solid. 156-158°C.

Intermediate 3a

A three neck round bottom flask with $CuCl_2$ (3 g, 22 mmol) equipped with addition funnel and gas outlet tube was flame dried in vacuum and filled with argon (commercially available $CuCl_2$ vigorously "boils" releasing water). A thermometer was set up, stir bar dropped. 2-amino-4,6-bis(trifluoromethyl)isophthalonitrile **2** (4 g, 14 mmol) and acetonitrile (30 ml) were added. The obtained suspension was heated up to 80 °C under argon atmosphere and isoamyl nitrite (i-AmONO) (2.52 g, 3.0 ml, 22 mmol) was added dropwise within 3 minutes. The reaction mixture was heated at this temperature for 1.5 h, then cooled to room temperature, poured into diethyl ether and washed with brine (3 x 200 ml). The organic layer was dried over $MgSO_4$, filtrated, and evaporated. Column chromatography gave the product in form of colorless crystals (2.5 g, 59 %). Colorless crystals, m.p. 117-119 °C.

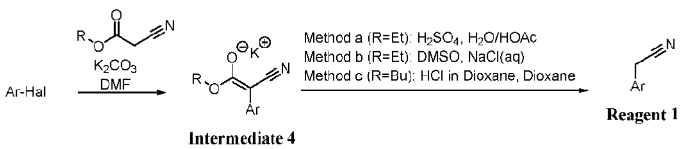


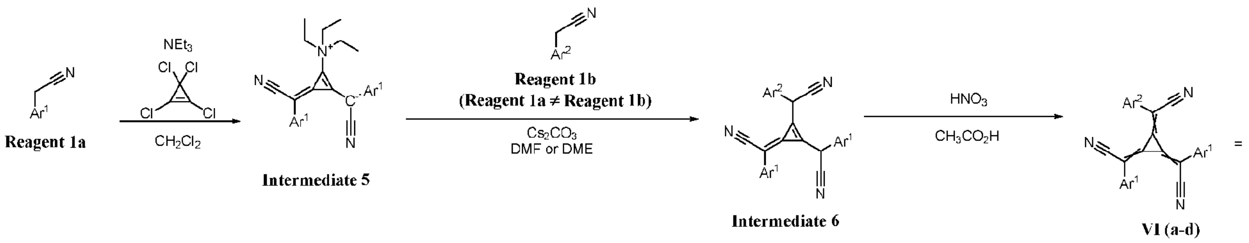


## General Procedures

Intermediate 4

To a solution of 1g aryl halogenide (e.g. intermediate 3a) dissolved in 10 mL DMF 1.2 equivalents potassium carbonate were added. After dropwise addition of 1.2 equivalents of Ethyl- or t-Butylcyanoacetate, the mixture was stirred for 3 days at 50°C. Solid by-products were removed by filtration and the filtrate was evaporated to dryness. The crude product was triturated with DCM (2 hours, rt) twice, the precipitated product was collected on a frit and washed with DCM three times. The final product was vacuum dried over night.

Reagent 1 (Method a: R=Ethyl)

To a solution of 1g of intermediate 4 in 3mL acetic acid (50%) 1.5 equivalents sulphuric acid (conc.) were added. The mixture was heated to reflux (130 °C bath temp.) for 1-3 days. After cooling to room temperature the mixture was poured into 10mL of ice water and stirred over a period of 30 min followed by addition of 10mL ethyl acetate. The aqueous phase was separated and washed with ethyl acetate three times. After drying the combined organic phases with sodium sulphate, the solvent was evaporated to dryness. The crude product was vacuum dried over night at ambient temperature and was purified by vacuum distillation.

Reagent 1 (Method b: R=Ethyl)

1 g of intermediate 4 was dissolved in 10 mL water and triturated with acetic acid until the solution was at pH=3. After addition of 5 mL ethyl acetate, the aqueous phase was extracted with 5 mL ethyl acetate twice. After drying the combined organic phases with sodium sulphate, the solvent was evaporated to dryness. The remaining oil was dissolved in 10 mL dimethylsulfoxid (DMSO) under inert gas atmosphere and 5 mL brine were added. The mixture was stirred under gentle reflux for 3-24 hours. After cooling to room temperature, 15 mL water and 25 mL ethyl acetate were added. The aqueous phase was separated and washed with ethyl acetate two times. After extracting with brine twice and drying the combined organic phases with sodium sulphate, the solvent was evaporated to dryness. The crude product was vacuum dried over night at ambient temperature and was purified by vacuum distillation.

Reagent 1 (Method c: R=*t*-Butyl)

5 g of intermediate 1 were dissolved in 10 mL dioxane in a pressure tube and 2 equivalents of a 4 M solution of hydrogen chloride in dioxane were added. The tube was sealed and stirred at 95°C for 2-4 hours. After cooling down to room temperature the mixture was poured on 40 mL water and extracted with 30 mL ethyl acetate three times. The combined organic phases were washed with 50 mL water, 50 mL 0.5 M sodium bicarbonate solution and 50 mL brine. After drying with sodium sulphate and evaporation of the solvent, the compound was purified via Kugelrohr distillation at 100-150°C and $10^{-3}$ mbar to give the final product.

Intermediate 5

A flame dried Schlenk flask was charged with 10g Reagent 1a and 0.5 equivalents tetrachlorocyclopropene and dry dichloromethane (DCM, 80 mL) were added subsequently. After cooled to -30 °C 3.2 equivalents triethyl amine were added dropwise over 30 min. The mixture was allowed to warm to room temperature over 1 h. Water (12 mL) was added dropwise and the mixture was filtered. The solid was washed with DCM three times (30 mL), methanol 2 times (30 mL) and water 4 times (30 mL). The vacuum dried crude product was recrystallized from acetonitrile.

Intermediate 6

A flame dried Schlenk flask was charged with anhydrous caesium carbonate (6 equivalents) under inert gas. The flask was cooled on ice and dry DMF (8 mL) was added. The mixture was stirred on ice for 10 minutes before a solution of intermediate 5 (1.05 equivalents) in DMF (2 mL) was added dropwise. Subsequently, 1g of Reagent 1b (≠ Reagent 1a) was added. After 20 minutes stirring on an ice bath, the cooling bath was removed and the mixture was allowed to warm to room temperature. The reaction was monitored via TLC (DCM/MeOH v:v 4:1) and stirring was continued until intermediate was visible anymore (usually 1-2 days). The base was filtered off and washed with t-Butylacetate (40 mL). The combined organic phases were washed with half-concentrated calcium chloride solution (3x 30 mL) dried over sodium sulfate and the solvent was removed.

This intermediate was not further purified, it was directly used in the last step.

Compound VI

Intermediate 6 (1g) was dissolved in glacial acetic acid (10 mL) added dropwise to aqueous nitric acid (65 % w/w, 13 mL and 3 mL acetic acid) stirring at 0°C. The solution turned from black/green to red/orange. After stirring for 30 minutes at 0°C, the solution was allowed to warm to room temperature and was stirred for additional 1-4 h. The crude product was precipitated by adding 10 mL of water and the mixture was stirred for 15 min. Filtration gave an orange solid, which was washed with cold water until the filtrate was neutral.

Work-up and purification of VI

The crude product was dissolved in DCM and washed with water two times to get rid of remaining acid. In some cases the product was partially insoluble in DCM. The insoluble fraction was filtered off has a satisfying purity to be sublimed

The soluble fraction was concentrated by evaporation and precipitated from an excess cyclohexane, filtered over a glass frit and dried in oil pump vacuum.

HOMO and LUMP calculations

The HOMO and LUMO are calculated with the program package ORCA V5.0.3 (Max Planck Institute für Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The dipole moment, the HOMO and LUMO energy levels of the molecular structures are determined by applying the hybrid functional B3LYP with a 6-31G* basis set from the optimized geometries obtained by applying the hybrid functional B3LYP with a 6-31G* basis set in the gas phase. All the calculations were performed in the gas phase. If more than one conformation is viable, the conformation with the lowest total energy is selected.

*Glass transition temperature*

The glass transition temperature (Tg) is measured under nitrogen and using a heating rate of 10 K per min in a Mettler Toledo DSC 822e differential scanning calorimeter as described in DIN EN ISO 11357, published in March 2010.

*Melting point (mp)*

Melting point (mp) temperatures were measured by DSC at heating rate 10 K/min, the reported values correspond the peak temperature for the observed melting endotherm on the DSC curve.

*Thermogravimetric analysis*

The term "TGA5%" denotes the temperature at which 5 % weight loss occurs during thermogravimetric analysis and is measured in °C.

The TGA5% value may be determined by heating a 9-11 mg sample in a thermogravimetric analyzer at a heating rate of 10 K/min in an open 100 μL aluminum pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

*UV-VIS absorption measurement in solution*

Experimental absorption spectra were recorded on a Thermo Fisher Evolution Pro UV-Vis Spectrophotometer. For sample preparation the material is weighed into an aluminum crucible, which is then inserted into a 25 mL measuring flask. The related micro balance has a mass change readability in the 1-2 μg range. The flask is then filled up to the mark with dichloromethane (Spectroscopy grade, transmission ≥90% for $\lambda \geq 248$ nm according to manufacturer) and shaken until the material is completely dissolved, yielding a solution with a concentration of 10-4 - 10-5 mol/L. For measurement the solution is put into a standard cuvette (Hellma 110-QS: quartz, d=10 mm, with PTFE stopper). The spectrum is recorded at a slit width of 1 nm at a sampling interval of 1 nm at an ambient temperature of 20 °C. From all spectra the background absorption of the pure solvent is subtracted which was measured immediately prior to the measurement using the same measurement conditions.

*Optical absorptance measurement of organic semiconductor layer*

Mixed films of N-([1,1'-biphenyl]-2-yl)-N-(9,9-dimethyl-9H-fluoren-2-yl)-9,9'-spirobi[fluoren]-2-amine and a p-dopant according to Table 2 with a thickness of 35 nm on quartz substrates (EN08, ≥ 99.98% SiO2, GVB GmbH) are prepared by thermal evaporation in a vacuum system (Cluster Tool, Sunic System Ltd.) at a deposition rate of 1 Å/s and a pressure of approximately 3e-7 mbar. The samples are stored in glovebox with pure nitrogen atmosphere until the measurement takes place (maximum 1 hour of air exposure). Reflectance and transmittance are measured using a

Filmetrics F10-RT Spectrometer with a spectral range of 380 nm to 1050 nm. An empty quartz substrate is used for reflectance standard. Absorptance is automatically calculated by subtracting reflectance and transmittance values from 100%..

*Calculated absorption area or maximal absorption*

All the calculations were performed with the program package ORCA Version 5.0.3 (Department of theory and spectroscopy, Max Planck Institute für Kohlenforschung Kaiser Wilhelm Platz 1, 45470 Muelheim/Ruhr, Germany). LUMO and HOMO energies and the resulting HOMO-LUMO-gap were calculated using the hybrid functional B3LYP with a 6-31G* basis set in the gas phase. The thus obtained optimized geometries were used to run TDDFT calculations applying the hybrid functional PBF0 with a def2-SVP basis set in the gas phase and including the first 30 singlet transitions. The calculated singlet transitions were used to calculate the absorption spectra by applying a Gaussian fit ($\lambda$ = 215-850 nm, SD=20, 200 sampling points) from which transitions below 350 nm were excluded.
To calculate the general absorption in a relevant wavelength area ($\lambda$ = 400-650 nm, blue and green emission) the integral below the calculated UV TDDFT spectrum was determined.

*Calculated bond dissociation energy (BDE)*

All the calculations were performed with the program package ORCA Version 5.0.3 (Department of theory and spectroscopy, Max Planck Institute für Kohlenforschung Kaiser Wilhelm Platz 1, 45470 Muelheim/Ruhr, Germany).
Homolytic Bond dissociation Energies (BDE), the amount of energy needed to break apart one mole of covalently bonded gases into a pair of radicals, were calculated accordingly a reported procedure (J. Phys. Chem. A, 1999, 103, 11, 1653-1661).
Molecular geometries were optimized with the DFT functional BP86 and the Def2-SVP basis set in the gas phase, if more than one conformer was available, we selected the conformer at lower energy. The optimized geometries were identified as minima by frequency analysis.
From the optimized geometry, $\Delta G^{(BP86/Def2SVP)}$ and Electronic Energy$^{(BP86/Def2SVP)}$ were obtained at the same level of geometry. In a second calculation the Electronic Energy$^{(B3LYP\ /Def2TZVP)}$, was obtained from a single point calculation performed with the DFT functional B3LYP and the Def2-TZVP basis set in the gas phase.
Energy corrected values were obtained as:

$$(\Delta G^{(BP86/Def2SVP)} - \text{Electronic Energy}^{(BP86/Def2SVP)}) + \text{Electronic Energy}^{(B3LYP\ /Def2TZVP)}.$$

General procedure for fabrication of OLEDs

**Preparation of an OLED device containing a hole injection layer comprising a compound of the invention**

For the present invention the following setup for OLEDs having a hole injection layer and one emission layer may be used.
A glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment, see Table 2, to prepare the anode layer. The plasma treatment was performed in an atmosphere comprising 97.6 vol.-% nitrogen and 2.4 vol.-% oxygen.
Then, N-([1,1'-biphenyl]-4-yl)-N-(2-(9,9-diphenyl-9H-fluoren-4-yl)phenyl)-9,9-dimethyl-9H-fluoren-2-amine [1792219-00-1] and 20 vol% of a compound of formula (I) or a comparative compound according to Table 4 were vacuum deposited on the anode, to form a HIL having a thickness of 10 nm.
Then, N-([1,1'-biphenyl]-4-yl)-N-(2-(9,9-diphenyl-9H-fluoren-4-yl)phenyl)-9,9-dimethyl-9H-fluoren-2-amine [1792219-00-1] was vacuum deposited on the HIL, to form a first HTL having a thickness of 128 nm.
Then N,N-di([1,1'-biphenyl]-4-yl)-3'-(9H-carbazol-9-yl)-[1,1'-biphenyl]-4-amine [1464822-27-2]was vacuum deposited on the HTL, to form an electron blocking layer (EBL) having a thickness of 5 nm.
Then, a first emission layer (EML1) having a thickness of 20 nm is formed on the EBL1 by co-depositing 99 vol.-% Dibenzofuran, 7-(phenyl-2,3,4,5,6-d)-1-[10-(phenyl-2,3,4,5,6-d)-9-anthracenyl] [2457172-82-4] as EML host and 1 vol.-% 5H,9H-[1]Benzothieno[2',3': 5,6][1,4]azaborino[2,3,4-kl]phenazaborine, 2,7,11-tris(1,1-dimethylethyl)-5,9-bis[4-(1,1-dimethylethyl)phenyl] [2482607-57-6] as blue dopant.
Then the hole blocking layer is formed with a thickness of 5 nm by depositing 22-(3'-(9,9-dimethyl-9H-fluoren-2-yl)-[1,1'-biphenyl]-3-yl)-4,6-diphenyl-1,3,5-triazine [1955543-57-3]onthe emission layer.

Then, the electron transporting layer (ETL) having a thickness of 31 nm is formed on the hole blocking layer by depositing 50 wt.-% 2-(2',6'-diphenyl-[1,1':4',1"-terphenyl]-4-yl)-4-phenyl-6-(3-(pyridin-4-yl)phenyl)-1,3,5-triazine [2869796-06-3] and 50 wt.-% LiQ.

Then Yb was evaporated at a rate of 0.01 to 1 Å/s at $10^{-7}$ mbar to form an electron injection layer with a thickness of 2nm on the electron transporting layer.

Ag/Mg (90:10 vol%) is evaporated at a rate of 0.01 to 1 Å/s at $10^{-7}$ mbar to form a cathode with a thickness of 13 nm.

Then, N-({[1,1-'biphenyl]-4-yl)-9,9,dimethyl-N-(4-(9-phenyl-9H-carbazol-3 -yl)phenyl)-9H-fluoren-2-amine} was vacuum deposited on the cathode layer to form a capping layer with a thickness of 75 nm.

The OLED stack is protected from ambient conditions by encapsulation of the device with a glass slide. Thereby, a cavity is formed, which includes a getter material for further protection.

**Preparation of an OLED device containing p-type charge generation comprising a compound of the invention**

For the present invention the following setup for OLEDs having a charge generation layer may be used.

A 15Ω/$cm^2$ glass substrate with 90 nm ITO (available from Corning Co.) should be cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode.

Then N-([1,1'-biphenyl]-4-yl)-N-(2-(9,9-diphenyl-9H-fluoren-4-yl)phenyl)-9,9-dimethyl-9H-fluoren-2-amine should be vacuum deposited with 20 vol% of the compound according to the invention or a comparative compound according to Table 3 to form a hole injection layer (p-HIL) having a thickness 10 nm.

Then N-([1,1'-biphenyl]-4-yl)-N-(2-(9,9-diphenyl-9H-fluoren-4-yl)phenyl)-9,9-dimethyl-9H-fluoren-2-amine should be vacuum deposited, to form a first hole transport layer having a thickness of 130 nm

Then N,N-bis(4-(dibenzo[b,d]furan-4-yl)phenyl)-[1,1':4',1"-terphenyl]-4-amine should be vacuum deposited on the HTL, to form an electron blocking layer (EBL) having a thickness of 5 nm.

Then 97 wt.-% H09 (Sun Fine Chemicals, Korea) as EML host and 3 wt.-% BD200 (Sun Fine Chemicals, Korea) as fluorescent blue dopant should be deposited on the EBL, to form a first blue-emitting emission layer (EML) with a thickness of 20 nm.

Then, a first hole blocking layer (HBL1) having a thickness of 25 nm should be formed on the first emission layer by depositing 2-(3'-(9,9-dimethyl-9H-fluoren-2-yl)-[1,1'-biphenyl]-3-yl)-4,6-diphenyl-1,3,5-triazine.

Then an n-type CGL (n-type charge generation layer) having a thickness of 15 nm should be formed on the first hole blocking layer (HBL1) by co-depositing 99 vol% 3-Phenyl-3H-benzo[b]dlnaphtho[2,1-d:1',2'-f]phosphepine-3-oxide and 1 vol% Yb.

Then a p-type CGL having a thickness of 10 nm should formed on the n-type CGL by co-depositing N-([1,1'-biphenyl]-4-yl)-N-(2-(9,9-diphenyl-9H-fluoren-4-yl)phenyl)-9,9-dimethyl-9H-fluoren-2-amine with 20 vol% of the compound according to the invention or a comparative compound according to Table 3.

Then a second hole transport layer having a thickness of 21 nm should be formed on the first p-type CGL by depositing N-([1,1'-biphenyl]-4-yl)-N-(2-(9,9-diphenyl-9H-fluoren-4-yl)phenyl)-9,9-dimethyl-9H-fluoren-2-amine.

Then a second electron blocking layer having a thickness of 5 nm should be formed on the second hole transport layer by depositing N,N-bis(4-(dibenzo[b,d]furan-4-yl)phenyl)-[1,1: 4', 1 "-terphenyl]-4-amine.

Then 97 wt.-% H09 (Sun Fine Chemicals, Korea) as EML host and 3 wt.-% BD200 (Sun Fine Chemicals, Korea) as fluorescent blue dopant should be deposited on the second EBL, to form a second blue-emitting EML with a thickness of 20 nm.

Then 2-(3'-(9,9-dimethyl-9H-fluoren-2-yl)-[1,1'-biphenyl]-3-yl)-4,6-diphenyl-1,3,5-triazine should be vacuum deposited to form a second hole blocking layer having a thickness of 10 nm is formed on the second blue-emitting EML.

Then, 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ should be vacuum deposited on the second hole blocking layer to form a second electron transport layer having a thickness of 25 nm.

Al should be evaporated at a rate of 0.01 to 1 Å/s at $10^{-7}$ mbar to form a cathode with a thickness of 100 nm.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0. 1V in the range between 0V and 10V. Likewise, the luminance-voltage characteristics and CIE coordinates are determined by measuring the luminance in cd/$m^2$ using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a

calibrated photodiode at 10 mA/cm2.

In top emission devices, the emission is forward directed, non-Lambertian and also highly dependent on the mirco-cavity. Therefore, the efficiency EQE will be higher compared to bottom emission devices. To determine the efficiency EQE in % the light output of the device is measured using a calibrated photodiode at 10 mA/cm$^2$.

Lifetime LT of the device is measured at room temperature (20°C) and 30 mA/cm$^2$, using a Keithley 2400 sourcemeter, and recorded in hours.

The brightness of the device is measured using a calibrated photo diode. The lifetime LT is defined as the time till the brightness of the device is reduced to 97 % of its initial value.

The increase in operating voltage ΔV is used as a measure of the operational voltage stability of the device. This increase is determined during the LT measurement and by subtracting the operating voltage after 1 hour after the start of operation of the device from the operating voltage after 100 hours.

$$\Delta V = [V(100\ h) - V(1h)].$$

The smaller the value of ΔV the better is the operating voltage stability.

<u>Luminous flux</u>

The samples were placed individually into an integrating sphere and the luminous flux with and without an hemispherical lense (HSL) has been measured at a current density of 10 mA/cm$^2$ using an Instrument Systems CAS-140CT array spectrometer (calibrated by Deutsche Akkreditierungsstelle (DAkkS)). As a constant current source a Keithley 2635 source measure unit has been used.

Tables 1a and 1b show selected properties (HOMO and Egap, i.e. difference in HOMO and LUMO of comparative compounds and compounds according to the invention:

**Table 1a:**

| Name | $A^1$ | $A^2$ | $A^3$ | **LUMO [eV]** | **E-Gap [eV]** | **λabs(max) 300-650 nm [nm] TDDFT** | **Absorption Area 400-650 nm TDDFT [a.u. x nm]** |
|---|---|---|---|---|---|---|---|
| CE-1 | 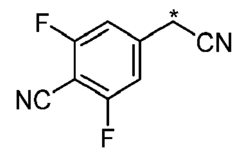 | 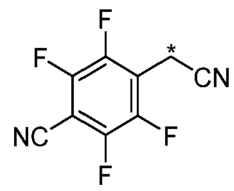 | 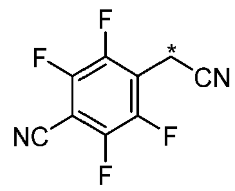 | -5.19 | 2.44 | 507 | 54.84 |
| CE-2 | 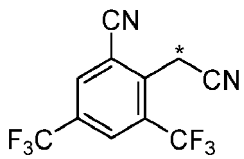 | 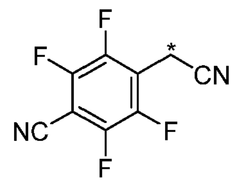 | 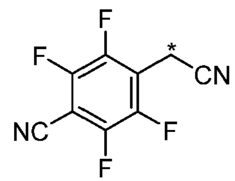 | -5.06 | 2.69 | 472 | 55.76 |
| CE-3 | 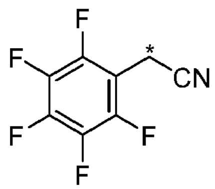 | 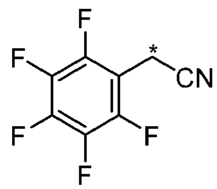 | 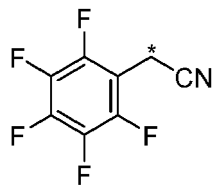 | -4.58 | 2.64 | 495 | 48.66 |

**Table 1b**

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A1 | 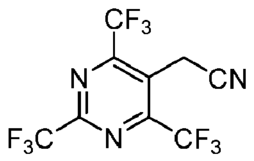 | 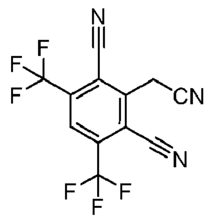 | 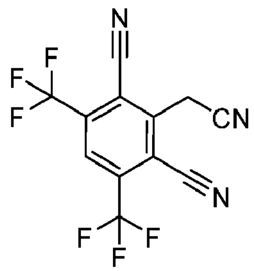 | -5.29 | 2.97 | 329 | 9.73 |
| A2 | 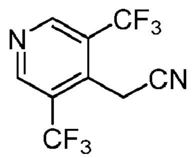 | 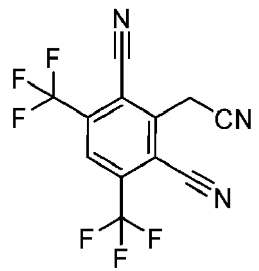 | 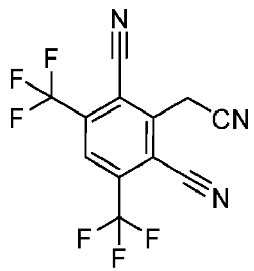 | -5.04 | 3.08 | 417 | 11.24 |
| A3 | 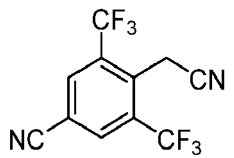 | 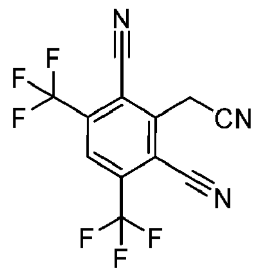 | 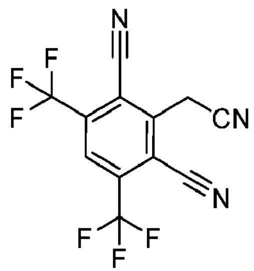 | -5.09 | 3.10 | 414 | 10.67 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A4 | 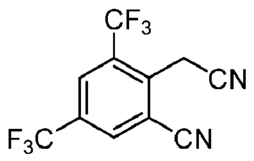 | 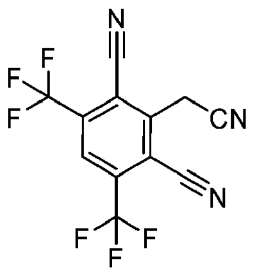 | 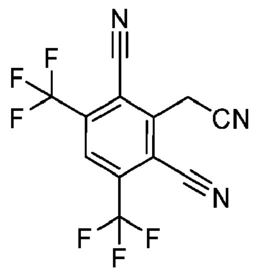 | -5.09 | 3.07 | 328 | 4.72 |
| A5 | 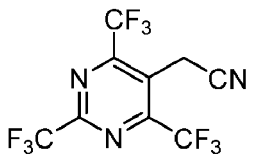 | 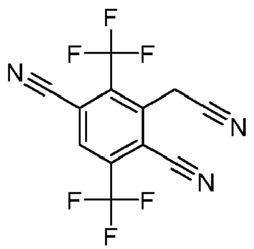 | 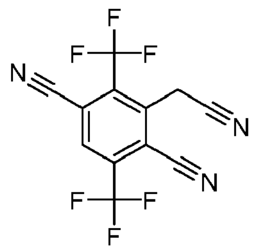 | -5.16 | 3.13 | 348 | 4.40 |
| A6 | 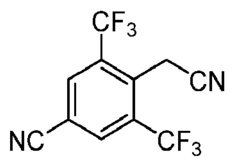 | 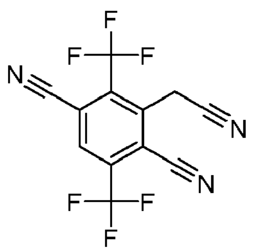 | 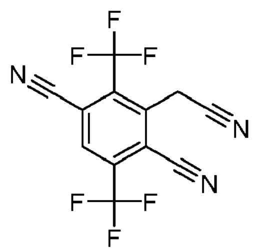 | -5.04 | 3.27 | 365 | 1.74 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A7 | 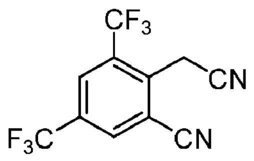 | 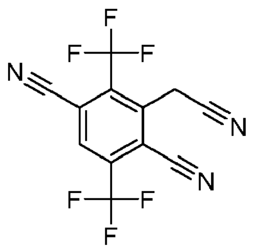 | 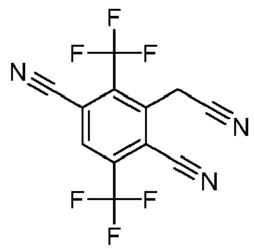 | -5.04 | 3.26 | 366 | 2.00 |
| A8 | 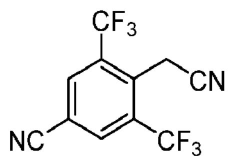 | 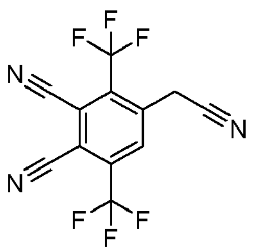 | 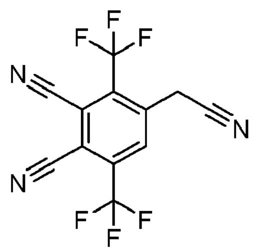 | -5.26 | 3.03 | 429 | 10.64 |
| A9 | 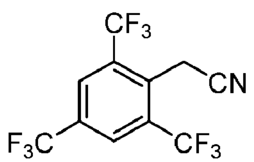 | 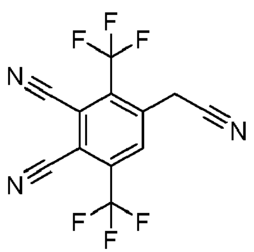 | 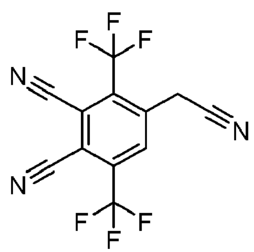 | -5.10 | 3.23 | 340 | 9.16 |
| A10 | 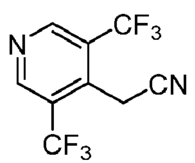 | 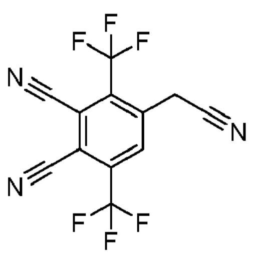 | 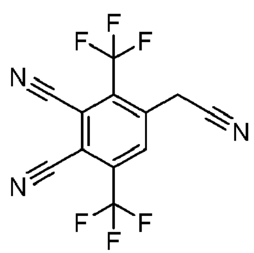 | -5.25 | 3.00 | 436 | 14.56 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A11 | 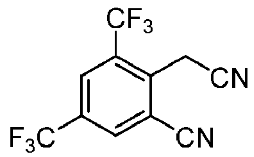 | 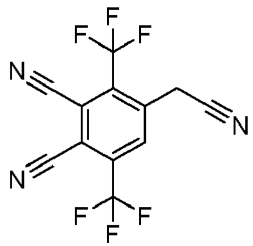 | 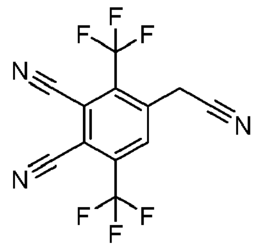 | -5.31 | 2.96 | 445 | 14.63 |
| A12 | 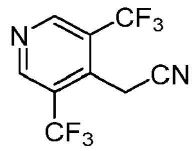 | 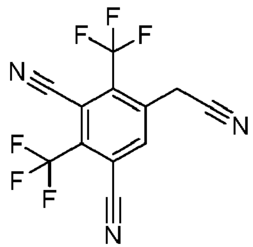 | 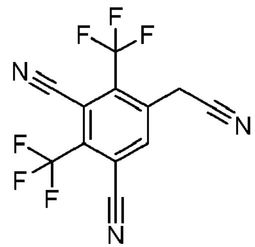 | -5.17 | 3.20 | 395 | 3.83 |
| A13 | 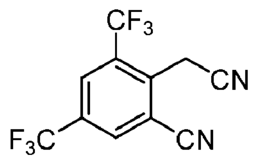 | 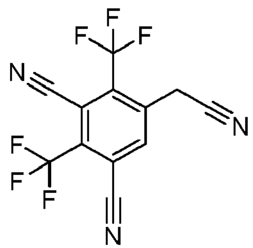 | 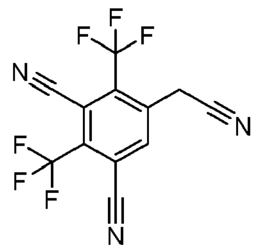 | -5.22 | 3.18 | 398 | 5.70 |
| A14 | 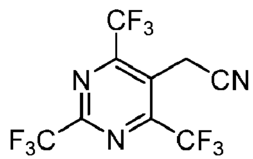 | 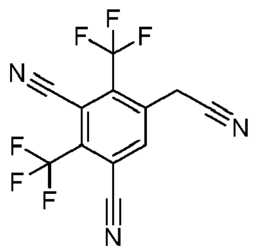 | 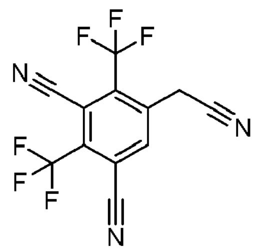 | -5.38 | 3.08 | 416 | 14.55 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A15 | 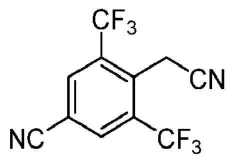 | 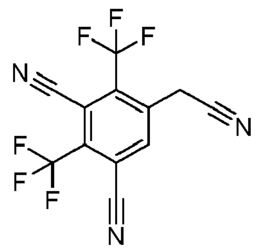 | 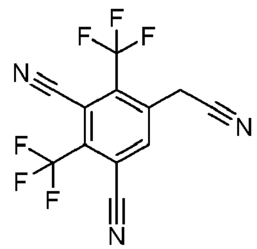 | -5.18 | 3.24 | 377 | 2.12 |
| A16 | 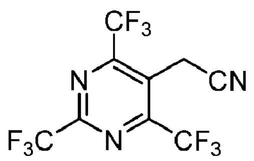 | 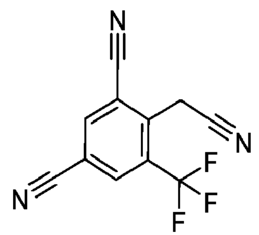 | 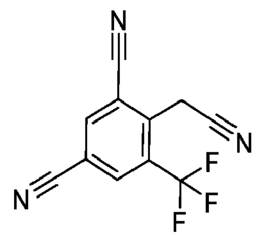 | -5.08 | 3.04 | 429 | 9.62 |
| A18 | 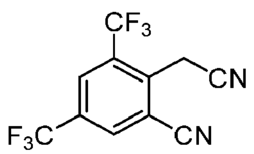 | 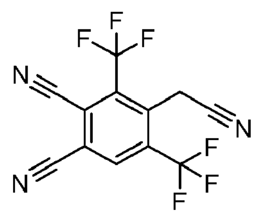 | 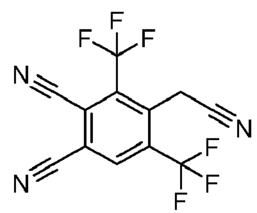 | -5.05 | 3.30 | 330 | 0.24 |
| A19 | 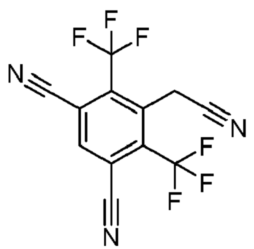 | 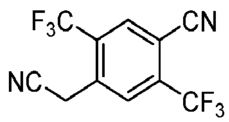 | 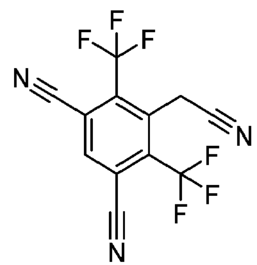 | -5.07 | 3.22 | 328 | 2.38 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A20 | 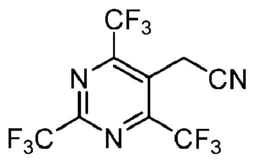 | 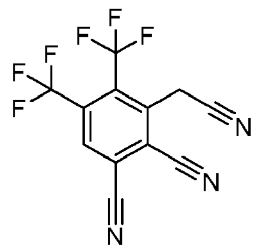 | 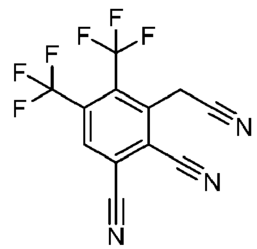 | -5.15 | 3.13 | 325 | 1.38 |
| A21 | 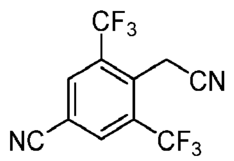 | 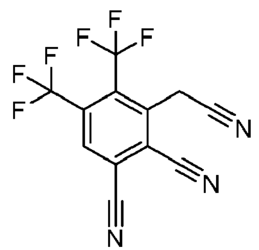 | 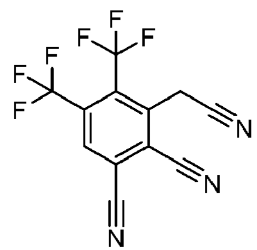 | -5.03 | 3.31 | 330 | 0.08 |
| A22 | 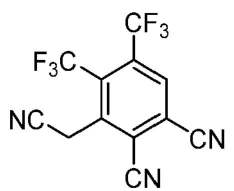 | 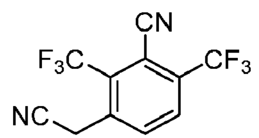 | 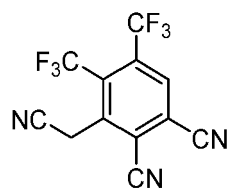 | -5.05 | 3.22 | 327 | 0.95 |
| A23 | 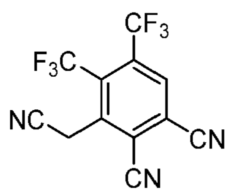 | 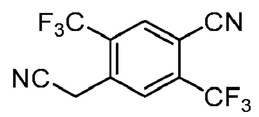 | 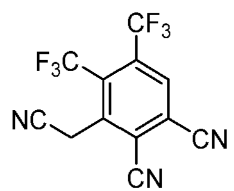 | -5.10 | 3.12 | 329 | 4.02 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A24 | 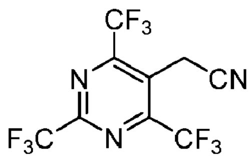 | 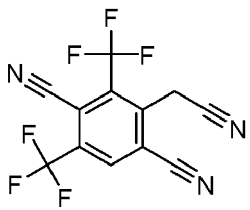 | 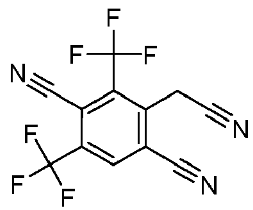 | -5.17 | 3.14 | 323 | 1.39 |
| A25 | 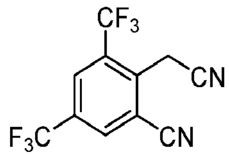 | 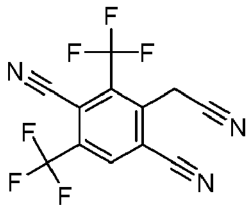 | 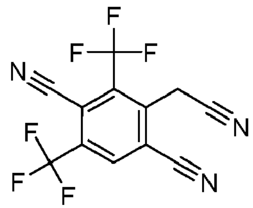 | -5.06 | 3.25 | 331 | 0.82 |
| A26 | 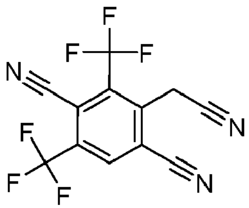 | 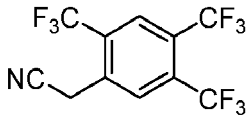 | 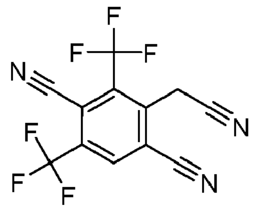 | -5.04 | 3.22 | 331 | 0.69 |
| A27 | 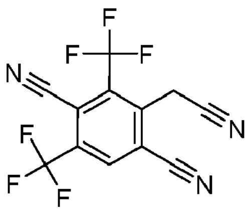 | 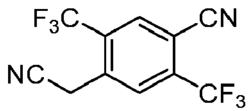 | 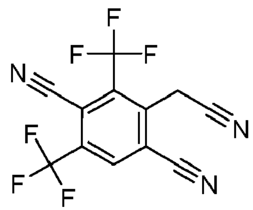 | -5.22 | 3.15 | 333 | 2.91 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A28 | 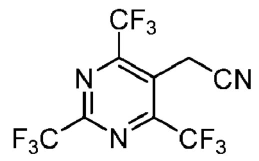 | 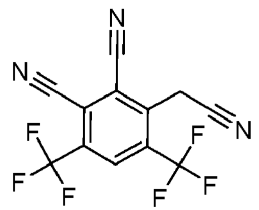 | 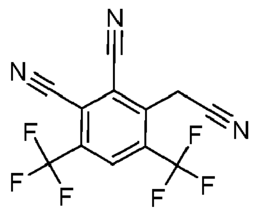 | -5.46 | 3.38 | 325 | 3.40 |
| A29 | 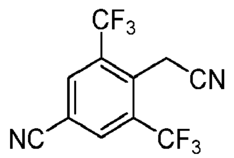 | 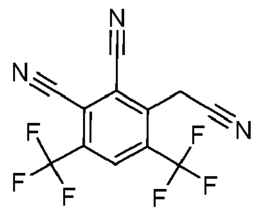 | 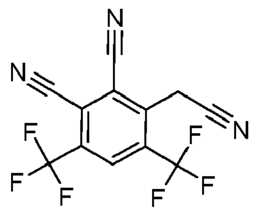 | -5.10 | 3.26 | 330 | 0.26 |
| A30 | 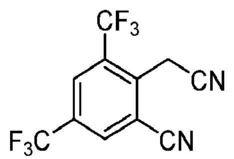 | 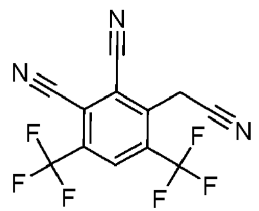 | 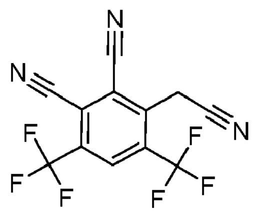 | -5.11 | 3.28 | 329 | 0.93 |
| A31 | 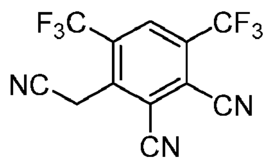 | 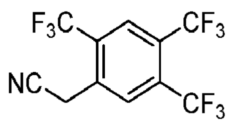 | 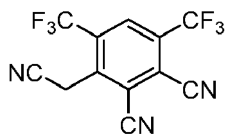 | -5.09 | 3.08 | 327 | 4.24 |
| A32 | 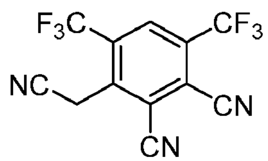 | 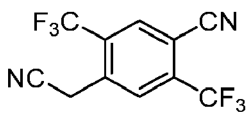 | 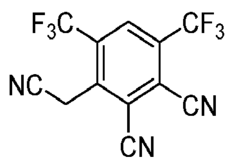 | -5.18 | 3.07 | 330 | 5.07 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A33 | 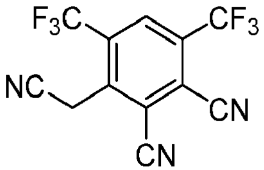 | 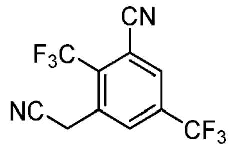 | 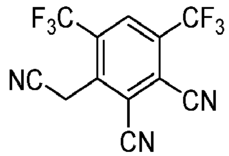 | -5.13 | 3.00 | 394 | 6.83 |
| A34 | 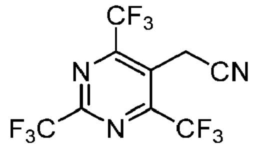 | 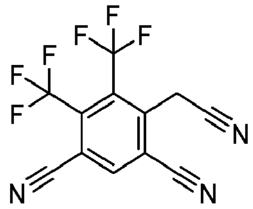 | 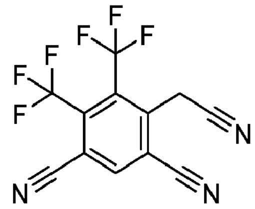 | -5.19 | 3.11 | 328 | 4.26 |
| A35 | 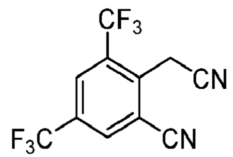 | 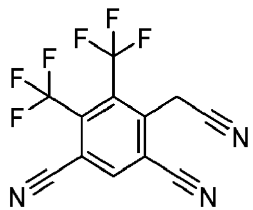 | 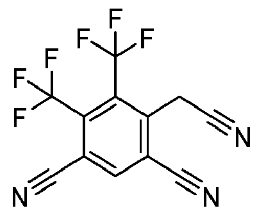 | -5.08 | 3.22 | 332 | 1.29 |
| A36 | 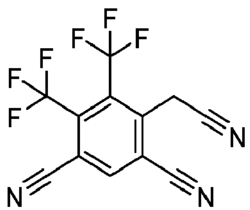 | 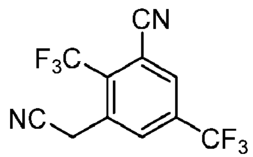 | 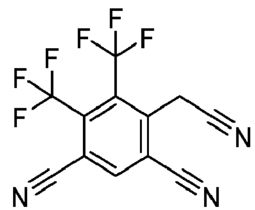 | -5.12 | 3.18 | 330 | 2.33 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A37 | 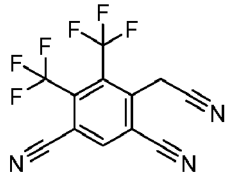 | 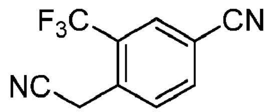 | 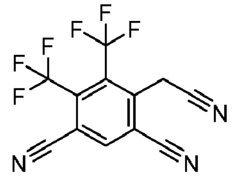 | -5.05 | 3.02 | 331 | 3.84 |
| A38 | 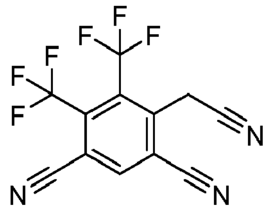 | 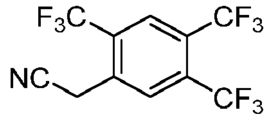 | 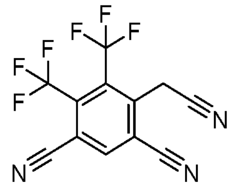 | -5.06 | 3.18 | 330 | 1.91 |
| A39 | 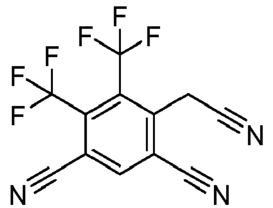 | 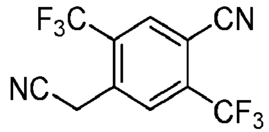 | 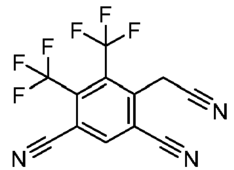 | -5.15 | 3.04 | 401 | 10.71 |
| A40 | 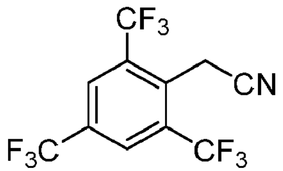 | 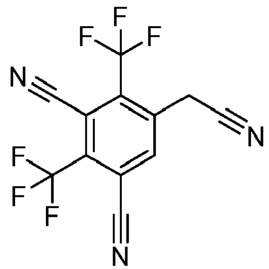 | 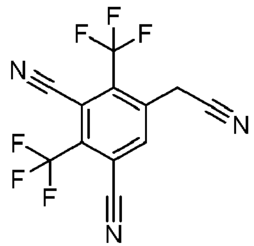 | -5.15 | 3.17 | 370 | 1.08 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A41 | 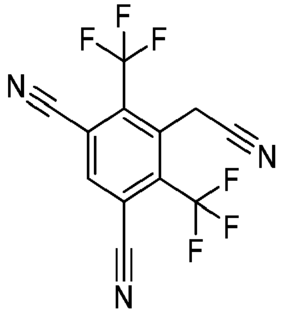 | 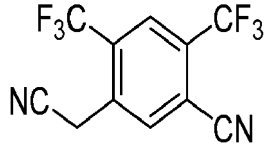 | 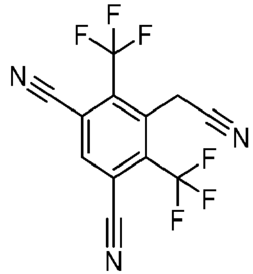 | -5.14 | 3.05 | 419 | 9.55 |
| A42 | 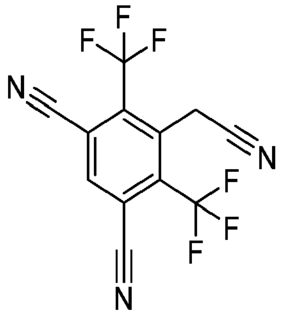 | 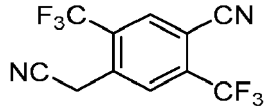 | 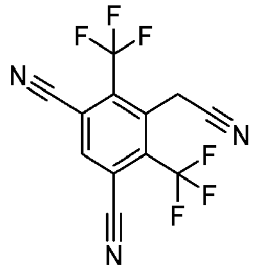 | -5.15 | 2.96 | 437 | 9.56 |
| A43 | 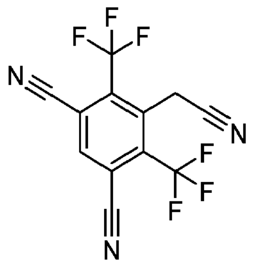 | 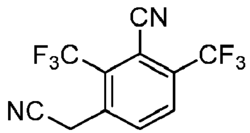 | 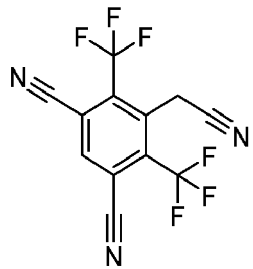 | -5.05 | 3.25 | 370 | 2.09 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A44 | 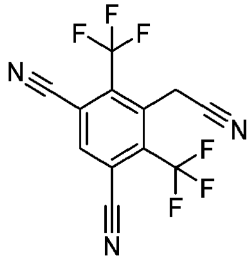 | 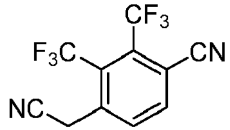 | 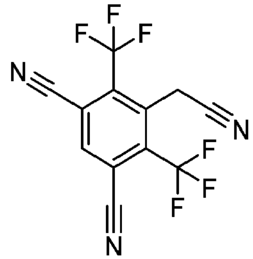 | -5.15 | 2.96 | 437 | 10.30 |
| A45 | 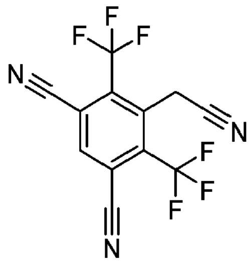 | 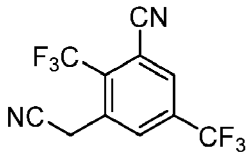 | 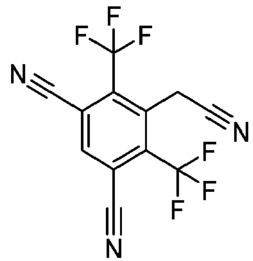 | -5.11 | 3.09 | 373 | 7.64 |
| A46 | 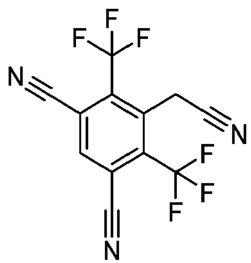 | 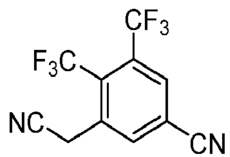 | 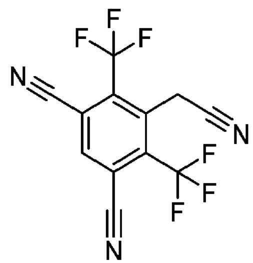 | -5.08 | 3.15 | 407 | 6.10 |
| A47 | 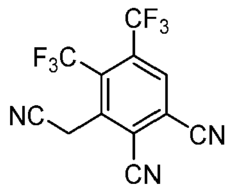 | 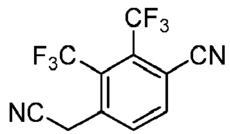 | 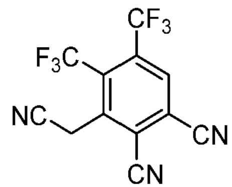 | -5.12 | 2.97 | 437 | 8.91 |

(continued)

| Name | A1 | A2 | A3 | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A48 | 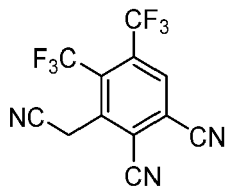 | 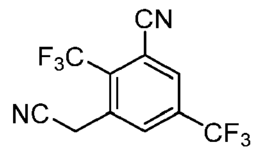 | 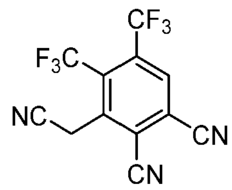 | -5.09 | 3.07 | 331 | 5.16 |
| A49 | 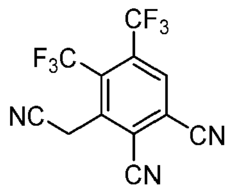 | 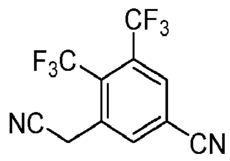 | 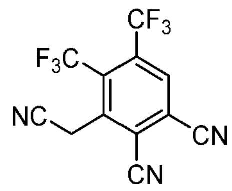 | -5.05 | 3.20 | 381 | 3.50 |
| A50 | 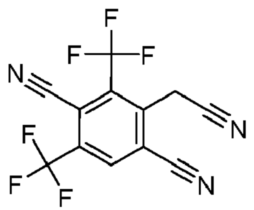 | 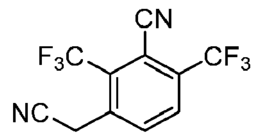 | 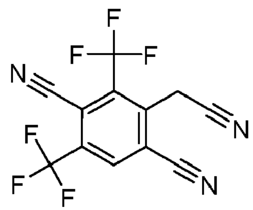 | -5.15 | 3.07 | 365 | 5.91 |
| A51 | 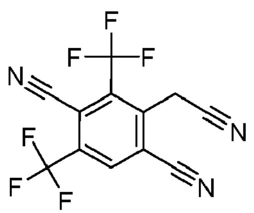 | 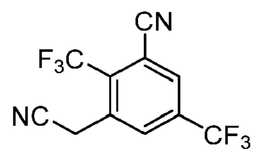 | 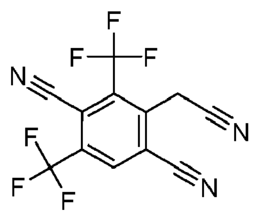 | -5.09 | 3.22 | 362 | 1.76 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A52 | 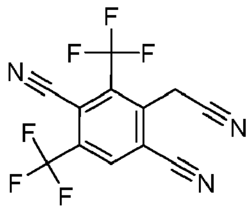 | 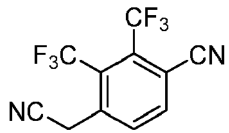 | 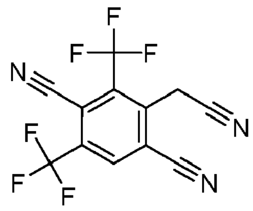 | -5.09 | 3.06 | 364 | 7.30 |
| A53 | 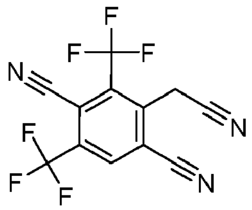 | 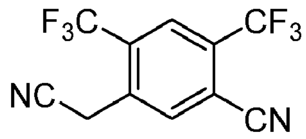 | 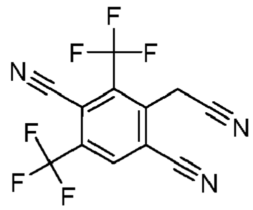 | -5.12 | 3.22 | 327 | 0.62 |
| A54 | 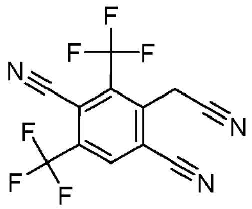 | 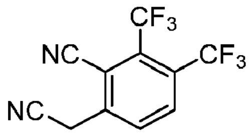 | 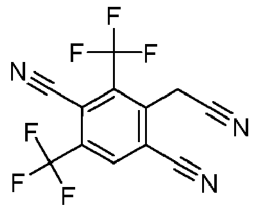 | -5.19 | 2.96 | 341 | 6.10 |
| A56 | 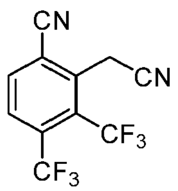 | 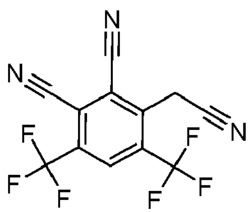 | 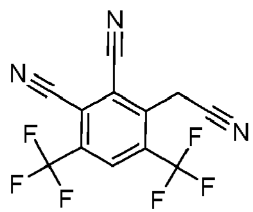 | -5.05 | 3.19 | 371 | 20.88 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A57 | 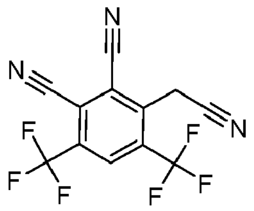 | 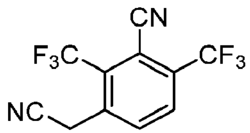 | 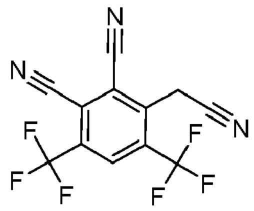 | -5.13 | 3.15 | 425 | 1.20 |
| A59 | 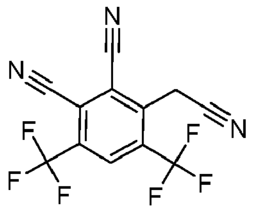 | 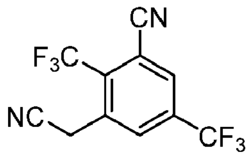 | 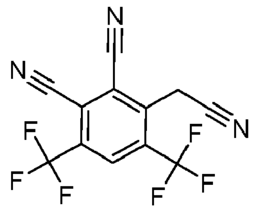 | -5.14 | 3.16 | 329 | 0.89 |
| A60 | 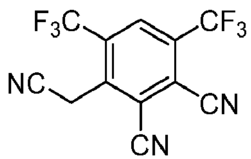 | 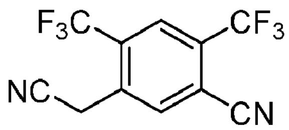 | 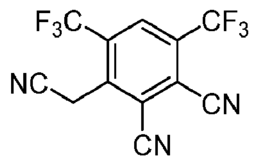 | -5.18 | 2.98 | 370 | 1.08 |
| A61 | 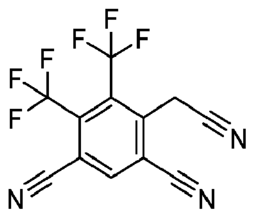 | 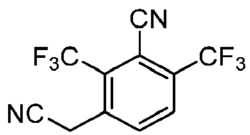 | 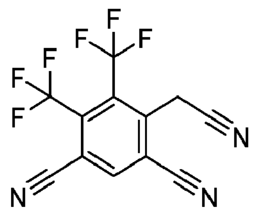 | -5.15 | 3.07 | 332 | 5.21 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A62 | 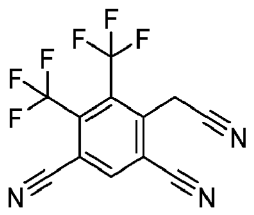 | 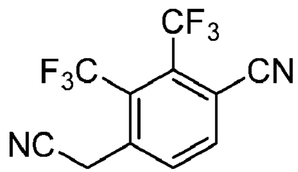 | 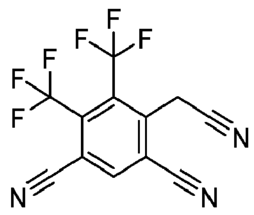 | -5.12 | 3.03 | 332 | 6.86 |
| A63 | 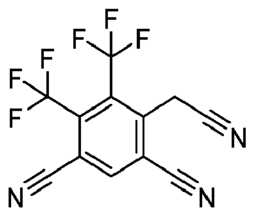 | 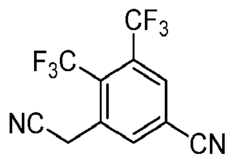 | 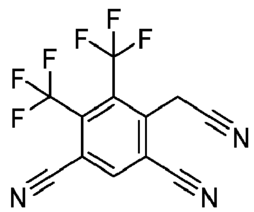 | -5.11 | 3.20 | 329 | 1.60 |
| A64 | 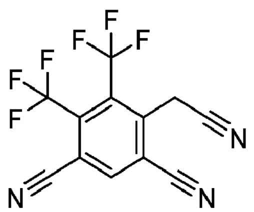 | 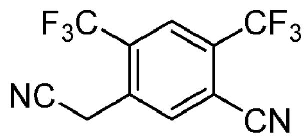 | 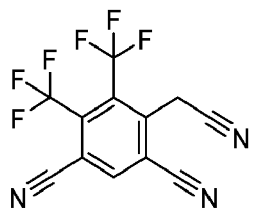 | -5.15 | 3.09 | 399 | 8.85 |
| A65 | 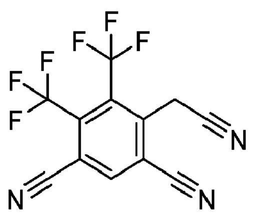 | 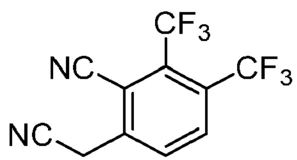 | 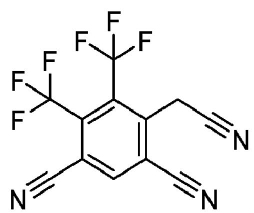 | -5.19 | 2.93 | 406 | 15.20 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A66 | 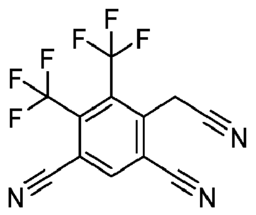 | 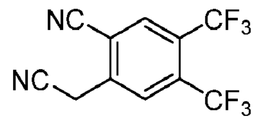 | 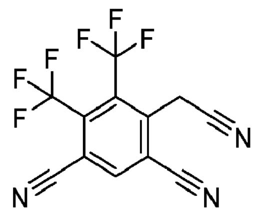 | -5.21 | 2.92 | 415 | 11.74 |
| A68 | 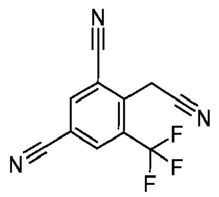 | 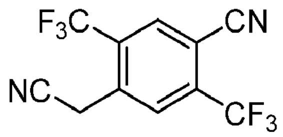 | 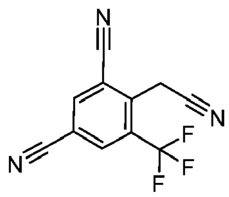 | -5.02 | 3.10 | | |
| A69 | 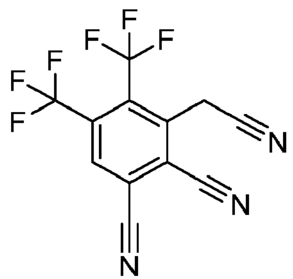 | 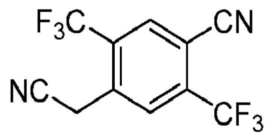 | 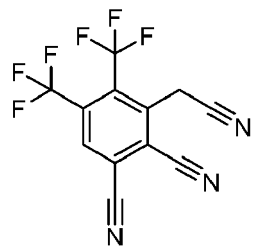 | -5.15 | 2.92 | | |
| A70 | 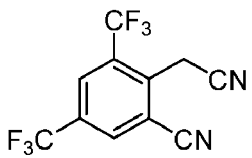 | 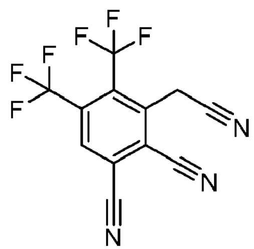 | 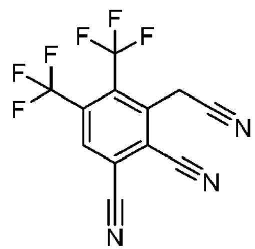 | -5.05 | 3.11 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A71 | 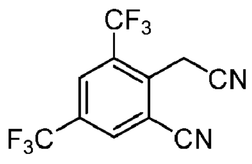 | 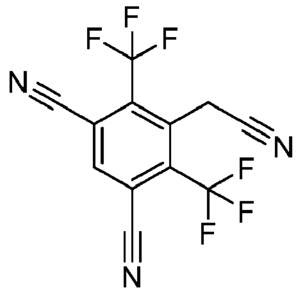 | 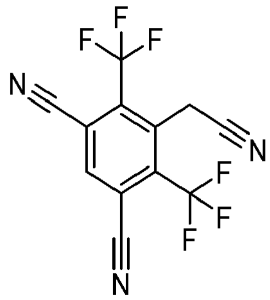 | -5.03 | 3.22 | | |
| A74 | 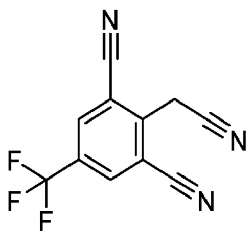 | 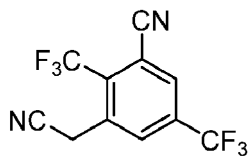 | 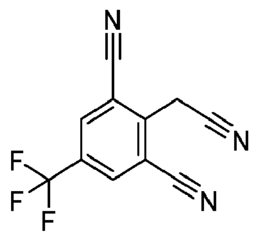 | -5.04 | 2.96 | | |
| A75 | 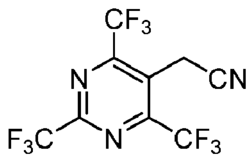 | 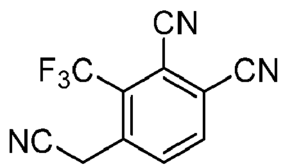 | 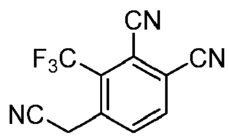 | -5.13 | 3.11 | | |
| A77 | 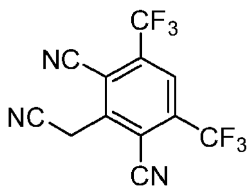 | 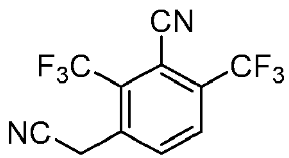 | 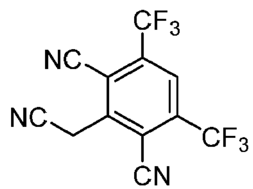 | -5.12 | 3.01 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A78 | 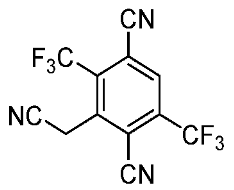 | 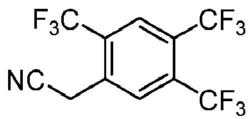 | 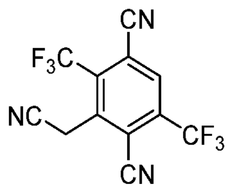 | -5.06 | 2.99 | | |
| A80 | 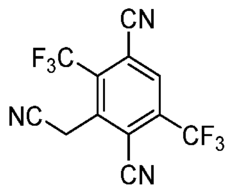 | 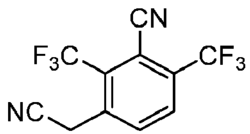 | 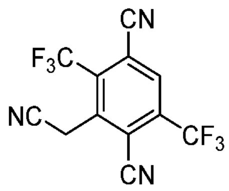 | -5.06 | 3.21 | | |
| A81 | 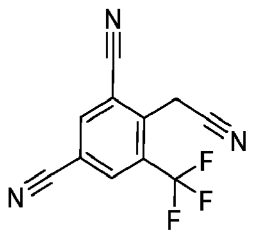 | 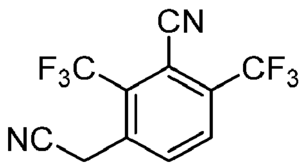 | 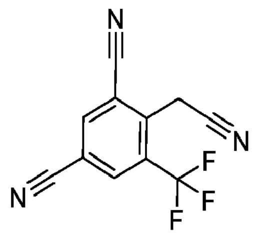 | -4.98 | 3.18 | | |
| A82 | 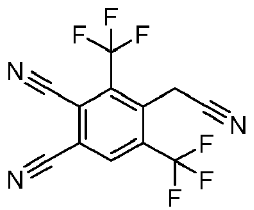 | 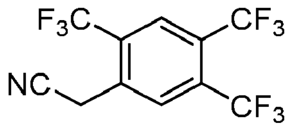 | 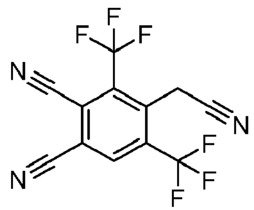 | -5.05 | 3.17 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A83 | 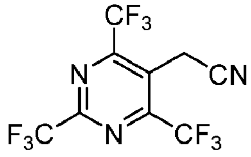 | 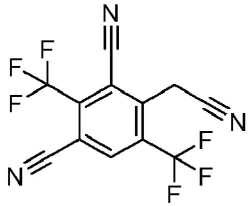 | 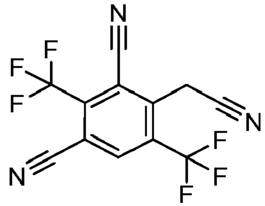 | -5.21 | 3.05 | | |
| A84 | 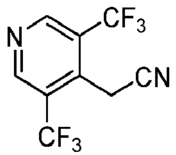 | 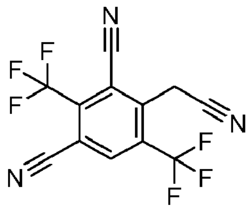 | 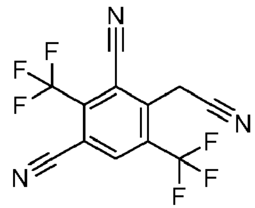 | -5.08 | 3.12 | | |
| A85 | 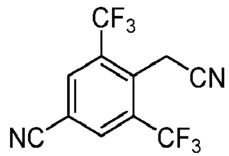 | 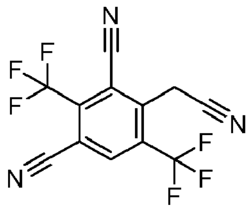 | 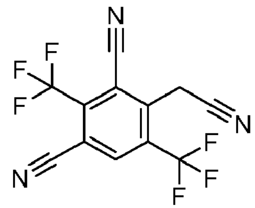 | -5.11 | 3.16 | | |
| A86 | 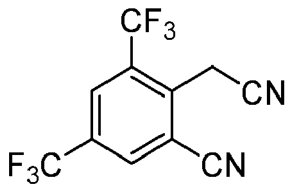 | 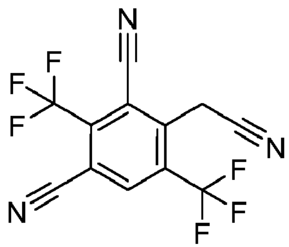 | 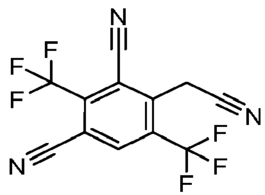 | -5.12 | 3.05 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A88 | 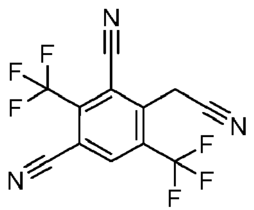 | 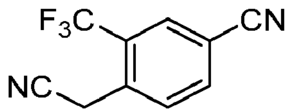 | 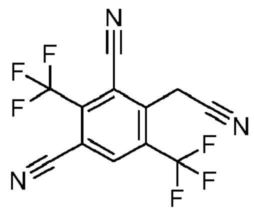 | -5.06 | 2.98 | | |
| A89 | 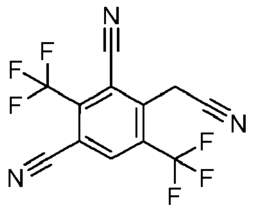 | 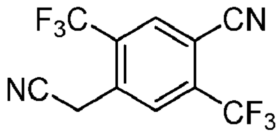 | 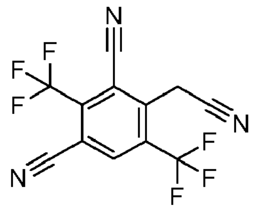 | -5.19 | 2.99 | | |
| A90 | 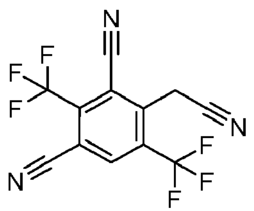 | 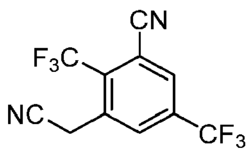 | 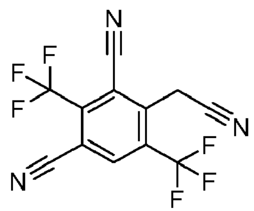 | -5.16 | 3.00 | | |
| A91 | 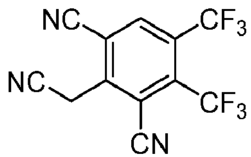 | 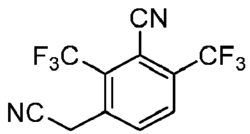 | 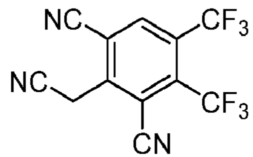 | -5.21 | 2.93 | 419 | 7.68 |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A92 | 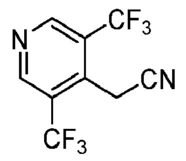 | 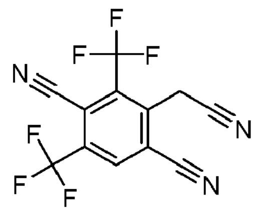 | 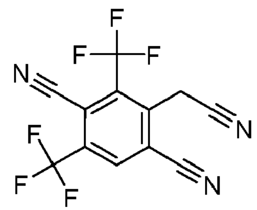 | -5.01 | 3.25 | | |
| A93 | 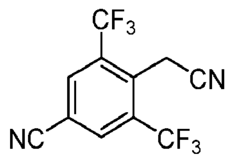 | 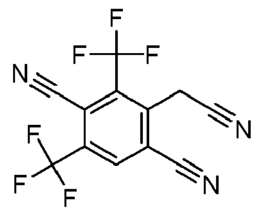 | 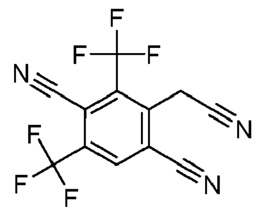 | -5.06 | 3.29 | | |
| A94 | 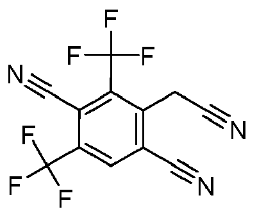 | 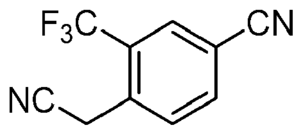 | 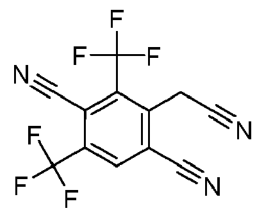 | -5.02 | 3.04 | | |
| A95 | 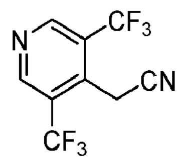 | 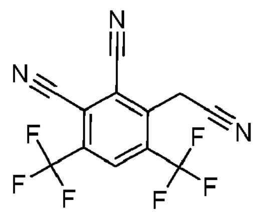 | 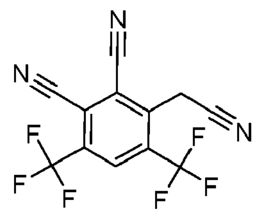 | -5.05 | 3.17 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A96 | 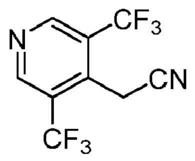 | 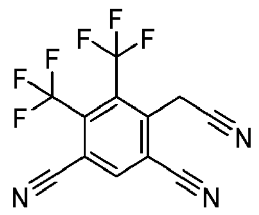 | 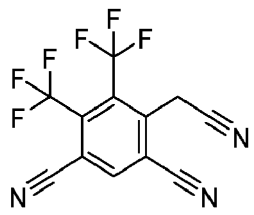 | -5.05 | 3.11 | 411 | 13.97 |
| A98 | 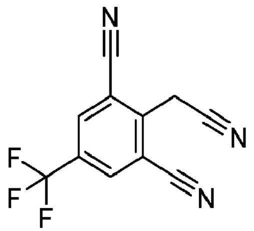 | 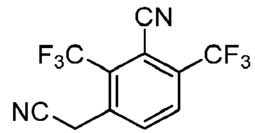 | 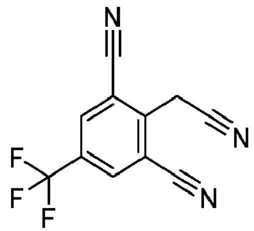 | -5.04 | 2.98 | | |
| A100 | 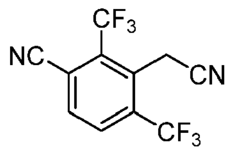 | 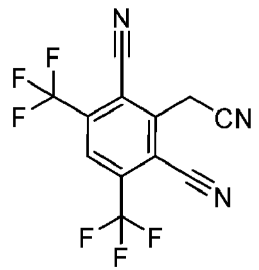 | 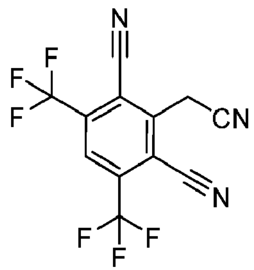 | -5.06 | 3.10 | | |
| A101 | 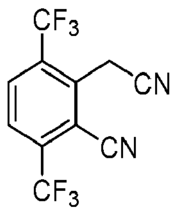 | 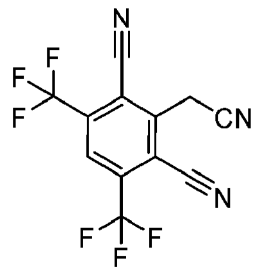 | 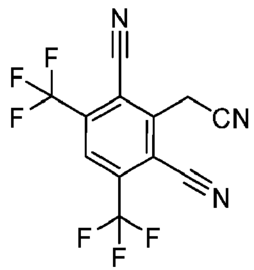 | -5.06 | 3.08 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A102 | 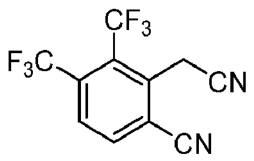 | 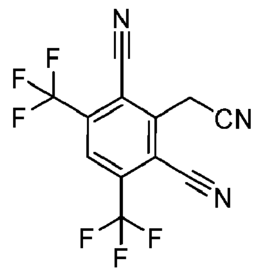 | 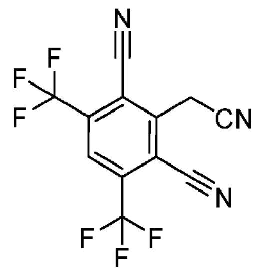 | -5.03 | 3.01 | | |
| A105 | 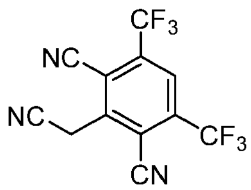 | 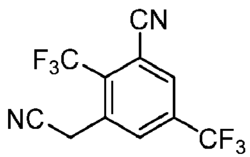 | 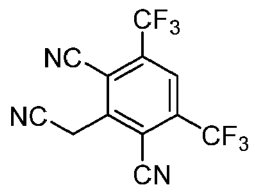 | -5.13 | 2.98 | | |
| A106 | 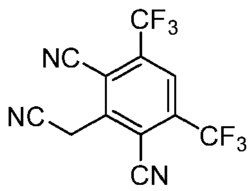 | 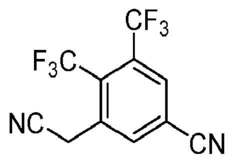 | 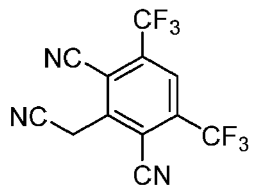 | -5.14 | 3.00 | | |
| A107 | 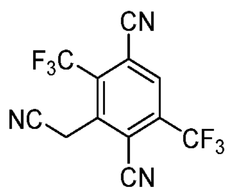 | 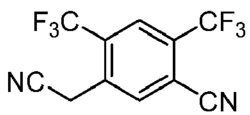 | 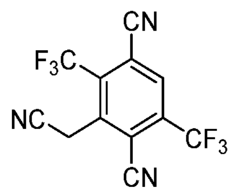 | -5.11 | 3.23 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A108 | 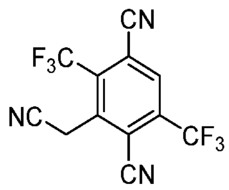 | 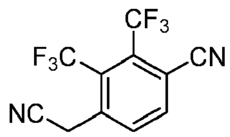 | 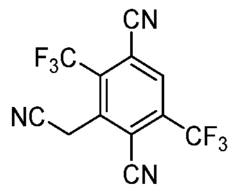 | -5.08 | 3.04 | | |
| A109 | 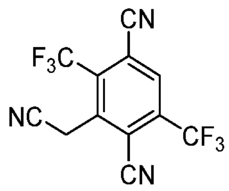 | 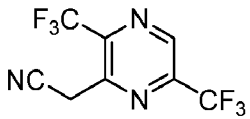 | 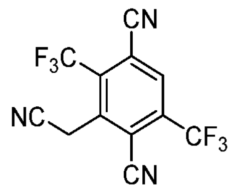 | -5.07 | 3.04 | | |
| A110 | 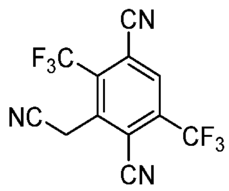 | 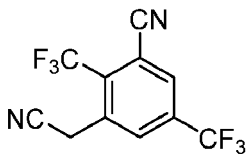 | 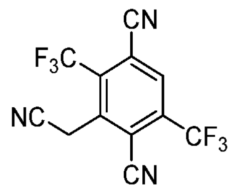 | -5.10 | 3.08 | | |
| A111 | 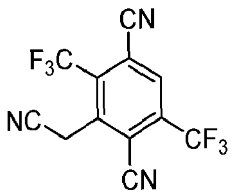 | 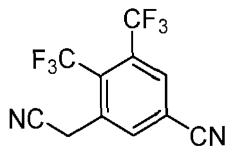 | 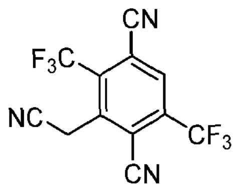 | -5.08 | 3.17 | | |

(continued)

| Name | A1 | A2 | A3 | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A112 | 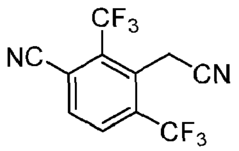 | 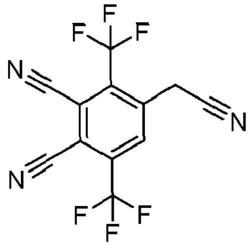 | 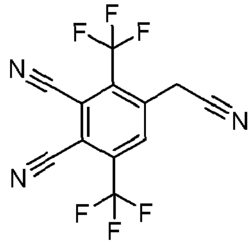 | -5.15 | 3.24 | | |
| A113 | 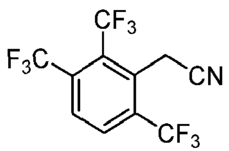 | 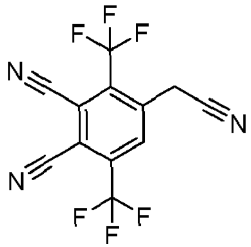 | 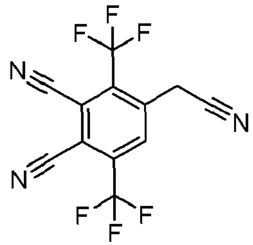 | -5.05 | 3.20 | | |
| A114 | 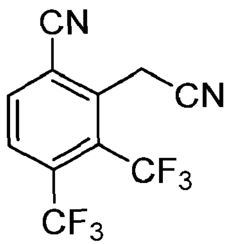 | 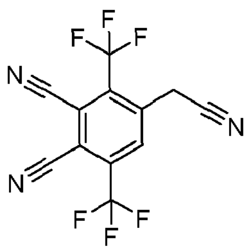 | 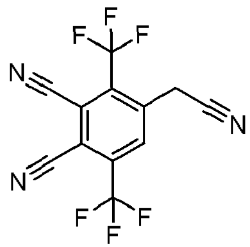 | -5.13 | 3.13 | | |
| A115 | 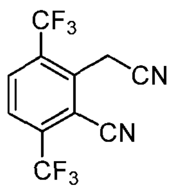 | 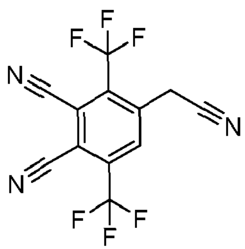 | 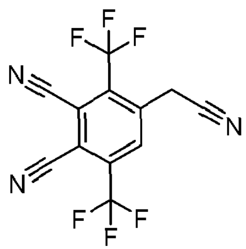 | -5.15 | 3.23 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A116 | 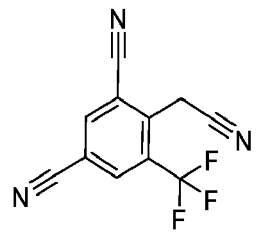 | 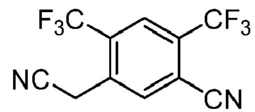 | 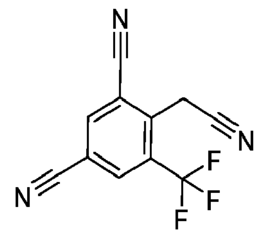 | -5.04 | 3.03 | | |
| A117 | 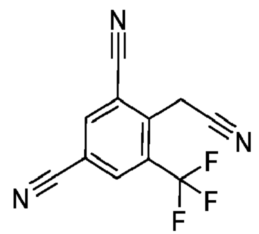 | 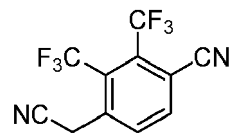 | 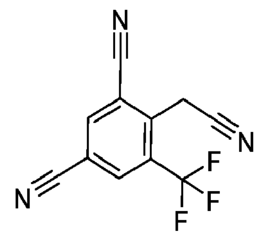 | -5.01 | 2.99 | | |
| A118 | 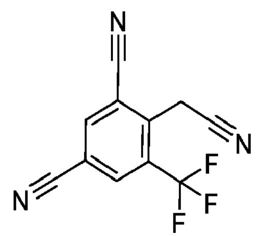 | 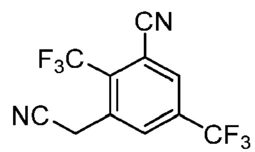 | 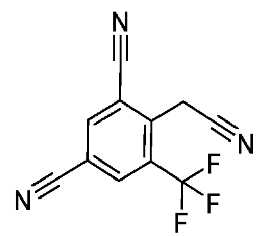 | -4.99 | 3.17 | | |
| A119 | 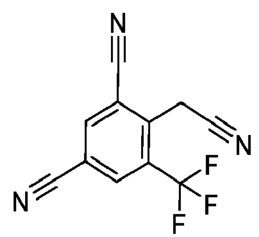 | 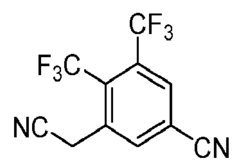 | 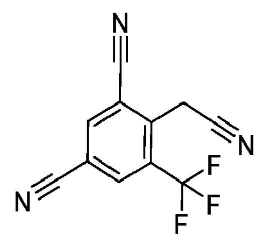 | -5.01 | 3.04 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A120 | 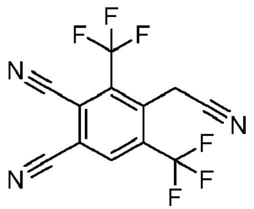 | 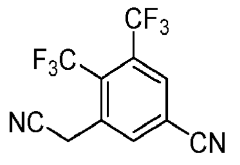 | 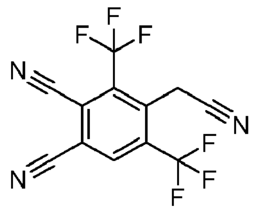 | -5.10 | 3.18 | | |
| A121 | 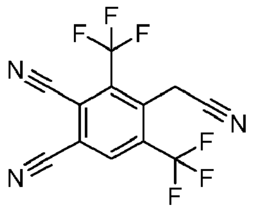 | 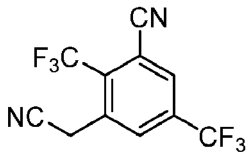 | 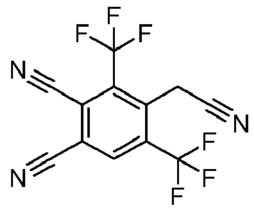 | -5.15 | 3.07 | | |
| A122 | 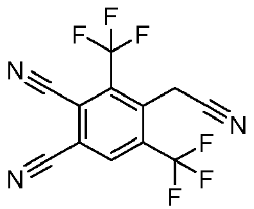 | 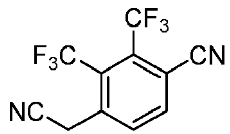 | 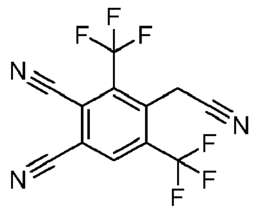 | -5.15 | 3.00 | | |
| A123 | 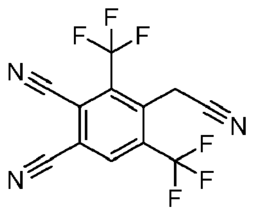 | 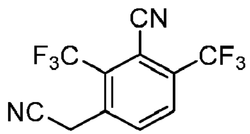 | 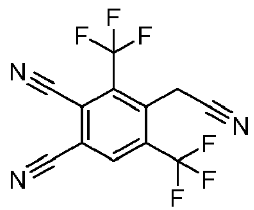 | -5.07 | 3.23 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A124 | 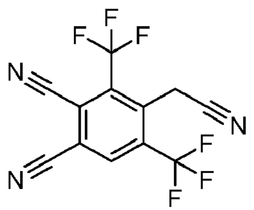 | 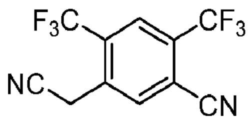 | 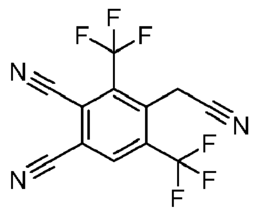 | -5.14 | 3.24 | | |
| A125 | 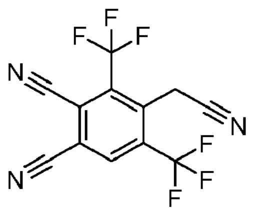 | 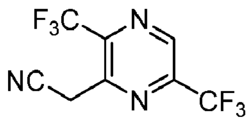 | 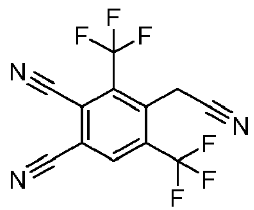 | -5.07 | 3.21 | | |
| A126 | 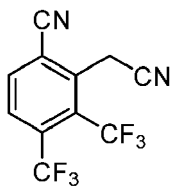 | 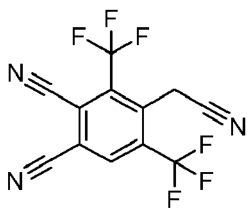 | 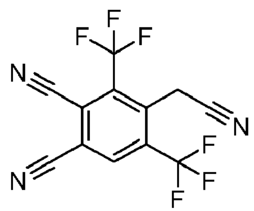 | -5.03 | 3.25 | | |
| A127 | 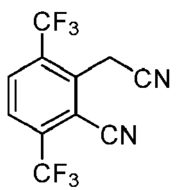 | 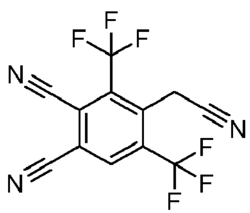 | 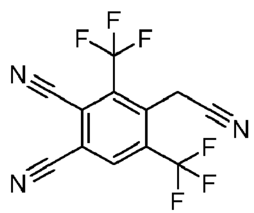 | -5.05 | 3.30 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A128 | 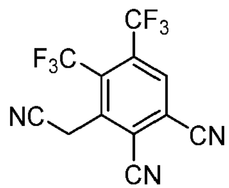 | 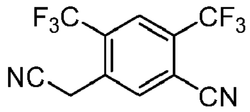 | 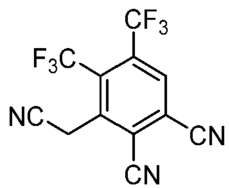 | -5.11 | 3.07 | | |
| A129 | 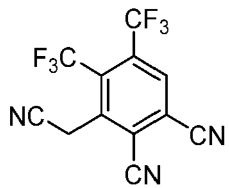 | 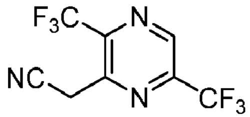 | 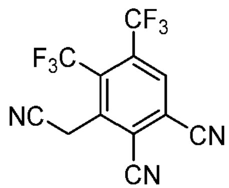 | -5.05 | 3.06 | | |
| A130 | 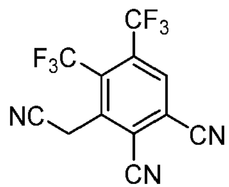 | 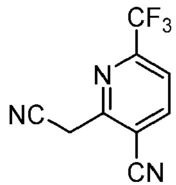 | 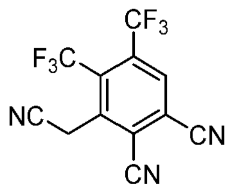 | -5.02 | 2.97 | | |
| A131 | 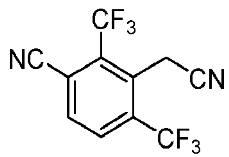 | 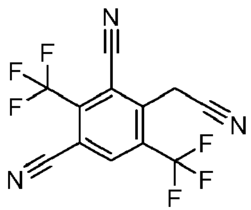 | 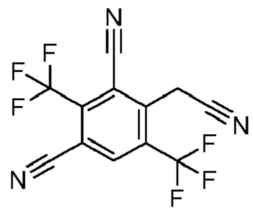 | -5.07 | 3.16 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A132 | 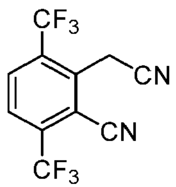 | 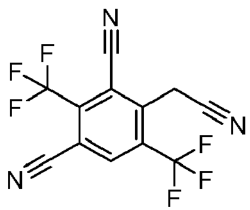 | 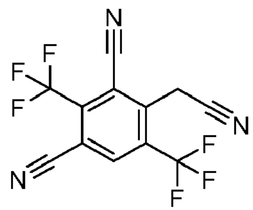 | -5.06 | 3.05 | | |
| A133 | 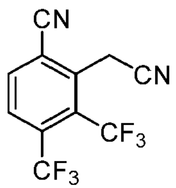 | 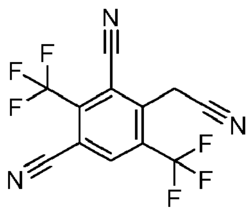 | 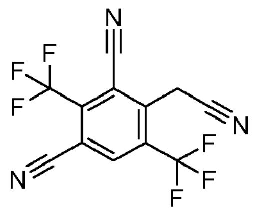 | -5.06 | 3.07 | | |
| A134 | 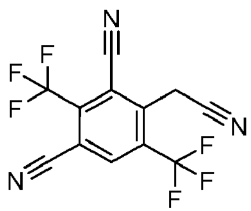 | 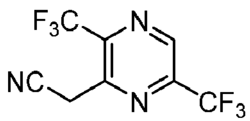 | 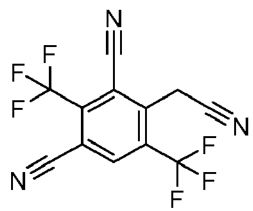 | -5.13 | 2.98 | | |
| A135 | 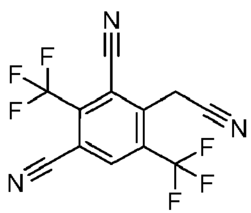 | 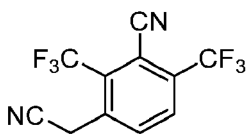 | 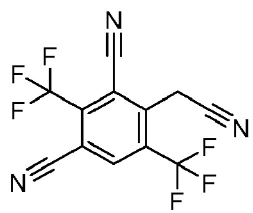 | -5.18 | 3.01 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A136 | 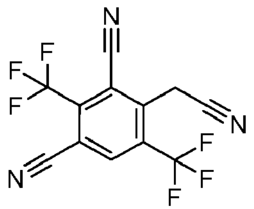 | 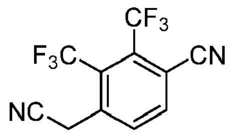 | 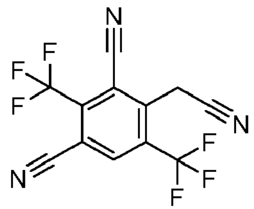 | -5.11 | 3.07 | | |
| A137 | 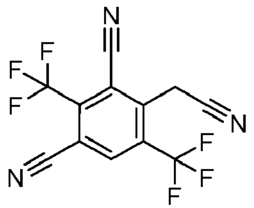 | 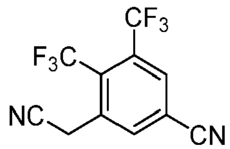 | 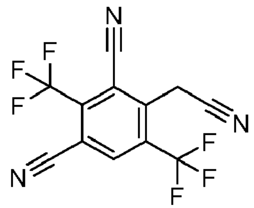 | -5.12 | 3.19 | | |
| A138 | 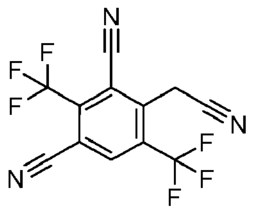 | 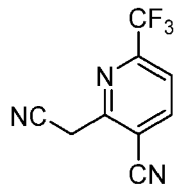 | 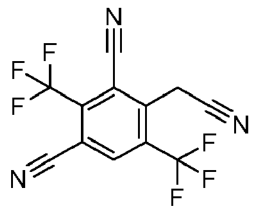 | -5.10 | 2.99 | | |
| A139 | 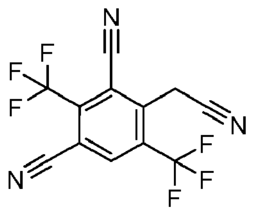 | 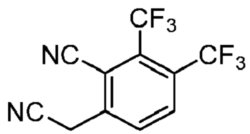 | 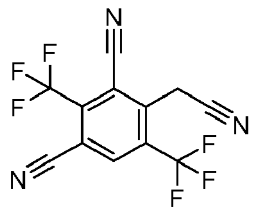 | -5.22 | 2.89 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A140 | 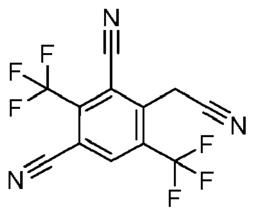 | 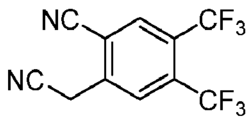 | 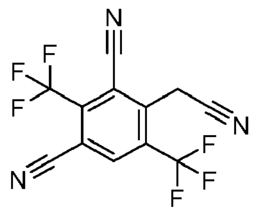 | -5.18 | 2.97 | | |
| A141 | 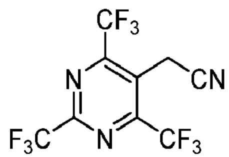 | 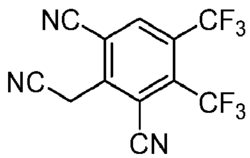 | 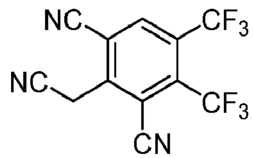 | -5.23 | 2.97 | | |
| A142 | 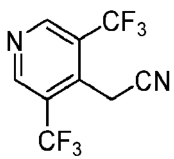 | 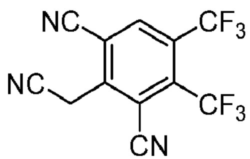 | 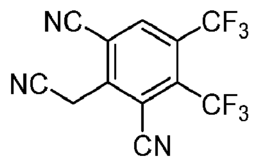 | -5.09 | 3.08 | | |
| A143 | 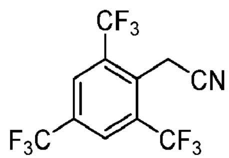 | 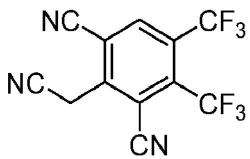 | 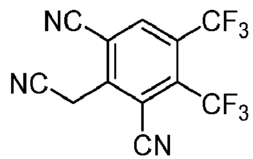 | -5.06 | 3.07 | | |
| A144 | 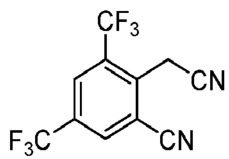 | 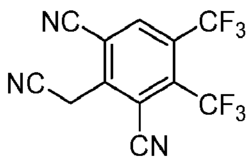 | 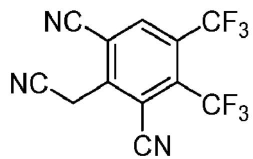 | -5.10 | 2.95 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A145 | 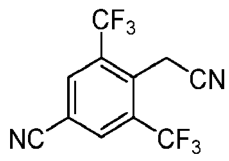 | 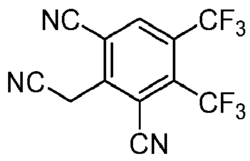 | 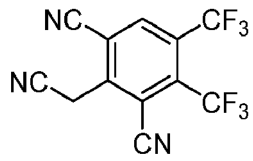 | -5.14 | 3.06 | | |
| A146 | 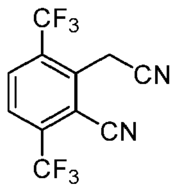 | 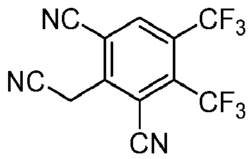 | 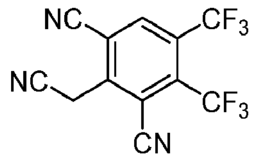 | -5.10 | 3.05 | | |
| A147 | 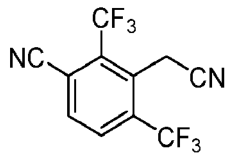 | 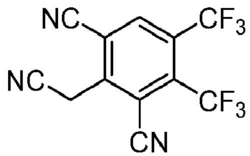 | 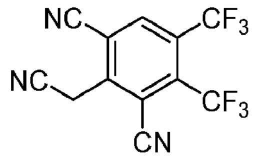 | -5.10 | 3.05 | | |
| A148 | 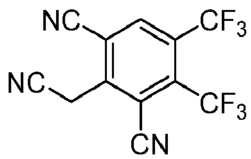 | 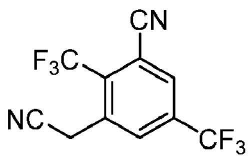 | 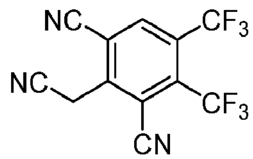 | -5.20 | 2.91 | | |
| A149 | 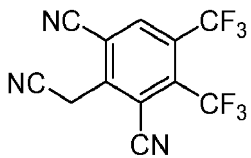 | 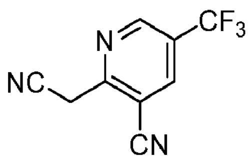 | 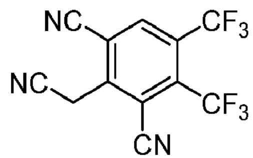 | -5.12 | 2.93 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A150 | 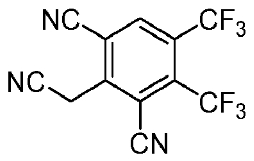 | 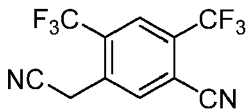 | 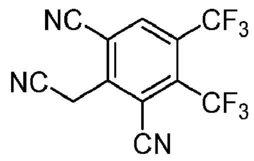 | -5.21 | 2.94 | | |
| A151 | 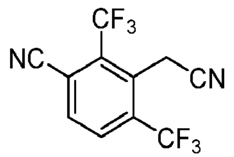 | 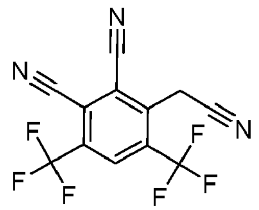 | 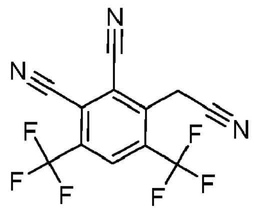 | -5.07 | 3.26 | | |
| A152 | 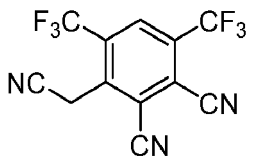 | 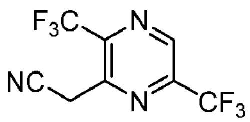 | 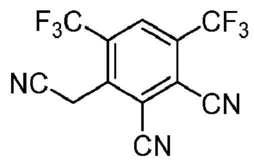 | -5.09 | 3.08 | | |
| A153 | 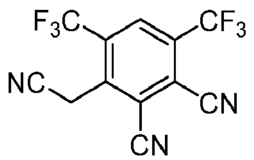 | 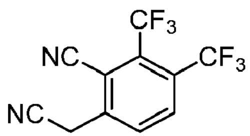 | 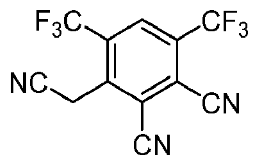 | -5.12 | 2.99 | | |
| A154 | 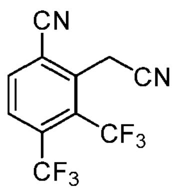 | 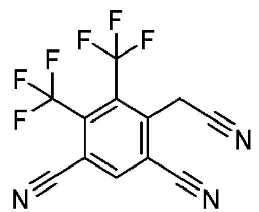 | 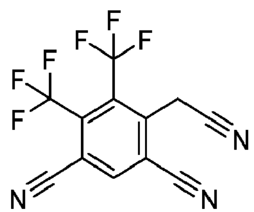 | -5.03 | 3.14 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A155 | 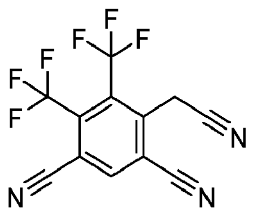 | 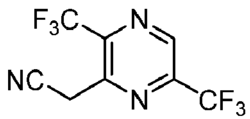 | 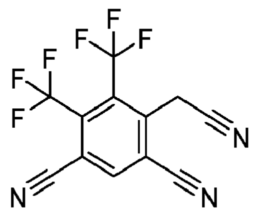 | -5.10 | 3.09 | | |
| A164 | 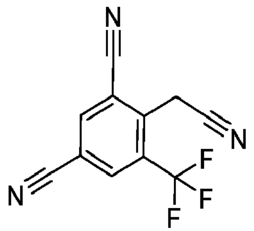 | 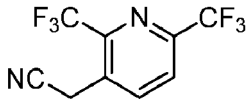 | 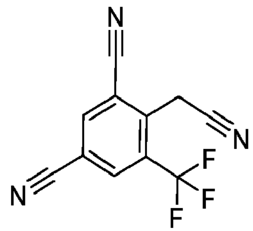 | -5.00 | 2.95 | | |
| A165 | 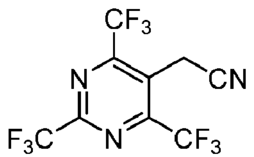 | 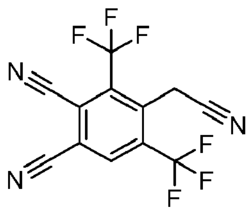 | 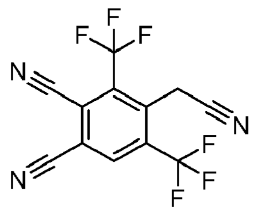 | -5.18 | 3.18 | | |
| A166 | 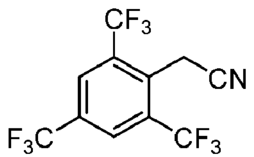 | 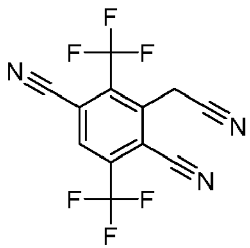 | 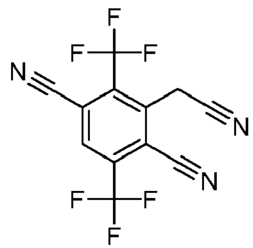 | -4.96 | 3.19 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A167 | 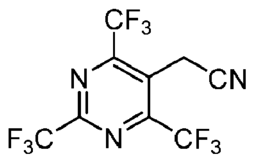 | 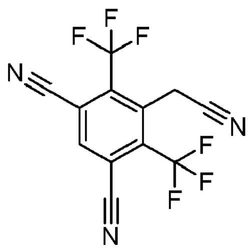 | 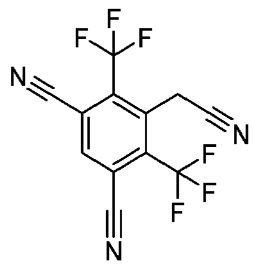 | -5.13 | 3.20 | | |
| A168 | 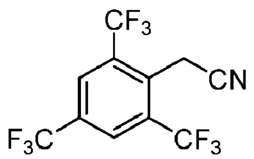 | 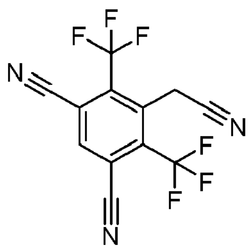 | 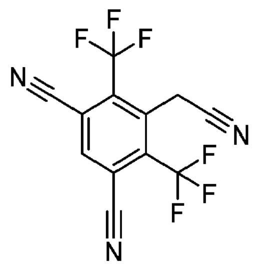 | -4.94 | 3.32 | | |
| A169 | 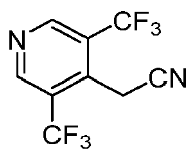 | 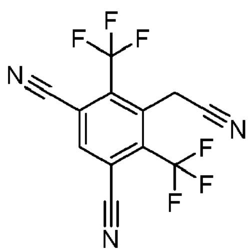 | 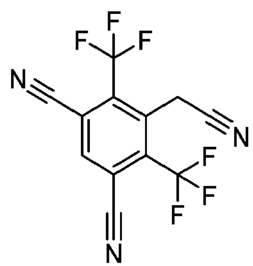 | -4.97 | 3.33 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A170 | 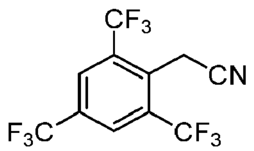 | 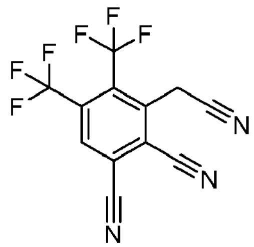 | 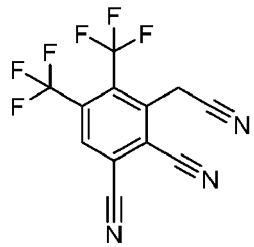 | -4.93 | 3.31 | | |
| A171 | 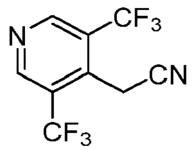 | 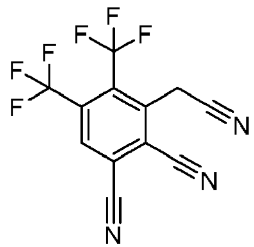 | 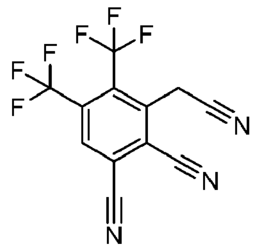 | -4.98 | 3.28 | | |
| A172 | 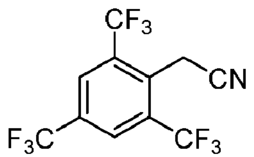 | 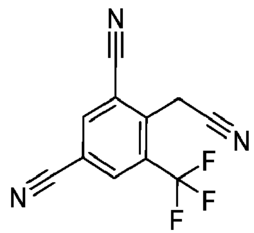 | 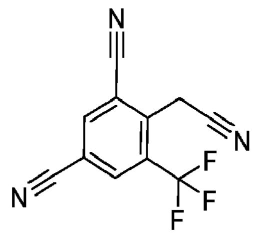 | -4.97 | 3.09 | | |
| A173 | 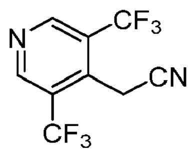 | 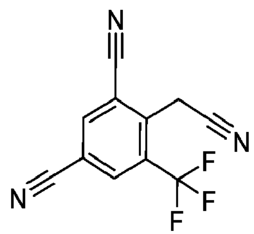 | 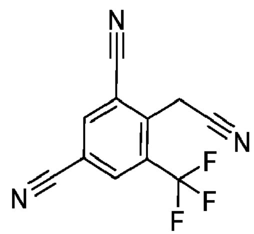 | -4.92 | 3.14 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A174 | 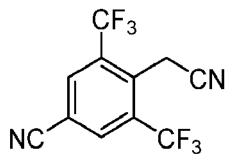 | 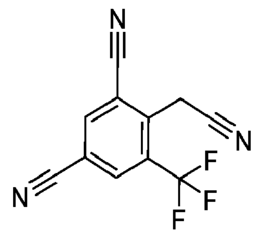 | 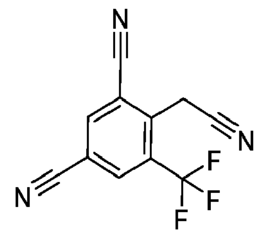 | -4.95 | 3.25 | | |
| A175 | 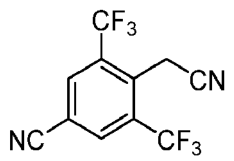 | 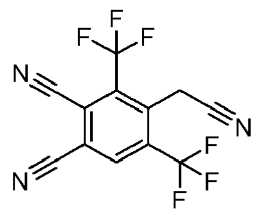 | 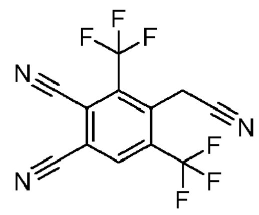 | -5.05 | 3.30 | | |
| A176 | 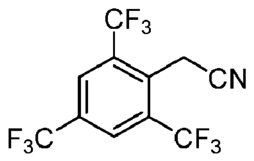 | 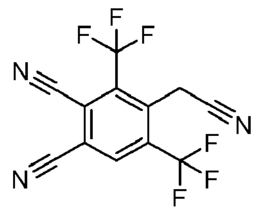 | 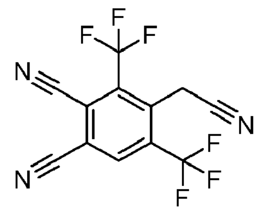 | -4.95 | 3.34 | | |
| A177 | 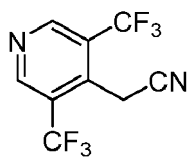 | 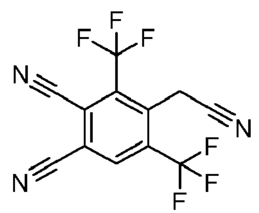 | 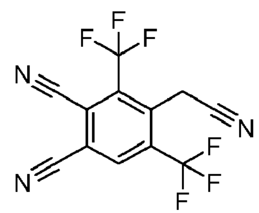 | -5.03 | 3.32 | | |

(continued)

| Name | $A^1$ | $A^2$ | $A^3$ | LUMO | E-Gap | λabs(max) 300-650 nm TDDFT of organic p-dopant | Absorption Area 400-650 nm TDDFT [a.u. x nm] of organic p-dopant |
|---|---|---|---|---|---|---|---|
| A178 | 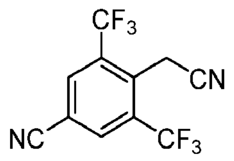 | 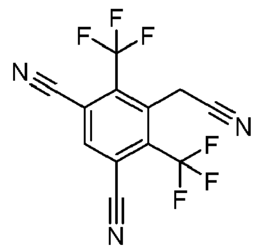 | 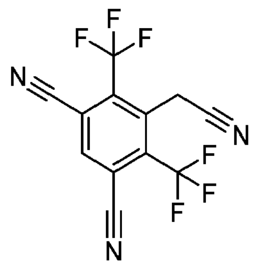 | -5.00 | 3.34 | | |
| A187 | 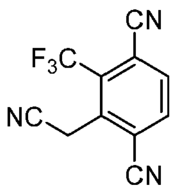 | 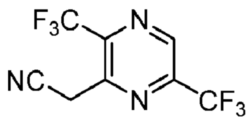 | 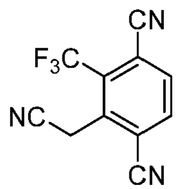 | -4.91 | 2.95 | | |

**Table 2: Optical absorptance of p-type charge generation layer (solid film)**

| | p-Dopant* | E-gap | **λabs(max) 300-650 nm TDDFT of organic p-dopant** | Absorption Area 400-650 nm TDDFT [a.u. x nm] of organic p-dopant | Abs. Integral visible 380-780nm [a.u. x nm] of solid film | Abs. Integral blue 450-500nm [a.u. x nm] of solid film | Abs. Integral green 500-600nm [a.u. x nm] of solid film |
|---|---|---|---|---|---|---|---|
| Comp.1 | CE-1 | 2.44 | 507 | 54.84 | 3509 | 604 | 818 |
| Comp.2 | CE-2 | 2.69 | 472 | 55.76 | 2723 | 484 | 584 |
| Inv. 1 | A1 | 2.97 | 329 | 9.73 | 2538 | 348 | 542 |

The inventive compounds exhibit a low energy gap (Egap) which is the difference between HOMO and LUMO energy level, and thus exhibit a low absorption in the range of 300 to 650 nm.

Moreover, the inventive compounds having a high energy gap exhibit a low absorption in the range of 300 to 650 nm.

A low absorption results in a reduction of the external quantum efficiency, the current density, and the luminous flux.

Thus, the compounds may be beneficial for providing an organic electronic device or a display device with increased brightness of a display or when a lower current density is used, the lifetime of the display can be increased.

Moreover, a high efficiency such as current efficiency and external quantum efficiency may be beneficial for reduced power consumption and improved battery life, in particular in mobile devices.

It can be seen from Table 2 (above) that a compound containing a radialene having a larger E-Gap (difference between the HOMO energy level and LUMO energy level) than a comparative compound CE1 to CE-2 exhibits a lower λabs(max), and a layer containing said radialene and an organic hole transport compound exhibits a lower absorption of visible light in particular of the green and blue part of the visible light than a comparative compound CE1 to CE-2 and an organic hole transport compound.

This may be beneficial for increasing the brightness of a display. If a lower current density is used, the lifetime of the display may be increased.

Moreover, the compounds may be beneficial for providing an organic electronic device or a display device with increased efficiency such as external quantum efficiency and current efficiency.

Table 3 shows the properties of selected devices in a stacked OLED comprising a p-CGL according to the invention and comparative data, Table 4 shows the properties of selected devices in a single-unit OLED according to the invention and comparative data:

**Table 3:**

| Example | Voltage at $10mA/cm^2$ [V] | Ceff/CIEy at $10mA/cm^2$ [cd/A] | EQE at $10mA/cm^2$ [%] | IS at $10mA/cm^2$: EQE [%] | IS at $10mA/cm^2$ with HSL: EQE [%] | luminous flux $10mA/cm^2$ [lm] | luminous flux with HSL $10mA/cm^2$ [lm] | LT97 at $30mA/cm^2$, RT [h] | ΔV at $30mA/cm^2$, 60° (1-100h) [V] |
|---|---|---|---|---|---|---|---|---|---|
| C-1 | 7.49 | 109 | 11.5 | 8.6 | 18.0 | 0.0179 | 0.0382 | 140 | 0.400 |
| C-2 | 7.57 | 116 | 12.2 | 9.0 | 19.0 | 0.0191 | 0.0411 | 140 | 0.324 |
| Inv-1 | 7.55 | 118 | 12.4 | 9.4 | 19.7 | 0.0197 | 0.0427 | 140 | 0.268 |

Table 4:

| Example | p-HIL | Voltage at 10mA/cm$^2$ [V] | Ceff/CIEy at 10mA/cm$^2$ [cd/A] | EQE at 10mA/cm$^2$ [%] | LT97 at 30mA/cm$^2$, RT [h] |
|---|---|---|---|---|---|
| C-1 | CE-1 | 3.32 | 165 | 15.6 | 125 |
| C-2 | CE-2 | 3.41 | 170 | 16.2 | 114 |
| Inv-1 | A1 | 3.38 | 179 | 16.7 | 125 |

Inventive device Inv-1 exhibits a good operational voltage and lower voltage rise (ΔV) than comparative device C-1 and C-2.

The compound A1 having no fluorine directly bound to an aryl moiety may exhibit a lower voltage rise over time when used in the p-type charge generation layer of the inventive devices Inv-1 than the comparative compounds CE-1 and CE-2 containing a fluorine directly bound to aryl moiety when used in the p-type charge generation of the comparative devices C-1 and C-2 (Table 3).

A low voltage rise over time may result in an improved long-term stability of the organic electronic device.

Moreover, a higher luminous flux could be measured when using a compound or an organic electronic device according to the invention (Inv-1). The organic electronic device according to the invention (Inv-1) contains a compound of formula (I) or (Ia) having a E-Gap (difference between the HOMO energy level and LUMO energy level) which is higher than the E-Gap for compound CE-1 or CE-2 used in the comparative device C-1 or C-2, respectively. This might be beneficial for increasing the brightness of a display or when a lower current density is used, the lifetime of the display can be increased. Moreover, a higher efficiency such as external quantum efficiency and current efficiency could be measured when using a compound or an organic electronic device according to the invention. The organic electronic device according to the invention (Inv-1) contains a compound of formula (I) or (Ia) having a E-Gap (difference between the HOMO energy level and LUMO energy level) which is higher than the compounds E-Gap of CE-1 or CE-2 used in the comparative devices C-1 or C-2, respectively. A high efficiency may be beneficial for reduced power consumption and improved battery life, in particular in mobile devices.

A higher efficiency such as external quantum efficiency and current efficiency could be measured when using a compound or an organic electronic device according to the invention. The organic electronic device according to the invention (Inv-1) contains a compound of formula (I) or (Ia) having a E-Gap (difference between the HOMO energy level and LUMO energy level) which is higher than the compounds E-Gap of CE-1 or CE-2 used in the comparative devices C-1 or C-2, respectively. A high efficiency may be beneficial for reduced power consumption and improved battery life, in particular in mobile devices.

Furthermore the TGA5%-values were measured for one inventive and one comparative compound, the results are shown in Table 5:

Table 5: TGA5%-values for selected compounds

| | Name | TGA5% [°C] |
|---|---|---|
| Comparative | CE-3 | 264 |
| Inventive | A1 | 277 |

It is apparent that the inventive compound exhibits a higher TGA5% than the comparative compound.

Since the TGA5% value can be regarded as an indirect measure of the volatility and/or decomposition temperature of a compound. In first approximation, the higher the TGA5% value the lower is the volatility of a compound and/or the higher the decomposition temperature. Thus, the inventive compounds exhibit a lower volatility, and a higher thermal stability, i.e. possess a higher decomposition temperature.

A higher TGA5% (lower volatility) may be beneficial for better controlling the processing of the organic electronic device by a thermal evaporation process, in particular in mass production, while the LUMO energy level, and thus the doping strength is almost unchanged or higher.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different

dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

**1.** A compound of formula (I)

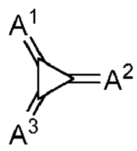

(I)

wherein

$A^1$ is selected from formula (II)

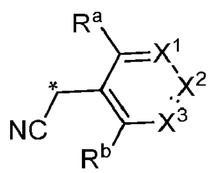

(II)

wherein
$X^1$ is selected from $CR^1$ or N,
$X^2$ is selected from $CR^2$ or N,
$X^3$ is selected from $CR^3$ or N,
$R^a$, and $R^b$ are independently selected from CN, $CF_3$, partially fluorinated $C_1$ to $C_8$ alkyl or perfluorinated $C_1$ to $C_8$ alkyl,
$R^1$, $R^2$, and $R^3$, if present, are independently selected from H, D, CN, $CF_3$, partially fluorinated $C_1$ to $C_8$ alkyl or perfluorinated $C_1$ to $C_8$ alkyl,
whereby if in formula (II) any of $X^1$ to $X^3$ is N then there is no CN moiety in $\alpha$-position,
$A^2$ is different from $A^1$ and selected from formula (III)

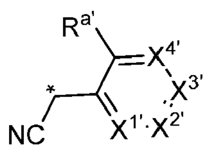

(III)

wherein

$X^1$ is selected from $CR^{1'}$ or N,
$X^{2'}$ is selected from $CR^{2'}$ or N,
$X^{3'}$ is selected from $CR^{3'}$ or N,
$X^{4'}$ is selected from $CR^{4'}$ or N,
$R^{a'}$ is selected from CN, $CF_3$, partially fluorinated $C_1$ to $C_8$ alkyl or perfluorinated $C_1$ to $C_8$ alkyl,
$R^{1'}$, $R^{2'}$, $R^{3'}$ and $R^{4'}$, if present, are independently selected from H, D, CN, $CF_3$, partially fluorinated $C_1$ to $C_8$ alkyl or perfluorinated $C_1$ to $C_8$ alkyl,
whereby if in formula (III) any $X^{1'}$ to $X^{4'}$ is N then there is no CN moiety in $\alpha$-position, $A^3$ is independently selected from formula (II), or formula (III),
wherein the compounds with the following combination of $A^1$ to $A^3$ are excluded:

| $A^1$ | $A^2$ | $A^3$ |
|---|---|---|
| 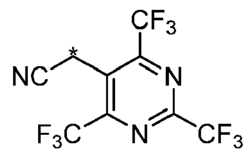 | 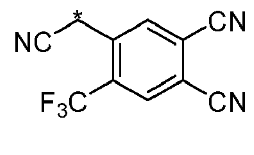 | 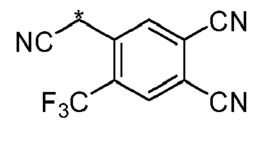 |
| 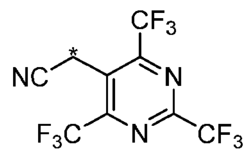 | 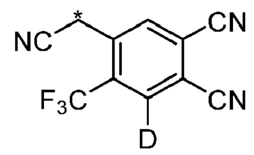 | 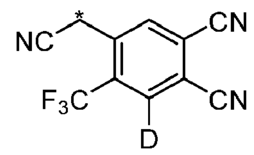 |
| 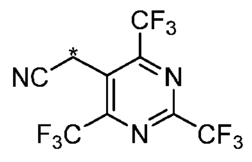 | 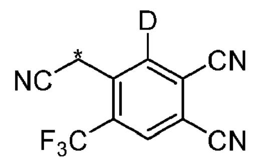 | 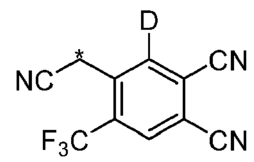 |
| 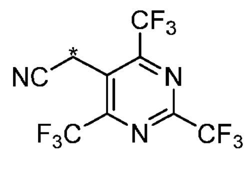 | 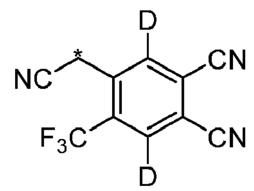 | 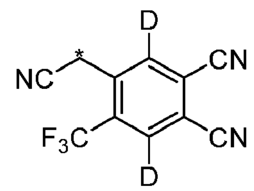 |
| 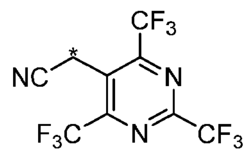 | 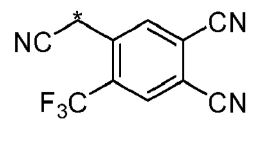 | 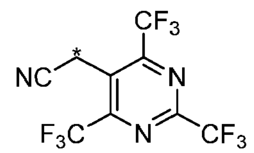 |
| 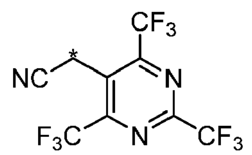 | 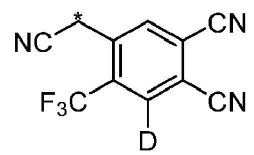 | 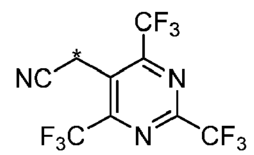 |
| 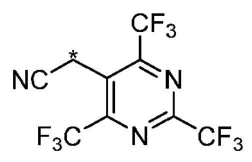 | 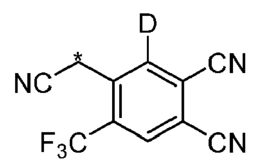 | 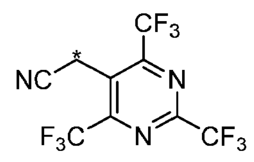 |
| 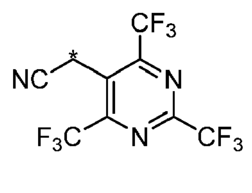 | 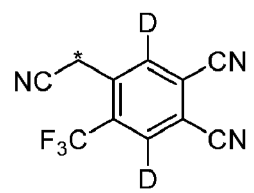 | 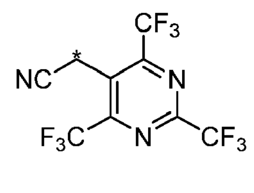 |
| 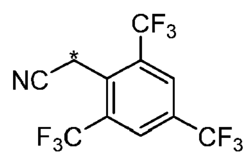 | 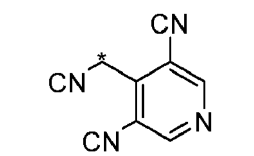 | 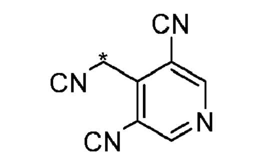 |

(continued)

| $A^1$ | $A^2$ | $A^3$ |
|---|---|---|
| 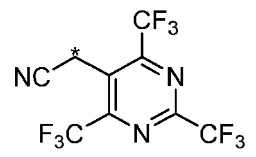 | 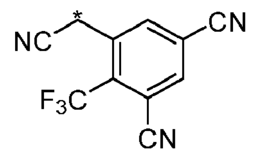 | 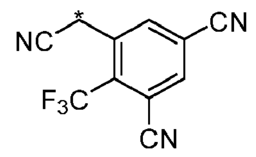 |

**2.** The compound of claim 1, comprising at least 6 CN groups

**3.** The compound of claim 1 or 2, wherein $A^3$ is identical with either $A^1$ or $A^2$.

**4.** The compound of any of the claims 1 to 3, wherein the compound of formula (I) has a LUMO energy level and a HOMO energy level, wherein the difference between the LUMO energy level and the HOMO energy level is $\geq$ 2.89 eV, preferably $\geq$ 2.92 eV, more preferably $\geq$ 2.95 eV, and most preferably $\geq$ 2.97 eV.

**5.** The compound of any of the claims 1 to 4, wherein the compound of formula (I) has a LUMO energy level of $\leq$ -4.90 eV, preferably $\leq$ -5.00 eV, more preferably $\leq$ -5.02 eV, and most preferably $\leq$ -5.04 eV.

**6.** The compound of any of the claims 1 to 5, wherein when in formula (II) one to three of X is N then $A^3$ is selected from formula (III), and none of the $X^{1'}$ to $X^{4'}$ in formula (III) is N, or when in formula (III) one to four of X is N then $A^3$ is selected from formula (II) and none of the $X^1$ to $X^3$ in formula (II) is N.

**7.** The compound of any of the claims 1 to 6, whereby when in formula (II) one or two of X is N then formula (III) is selected from formula (IIIc)

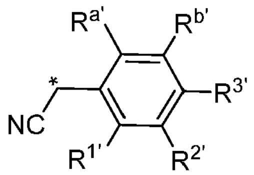 (IIIc)

$R^{a'}$, and $R^{b'}$ are independently selected from CN, $CF_3$, partially fluorinated $C_1$ to $C_8$ alkyl or perfluorinated $C_1$ to $C_8$ alkyl,
$R^1$, $R^{2'}$, and $R^{3'}$ are independently selected from H, D, CN, $CF_3$, partially fluorinated $C_1$ to $C_8$ alkyl or perfluorinated $C_1$ to $C_8$ alkyl.

**8.** The compound of any of the claims 1 to 7, whereby when in formula (II) one or two of X is N then in formula (III) none of the $X^{1'}$ to $X^{4'}$ is N and only one of $R^{1'}$, $R^{2'}$, $R^{3'}$ and $R^{4'}$ is H or D.

**9.** The compound of any of the clams 1 to 8, whereby the compound of formula (I) comprises 3 to 9 $CF_3$ groups or partially fluorinated $C_1$ to $C_8$ alkyl groups or perfluorinated $C_1$ to $C_8$ alkyl groups, preferably 4 to 8, more preferably 5 to 7, and most preferably 6 to 7.

**10.** The compound of any of the claims 1 to 9, wherein the compound of formula (I) comprises 6 to 9 CN groups, preferably 6 to 8, and more preferably 7 to 8.

**11.** A composition comprising a compound of formula (IV) and at least one compound of formula (IVa) to (IVd)

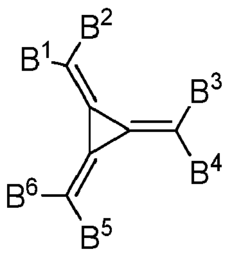

(IV)

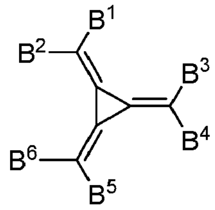

(IVa)

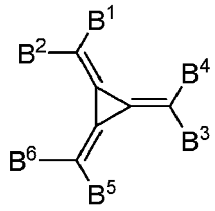

(IVb)

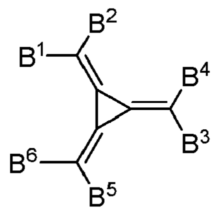

(IVc)

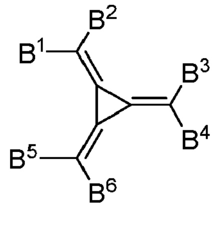

(IVd).

whereby $B^1$ is selected from formula (II), $B^3$ is selected from formula (III), $B^5$ is selected from formula (II) or (III) and $B^2$, $B^4$ and $B^6$ are CN.

**12.** An organic semiconductor layer, whereby the organic semiconductor layer comprises a compound of any of the claims 1 to 10 or a composition according to claim 11.

**13.** An organic electronic device comprising an anode layer, a cathode layer, and at least one organic semiconductor layer, wherein the organic semiconductor layer is arranged between the anode layer and the cathode layer, and wherein the organic semiconductor layer is an organic semiconductor layer according to claim 12.

**14.** The organic electronic device of claim 13, wherein the organic electronic device further comprises a charge generation layer, wherein the charge generation layer comprises a p-type charge generation layer and a n-type charge generation layer, wherein the p-type charge generation layer is an organic semiconductor layer according to claim 11.

**15.** The organic electronic device of claims 13 or 14, wherein the organic electronic device further comprises a hole injection layer.

**16.** The organic electronic device of claim 15, wherein the p-type charge generation layer and the hole injection layer comprise the same compound of formula (I).

**17.** The organic electronic device of any of the claims 13 to 16, wherein the p-type charge generation layer and the hole injection layer comprise an identical substantially covalent matrix compound.

**18.** The organic electronic device of any of the claims 13 to 17, wherein the organic electronic device is an electroluminescent device, an organic electroluminescent device, an organic light emitting diode (OLED), a light emitting device, thin film transistor, a battery, an organic photovoltaic cell (OPV), or an organic solar cell, preferably an organic electroluminescent device, an organic light emitting diode (OLED), a light emitting device, more preferably an organic electroluminescent device or an organic light emitting diode (OLED).

**19.** A display device comprising an organic electronic device according to any of the claims 13 to 18.

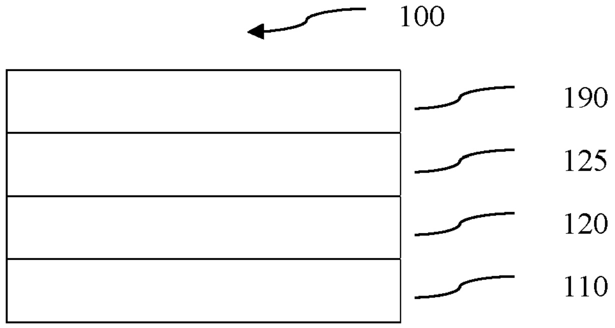


Fig. 1

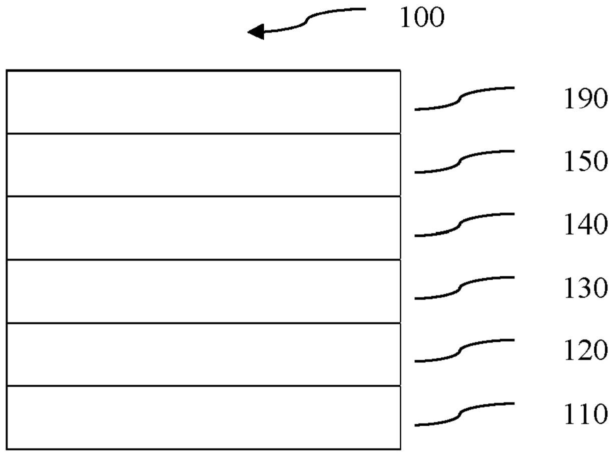


Fig. 2

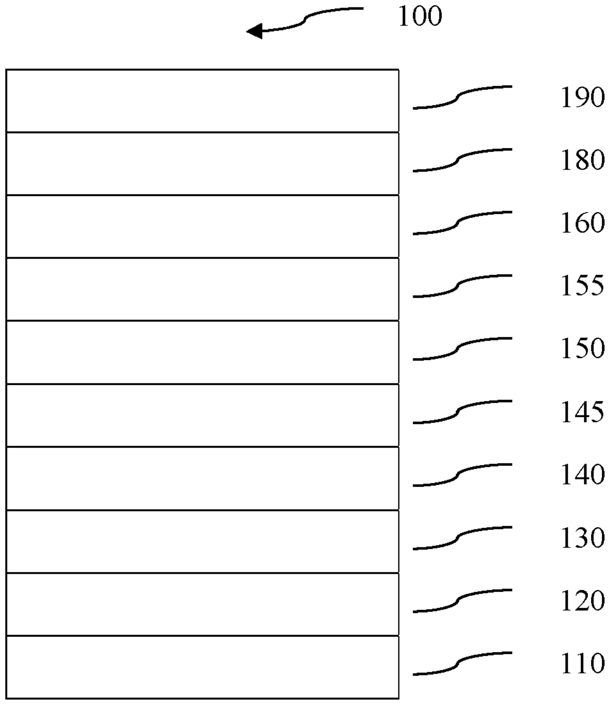
100
190
180
160
155
150
145
140
130
120
110

**Fig. 3**

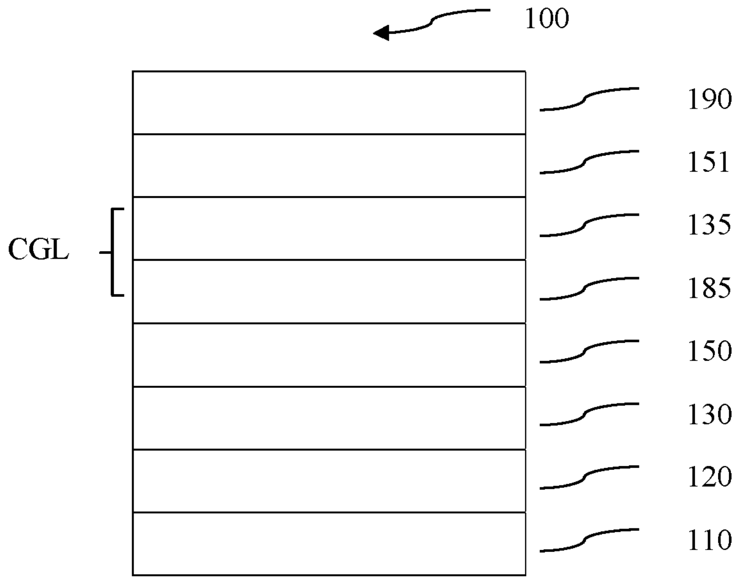
100
CGL
190
151
135
185
150
130
120
110

**Fig. 4**

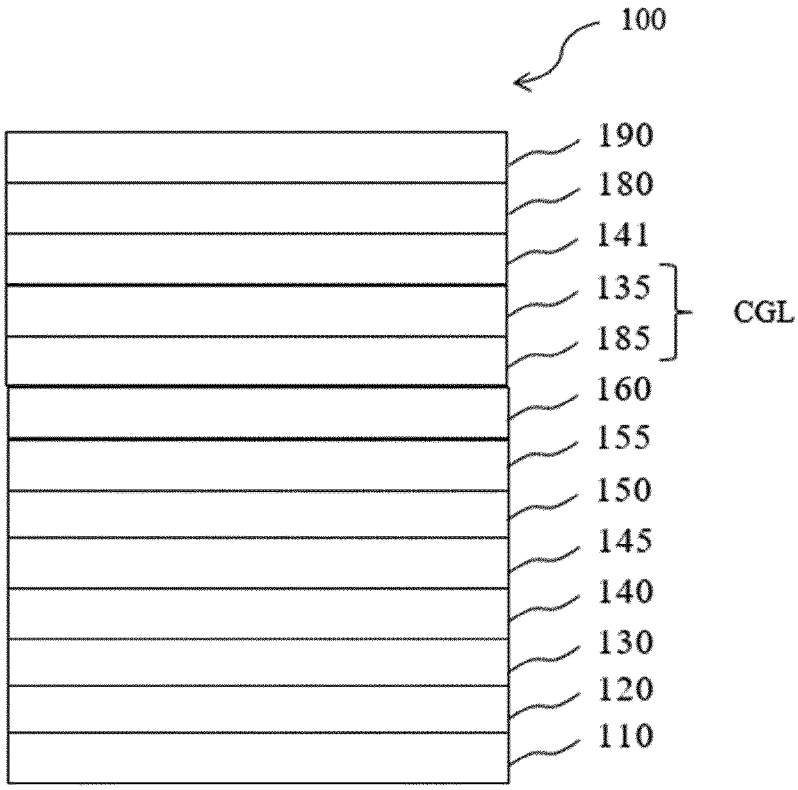


Fig. 5

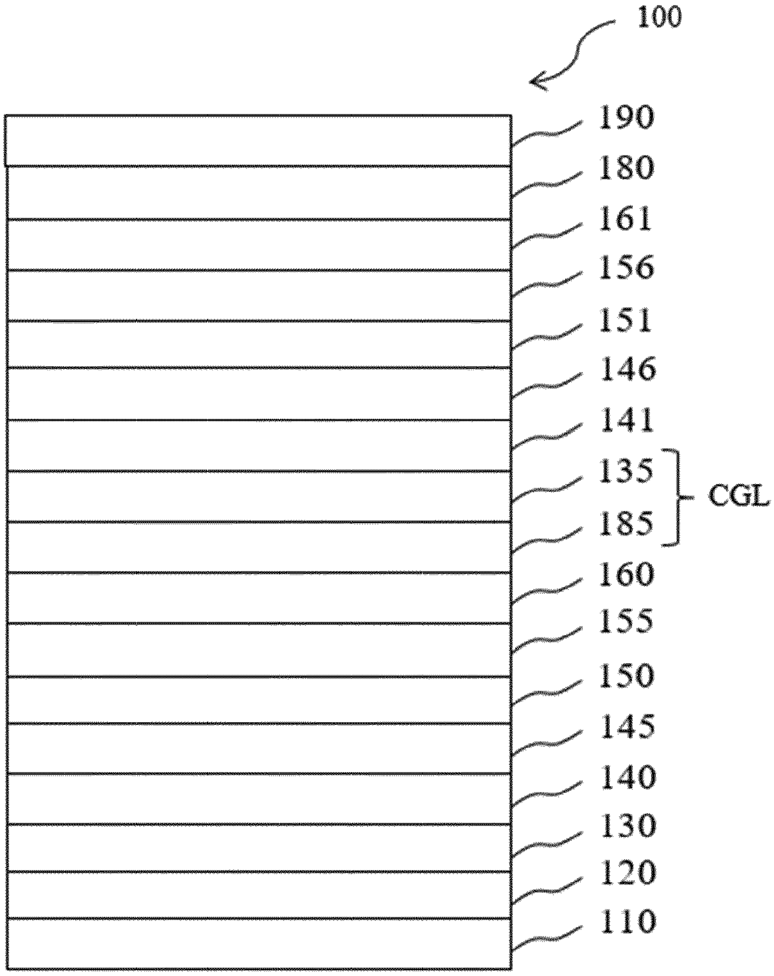
100
190
180
161
156
151
146
141
135
185
CGL
160
155
150
145
140
130
120
110

**Fig. 6**

Europäisches Patentamt
European Patent Office
Office européen des brevets

# EUROPEAN SEARCH REPORT

Application Number
EP 23 18 0120

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | CN 108 774 515 A (CHANGCHUN HAIPURUNSI TECH CO LTD) 9 November 2018 (2018-11-09)<br>* compounds 2-16 * | 1,3,6-9<br>11 | INV.<br>H10K85/60<br>C09K11/06 |
| X<br>Y | EP 3 799 143 A1 (NOVALED GMBH [DE]) 31 March 2021 (2021-03-31)<br>* page 18; compound A9 * | 1,3<br>11 | |
| X<br>Y | EP 3 034 489 A1 (NOVALED GMBH [DE]) 22 June 2016 (2016-06-22)<br>* page 6; compound A3 * | 1,3<br>11 | |
| X<br>Y | CN 109 081 791 A (AAC TECH NANJING INC) 25 December 2018 (2018-12-25)<br>* compounds P-7 * | 1-3,10, 12-19<br>11 | |
| X<br>Y | WO 2022/148464 A1 (ZHEJIANG BRILLIANT OPTOELECTRONIC TECHNO) 14 July 2022 (2022-07-14)<br>* page 21; compound 3 * | 1,3-5<br>11 | |
| Y | EP 3 930 022 A1 (NOVALED GMBH [DE]) 29 December 2021 (2021-12-29)<br>* claim 1 * | 11 | TECHNICAL FIELDS SEARCHED (IPC)<br>H10K<br>C07D<br>H05B<br>C09K |

The present search report has been drawn up for all claims

2

| Place of search | Date of completion of the search | Examiner |
|---|---|---|
| The Hague | 6 November 2023 | Fratiloiu, Silvia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 18 0120

This annex lists the patent family members relating to the patent documents cited in the above-mentioned European search report. The members are as contained in the European Patent Office EDP file on
The European Patent Office is in no way liable for these particulars which are merely given for the purpose of information.

06-11-2023

| Patent document cited in search report | | Publication date | Patent family member(s) | | | Publication date |
|---|---|---|---|---|---|---|
| CN 108774515 | A | 09-11-2018 | NONE | | | |
| EP 3799143 | A1 | 31-03-2021 | CN | 114503297 | A | 13-05-2022 |
| | | | EP | 3799143 | A1 | 31-03-2021 |
| | | | EP | 4035217 | A1 | 03-08-2022 |
| | | | KR | 20220069959 | A | 27-05-2022 |
| | | | US | 2023240136 | A1 | 27-07-2023 |
| | | | WO | 2021058761 | A1 | 01-04-2021 |
| EP 3034489 | A1 | 22-06-2016 | CN | 107207420 | A | 26-09-2017 |
| | | | CN | 116789565 | A | 22-09-2023 |
| | | | CN | 116789566 | A | 22-09-2023 |
| | | | EP | 3034489 | A1 | 22-06-2016 |
| | | | EP | 3233787 | A1 | 25-10-2017 |
| | | | JP | 6649952 | B2 | 19-02-2020 |
| | | | JP | 2018506137 | A | 01-03-2018 |
| | | | KR | 20170097707 | A | 28-08-2017 |
| | | | TW | 201638366 | A | 01-11-2016 |
| | | | US | 2017373251 | A1 | 28-12-2017 |
| | | | WO | 2016097017 | A1 | 23-06-2016 |
| CN 109081791 | A | 25-12-2018 | NONE | | | |
| WO 2022148464 | A1 | 14-07-2022 | CN | 116783159 | A | 19-09-2023 |
| | | | WO | 2022148464 | A1 | 14-07-2022 |
| EP 3930022 | A1 | 29-12-2021 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2722908 A1
- EP 2180029 A1
- WO 2016097017 A1

### Non-patent literature cited in the description


- **YASUHIKO SHIROTA** ; **HIROSHI KAGEYAMA**. *Chem. Rev.*, 2007, vol. 107, 953-1010
- *J. Phys. Chem. A*, 1999, vol. 103 (11), 1653-1661